# EUROPÄISCHE PATENTSCHRIFT

(11) **EP 2 480 539 B1**
(45) Veröffentlichungstag und Bekanntmachung des Hinweises auf die Patenterteilung: **31.07.2013**
(21) Anmeldenummer: 10754896.8
(22) Anmeldetag: 18.09.2010
(51) Int. Cl.: C07D 271/08, C07D 413/04, C07D 413/12, C07D 413/14, A01N 43/82

(54) **N-(1,2,5-OXADIAZOL-3-YL)BENZAMIDE UND IHRE VERWENDUNG ALS HERBIZIDE**
N-(1,2,5-OXADIAZOL-3-YL)BENZAMIDES AND THEIR USE AS HERBICIDES
N-(1,2,5-OXADIAZOL-3-YL)BENZAMIDES ET LEUR UTILISATION COMME HERBICIDES

(30) Priorität: 25.09.2009 EP 09012169; 28.09.2009 US 246295 P
(43) Veröffentlichungstag der Anmeldung: 01.08.2012
(73) Patentinhaber: Bayer CropScience AG, 40789 Monheim (DE)
(72) Erfinder: KÖHN , Arnim, 55270 Klein-Winternheim (DE); TIEBES , Jörg, 60431 Frankfurt (DE); VAN ALMSICK, Andreas, 61184 Karben (DE); AHRENS, Hartmut, 63329 Egelsbach (DE); HEINEMANN, Ines, 65719 Hofheim (DE); BRAUN, Ralf, 76857 Ramberg (DE); SCHMITT, Monika, H., 60318 Frankfurt (DE); WILLMS, Lothar, 65719 Hofheim (DE); FEUCHT, Dieter, 65760 Eschborn (DE); ROSINGER, Christopher, Hugh, 65719 Hofheim (DE); HÄUSER-HAHN, Isolde, 51375 Leverkusen (DE); DREWES, Mark, 40764 Langenfeld (DE); DÖRNER-RIEPING, Simon, 61267 Neu-Anspach (DE); DITTGEN, Jan, 60316 Frankfurt (DE); ADAMCZEWSKI, Martin, 51067 Köln (DE)
(86) Internationale Anmeldenummer: PCT/EP2010/005739
(87) Internationale Veröffentlichungsnummer: WO 2011/035874

(56) Entgegenhaltungen:
- EP-A2- 0 173 657
- WO-A1-2006/122150

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

Aus EP 0 173 657 A1 sind herbizide Mischungen bekannt, die bestimmte N-(1,2,5-Oxadiazol-3-yl)benzamide enthalten. Dort ist auch die Verbindung N-(4-Methylfurazan-3-yl)-2,4-dichlorphenylcarboxamid beschrieben .

Die aus dieser Schrift bekannten herbizide Mischungen und N-(1,2,5-Oxadiazol-3-yl)benzamide zeigen jedoch häufig eine nicht ausreichende herbizide Wirksamkeit. Aufgabe der vorliegenden Erfindung ist daher die Bereitstellung von weiteren herbizid wirksamen Verbindungen.

Es wurde nun gefunden, daß N-(1,2,5-Oxadiazol-3-yl)benzamide, deren Phenylring in 2-, 3- und 4-Position durch ausgewählte Reste substituiert sind, als Herbizide besonders gut geeignet sind.

Ein Gegenstand der vorliegenden Erfindung sind N-(1,2,5-Oxadiazol-3-yl)benzamide der Formel (I) oder deren Salze 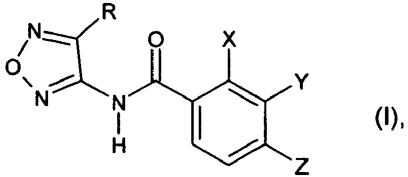 worin
R bedeutet Wasserstoff, (C₁-C₆)-Alkyl, (C₃-C₇)-cyclo-Alkyl, Halogen-(C₁-C₆)-alkyl, (C₁-C₆)-Alkoxy, Halogen-(C₁-C₆)-alkoxy, (C₂-C₆)-Alkenyl, (C₂-C₆)-Alkenyloxy, (C₂-C₆)-Halogenalkenyl, (C₂-C₆)-Alkinyl, (C₂-C₆)-Alkinyloxy, (C₂-C₆)-Halogenalkinyl, Cyano, Nitro, Methylsulfenyl, Methylsulfinyl, Methylsulfonyl, Acetylamino, Benzoylamino, Methoxycarbonyl, Ethoxycarbonyl, Methoxycarbonylmethyl, Ethoxycarbonylmethyl, Benzoyl, Methylcarbonyl, Piperidinylcarbonyl, Trifluormethylcarbonyl, Halogen, Amino, Aminocarbonyl, Methylaminocarbonyl, Dimethylaminocarbonyl, Methoxymethyl, oder
   jeweils durch s Reste aus der Gruppe Methyl, Ethyl, Methoxy, Trifluormethyl und Halogen substituiertes Heteroaryl, Heterocyclyl oder Phenyl,
X und Z bedeuten unabhängig voneinander jeweils Nitro, Halogen, Cyano, Formyl, Rhodano, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₃-C₆)-Halogenalkinyl, (C₃-C₆)-Cycloalkyl, (C₃-C₆)-Halogencycloalkyl, (C₃-C₆)-Cycloalkyl-(C₁-C₆)-alkyl, (C₃-C₆)-Halogencycloalkyl-(C₁-C₆)-alkyl, COR¹, OR¹, OCOR², OSO₂R², S(O)ₙR², SO₂OR¹, SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-COOR¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², NR₁R₂, P(O)(OR⁵)₂, oder
   jeweils durch s Reste aus der Gruppe Methyl, Ethyl, Methoxy, Nitro, Trifluormethyl und Halogen substituiertes Heteroaryl, Heterocyclyl oder Phenyl,
Y bedeutet Nitro, Halogen, Cyano, Rhodano, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₃-C₆)-Halogenalkinyl, (C₃-C₆)-Cycloalkyl, (C₃-C₆)-Halogencycloalkyl, (C₃-C₆)-Cycloalkyl-(C₁-C₆)-alkyl, (C₃-C₆)-Halogencycloalkyl-(C₁-C₆)-alkyl, COR¹,CO₂R¹ , OR¹, OCOR², OSO₂R², S(O)ₙR², SO₂OR¹, SO₂N(R¹)₂, NR¹SO₂R², NR¹COR¹, (C₁-C₆)-Alkyl-Heteroaryl, O-(C₁-C₆)-Alkyl-Heterocyclyl, O-(C₁-C₆)-Alkyl-Heteroaryl, (C₁-C₆)-Alkyl-Heterocyclyl, (C₁-C₆)-Alkyl-S(O)ₙR², (C₁-C₆)-Alkyl-OR¹, (C₁-C₆)-Alkyl-OCOR², (C₁-C₆)-Alkyl-OSO₂R², (C₁-C₆)-Alkyl-COOR¹, (C₁-C₆)-Alkyl-CN, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², NR¹R², P(O)(OR⁵)₂, Tetrahydrofuranyloxymethyl, Tetrahydrofuranylmethoxymethyl, O(CH₂)-3,5-dimethyl-1,2-oxazol-4-yl, O(CH₂)₂-O(3,5-dimethoxypyrimidin-2-yl, O(CH₂)-5-pyrrolidin-2-on, O(CH₂)-5-2,4-dimethyl-2,4-dihydro-3H-1,2,4-triazol-3-on, oder
   jeweils durch s Reste aus der Gruppe Methyl, Ethyl, Methoxy, Halogen und Cyanomethyl substituiertes Heteroaryl oder Heterocyclyl,
R¹ bedeutet Wasserstoff, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl oder Phenyl-(C₁-C₆)-alkyl, wobei die 12 letztgenannten Reste durch s Reste aus der Gruppe bestehend aus Cyano, Halogen, Nitro, Rhodano, OR³, S(O)ₙR⁴, N(R³)₂, NR³OR³, COR³, OCOR³, SCOR³, NR³COR³, CO₂R³, COSR³, CON(R³)₂ und (C₁-C₄)-Alkoxy-(C₂-C₆)-alkoxycarbonyl substituiert sind,
R² bedeutet (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, Phenyl oder Phenyl-(C₁-C₆)-alkyl, wobei die sieben letztgenannten Reste durch s Reste aus der Gruppe bestehend aus Cyano, Halogen, Nitro, Rhodano, OR³, S(O)ₙR⁴, N(R³)₂, NR³OR³, COR³, OCOR³, SCOR³, NR³COR³, CO₂R³, COSR³, CON(R³)₂ und (C₁-C₄)-Alkoxy-(C₂-C₆)-alkoxycarbonyl substituiert sind,
R³ bedeutet Wasserstoff, (C₁-C₆)-Alkyl, (C₂-C₆)-Alkenyl oder (C₂-C₆)-Alkinyl,
R⁴ bedeutet (C₁-C₆)-Alkyl, (C₂-C₆)-Alkenyl oder (C₂-C₆)-Alkinyl,
R⁵ bedeutet Methyl oder Ethyl,
n bedeutet 0, 1 oder 2,
s bedeutet 0,1,2 oder 3.

In der Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl. Halogen steht für Fluor, Chlor, Brom oder lod.

Heterocyclyl bedeutet einen gesättigten, teilgesättigten oder vollständig ungesättigten cyclischen Rest, der 3 bis 6 Ringatome enthält, von denen 1 bis 4 aus der Gruppe Sauerstoff, Stickstoff und Schwefel stammen, und der zusätzlich durch einen Benzoring annelliert sein kann. Beispielsweise steht Heterocyclyl für Piperidinyl, Pyrrolidinyl, Tetrahydrofuranyl, Dihydrofuranyl, 4,5-Dihydro-1,2-oxazol-3-yl und Oxetanyl.

Heteroaryl bedeutet einen aromatischen cyclischen Rest, der 3 bis 6 Ringatome enthält, von denen 1 bis 4 aus der Gruppe Sauerstoff, Stickstoff und Schwefel stammen, und der zusätzlich durch einen Benzoring annelliert sein kann. Beispielsweise steht Heteroaryl für Benzimidazol-2-yl, Furanyl, Imidazolyl, Isoxazolyl, Isothiazolyl, Oxazolyl, Pyrazinyl, Pyrimidinyl, Pyridazinyl, Pyridinyl, Benzisoxazolyl, Thiazolyl, Pyrrolyl, Pyrazolyl, Thiophenyl, 1,2,3-Oxadiazolyl, 1,2,4-Oxadiazolyl, 1,2,5-Oxadiazolyl, 1,3,4-Oxadiazolyl, 1,2,4-Triazolyl, 1,2,3-Triazolyl, 1,2,5-Triazolyl, 1,3,4-Triazolyl, 1,2,4-Triazolyl, 1,2,4-Thiadiazolyl, 1,3,4-Thiadiazolyl, 1,2,3-Thiadiazolyl, 1,2,5-Thiadiazolyl, 2H-1,2,3,4-Tetrazolyl, 1H-1,2,3,4-Tetrazolyl, 1,2,3,4-Oxatriazolyl, 1,2,3,5-Oxatriazolyl, 1,2,3,4-Thiatriazolyl und 1,2,3,5-Thiatriazolyl.

Ist eine Gruppe mehrfach durch Reste substituiert, so ist darunter zu verstehen, daß diese Gruppe durch ein oder mehrere gleiche oder verschiedene der genannten Reste substituiert ist.

Die Verbindungen der allgemeinen Formel (I) können je nach Art und Verknüpfung der Substituenten als Stereoisomere vorliegen. Sind beispielsweise ein oder mehrere asymmetrische Kohlenstoffatome vorhanden, so können Enantiomere und Diastereomere auftreten. Ebenso treten Stereoisomere auf, wenn n für 1 steht (Sulfoxide). Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfasst, jedoch nicht spezifisch definiert sind.

Bevorzugt sind Verbindungen der allgemeinen Formel (I), worin
R bedeutet Wasserstoff, (C₁-C₆)-Alkyl, (C₃-C₇)-cyclo-Alkyl, Halogen-(C₁-C₆)-alkyl, (C₁-C₆)-Alkoxy, Halogen-(C₁-C₆)-alkoxy, Cyano, Nitro, Methylsulfenyl, Methylsulfinyl, Methylsulfonyl, Acetylamino, Benzoylamino, Methoxycarbonyl, Ethoxycarbonyl, Benzoyl, Methylcarbonyl, Piperidinylcarbonyl, Trifluormethylcarbonyl, Halogen, Amino, Aminocarbonyl, Methylaminocarbonyl, Dimethylaminocarbonyl, Methoxymethyl,
   einen durch s Reste aus der Gruppe Methyl, Methoxy, Trifluormethyl und Halogen substituierten Heterocyclus aus der Gruppe umfassend Pyridin-2-yl, Pyridin-3-yl, Pyridin-4-yl, Piperidin-2-yl, Piperidin-3-yl, Piperidin-4-yl, Benzisoxazol-2-yl, 1,2,4-Oxadiazol-3-yl, 1,2,4-Triazol-3-yl, 1-Ethylbenzimidazol-2-yl, 4-Methylthiazol-2-yl, Thiophen-2-yl, Furan-2-yl, Furan-3-yl, Tetrahydrofuran-2-yl, Tetrahydrofuran-3-yl, Isoxazol-2-yl, Isoxazol-3-yl, Oxazol-2-yl, Oxazol-3-yl, Pyrrol-2-yl, Pyrrol-3-yl, Imidazol-2-yl, Imidazol-5-yl, Imidazol-4-yl, Pyrazol-3-yl, Pyrazol-5-yl, Pyrazol-4-yl, Isoxazol-3-yl, Isoxazol-4-yl, Isoxazol-5-yl, Oxazol-2-yl, Oxazol-4-yl, Oxazol-5-yl, Isothiazol-3-yl, Isothiazol-4-yl, Isothiazol-5-yl, Thiazol-2-yl, Thiazol-4-yl, Thiazol-5-yl 1,2,3-Triazol-4-yl, 1,2,3-Triazol-5-yl, 1,2,5-Triazol-3-yl, 1,3,4-Triazol-2-yl, 1,2,4-Triazol-3-yl, 1,2,4-Triazol-5-yl, 1,2,4-Oxadiazol-3-yl, 1,2,4-Oxadiazol-5-yl, 1,3,4-Oxadiazol-2-yl, 1,2,3-Oxadiazol-4-yl, 1,2,3-Oxadiazol-5-yl, 1,2,5-Oxadiazol-3-yl, 1,2,4-Thiadiazol-3-yl, 1,2,4-Thiadiazol-5-yl, 1,3,4-Thiadiazol-2-yl, 1,2,3-Thiadiazol-4-yl, 1,2,3-Thiadiazol-5-yl, 1,2,5-Thiadiazol-3-yl, 2H-1,2,3,4-Tetrazol-5-yl, 1H-1,2,3,4-Tetrazol-1-yl, 1,2,3,4-Oxatriazol-5-yl, 1,2,3,5-Oxatriazol-4-yl, 1,2,3,4-Thiatriazol-5-yl, 1,2,3,5-Thiatriazol-4-yl, Pyrazin-2-yl, Pyrazin-3-yl, Pyrimidin-2-yl, Pyrimidin-4-yl, Pyrimidin-5-yl, Pyridazin-3-yl und Pyridazin-4-yl, oder
   durch s Reste aus der Gruppe Methyl, Methoxy, Trifluormethyl und Halogen substituiertes Phenyl,
X und Z bedeuten unabhängig voneinander jeweils Nitro, Halogen, Cyano, Rhodano, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₃-C₆)-Halogenalkinyl, (C₃-C₆)-Cycloalkyl, (C₃-C₆)-Halogencycloalkyl, (C₃-C₆)-Cycloalkyl-(C₁-C₆)-alkyl, (C₃-C₆)-Halogencycloalkyl-(C₁-C₆)-alkyl, COR¹, OR¹, OCOR², OSO₂R², S(O)ₙR², SO₂OR¹, SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-COOR¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², Benzoxazol-2-yl, 1-Ethyl-benzimidazol-2-yl, Piperidin-1-yl oder 1,2,4-Triazol-1-yl,
Y bedeutet Nitro, Halogen, Cyano, Rhodano, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₃-C₆)-Halogenalkinyl, (C₃-C₆)-Cycloalkyl, (C₃-C₆)-Halogencycloalkyl, (C₃-C₆)-Cycloalkyl-(C₁-C₆)-alkyl, (C₃-C₆)-Halogencycloalkyl-(C₁-C₆)-alkyl, COR¹, OR¹, OCOR², OSO₂R², S(O)ₙR², SO₂OR¹, SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-COOR¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², Tetrahydrofuranyl-oxymethyl, Tetrahydrofuranylmethoxymethyl, O(CH₂)-3,5-dimethyl-1,2-oxazol-4-yl, O(CH₂)₂-O(3,5-dimethoxypyrimidin-2-yl, O(CH₂)-5-pyrrolidin-2-on oder O(CH₂)-5-2,4-dimethyl-2,4-dihydro-3H-1,2,4-triazol-3-on,
R¹ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, Phenyl oder Phenyl-(C₁-C₆)-alkyl, wobei die sieben letztgenannten Reste durch s Reste aus der Gruppe bestehend aus Cyano, Halogen, Nitro, Rhodano, OR³, S(O)ₙR⁴, N(R³)₂, NR³OR³, COR³, OCOR³, SCOR³, NR³COR³, CO₂R³, COSR³, CON(R³)₂ und (C₁-C₄)-Alkoxy-(C₂-C₆)-alkoxycarbonyl substituiert sind,
R² bedeutet (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, Phenyl oder Phenyl-(C₁-C₆)-alkyl, wobei die sieben letztgenannten Reste durch s Reste aus der Gruppe bestehend aus Cyano, Halogen, Nitro, Rhodano, OR³, S(O)ₙR⁴, N(R³)₂, NR³OR³, COR³, OCOR³, SCOR³, NR³COR³, CO₂R³, COSR³, CON(R³)₂ und (C₁-C₄)-Alkoxy-(C₂-C₆)-alkoxycarbonyl substituiert sind,
R³ bedeutet Wasserstoff, (C₁-C₆)-Alkyl, (C₂-C₆)-Alkenyl oder (C₂-C₆)-Alkinyl,
R⁴ bedeutet (C₁-C₆)-Alkyl, (C₂-C₆)-Alkenyl oder (C₂-C₆)-Alkinyl,
n bedeutet 0, 1 oder 2,
s bedeutet 0, 1, 2 oder 3:

Besonders bevorzugt sind Verbindungen der allgemeinen Formel (I), worin
R bedeutet Wasserstoff, (C₁-C₆)-Alkyl, (C₃-C₇)-cyclo-Alkyl, Halogen-(C₁-C₆)-alkyl, (C₁-C₆)-Alkoxy, Halogen-(C₁-C₆)-alkoxy, Cyano, Nitro, Methylsulfenyl, Methylsulfinyl, Methylsulfonyl, Acetylamino, Benzoylamino, Methoxycarbonyl, Ethoxycarbonyl, Benzoyl, Methylcarbonyl, Piperidinylcarbonyl, Trifluormethylcarbonyl, Halogen, Amino, Aminocarbonyl, Methylaminocarbonyl, Dimethylaminocarbonyl, Methoxymethyl,
X und Z bedeuten unabhängig voneinander jeweils Nitro, Halogen, Cyano, (C₁-C₆)-Alkyl, (C₁-C₆)-Halogenalkyl, (C₃-C₆)-Cycloalkyl, OR¹, S(O)ₙR², (C₁-C₆)-Alkyl-S(O)ₙR², (C₁-C₆)-Alkyl-OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R² oder 1,2,4-Triazol-1-yl,
Y bedeutet Nitro, Halogen, Cyano, (C₁-C₆)-Alkyl, (C₁-C₆)-Halogenalkyl, OR¹, S(O)ₙR², SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², Tetrahydrofuranyl-oxymethyl, Tetrahydrofuranylmethoxymethyl, O(CH₂)-3,5-dimethyl-1,2-oxazol-4-yl, O(CH₂)₂-O(3,5-dimethoxypyrimidin-2-yl, O(CH₂)-5-pyrrolidin-2-on oder O(CH₂)-5-2,4-dimethyl-2,4-dihydro-3H-1,2,4-triazol-3-on,
R¹ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, Phenyl oder Phenyl-(C₁-C₆)-alkyl, wobei die sieben letztgenannten Reste durch s Reste aus der Gruppe bestehend aus Halogen und OR³ substituiert sind,
R² bedeutet (C₁-C₆)-Alkyl, (C₃-C₆)-Cycloalkyl oder (C₃-C₆)-Cycloalkyl-(C₁-C₆)-alkyl, wobei die drei letztgenannten Reste durch s Reste aus der Gruppe bestehend aus Halogen und OR³ substituiert sind,
R³ bedeutet Wasserstoff oder (C₁-C₆)-Alkyl,
n bedeutet 0, 1 oder 2,
s bedeutet 0, 1, 2 oder 3.

Besonders bevorzugt sind auch Verbindungen der allgemeinen Formel (I), worin
R bedeutet Wasserstoff, (C₁-C₆)-Alkyl, (C₃-C₇)-cyclo-Alkyl, Halogen-(C₁-C₆)-alkyl, (C₁-C6)-Alkoxy, Halogen-(C₁-C₆)-alkoxy, Cyano, Nitro, Methylsulfenyl, Methylsulfinyl, Methylsulfonyl, Acetylamino, Benzoylamino, Methoxycarbonyl, Ethoxycarbonyl, Benzoyl, Methylcarbonyl, Piperidinylcarbonyl, Trifluormethylcarbonyl, Halogen, Amino, Aminocarbonyl, Methylaminocarbonyl, Dimethylaminocarbonyl, Methoxymethyl,
X und Z bedeuten unabhängig voneinander jeweils Nitro, Halogen, Cyano, (C₁-C₆)-Alkyl, (C₁-C₆)-Halogenalkyl, (C₃-C₆)-Cycloalkyl, OR¹, S(O)ₙR², (C₁-C₆)-Alkyl-S(O)ₙR², (C₁-C₆)-Alkyl-OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R² oder 1,2,4-Triazol-1-yl,
Y bedeutet S(O)ₙR², 4,5-Dihydro-1,2-oxazol-3-yl, 5-Cyanomethyl-4,5-dihydro-1,2-oxazol-3-yl oder 5-Methoxymethyl-4,5-dihydro-1,2-oxazol-3-yl,
R¹ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, Phenyl oder Phenyl-(C₁-C₆)-alkyl, wobei die sieben letztgenannten Reste durch s Reste aus der Gruppe bestehend aus Halogen und OR³ substituiert sind,
R² bedeutet (C₁-C₆)-Alkyl, (C₃-C₆)-Cycloalkyl oder (C₃-C₆)-Cycloalkyl-(C₁-C₆)-alkyl, wobei die drei letztgenannten Reste durch s Reste aus der Gruppe bestehend aus Halogen und OR³ substituiert sind,
R³ bedeutet Wasserstoff oder (C₁-C₆)-Alkyl,
n bedeutet 0, 1 oder 2,
s bedeutet 0, 1, 2 oder 3.

In allen nachfolgend genannten Formeln haben die Substituenten und Symbole, sofern nicht anders definiert, dieselbe Bedeutung wie unter Formel (I) beschrieben.

Erfindungsgemäße Verbindungen können beispielsweise nach der in Schema 1 angegebenen Methode durch basenkatalysierte Umsetzung eines Benzoesäurechlorids (II) mit einem 4-Amino-1,2,5-oxadiazol (III) hergestellt werden:

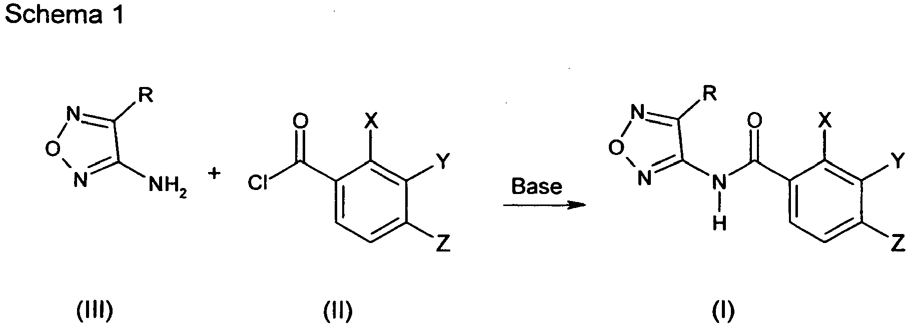

Die Benzoesäurechloride der Formel (II) beziehungsweise die ihnen zugrunde liegenden Benzoesäuren sind grundsätzlich bekannt und können beispielsweise gemäß den in US 6,376,429 B1, EP 1 585 742 A1 und EP 1 202 978 A1 beschriebenen Methoden hergestellt werden.

Erfindungsgemäße Verbindungen können auch nach der in Schema 2 angegebenen Methode durch Umsetzung einer Benzoesäure der Formel (IV) mit einem 4-Amino-1,2,5-oxadiazol (III) hergestellt werden:

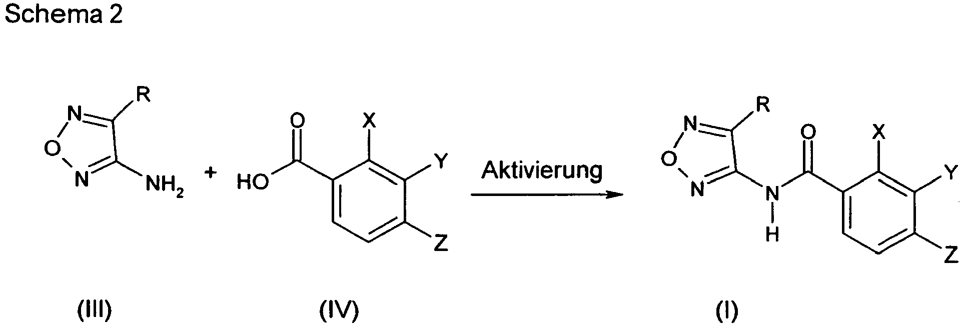

Für die Aktivierung können wasserentziehende Reagenzien eingesetzt werden, die üblicherweise fürAmidierungsreaktionen, wie z. B. 1,1' -Carbonyldiimidazol (CDI), Dicyclohexyl-carbodiimid (DCC), 2,4,6-Tripropyl-1,3,5,2,4,6-trioxatriphosphinane 2,4,6-trioxide (T3P) etc. verwendet werden.

Die 4-Amino-1,2,5-oxadiazole der Formel (III) sind entweder käuflich erhältlich oder bekannt oder können analog zu literaturbekannten Methoden hergestellt werden. Beispielsweise können 3-Alkyl-4-amino-1,2,5-oxadiazole nach der in Russian Chemical Bulletin, Int. Ed., Vol. 54, No. 4, S. 1032-1037 (2005) beschriebenen Methode aus ß-Ketoestern hergestellt werden:

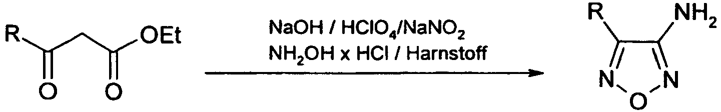

In der oben genannten Formel steht R beispielsweise für einen Alkyl- oder Phenylrest. 3-Aryl-4-amino-1,2,5-oxadiazofen können zum Beispiel wie in Russian Chemical Bulletin, 54(4), 1057-1059, (2005) oder Indian Journal of Chemistry, Section B: Organic Chemistry Including Medicinal Chemistry, 26B(7), 690-2, (1987) beschrieben, synthetisiert werden: 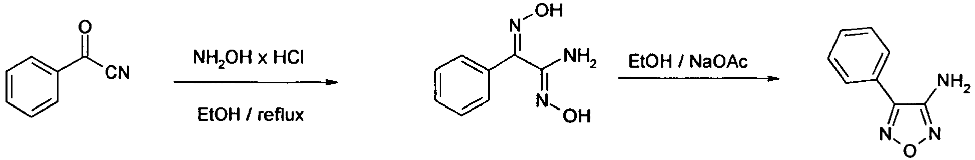 3-Amino-4-Halogen-1,2,5-oxadiazole können beispielsweise nach der in Heteroatom Chemistry 15(3), 199-207 (2004) beschrieben Methode aus dem käuflich erhätlichen 3,4-Diamino-1,2,5-oxadiazol hergestellt werden:

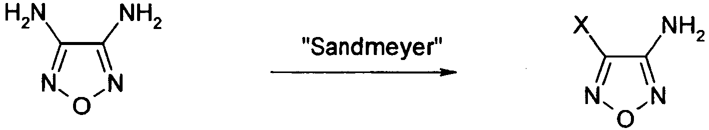

Nucleophile Reste R können wie in Journal of Chemical Research, Synopses, (6), 190, 1985 oder in oder lzvestiya Akademii Nauk SSSR, Seriya Khimicheskaya, (9), 2086-8, 1986 oder in Russian Chemical Bulletin (Translation of Izvestiya Akademii Nauk, Seriya Khimicheskaya), 53(3), 596-614, 2004 beschrieben, durch Substitution der Austrittsgruppe L in 3-Amino-1,2,5-oxadiazolen der allgemeinen Formel V eingeführt werden:

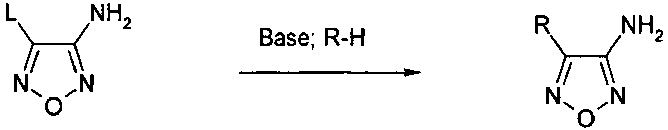

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry ― Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, lowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der allgemeinen Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der allgemeinen Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasen- unterstützte Synthesemethoden sind in der Fachliteratur hinreichend beschrieben, z.B. Barry A. Bunin in "The Combinatorial Index", Verlag Academic Press, 1998 und Combinatorial Chemistry ― Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und a. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Die erfindungsgemäßen Verbindungen der Formel (I) (und/oder deren Salze), im folgenden zusammen als "erfindungsgemäße Verbindungen" bezeichnet, weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, lmperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, lpomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, lpomoea, Lactuca, Linum, Lycopersicon, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt bezüglich transgener Kulturen ist die Anwendung der erfindungsgemäßen Verbindungen in wirtschaftlich bedeutenden transgenen Kulturen von Nutz- und Zierpflanzen, z. B. von Getreide wie Weizen, Gerste, Roggen, Hafer, Hirse, Reis und Mais oder auch Kulturen von Zuckerrübe, Baumwolle, Soja, Raps, Kartoffel, Tomate, Erbse und anderen Gemüsesorten.Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Bevorzugt ist die Anwendung der erfindungsgemäßen Verbindungen oder deren Salze in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten. Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398).
- Transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt, siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY, oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet. Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227, Wolter et al., Proc. Natl. Acad. Sci. USA 85 (1988), 846-850, Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydroxyphenylpyruvat Dioxygenasen (HPPD) hemmen, respektive gegen Herbizide aus der Gruppe der Sulfonylharnstoffe, Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973, K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry", 2nd Ed., J. Wiley & Sons, N.Y., C. Marsden, "Solvents Guide", 2nd Ed., Interscience, N.Y. 1963, McCutcheon's "Detergents and Emulsifiers Annual", MC Publ. Corp., Ridgewood N.J., Sisley and Wood, "Encyclopedia of Surface Active Agents", Chem. Publ. Co. Inc., N.Y. 1964, Schönfeldt, "Grenzflächenaktive Äthylenoxidaddukte", Wiss. Verlagsgesell., Stuttgart 1976, Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix. Geeignete Safener sind beispielsweise Mefenpyr-diethyl, Cyprosulfamid, Isoxadifen-ethyl, Cloquintocet-mexyl und Dichlormid.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührem, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London, J.E. Browning, "Agglomeration", Chemical and Engineering 1967, Seiten 147 ff, "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen.
In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441-445 oder "The Pesticide Manual", 14th edition, The British Crop Protection Council and the Royal Soc. of Chemistry, 2003 und dort zitierter Literatur beschrieben sind. Als bekannte Herbizide oder Pflanzenwachstumsregulatoren, die mit den erfindungsgemäßen Verbindungen kombiniert werden können, sind z.B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere wie Stereoisomere und optische Isomere. Dabei sind beispielhaft eine und zum Teil auch mehrere Anwendungsformen genannt:
Acetochlor, Acibenzolar, Acibenzolar-S-methyl, Acifluorfen, Acifluorfen-sodium, Aclonifen, Alachlor, Allidochlor, Alloxydim, Alloxydim-sodium, Ametryn, Amicarbazone, Amidochlor, Amidosulfuron, Aminopyralid, Amitrole, Ammoniumsulfamat, Ancymidol, Anilofos, Asulam, Atrazine, Azafenidin, Azimsulfuron, Aziprotryn, BAH-043, BAS-140H, BAS-693H, BAS-714H, BAS-762H, BAS-776H, BAS-800H, Beflubutamid, Benazolin, Benazolin-ethyl, bencarbazone, Benfluralin, Benfuresate, Bensulide, Bensulfuron-methyl, Bentazone, Benzfendizone, Benzobicyclon, Benzofenap, Benzofluor, Benzoylprop, Bifenox, Bilanafos, Bilanafosnatrium, Bispyribac, Bispyribac-natrium, Bromacil, Bromobutide, Bromofenoxim, Bromoxynil, Bromuron, Buminafos, Busoxinone, Butachlor, Butafenacil, Butamifos, Butenachlor, Butralin, Butroxydim, Butylate, Cafenstrole, Carbetamide, Carfentrazone, Carfentrazone-ethyl, Chlomethoxyfen, Chloramben, Chlorazifop, Chlorazifop-butyl, Chlorbromuron, Chlorbufam, Chlorfenac, Chlorfenac-natrium, Chlorfenprop, Chlorflurenol, Chlorflurenol-methyl, Chloridazon, Chlorimuron, Chlorimuron-ethyl, Chlormequat-chlorid, Chlornitrofen, Chlorophthalim, Chlorthaldimethyl, Chlorotoluron, Chlorsulfuron, Cinidon, Cinidon-ethyl, Cinmethylin, Cinosulfuron, Clethodim, Clodinafop Clodinafop-propargyl, Clofencet, Clomazone, Clomeprop, Cloprop, Clopyralid, Cloransulam, Cloransulam-methyl, Cumyluron, Cyanamide, Cyanazine, Cyclanilide, Cycloate, Cyclosulfamuron, Cycloxydim, Cycluron, Cyhalofop, Cyhalofop-butyl, Cyperquat, Cyprazine, Cyprazole, 2,4-D, 2,4-DB, Daimuron/Dymron, Dalapon, Daminozide, Dazomet, n-Decanol, Desmedipham, Desmetryn, Detosyl-Pyrazolate (DTP), Diallate, Dicamba, Dichlobenil, Dichlorprop, Dichlorprop-P, Diclofop, Diclofop-methyl, Diclofop-P-methyl, Diclosulam, Diethatyl, Diethatyl-ethyl, Difenoxuron, Difenzoquat, Diflufenican, Diflufenzopyr, Diflufenzopyrnatrium, Dimefuron, Dikegulac-sodium, Dimefuron, Dimepiperate, Dimethachlor, Dimethametryn, Dimethenamid, Dimethenamid-P, Dimethipin, Dimetrasulfuron, Dinitramine, Dinoseb, Dinoterb, Diphenamid, Dipropetryn, Diquat, Diquat-dibromide, Dithiopyr, Diuron, DNOC, Eglinazine-ethyl, Endothal, EPTC, Esprocarb, Ethalfluralin, Ethametsulfuron-methyl, Ethephon, Ethidimuron, Ethiozin, Ethofumesate, Ethoxyfen, Ethoxyfen-ethyl, Ethoxysulfuron, Etobenzanid, F-5331, d.h. N-[2-Chlor-4-fluor-5-[4-(3fluorpropyl)-4,5-dihydro-5-oxo-1 H-tetrazol-1-yl]-phenyl]-ethansulfonamid, Fenoprop, Fenoxaprop, Fenoxaprop-P, Fenoxaprop-ethyl, Fenoxaprop-P-ethyl, Fentrazamide, Fenuron, Flamprop, Flamprop-M-isopropyl, Flamprop-M-methyl, Flazasulfuron, Florasulam, Fluazifop, Fluazifop-P, Fluazifop-butyl, Fluazifop-P-butyl, Fluazolate, Flucarbazone, Flucarbazone-sodium, Flucetosulfuron, Fluchloralin, Flufenacet (Thiafluamide), Flufenpyr, Flufenpyr-ethyl, Flumetralin, Flumetsulam, Flumiclorac, Flumiclorac-pentyl, Flumioxazin, Flumipropyn, Fluometuron, Fluorodifen, Fluoroglycofen, Fluoroglycofen-ethyl, Flupoxam, Flupropacil, Flupropanate, Flupyrsulfuron, Flupyrsulfuron-methyl-sodium, Flurenol, Flurenol-butyl, Fluridone, Flurochloridone, Fluroxypyr, Fluroxypyr-meptyl, Flurprimidol, Flurtamone, Fluthiacet, Fluthiacet-methyl, Fluthiamide, Fomesafen, Foramsulfuron, Forchlorfenuron, Fosamine, Furyloxyfen, Gibberellinsäure, Glufosinate, L-Glufosinate, L-Glufosinate-ammonium, Glufosinate-ammonium, Glyphosate, Glyphosate-isopropylammonium, H-9201, Halosafen, Halosulfuron, Halosulfuronmethyl, Haloxyfop, Haloxyfop-P, Haloxyfop-ethoxyethyl, Haloxyfop-P-ethoxyethyl, Haloxyfop-methyl, Haloxyfop-P-methyl, Hexazinone, HNPC-9908, HOK-201, HW-02, Imazamethabenz, Imazamethabenz-methyl, Imazamox, lmazapic, lmazapyr, Imazaquin, Imazethapyr, Imazosulfuron, Inabenfide, Indanofan, Indolessigsäure (IAA), 4-Indol-3-ylbuttersäure (IBA), lodosulfuron, lodosulfuron-methyl-natrium, loxynil, Isocarbamid, Isopropalin, Isoproturon, Isouron, Isoxaben, Isoxachlortole, Isoxaflutole, Isoxapyrifop, IDH-100, KUH-043, KUH-071, Karbutilate, Ketospiradox, Lactofen, Lenacil, Linuron, Maleinsäurehydrazid, MCPA, MCPB, MCPB-methyl, - ethyl und -natrium, Mecoprop, Mecoprop-natrium, Mecoprop-butotyl, Mecoprop-P-butotyl, Mecoprop-P-dimethylammonium, Mecoprop-P-2-ethylhexyl, Mecoprop-P-kalium, Mefenacet, Mefluidide, Mepiquat-chlorid, Mesosulfuron, Mesosulfuronmethyl, Mesotrione, Methabenzthiazuron, Metam, Metamifop, Metamitron, Metazachlor, Methazole, Methoxyphenone, Methyldymron, 1-Methylcyclopropen, Methylisothiocyanat, Metobenzuron, Metobenzuron, Metobromuron, Metolachlor, S-Metolachlor, Metosulam, Metoxuron, Metribuzin, Metsulfuron, Metsulfuron-methyl, Molinate, Monalide, Monocarbamide, Monocarbamide-dihydrogensulfat, Monolinuron, Monosulfuron, Monuron, MT 128, MT-5950, d. h. N-[3-Chlor-4-(1-methylethyl)-phenyl]-2-methylpentanamid, NGGC-011, Naproanilide, Napropamide, Naptalam, NC-310, d.h. 4-(2,4-dichlorobenzoyl)-1-methyl-5-benzyloxypyrazole, Neburon, Nicosulfuron, Nipyraclofen, Nitralin, Nitrofen, Nitrophenolat-natrium (Isomerengemisch), Nitrofluorfen, Nonansäure, Norflurazon, Orbencarb, Orthosulfamuron, Oryzalin, Oxadiargyl, Oxadiazon, Oxasulfuron, Oxaziclomefone, Oxyfluorfen, Paclobutrazol, Paraquat, Paraquat-dichlorid, Pelargonsäure (Nonansäure), Pendimethalin, Pendralin, Penoxsulam, Pentanochlor, Pentoxazone, Perfluidone, Pethoxamid, Phenisopham, Phenmedipham, Phenmedipham-ethyl, Picloram, Picolinafen, Pinoxaden, Piperophos, Pirifenop, Pirifenop-butyl, Pretilachlor, Primisuffuron, Primisulfuron-methyl, Probenazole, Profluazol, Procyazine, Prodiamine, Prifluraline, Profoxydim, Prohexadione, Prohexadione-calcium, Prohydrojasmone, Prometon, Prometryn, Propachlor, Propanil, Propaquizafop, Propazine, Propham, Propisochlor, Propoxycarbazone, Propoxycarbazone-natrium, Propyzamide, Prosulfalin, Prosulfocarb, Prosulfuron, Prynachlor, Pyraclonil, Pyraflufen, Pyraflufen-ethyl, Pyrasulfotole, Pyrazolynate (Pyrazolate), Pyrazosulfuron-ethyl, Pyrazoxyfen, Pyribambenz, Pyribambenz-isopropyl, Pyribenzoxim, Pyributicarb, Pyridafol, Pyridate, Pyriftalid, Pyriminobac, Pyriminobacmethyl, Pyrimisulfan, Pyrithiobac, Pyrithiobac-natrium, Pyroxasulfone, Pyroxsulam, Quinclorac, Quinmerac, Quinoclamine, Quizalofop, Quizalofop-ethyl, Quizalofop-P, Quizalofop-P-ethyl, Quizalofop-P-tefuryl, Rimsulfuron, Secbumeton, Sethoxydim, Siduron, Simazine, Simetryn, SN-106279, Sulcotrione, Sulfallate (CDEC), Sulfentrazone, Sulfometuron, Sulfometuron-methyl, Sulfosate (Glyphosatetrimesium), Sulfosulfuron, SYN-523, SYP-249, SYP-298, SYP-300, Tebutam, Tebuthiuron, Tecnazene, Tefuryltrione, Tembotrione, Tepraloxydim, Terbacil, Terbucarb, Terbuchlor, Terbumeton, Terbuthylazine, Terbutryn, TH-547, Thenylchlor, Thiafluamide, Thiazafluron, Thiazopyr, Thidiazimin, Thidiazuron, Thiencarbazone, Thiencarbazone-methyl, Thifensuffuron, Thifensulfuron-methyl, Thiobencarb, Tiocarbazil, Topramezone, Tralkoxydim, Triallate, Triasulfuron, Triaziflam, Triazofenamide, Tribenuron, Tribenuron-methyl, Trichloressigsäure (TCA), Triclopyr, Tridiphane, Trietazine, Trifloxysulfuron, Trifloxysulfuron-natrium, Trifluralin, Triflusulfuron, Triflusulfuron-methyl, Trimeturon, Trinexapac, Trinexapacethyl, Tritosulfuron, Tsitodef, Uniconazole, Uniconazole-P, Vemolate, ZJ-0166, ZJ-0270, ZJ-0543, ZJ-0862 sowie die folgenden Verbindungen

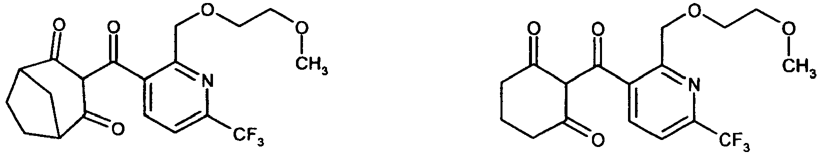

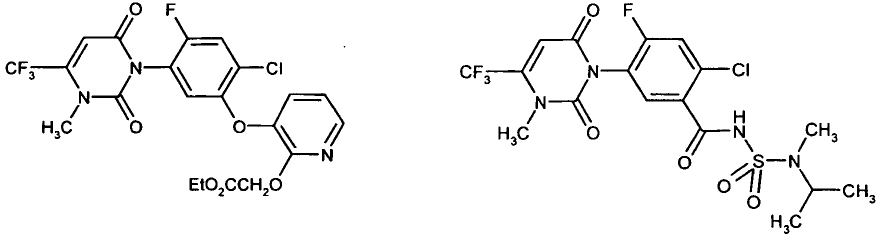

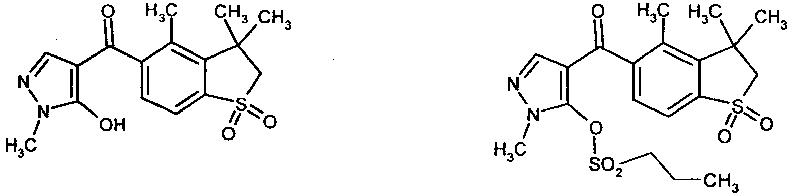

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die nachstehenden Beispiele erläutern die Erfindung.

### A. Chemische Beispiele

### Synthese von N-(4-Methyl-1,2,5-oxadiazol-3-yl)-4-(methylsulfonyl)-2-nitrobenzamid, (Tabellenbeispiel Nr. 1-18)

495 mg (2,02 mmol) 2-Nitro-4-methylsulfonylbenzoesaeure und 200 mg (2,02 mmol) 4-Methyl-1,2,5-oxadiazol-3-yl-amin werden bei Raumtemperatur (RT) in 5 ml CH₂Cl₂ gelöst und mit 0,28 ml (2,02 mmol) Triethylamin, 49 mg (0,40 mmol) 4-Dimethylaminopyridin (DMAP) und 1,93 g (3,03 mmol) 2,4,6-Tripropyl-1,3,5,2,4,6-trioxatriphosphinan-2,4,6-trioxid (50%ige Lösung in THF) versetzt. Das Reaktionsgemisch wird 30 h bei RT gerührt und anschließend zweimal mit jeweils 5 ml Wasser gewaschen. Die organische Phase wird über Na₂SO₄ getrocknet und eingedampft. Der Rückstand wird säulenchromatographisch (Kieselgel, Heptan/Essigester) gereinigt. Ausbeute 350 mg (53%).

### Synthese von 2-Chlor-N-(4-methoxy-1,2,5-oxadiazol-3-yl)-4-(methylsulfonyl)-3-[(2,2,2-trifluorethoxy)methyl]benzamid, (Tabellenbeispiel Nr. 9-174)

200 mg (0,58 mmol) 2-Chlor-4-(methylsulfonyl)-3-[(2,2,2-trifluorethoxy) methyl]benzoesäure und 66 mg (0,58 mmol) 4-Methoxy-1,2,5-oxadiazol-3-amin werden bei RT in 10 ml CH₂Cl₂ gelöst und mit 0,08 ml (0,6 mmol) Triethylamin, 14 mg (0,12 mmol) DMAP und 511 mg (0,87 mmol) 2,4,6-Tripropyl-1,3,5,2,4,6-trioxatriphosphinan-2,4,6-trioxid (50%ige Lösung in THF) versetzt. Das Reaktionsgemisch wird 20 h bei RT gerührt und anschließend zweimal mit jeweils 5ml Wasser gewaschen. Die organische Phase wird über Na₂SO₄ getrocknet und eingedampft. Der Rückstand wird säulenchromatographisch (Kieselgel, Heptan/Essigester) gereinigt. Ausbeute 90 mg (33%).

### Synthese von 2-Chlor-3-[5-(cyanmethyl)-4,5-dihydro-1,2-oxazol-3-yl]-N-(4-cyan-1,2,5-oxadiazol-3-yl)-4-(ethylsulfonyl)benzamid, (Tabellenbeispiel Nr. 8-120)

200 mg (0,56 mmol) 2-Chlor-3-[5-(cyanmethyl)-4,5-dihydro-1,2-oxazol-3-yl]-4-(ethylsulfonyl)benzoesäure und 62 mg (0,58 mmol) 4-Methoxy-1,2,5-oxadiazol-3-amin werden bei RT in 24 ml CH₂Cl₂ gelöst und mit 0,08 ml (0,6 mmol) Triethylamin, 14 mg (0,12 mmol) DMAP und 535 mg (0,84 mmol) 2,4,6-Tripropyl-1,3,5,2,4,6-trioxatriphosphinan-2,4,6-trioxid (50%ige Lösung in THF) versetzt. Das Reaktionsgemisch wird 20 h bei RT gerührt und anschließend zweimal mit jeweils 10 ml Wasser gewaschen. Die organische Phase wird über Na₂SO₄ getrocknet und eingedampft. Der Rückstand wird säulenchromatographisch (Kieselgel, Heptan/Essigester) gereinigt. Ausbeute 120 mg (45%).

### Synthese von N-(4-Benzoyl-1,2,5-oxadiazol-3-yl)-2-chlor-4-(methylsulfonyl)-3-[(2,2,2-trifluorethoxy)methyl]benzamid, (Tabellenbeispiel Nr. 11-5)

204 mg (0,59 mmol) 2-Chlor-4-(methylsulfonyl)-3-[(2,2,2-trifluorethoxy)-methyl]benzoesäure und 111 mg (0,59 mmol) (4-Amino-1,2,5-oxadiazol-3-yl)(phenyl)methanon werden bei RT in 10 ml Dichlormethan gelöst und mit 59 mg (0,599 mmol) Triethylamin, 14 mg (0,19 mmol) DMAP und 561 mg (0,882 mmol) 2,4,6-tripropyl-1,3,5,2,4,6-trioxatriphosphinane 2,4,6-trioxide (50%ige Lösung in THF) versetzt. Das Reaktionsgemisch wird 30 h bei RT gerührt und anschließend zweimal mit jeweils 5ml Wasser gewaschen. Die organische Phase wird über Na₂SO₄ getrocknet und eingedampft. Der Rückstand wird säulen-chromatographisch (Kieselgel, Heptan/Essigester) gereinigt. Ausbeute 50 mg (15%).

### Synthese von 2-Chlor-N-[4-(4-chlorphenyl)-1,2,5-oxadiazol-3-yl]-4-(methylsulfonyl)benzamid, (Tabellenbeispiel Nr. 10-4)

200 mg (0,85 mmol) 2-Chlor-4-(methylsulfonyl)-benzoesäure und 167 mg (0,85 mmol) 4-(4-Chlorphenyl)-1,2,5-oxadiazol-3-yl-amin werden bei RT in 24 ml CH₂Cl₂ gelöst und mit 0,12 ml (0,85 mmol) Triethylamin, 21 mg (0,17 mmol) DMAP und 1814 mg (1,28 mmol) 2,4,6-Tripropyl-1,3,5,2,4,6-trioxatriphosphinan-2,4,6-trioxid (50%ige Lösung in THF) versetzt. Das Gemisch wird 30 h bei RT gerührt und dann zweimal mit jeweils 5 ml Wasser gewaschen. Die organische Phase wird über Na₂SO₄ getrocknet und eingedampft. Der Rückstand wird säulenchromatographisch (Kieselgel, Heptan/Essigester) gereinigt. Ausbeute 140 mg (36%).

### Synthese von N-(4-Acetamido-1,2,5-oxadiazol-3-yl)-2-chlor-4-(methylsulfonyl) benzamid, (Tabellenbeispiel Nr. 11-31)

173 mg (0,5 mmol) 2-Chlor-4-(methylsulfonyl)-benzoesaeure und 191 mg (0,505 mmol) N-(4-Amino-1,2,5-oxadiazol-3-yl)acetamid werden bei RT in 5 ml Dichloethan gelöst und mit 0,071 ml (0,5 mmol) Triethylamin, 31 mg (0,25 mmol) DMAP und 483 mg (0,76 mmol) 2,4,6-Tripropyl-1,3,5,2,4,6-trioxatriphosphinan-2,4,6-trioxid (50%ige Lösung in THF) versetzt. Das Reaktionsgemisch wird 24 h bei RT gerührt und anschließend über eine Glasfritte filtriert. Der Rückstand wird zweimal mit jeweils 1 ml CH₂Cl₂ gewaschen. Das Filtrat wird über eine mit Kieselgel bestückte SPE-Kartusche gegeben und zweimal mit jeweils 5 ml eines Gemisches aus Essigsäureethylester/ Methyl-tert. Butylester (1:1) eluiert. Die organische Phase wird über Na₂SO₄ getrocknet und eingedampft. Der Rückstand wird durch präparative HPLC mit Acetonitril/Wasser gereinigt. Ausbeute 16 mg (9%).

Die in den nachfolgenden Tabellen aufgeführten Beispiele wurden analog oben genannten Methoden hergestellt beziehungsweise sind analog oben genannten Methoden erhältlich. Die in den nachfolgenden Tabellen aufgeführten Verbindungen sind ganz besonders bevorzugt

Die verwendeten Abkürzungen bedeuten:

| | | | | | | | |
|---|---|---|---|---|---|---|---|
| Et | = Ethyl | Me | = Methyl | n-Pr | = n-Propyl | i-Pr | = Isopropyl |
| Pen | = Pentyl | Ph | = Phenyl | Ac | = Acetyl | Bz | = Benzoyl |
| c-Pr | = Cyclopropyl | | | | | | |

**Tabelle 1: Erfindungsgemäße Verbindungen der allgemeinen Formel (I), worin R für Methyl steht**

| | | | | |
|---|---|---|---|---|
| 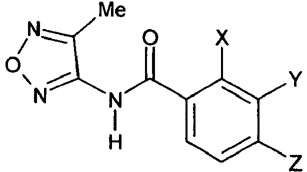 | | | | |

| **Nr.** | **X** | **Y** | **Z** | **Physikalische Daten** |
|---|---|---|---|---|
| 1-1 | CF₃ | OCH₂CON(Me)Et | SO₂Me | ¹H-NMR, CDCl₃, 400 MHz |
| | | | | 9,05 (bs, 1H), 8,29 (d, 1H), 7,60 (d, 1 H), 4,92 (s, 1H), 4,85 (s, 1H), 3,43 (s, 3H), 3,43 (q, 1H), 3,16 (q, 1H), 2,94 (s, 3H), 2,85 (s, 3H), 2,50 (s, 3H), 1,17 (t, 1,5H), 1,12 (t, 1,5H) |
| 1-2 | CF₃ | OCH₂CON(Me)Et | SO₂Et | ¹H-NMR, CDCl₃, 400 MHz |
| | | | | 9,89 (bs, 1H), 8,25 (d, 1H), 7,59 (d, 1H), 4,41 (s, 1H), 4,33 (s, 1H), 3,66 (q, 2H), 3,43 (q, 1H), 3,16 (q, 1H), 2,93 (s, 1,5H), 2,85 (s, 1,5H), 2,49 (s, 3H), 1,25 (t, 3H), 1,18 (t, 1,5H), 1,12 (t, 1,5H) |
| 1-4 | CF₃ | 2-(1H-Pyrazol-1-yl)ethoxyl | SO₂Me | ¹H-NMR, CDCl₃, 400 MHz |
| | | | | 8,29 (d, 1H), 8,24 (bs, 1H), 7,57 (bd, 1 H), 7,52 (d, 2H), 6,31 (t, 1H), 4,62 (s, 4H), 3,07 (s, 3H), 2,49 (s, 3H), |
| 1-5 | CF₃ | 2-(1H-Pyrazol-1-yl)ethoxyl | SO₂Et | ¹H-NMR, CDCl₃, 400 MHz |
| | | | | 8,16 (d, 1H), 7,78 (d, 1H), 7,60 (d, 1H), 7,56 (d, 1H), 6,38 (t, 1H), 4,60-4,68 (m, 4H), 3,21 (q, 2H), 2,32 (s, 3H), 1,12 (t, 3H) |
| 1-6 | CF₃ | Tetrahydrofuran-2-yl-methoxy | SO₂Me | ¹H-NMR, CDCl₃, 400 MHz |
| | | | | 8,31 (d, 1H), 8,00 (bs, 1H), 7,55 (d, 1 H), 4,38-4,45 (m, 1H), 4,29 (t, 1H), 4,15 (dd, 1H), 3,90 (ddd, 2H), 3,39 (s, 3H), 2,48 (s, 3H), 2,02-2,12 (m, 1H), 1,90-2,00 (m, 2H), 1,62-1,72 (m, 1H) |
| 1-7 | CF₃ | Tetrahydrofuran-2-yl-methoxy | SO₂Et | ¹H-NMR, CDCl₃, 400 MHz |
| | | | | 8,26 (d, 1H), 8,22 (bs, 1H), 7,54 (d, 1H), 4,37-4,43 (m, 1H), 4,22 (t, 1H), 4,08-4,13 (m, 2H), 3,90 (ddd, 2H), 3,52-3,60 (m, 2H), 2,50 (s, 3H), 2,04-2,12 (m, 1H), 1,90-1,99 (m, 2H), 1,62-1,71 (m, 1H), 1,24 (t, 3H) |
| 1-8 | CF₃ | OH | SO₂Me | ¹H-NMR, CD₃OD, 400 MHz |
| | | | | 8,18 (d, 1H), 7,34 (d, 1H), 3,35 (s, 3H), |
| | | | | 2,44 (s, 3H) |
| 1-9 | CF₃ | OH | SO₂Et | ¹H-NMR, CDCl₃, 400 MHz |
| | | | | 9,90 (s, 1H), 7,93 (d, 1H), 7,83 (bs, 1H), 7,21 (d, 1H), 3,25 (q, 2H), 2,50 (s, 3H), 1,38 (t, 3H) |
| 1-10 | CF₃ | SH | SO₂Me | ¹H-NMR, CD₃OD, 400 MHz |
| | | | | 8,34 (pt, 1H), 7,79 (d, 1H), 3,38 (s, 3H), 2,44 (s, 3H) |
| 1-11 | CF₃ | SH | SO₂Et | ¹H-NMR, CD₃OD, 400 MHz |
| | | | | 8,42 (d, 1H), 7,70 (d, 1H), 3,62 (q, 2H), 2,46 (s, 3H), 1,28 (t, 3H) |
| 1-15 | CF₃ | SMe | SO₂Me | ¹H-NMR, CD₃OD, 400 MHz |
| | | | | 8,58 (d, 1H), 7,97 (d, 1H), 7,55 (d, 1H), 3,58 (s, 3H), 2,59 (s, 3H), 2,46 (s, 3H) |
| 1-16 | CF₃ | SMe | SO₂Et | ¹H-NMR, CDCl₃, 400 MHz |
| | | | | 8,57 (bs, 1H), 8,33 (d, 1H), 7,75 (d, 1 H), 3,74 (q, 2H), 2,50 (s, 3H), 1,25 (t, 3H), |
| 1-17 | CF₃ | S(O)Me | SO₂Me | ¹H-NMR, CDCl₃, 400 MHz |
| | | | | 8,53 (d, 1H), 7,92 (d, 1H), 3,45 (s, 3H), 3,40 (s, 3H), 2,48 (s, 3H), |
| 1-24 | CF₃ | S(O)Me | SO₂Et | ¹H-NMR, CDCl₃, 400 MHz |
| | | | | 8,62 (d, 1H), 8,26 (d, 1H), 3,85 (q, 2H), 3,72 (s, 3H), 2,45 (s, 3H), 1,42 (t, 3H), |
| 1-25 | CF₃ | S(O)₂Me | SO₂Me | ¹H-NMR, CD₃OD, 400 MHz |
| | | | | 8,68 (d, 1H), 8,26 (d, 1H), 3,70 (s, 3H), 3,60 (s, 3H), 2,44 (s, 3H) |
| 1-26 | CF₃ | S(O)₂Me | SO₂Et | ¹H-NMR, CD₃OD, 400 MHz |
| | | | | 8,59 (d, 1H), 8,19 (d, 1H), 3,82 (q, 2H), 3,71 (s, 3H), 2,44 (s, 3H), 1,42 (t, 3H), |
| 1-27 | CF₃ | 2-[(Methylsulfonyl) amino]ethoxy | SO₂Me | ¹H-NMR, DMSO-d₆, 400 MHz |
| | | | | 8,11 (d, 1H), 7,35-7,42 (m, 2H), 3,50 (t, 2H), 3,39 (s, 3H), 3,25-3,35 (m, 2H), 2,95 (s, 3H), 2,37 (s, 3H) |
| 1-28 | CF₃ | 2-[(Methylsulfonyl) amino]ethyl}sulfanyl | SO₂Me | ¹H-NMR, CD₃OD, 400 MHz |
| | | | | 8,56 (d, 1H), 7,95 (d, 1H), 3,56 (s, 3H), 3,43 (t, 2H), 3,23 (t, 2H), 2,96 (s, 3H), 2,42 (s, 3H) |
| 1-29 | CF₃ | 2-[(Methylsulfonyl) amino]ethyl}sulfanyl | SO₂Et | ¹H-NMR, CD₃OD, 400 MHz |
| | | | | 8,58 (d, 1H), 8,00 (d, 1H), 3,86 (q, 2H), 3,46 (t, 2H), 3,26 (t, 2H), 3,00 (s, 3H), 2,47 (s, 3H), 1,27 (t, 3H) |
| 1-30 | NO₂ | O(CH₂)₂OMe | OMe | ¹H-NMR, CDCl₃, 400 MHz |
| | | | | 8,35 (bs, 1H), 7,45 (m, 2H), 4,15 (m, 2H), 3,70 (m, 2H), 3,42 (s, 3H), 2,46 (s, 3H), 2,43 (s, 3H) |
| 1-31 | NO₂ | OMe | Me | ¹H-NMR, DMSO-d₆, 400 MHz |
| | | | | 11,64 (s, 1H), 7,72 (d, 1H), 7,67 (d, 1 H), 3,82 (s, 3H), 2,41 (s, 3H), 2,31 (s, 3H) |
| 1-32 | NO₂ | NH₂ | OMe | |
| 1-33 | NO₂ | NH₂ | SO₂Et | |
| 1-34 | NO₂ | NH₂ | Cl | ¹H-NMR, CDCl₃, 400 MHz |
| | | | | 7.72 (s, 1H), 7.59 (d, 1H), 6.83 (d, 1 H), 6.49 (s, 2H), 2.53 (s, 3H) |
| 1-35 | NO₂ | NHMe | Cl | |
| 1-36 | NO₂ | NMe₂ | Cl | ¹H-NMR, CDCl₃, 400 MHz |
| | | | | 9.79 (bs, 1H), 8.18 (s, br, 1H), 7.60 (d, 1 H), 7.46 (d, 1H), 2.82 (s, 6H), 2.42 (s, 3H) |
| 1-37 | NO₂ | NH₂ | Br | |
| 1-38 | NO₂ | NHMe | Br | |
| 1-39 | NO₂ | NMe₂ | Br | |
| 1-40 | NO₂ | NH₂ | F | |
| 1-41 | NO₂ | NHMe | F | |
| 1-42 | NO₂ | NMe₂ | F | |
| 1-43 | NO₂ | NH₂ | SO₂Me | |
| 1-44 | NO₂ | NHMe | SO₂Me | |
| 1-45 | NO₂ | NMe₂ | SO₂Me | |
| 1-46 | NO₂ | NH₂ | 1H-1,2,4-triazol-1-yl | |
| 1-47 | NO₂ | NHMe | 1H-1,2,4-triazol-1-yl | |
| 1-48 | NO₂ | NMe₂ | 1H-1,2,4-triazol-1-yl | |
| 1-49 | Me | F | F | ¹H-NMR, DMSO-d₆, 400 MHz |
| | | | | 11,30 (s, 1H), 7,40-7,60 (m, 2H), 2,50 (s, 3H), 2,39 (s, 3H) |
| 1-50 | Me | F | Cl | ¹H-NMR, DMSO-d₆, 400 MHz |
| | | | | 11,38 (s, 1H), 7,49 (d, 1H), 7,62 (dd, 1H), 2,50 (s, 3H), 2,39 (s, 3H) |
| 1-51 | Me | SMe | CF₃ | ¹H-NMR, DMSO-d₆, 400 MHz |
| | | | | 11,40 (s, 1H), 7,81 (d, 1H), 7,74 (d, 1 H), 2,68 (s, 3H), 2,41 (s, 3H), 2,33 (s, 3H) |
| 1-52 | Me | Cl | SO₂Me | ¹H-NMR, DMSO-d₆, 400 MHz |
| | | | | 11,51 (s, 1H), 8,05 (d, 1 H), 7,82 (d, 1 H), |
| | | | | 3,45 (s, 3H), 2,50 (s, 3H), 2,42 (s, 3H) |
| 1-53 | Me | SO₂Me | SO₂Me | ¹H-NMR, DMSO-d₆, 400 MHz |
| | | | | 11,53 (s, 1H), 8,26 (d, 1H), 8,08 (d, 1H), 3,61 (s, 3H), 3,57 (s, 3H), 2,72 (s, 3H), 2,41 (s, 3H) |
| 1-54 | Me | SO₂Me | CF₃ | ¹H-NMR, DMSO-d₆, 400 MHz |
| | | | | 11,52 (bs, 1H), 8,04 (m, 2H), 3,44 (s, 3H), 2,75 (s, 3H), 2,41 (s, 3H) |
| 1-55 | Me | Cl | CF₃ | ¹H-NMR, DMSO-d₆, 400 MHz |
| | | | | 11,46 (s, 1H), 7,88 (d, 1H), 7,74 (d, 1H), 2,50 (s, 3H), 2,42 (s, 3H) |
| 1-56 | Me | S(O)Me | CF₃ | ¹H-NMR, DMSO-d₆, 400 MHz |
| | | | | 11,50 (s, 1H), 7,88 (m, 2H), 3,07 (s, 3H), 2,87 (s, 3H), 2,42 (s, 3H) |
| 1-57 | Me | SEt | OMe | ¹H-NMR, CDCl₃, 400 MHz |
| | | | | 8,02 (bs, 1H), 7,52 (d, 1H), 6,80 (d, 1H), 3,95 (s, 3H), 2,83 (q, 2H), 2,69 (s, 3H), 2,49 (s, 3H), 1,15 (t, 3H) |
| 1-58 | Me | NMe₂ | SO₂Me | ¹H-NMR, DMSO-d₆, 400 MHz |
| | | | | 11,38 (s, 1H), 7,86 (d, 1H), 7,61 (d, 1H), 3,35 (s, 3H), 2,84 (s, 6H), 2,40 (s, 6H) |
| 1-59 | Me | NH(CH₂)₂OMe | SO₂Me | ¹H-NMR, DMSO-d₆, 400 MHz |
| | | | | 11,33 (s, 1H), 7,72 (d, 1 H), 7,23 (d, 1 H), 5,70 (t, 1 H), 3,55 (m, 2H), 3,33 (m, 5H), 2,40 (s, 3H), 2,31 (s, 3H) |
| 1-60 | Me | O(CH₂)₄OMe | SO₂Me | ¹H-NMR, CDCl₃, 400 MHz |
| | | | | 8,45 (bs, 1H), 7,75 (d, 1 H), 7,45 (d, 1 H), 4,01 (t, 2H), 3,46 (t, 2H), 3,35 (s, 3H), 3,23 (s, 3H), 2,50 (s, 3H), 2,46 (s, 3H), 1,94 (m, 2H), 1,77 (m, 2H) |
| 1-61 | Me | NH₂ | SO₂Me | ¹H-NMR, DMSO-d₆, 400 MHz |
| | | | | 11,28 (s, 1H), 7,57 (d, 1H), 6,88 (d, 1 H), 6,06 (bs, 2H), 3,16 (s, 3H), 2,39 (s, 3H), 2,19 (s, 3H) |
| 1-62 | Me | O(CH₂)₂-O(3,5-Dimethoxypyrimidin-2-yl | SO₂Me | ¹H-NMR, CDCl₃, 400 MHz |
| | | | | 8,60 (bs, 1H), 7,80 (d, 1H), 7,45 (d, 1 H), 5,71 (s, 1H), 4,76 (t, 2H), 4,43 (t, 2H), 3,90 (s, 6H), 3,28 (s, 3H), 2,49 (s, 3H), 2,50 (s, 3H) |
| 1-63 | Me | O(CH₂)₂-O-NMe₂ | Cl | ¹H-NMR, CDCl₃, 400 MHz |
| | | | | 7,88 (bs, 1H), 7,33 (d, 1H), 7,27 (d, 1H), 4,38 (t, 2H), 4,20 (t, 2H), 2,49 (s, 3H), 2,45 (s, 3H), 1,88 (s, 6H) |
| 1-64 | Me | O(CH₂)₂-NH(CO)NMe₂ | Cl | ¹H-NMR, DMSO-d₆, 400 MHz |
| | | | | 11,25 (s, 1H), 7,48 (d, 1 H), 7,37 (d, 1H), 6,54 (t, 1H), 3,91 (t, 2H), 3,44 (q, 2H), |
| | | | | 2,80 (s, 6H), 2,38 (s, 3H), 2,34 (s, 3H) |
| 1-65 | Me | O(CH₂)-5-Pyrrolidin-2-on | Br | ¹H-NMR, CDCl₃, 400 MHz |
| | | | | 9,44 (bs, 1H), 7,51 (d, 1H), 7,25 (d, 1H), 6,0 (bs, 1H), 4,10 (m, 1H), 3,76-3,89 (m, 2H), 2,47 (s, 3H), 2,44 (s, 3H), 2,30 (m, 3H), 1,88 (m, 1 H) |
| 1-66 | Me | O(CH₂)₂-NH(CO)NHCO₂Et | Cl | ¹H-NMR, CDCl₃, 400 MHz |
| | | | | 8,53 (bs, 1H), 8,24 (t, 1H), 7,30 (m, 3H), 4,22 (q, 2H), 4,03 (t, 2H), 3,70 (q, 2H), 2,49 (s, 3H), 2,45 (s, 3H), 1,31 (t, 3H) |
| 1-67 | Me | O(CH₂)-(CO)NEt₂ | Br | ¹H-NMR, DMSO-d₆, 400 MHz |
| | | | | 11,25 (s, 1H), 7,64 (d, 1H), 7,34 (d, 1 H), 4,59 (s, 2H), 3,35 (m, 4H), 2,37 (s, 6H), 1,14 (t, 3H), 1,08 (t, 3H) |
| 1-68 | Me | O(CH₂) -5-2,4-Dimethyl-2,4-dihydro-3H-1,2,4-triazol-3-on | Cl | ¹H-NMR, DMSO-d₆, 400 MHz |
| | | | | 11,30 (s, 1H), 7,52 (d, 1H), 7,44 (d, 1H), 4,90 (s, 2H), 3,35 (m, 6H), 2,38 (s, 3H), 2,33 (s, 3H) |
| 1-69 | Me | O(CH₂)-3,5-Dimethyl-1,2-oxazol-4-yl | Cl | ¹H-NMR, CDCl₃, 400 MHz |
| | | | | 8,0 (bs, 1H), 7,39 (d, 1H), 7,33 (d, 1 H), 4,75 (s, 2H), 2,49 (s, 3H), 2,44 (s, 3H), 2,42 (s, 3H), 2,36 (s, 3H) |
| 1-70 | Me | O(CH₂)₂-NHCO₂Me | Cl | ¹H-NMR, DMSO-d₆, 400 MHz |
| | | | | 11,23 (s, 1 H), 7,48 (d, 1H), 7,38 (d, 1H), 3,92 (t, 2H), 3,55 (s, 3H), 3,42 (q, 2H), 2,37 (s, 3H), 2,44 (s, 3H) |
| 1-71 | Me | 4,5-Dihydro-1,2-oxazol-3 yl | SO₂Me | ¹H-NMR, DMSO-d₆, 400 MHz |
| | | | | 11,48 (s, 1H), 8,03 (d, 1H), 7,92 (d, 1 H), 4,50 (t, 2H), 3,34 (t, 2H), 3,27 (s, 3H), 2,41 (s, 3H), 2,35 (s, 3H) |
| 1-72 | Me | Me | SO₂Me | ¹H-NMR, DMSO-d₆, 400 MHz |
| | | | | 11,32 (s, 1H), 7,91 (d, 1H), 7,59 (d, 1H), 3,23 (s, 3H), 2, 33 (s, 3H), 2,31 (s, 3H) |
| 1-73 | Me | OH | SO₂Me | |
| 1-74 | Me | O-CH₂-NHSO₂cPr | Cl | |
| 1-75 | Me | O(CH₂)₂NHSO2Me | SO₂Me | |
| 1-76 | Me | S(O)Me | SO₂Me | |
| 1-77 | Me | SMe | SO₂Me | |
| 1-78 | Me | SMe | OMe | |
| 1-79 | Me | S(O)Me | OMe | |
| 1-80 | Me | SO₂Me | OMe | |
| 1-81 | Me | SMe | Cl | |
| 1-82 | Me | S(O)Me | Cl | ¹H-NMR, CDCl₃, 400 MHz |
| | | | | 7,54 (d, 1H), 7,37 (d, 1H), 2,92 (s, 3H), |
| | | | | 2,67 (s, 3H), 2,47 (s, 3H) |
| 1-83 | Me | SO₂Me | Cl | |
| 1-84 | Me | [1,4]Dioxan-2-yl-methoxy | SO₂Me | |
| 1-85 | Me | [1,4]Dioxan-2-yl-methoxy | SO₂Et | |
| 1-86 | Me | O(CH₂)₄OMe | SO₂Et | |
| 1-87 | Me | O(CH₂)₃OMe | SO₂Me | |
| 1-88 | Me | O(CH₂)₃OMe | SO₂Et | |
| 1-89 | Me | O(CH₂)₂OMe | SO₂Me | |
| 1-90 | Me | O(CH₂)₂OMe | SO₂Et | |
| 1-91 | Me | S(O)Me | SO₂Me | ¹H-NMR, CDCl₃, 400 MHz |
| | | | | 8,05 (d, 1H), 7,76 (d, 1H), 3,36 (s, 3H), 3,16 (s, 3H), 2,91 (s, 3H), 2,52 (s, 3H) |
| 1-92 | Me | SMe | SO₂Me | ¹H-NMR, CDCl₃, 400 MHz |
| | | | | 8,11 (d, 1H), 7,67 (d, 1H), 3,46 (s, 3H), 2,80 (s, 3H), 2,52 (s, 3H), 2,42 (s, 3H) |
| 1-93 | Me | SMe | OMe | |
| 1-94 | Me | S(O)Me | OMe | |
| 1-95 | Me | SO₂Me | OMe | |
| 1-96 | Me | SMe | Cl | ¹H-NMR, CDCl₃, 400 MHz |
| | | | | 7,44 (d, 1H), 7,42 (d, 1H), 2,75 (s, 3H), 2,49 (s, 3H), 2,37 (s, 3H) |
| 1-97 | Me | S(O)Me | Cl | |
| 1-98 | Me | SO₂Me | Cl | |
| 1-99 | Me | SMe | Br | |
| 1-100 | Me | SOMe | Br | |
| 1-101 | Me | SO₂Me | Br | |
| 1-102 | Me | SMe | I | |
| 1-103 | Me | SOMe | I | |
| 1-104 | Me | SO₂Me | I | |
| 1-105 | Me | SEt | Cl | ¹H-NMR, CDCl₃, 400 MHz |
| | | | | 7,44 (d, 1H), 7,42 (d, 1H), 2,86 (q, 2H), 2,73 (s, 3H), 2,48 (s, 3H), 1,22 (t, 3H) |
| 1-106 | Me | SOEt | Cl | ¹H-NMR, CDCl₃, 400 MHz |
| | | | | 7,54 (d, 1H), 7,36 (d, 1 H), 3,26 (m, 1 H), 3,03 (m, 1H), 2,65 (s, 3H), 2,47 (s, 3H), 1,33 (t, 3H) |
| 1-107 | Me | SO₂Et | Cl | ¹H-NMR, CDCl₃, 400 MHz |
| | | | | 7,61 (d, 1H), 7,52 (d, 1H), 3,42 (q, 2H), 2,82 (s, 3H), 2,49 (s, 3H), 1,35 (t, 3H) |
| 1-108 | Me | SEt | Br | |
| 1-109 | Me | SOEt | Br | |
| 1-110 | Me | SO₂Et | Br | |
| 1-111 | Me | SEt | I | |
| 1-112 | Me | SOEt | I | |
| 1-113 | Me | SO₂Et | I | |
| 1-114 | Me | SEt | F | |
| 1-115 | Me | SOEt | F | |
| 1-116 | Me | SO₂Et | F | |
| 1-117 | Cl | OCH₂(CO)NMe₂ | Cl | ¹H-NMR, CDCl₃, 400 MHz |
| | | | | 7,39 (d, 2H), 7,26 (d, 1H), 4,62 (s, 2H), 3,12 (s, 3H), 3,02 (s, 3H), 2,41 (s, 3H) |
| 1-119 | Cl | CH₂OCH₂CF₃ | SO₂Me | ¹H-NMR, CDCl₃, 400 MHz |
| | | | | 11,25 (s, 1H), 8,18 (d, 1H), 7,75 (d, 1 H), 5,38 (s, 2H), 4,05 (q, 2H), 3,25 (s, 3H), 2,45 (s, 3H) |
| 1-120 | Cl | 5-Cyanomethyl- 4,5-dihydro-1,2-oxazol-3 yl | SO₂Et | ¹H-NMR, DMSO-d₆, 400 MHz |
| | | | | 11,69 (s, 1H), 8,12 (m, 2H), 5,20 (m, 1H), 3,61 (m, 1H), 3,43 (q, 2H), 3,19 (m, 1 H), 3,02 (m, 2H), 2,40 (s, 3H), 1,18 (t, 3H) |
| 1-121 | Cl | CH₂O-Tetrahydrofuran-3-yl | SO₂Me | ¹H-NMR, CDCl₃, 400 MHz |
| | | | | 8,88 (bs, 1H), 8,02 (d, 1H), 7,62 (d, 1H), 5,11 (m, 2H), 4,36 (m, 1H), 3,92-3,74 (m, 4H), 3,21 (s, 3H), 2,51 (s, 3H), 2,10 (m, 2H) |
| 1-122 | Cl | CH₂O-Tetrahydrofuran-2-yl | SO₂Me | |
| 1-123 | Cl | SMe | SO₂Me | ¹H-NMR, DMSO-d₆, 400 MHz |
| | | | | 11,62 (s, 1H), 8,14 (d, 1H), 7,95 (d, 1 H), 3,59 (s, 3H), 2,50 (s, 3H), 2,41 (s, 3H) |
| 1-124 | Cl | F | SMe | ¹H-NMR, DMSO-d₆, 400 MHz |
| | | | | 11,46 (bs, 1H), 7,58 (d, 1H), 7,46 (t, 1 H), 2,59 (s, 3H), 2,39 (s, 3H) |
| 1-125 | Cl | CH₂OCH₂-Tetrahydrofuran-2-yl | SO₂Me | ¹H-NMR, DMSO-d₆, 400 MHz |
| | | | | 11,65 (s, 1H), 8,12 (d, 1H), 7,91 (d, 1H), 5,10 (m, 2H), 3,98 (m, 1 H), 3,72 (dd, 1 H), 3,51-3,63 (m, 3H), 3,30 (s, 3H), 2,40 (s, 3H), 1,89 (m, 1H), 1,79 (m, 2H), 1,53 (m, 1H) |
| 1-126 | Cl | CH₂OCH₂-Tetrahydrofuran-3-yl | SO₂Et | |
| 1-127 | Cl | O(CH₂)-5-Pyrrolidin-2-on | Cl | ¹H-NMR, CDCl₃, 400 MHz |
| | | | | 9,49 (bs, 1H), 7,44 (s, 2H), 6,38 (bs, 1 H), 4,12 (m, 2H), 3,92 (t, 1H), 2,47 (s, 3H), 2,33 (m, 3H), 1,92 (m, 1H) |
| 1-128 | Cl | SMe | Cl | ¹H-NMR, DMSO-d₆, 400 MHz |
| | | | | 11,42 (s, 1H), 7,23 (d, 1H), 7,181 (d, 1H), 3,30 (s, 3H), 2,39 (s, 3H) |
| 1-129 | Cl | S(O)Me | SO₂Me | |
| 1-130 | Cl | CH₂O-Tetrahydrofuran-3-yl | SO₂Et | |
| 1-131 | Cl | O(CH₂)₂OMe | Cl | |
| 1-132 | Cl | O(CH₂)₂OMe | SO₂Me | |
| 1-133 | Cl | O(CH₂)₄OMe | SO₂Me | |
| 1-134 | Cl | O(CH₂)₄OMe | SO₂Et | |
| 1-135 | Cl | O(CH₂)₃OMe | SO₂Me | |
| 1-136 | Cl | O(CH₂)₃OMe | SO₂Et | |
| 1-137 | Cl | O(CH₂)₂OMe | SO₂Me | |
| 1-138 | Cl | O(CH₂)₂OMe | SO₂Et | |
| 1-139 | Cl | [1,4]Dioxan-2-yl-methoxy | SO₂Me | |
| 1-140 | Cl | [1,4]Dioxan-2-yl-methoxy | SO₂Et | |
| 1-141 | Cl | SO₂Me | Me | |
| 1-142 | Cl | SEt | Me | |
| 1-143 | Cl | SOEt | Me | |
| 1-144 | Cl | SO₂Et | Me | |
| 1-145 | Cl | 4,5-Dihydro-1,2-oxazol-3 yl | SO₂Me | |
| 1-146 | Cl | Cl | SO₂Me | ¹H-NMR, CDCl₃, 400 MHz |
| | | | | 8,30 (bs, 1H), 8,18 (d, 1H), 7,77 (d, 1H), 3,30 (s, 3H), 2,51 (s, 3H) |
| 1-147 | F | SMe | CF₃ | ¹H-NMR, DMSO-d₆, 400 MHz |
| | | | | 11,57 (s, 1H), 7,92 (dd, 1H), 7,81 (dd, 1 H), 2,50 (s, 3H), 2,39 (s, 3H) |
| 1-148 | F | S(O)Me | CF₃ | ¹H-NMR, DMSO-d₆, 400 MHz |
| | | | | 11,69 (s, 1H), 8,15 (dd, 1H), 7,90 (dd, 1 H), 3,14 (s, 3H), 2,40 (s, 3H), |
| 1-149 | OMe | SMe | CF₃ | ¹H-NMR, DMSO-d₆, 400 MHz |
| | | | | 11,36 (s, 1H), 7,78 (d, 1H), 7,70 (d, 1 H), 3,96 (s, 3H), 2,40 (s, 3H), 2,46 (s, 3H) |
| 1-150 | OMe | S(O)Me | CF₃ | ¹H-NMR, CDCl₃, 400 MHz |
| | | | | 8,32 (d, 1H), 7,72 (d, 1H), 4,21 (s, 3H), 3,14 (s, 3H), 2,51 (s, 3H) |
| 1-151 | OMe | SO₂Me | CF₃ | |
| 1-152 | Et | NH(CH₂)₂OMe | SO₂Me | ¹H-NMR, CDCl₃, 400 MHz |
| | | | | 8,64 (bs, 1H), 7,59 (d, 1H), 7,13 (d, 1H), 3,60 (m, 2H), 3,38 (m, 5H), 3,20 (s, 3H), |
| | | | | 2,87 (q, 2H), 2,52 (s, 3H), 1,25 (t, 3H) |
| 1-153 | Et | F | SO₂Me | ¹H-NMR, CDCl₃, 400 MHz |
| | | | | 8,62 (bs, 1H), 7,69 (dd, 1H), 7,49 (dd, 1H), 3,22 (s, 3H), 2,90 (m, 2H), 2,50 (s, 3H), 1,29 (t, 3H) |
| 1-154 | Et | SMe | CF₃ | |
| 1-155 | CF₃ | F | SO₂Me | ¹H-NMR, DMSO-d₆, 400 MHz |
| | | | | 11,75 (s, 1H), 8,32 (t, 1H), 7,90 (d, 1H), 3,50 (s, 3H), 2,39 (s, 3H) |
| 1-156 | CF₃ | F | SO₂Et | ¹H-NMR, DMSO-d₆, 400 MHz |
| | | | | 11,75 (s, 1H), 8,30 (t, 1H), 7,90 (d, 1H), 3,56 (q, 2H), 2,40 (s, 3H), 1,20 (t, 3H) |
| 1-157 | CF₃ | O(CH₂)₂OMe | SO₂Et | ¹H-NMR, CDCl₃, 400 MHz |
| | | | | 8,33 (bs, 1H), 8,23 (d, 1 H), 7,55 (d, 1 H), 4,39 (t, 2H), 3,81 (t, 2H), 3,55 (q, 2H), 3,43 (s, 3H), 2,50 (s, 3H), 1,25 (t, 3H), |
| 1-158 | CF₃ | O(CH₂)₃OMe | SO₂Et | ¹H-NMR, CDCl₃, 400 MHz |
| | | | | 8,40 (bs, 1H), 8,21 (d, 1H), 7,54 (d, 1H), 4,29 (t, 2H), 3,56 (t, 2H), 3,43 (q, 2H), 3,36 (s, 3H), 2,49 (s, 3H), 2,13 (quin, 2H), 1,25 (t, 3H) |
| 1-159 | CF₃ | O(CH₂)₂OMe | SO₂Me | ¹H-NMR, CDCl₃, 400 MHz |
| | | | | 8,88 (bs, 1H), 8,29 (d, 1H), 7,54 (d, 1H), 4,42 (t, 2H), 3,82 (t, 2H), 3,46 (s, 3H), 3,40 (s, 3H), 2,48 (s, 3H), |
| 1-160 | CF₃ | O(CH₂)₃OMe | SO₂Me | ¹H-NMR, CDCl₃, 400 MHz |
| | | | | 8,31 (bs, 1H), 8,30 (d, 1H), 7,55 (d, 1H), 4,36 (t, 2H), 3,58 (t, 2H), 3,38 (s, 3H), 3,29 (s, 3H), 2,49 (s, 3H), 2,17 (quin, 2H) |
| 1-161 | CF₃ | OCH₂CONMe₂ | SO₂Me | ¹H-NMR, CDCl₃, 400 MHz |
| | | | | 9,10 (bs, 1H), 8,29 (d, 1H), 7,60 (d, 1H), 4,89 (s, 2H), 3,43 (s, 3H), 2,98 (s, 3H), 2,88 (s, 3H), 2,48 (s, 3H) |
| 1-162 | CF₃ | OCH₂CONMe₂ | SO₂Et | ¹H-NMR, CDCl₃, 400 MHz |
| | | | | 9,35 (bs, 1H), 8,28 (d, 1H), 7,60 (d, 1H), 4,87 (s, 2H), 3,68 (q, 2H), 3,00 (s, 3H), 2,90 (s, 3H), 2,48 (s, 3H), 1,23 (t, 3H) |
| 1-163 | CF₃ | OCH₂CONMe₂ | Cl | |
| 1-164 | CF₃ | OCH₂CONMe₂ | Br | |
| 1-165 | CF₃ | OCH₂CONMe₂ | I | |
| 1-166 | CF₃ | OCH₂CONMe₂ | F | |
| 1-167 | CF₃ | O(CH₂)₂OMe | Cl | |
| 1-168 | CF₃ | O(CH₂)₃OMe | Cl | |
| 1-169 | CF₃ | O(CH₂)₂OMe | Br | |
| 1-170 | CF₃ | O(CH₂)₃OMe | Br | |
| 1-171 | CF₃ | O(CH₂)₂OMe | I | |
| 1-172 | CF₃ | O(CH₂)₃OMe | I | |
| 1-173 | CF₃ | O(CH₂)₂OMe | F | |
| 1-174 | CF₃ | O(CH₂)₃OMe | F | |
| 1-175 | CF₃ | [1,4]Dioxan-2-yl-methoxy | SO₂Me | ¹H-NMR, CDCl₃, 400 MHz |
| | | | | 8,59 (bs, 1H), 8,38 (d, 1H), 7,75 (d, 1H), 3,88-3,95 (m, 1H), 3,68-3,85 (m, 4H), 3,55-3,65 (m, 1H), 3,50 (s, 3H), 3,36 (dd, 1 H), 3,20 (dd, 1H), 2,94 (dd, 1H), 2,50 (s, 3H) |
| 1-176 | CF₃ | [1,4]Dioxan-2-yl-methoxy | SO₂Et | ¹H-NMR, CDCl₃, 400 MHz |
| | | | | 8,62 (bs, 1H), 8,32 (d, 1H), 7,75 (d, 1H), 3,88-3,95 (m, 1H), 3,68-3,82 (m, 6H), 3,56-3,64 (m, 1H), 3,36 (dd, 1H), 3,19 (dd, 1 H), 2,92-2,97 (m, 1 H), 2,50 (s, 3H), 1,25 (t, 3H) |
| 1-177 | CF₃ | [1,4]Dioxan-2-yl-methoxy | Cl | |
| 1-178 | CF₃ | [1,4]Dioxan-2-yl-methoxy | Br | |
| 1-179 | CF₃ | [1,4]Dioxan-2-yl-methoxy | I | |
| 1-180 | CF₃ | [1,4]Dioxan-2-yl-methoxy | F | |
| 1-181 | Br | OMe | Br | ¹H-NMR, DMSO-d₆, 400 MHz |
| | | | | 11,43 (s, 1H), 7,83 (d, 1H), 7,38 (d, 1H), 3,85 (s, 3H), 2,40 (s, 3H) |
| 1-182 | Br | O(CH₂)₂OMe | Br | ¹H-NMR, CDCl₃, 400 MHz |
| | | | | 8,17 (bs, 1H), 7,65 (d, 1H), 7,30 (d, 1H), 4,22 (m, 2H), 3,85 (m, 2H), 3,47 (s, 3H), 2,50 (s, 3H) |
| 1-183 | Br | O(CH₂)₄OMe | SO₂Me | |
| 1-184 | Br | O(CH₂)₄OMe | SO₂Et | |
| 1-185 | Br | O(CH₂)₃OMe | SO₂Me | |
| 1-186 | Br | O(CH₂)₃OMe | SO₂Et | |
| 1-187 | Br | O(CH₂)₂OMe | SO₂Me | |
| 1-188 | Br | O(CH₂)₂OMe | SO₂Et | |
| 1-189 | Br | [1,4]Dioxan-2-yl-methoxy | SO₂Me | |
| 1-190 | Br | [1,4]Dioxan-2-yl-methoxy | SO₂Et | |
| 1-191 | Br | SMe | Me | |
| 1-192 | Br | SOMe | Me | |
| 1-193 | Br | SO₂Me | Me | |
| 1-194 | Br | SEt | Me | |
| 1-195 | Br | SOEt | Me | |
| 1-196 | Br | SO₂Et | Me | |
| 1-197 | I | O(CH₂)₄OMe | SO₂Me | |
| 1-198 | I | O(CH₂)₄OMe | SO₂Et | |
| 1-199 | I | O(CH₂)₃OMe | SO₂Me | |
| 1-200 | I | O(CH₂)₃OMe | SO₂Et | |
| 1-201 | I | O(CH₂)₂OMe | SO₂Me | |
| 1-202 | I | O(CH₂)₂OMe | SO₂Et | |
| 1-203 | I | [1,4]Dioxan-2-yl-methoxy | SO₂Me | |
| 1-204 | I | [1,4]Dioxan-2-yl-methoxy | SO₂Et | |
| 1-205 | I | SMe | Me | |
| 1-206 | I | SOMe | Me | |
| 1-207 | I | SO₂Me | Me | |
| 1-208 | I | SEt | Me | |
| 1-209 | I | SOEt | Me | |
| 1-210 | I | SO₂Et | Me | |
| 1-211 | CH₂SMe | OMe | SO₂Me | ¹H-NMR, DMSO-d₆, 400 MHz |
| | | | | 11,50 (s, 1H), 7,88 (d, 1H), 7,66 (d, 1H), 4,04 (s, 2H), 3,97 (s, 3H), 3,33 (s, 3H), 2,41 (s, 3H), 2,05 (s, 3H) |
| 1-212 | CH₂OMe | OMe | SO₂Me | |
| 1-213 | CH₂O(CH₂)₂ OMe | NH(CH₂)₂OEt | SO₂Me | ¹H-NMR, CDCl₃, 400 MHz 9,79 (bs, 1 H), 7,95 (d, 1H), 7,38 (d, 1H), 4,65 (s, 2H), 3,75 (m, 2H), 3,51-3,67 (m, 8H), 3,19 (s, 3H), 3,18 (s, 3H), 2,46 (s, 3H), 1,23 (t, 3H) |
| 1-214 | CH₂O(CH₂)₂ OMe | NH(CH₂)₃OEt | SO₂Me | |
| 1-215 | CH₂O(CH₂)₃ OMe | OMe | SO₂Me | ¹H-NMR, DMSO-d₆, 400 MHz |
| | | | | 11,36 (s, 1H), 7,92 (d, 1H), 7,61 (d, 1H), 4,69 (s, 2H), 3,92 (s, 3H), 3,45 (t, 2H), 3,34 (s, 3H), 3,25 (t, 2H), 3,10 (s, 3H), 2,40 (s, 3H), 1,64 (m, 2H) |
| 1-216 | CH₂O(CH₂)₂ OMe | NH(CH₂)₂OMe | SO₂Me | |
| 1-217 | CH₂O(CH₂)₂ OMe | NH(CH₂)₃OMe | SO₂Me | |
| 1-218 | SO₂Me | NH₂ | CF₃ | |
| 1-219 | SO₂Me | F | CF₃ | |
| 1-220 | SO₂Me | NHEt | Cl | |
| 1-221 | SMe | SEt | F | |
| 1-222 | SMe | SMe | F | ¹H-NMR, DMSO-d₆, 400 MHz |
| | | | | 11,20 (s, 1H), 7,55 (dd, 1H), 7,42 (dd, 1H), 2,40 (s, 3H) |
| 1-223 | Me | NH₂ | Cl | |
| 1-224 | Me | NH₂ | Br | |
| 1-225 | Me | NHMe | Cl | |
| 1-226 | Me | NHMe | Br | |
| 1-227 | Me | NMe₂ | Cl | ¹H-NMR, CDCl₃, 400 MHz |
| | | | | 7,80 (bs, 1H), 7,28 (d, 1H), 7,26 (d, 1H), 2,88 (s, 6H), 2,49 (s, 3H), 2,47 (s, 3H) |
| 1-228 | Me | NMe₂ | Br | ¹H-NMR, CDCl₃, 400 MHz |
| | | | | 7,75 (bs, 1H), 7,50 (d, 1H), 7,18 (d, 1H), 2,88 (s, 6H), 2,49 (s, 3H), 2,47 (s, 3H) |
| 1-227 | Me | NMe₂ | Cl | |
| 1-228 | Me | NMe₂ | Br | |
| 1-229 | NO₂ | O(CH₂)₂OMe | Me | ¹H-NMR, CDCl₃, 400 MHz |
| | | | | 8,34 (bs, 1H), 7,46 (d, 1H), 7,44 (d, 1H), 4,15 (t, 2H), 3,70 (t, 2H), 3,41 (s, 3H), 2,46 (s, 3H), 2,41 (s, 3H) |
| 1-230 | CF₃ | S(O)₂Et | SO₂Me | ¹H-NMR, CD₃OD, 400 MHz |
| | | | | 8,69 (d, 1H), 8,25 (d, 1H), 4,00 (bs, 2H), 3,60 (s, 3H), 2,44 (s, 3H), 1,55 (t, 3H) |
| 1-231 | CF₃ | S(O)₂Et | SO₂Et | ¹H-NMR, CD₃OD, 400 MHz |
| | | | | 8,63 (d, 1H), 8,25 (d, 1H), 3,99 (bs, 2H), 3,84 (q, 2H), 2,43 (s, 3H), 1,55 (t, 3H), 1,42 (t, 3H) |
| 1-232 | CF₃ | SCH₂CONMe₂ | SO₂Me | ¹H-NMR, DMSO-d₆, 400 MHz |
| | | | | 8,48 (d, 1H), 8,09 (d, 1H), 4,04 (s, 2H), 3,60 (s, 3H), 2,91 (s, 3H), 2,85 (s, 3H), 2,39 (s, 3H) |
| 1-233 | CF₃ | SCH₂CONMe₂ | SO₂Et | ¹H-NMR, DMSO-d₆, 400 MHz |
| | | | | 8,46 (d, 1H), 8,09 (d, 1H), 4,05 (s, 2H), 4,80 (q, 2H), 3,90 (s, 3H), 3,85 (s, 3H), 2,38 (s, 3H), 1,12 (t, 3H) |
| 1-234 | CF₃ | SCH₂COOH | SO₂Me | ¹H-NMR, CD₃OD, 400 MHz |
| | | | | 8,59 (d, 1H), 8,00 (d, 1H), 4,11 (s, 2H), 3,56 (s, 3H), 2,44 (s, 3H) |
| 1-235 | CF₃ | SCH₂COOH | SO₂Et | ¹H-NMR, CD₃OD, 400 MHz |
| | | | | 8,50 (d, 1H), 7,94 (d, 1H), 4,10 (s, 2H), 3,81 (q, 2H), 2,40 (s, 3H), 1,22 (t, 3H) |
| 1-236 | Me | SO₂-CH₂-CH₂-CH=CH₂ | CF₃ | ¹H-NMR, CDCl₃, 400 MHz |
| | | | | 9,13 (bs, 1H), 7,84 (d, 1H), 7,78 (d, 1H), |
| | | | | 5,79 (m, 1H), 5,11 (t, 2H), 3,30 (dd, 2H), 2,71 (s, 3H), 2,65 (m, 2H), 2,51 (s, 3H) |
| 1-237 | Cl | Me | SO₂Et | ¹H-NMR, DMSO-d₆, 400 MHz |
| | | | | 11,58 (s, 1H), 8,02 (d, 1H), 7,77 (d, 1H), 4,42 (q, 2H), 2,73 (s, 3H), 2,39 (s, 3H), 1,12 (t, 3H) |
| 1-238 | CF₃ | SEt | SO₂Me | ¹H-NMR, CDCl₃, 400 MHz |
| | | | | 8,50 (d, 1H), 7,74 (d, 1H), 3,51 (s, 3H), 3,09 (q, 2H), 2,48 (s, 3H), 1,32 (t, 3H) |
| 1-239 | OMe | N02 | Cl | ¹H-NMR, DMSO-d₆, 400 MHz |
| | | | | 11,48 (s, 1H), 7,91 (d, 1H), 7,69 (d, 1H), 4,91 (s, 3H), 2,48 (s, 3H) |
| 1-240 | OMe | NH(CO)i-Pr | Cl | ¹H-NMR, DMSO-d₆, 400 MHz |
| | | | | 11,10 (s, 1H), 9,54 (s, 1H), 7,59 (d, 1 H), 7,42 (d, 1H), 3,78 (s, 3H), 2,68 (m, 1 H), 2,38 (s, 3H), 1,13 (d, 6H) |
| 1-241 | OMe | NH(CO)CH2Ph | Cl | ¹H-NMR, DMSO-d₆, 400 MHz |
| | | | | 11,08 (s, 1 H), 9,91 (s, 1H), 7,59 (d, 1H), 7,46-7,29 (m, 5H), 3,67 (s, 3H), 2,35 (s, 3H) |
| 1-242 | CF₃ | SEt | SO₂Et | ¹H-NMR, CD₃OD, 400 MHz |
| | | | | 8,55 (d, 1H), 7,96 (d, 1H), 3,81 (q, 2H), 3,11 (q, 2H), 2,45 (s, 3H), 1,31 (t, 3H), 1,24 (t, 3H) |
| 1-243 | CF₃ | S(O)Et | SO₂Me | ¹H-NMR, DMSO-d₆, 400 MHz |
| | | | | 8,49 (bs, 1H), 8,22 (d, 1H), 3,54 (s, 3H), 3,25-3,40 (m, 2H), 2,38 (s, 3H), 1,40 (t, 3H) |
| 1-244 | Cl | Me | Cl | ¹H-NMR, DMSO-d₆, 400 MHz |
| | | | | 11,41 (s, 1H), 7,62 (d, 1H), 7,54 (d, 1H), 2,38 (s, 3H) |
| 1-245 | Me | 3,5-Dimethylpyrazol-1-yl | SO₂Me | ¹H-NMR, DMSO-d₆, 400 MHz |
| | | | | 11,54 (s, 1H), 8,09 (d, 1H), 7,98 (d, 1H), 3,17 (s, 3H), 2,39 (s, 3H), 2,21 (s, 3H), 1,98 (s, 3H), 1,82 (s, 3H) |
| 1-247 | Me | 1,2,3-Triazol-1-yl | SO₂Me | ¹H-NMR, DMSO-d₆, 400 MHz |
| | | | | 11,60 (s, 1H), 8,53 (s, 1H), 8,17 (d, 1H), 8,11 (d, 1H), 8,04 (s, 1H), 3,12 (s, 3H), 2,39 (s, 3H), 1,90 (s, 1 H) |
| 1-248 | Me | Me | SMe | ¹H-NMR, DMSO-d₆, 400 MHz |
| | | | | 11,08 (s, 1H), 7,41 (d, 1H), 7,18 (d, 1H), 2,36 (s, 3H), 2,31 (s, 3H), 2,25 (s, 3H) |
| 1-249 | Me | Pyrolidin-2-on-1-yl | SO₂Me | ¹H-NMR, DMSO-d₆, 400 MHz |
| | | | | 11,49 (s, 1H), 8,00 (d, 1H), 7,86 (d, 1H), 3,78-3,60 (m, 2H), 3,19 (s, 3H), 2,27 (s, 3H) |
| 1-250 | CF₃ | S(O)Et | SO₂Et | ¹H-NMR, CD₃OD, 400 MHz |
| | | | | 8,56 (bs, 1H), 8,13 (d, 1H), 3,60-3,70 (m, 4H), 2,45 (s, 3H), 1,53 (t, 3H), 1,32 (t, 3H) |
| 1-251 | Cl | Pyrazol-1-yl | SO₂Me | ¹H-NMR, DMSO-d₆, 400 MHz |
| | | | | 11,72 (s, 1H), 8,24 (d, 1H), 8,17 (d, 1H), 8,04 (d, 1H), 7,88 (d, 1H), 3,18 (s, 3H), 2,39 (s, 3H) |
| 1-252 | Me | 3-Methyl-pyrazol-1-yl | SO₂Me | ¹H-NMR, DMSO-d₆, 400 MHz |
| | | | | 11,51 (s, 1H), 8,08 (d, 1H), 7,98 (d, 1H), 7,81 (d, 1H), 6,38 (d, 1H), 3,06 (s, 3H), 2,40 (s, 3H) |
| 1-253 | Cl | CH₂-N(Et)OMe | SO₂Me | ¹H-NMR, DMSO-d₆, 400 MHz |
| | | | | 11,62 (s, 1H), 8,14 (d, 1H), 7,91 (d, 1H), 4,53 (s, 2H), 3,57 (s, 3H), 3,14 (s, 3H), 2,87 (q, 2H), 2,40 (s, 3H), 1,13 (t, 3H) |
| 1-254 | Me | Me | Cl | ¹H-NMR, DMSO-d₆, 400 MHz |
| | | | | 11,18 (s, 1H), 7,43 (d, 1H), 7,39 (d, 1H), 2,38 (s, 3H), 2,37 (s, 3H), 2,35 (s, 3H) |
| 1-255 | OH | Cl | Cl | ¹H-NMR, DMSO-d₆, 400 MHz |
| | | | | 7,68 (d, 1H), 7,39 (d, 1H), 2,36 (s, 3H) |
| 1-256 | Me | 1,2,4-Triazol-1-yl | SO₂Me | |
| 1-257 | Me | 4-Methoxy-pyrazol-1-yl | SO₂Me | ¹H-NMR, DMSO-d₆, 400 MHz |
| | | | | 11,54 (s, 1H), 8,08 (d, 1H), 7,99 (d, 1H), 7,74 (s, 1H), 7,66 (s, 1H), 3,75 (s, 3H), 3,11 (s, 3H), 2,39 (s, 3H) |
| 1-258 | Me | 1,2,4-triazol-1-yl | CF₃ | |
| 1-259 | Me | Tetrahydropyrimidin-2(1H)-one-1-yl | SO₂Me | ¹H-NMR, DMSO-d₆, 400 MHz |
| | | | | 11,51 (s, 1H), 7,95 (d, 1H), 7,77 (d, 1H), 3,19 (s, 3H), 2,90 (s, 3H), 2,40 (s, 3H), 2,26 (s, 3H) |
| 1-260 | Me | NH-(CH₂)₂-O(CO)Et | SO₂Me | ¹H-NMR, DMSO-d₆, 400 MHz |
| | | | | 11,36 (s, 1H), 7,76 (d, 1H), 7,30 (d, 1H), 5,60 (t, 1H), 4,25 (t, 2H), 3,41 (m, 2H), 2,40 (s, 3H), 1,05 (t, 3H) |
| 1-261 | Me | NH-iPr | SO₂Me | ¹H-NMR, DMSO-d₆, 400 MHz |
| | | | | 11,36 (s, 1H), 7,78 (d, 1H), 7,20 (d, 1H), 5,58 (d, 1H), 3,69 (m, 1H), 3,28 (s, 3H), 2,40 (s, 3H), 1,14 (d, 6H) |
| 1-262 | Cl | NH-CH₂-(CO)NHEt | Cl | ¹H-NMR, DMSO-d₆, 400 MHz |
| | | | | 11,38 (s, 1H), 8,08 (t, 1H), 7,47 (d, 1H), 7,08 (d, 1H), 5,69 (t, 1H), 3,97 (d, 2H), 3,15 (m, 2H), 2,38 (s, 3H), 1,04 (t, 3H) |
| 1-263 | Me | NH-CH2-(CO)NMe2 | SO₂Me | ¹H-NMR, DMSO-d₆, 400 MHz |
| | | | | 11,32 (s, 1H), 7,70 (d, 1H), 7,20 (d, 1H), 6,41 (t, 1H), 4,10 (d, 2H), 3,25 (s, 3H), |
| 1-264 | Me | NH-CH₂-furan-2-yl | SO₂Me | ¹H-NMR, DMSO-d₆, 400 MHz |
| | | | | 13,39 (bs, !H), 11,30 (s, 1H), 7,66 (d, 1H), 7,32 (d, 1H), 5,60 (broad, 1H), 4,07-3,98 (m, 1H), 3,84-3,77 (m, 1H), 3,72-3,64 (m, 1 H), 2,38 (s, 3H), 2,03-1,92 (m,1H), 1,90-1,79 (m, 2H), 1,63-1,54 (m, 1H). |
| 1-265 | Me | NH-CH₂-(CO)NHEt | SO₂Me | ¹H-NMR, DMSO-d₆, 400 MHz |
| | | | | 11,32 (s, 1H), 8,12 (broad, 1H), 7,72 (d, 1 H), 7,22 (d, 1H), 6,18 (t, 1H), 3,87 (d, 2H),2,39 (s, 3H), 1,02 (t, 3H), |
| 1-266 | Me | F | SO₂Me | ¹H-NMR, DMSO-d₆, 400 MHz |
| | | | | 11,49 (s, 1H), 7,83 (t, 1H), 7,67 (d, 1H), 3,38 (s, 3H) |
| 1-267 | F | SO₂Me | SO₂Me | |
| 1-268 | Cl | (4-Cyclopropyl-3-methyl-5-oxo-4, 5-dihydro-1H-1,2,4-triazol-1-yl)-methyl | Cl | ¹H-NMR, DMSO-d₆, 400 MHz |
| | | | | 11,50 (s, 1H), 7,69 (d, 1H), 7,65 (d, 1H), 5,06 (s, 2H), 2,79 (m, 1H), 2,38 (s, 3H), 2,14 (s, 3H) |
| 1-269 | Cl | [4-Methyl-5-oxo-3-(2,2,2-trifluorethoxy)-4,5-dihydro-1H-1,2,4-triazol-1-yl]-methyl | Cl | ¹H-NMR, DMSO-d₆, 400 MHz |
| | | | | 11,50 (s, 1H), 7,70 (d, 1H), 7,68 (d, 1H), 5,12 (s, 2H), 4,85 (q, 2H), 3,07 (s, 3H), 2,38 (s, 3H) |
| 1-270 | Cl | (3-Isopropoxy- 4-methyl-5-oxo-4, 5-dihydro-1H-1,2,4-triazol-1-yl]-methyl | Cl | ¹H-NMR, DMSO-d₆, 400 MHz |
| | | | | 11,50 (s, 1H), 7,69 (d, 1H), 7,65 (d, 1H), 5,08 (s, 2H), 4,72 (m, 1H), 2,98 (m, 1H), 2,38 (s, 3H), 1,27 (d, 6H) |
| 1-271 | Cl | (4-Methyl-5-oxo-4,5-dihydro-1H-1,2,4-triazol-1-yl)-methyl | Cl | ¹H-NMR, DMSO-d₆, 400 MHz |
| | | | | 11,51 (s, 1H), 7,86 (s, 1H), 7,70 (d, 1H), 7,66 (d, 1H), 5,14 (s, 2H), 3,18 (m, 1H), 2,38 (s, 3H). |
| 1-272 | Me | Cl | SO₂Et | |
| 1-273 | SO₂Me | F | Cl | ¹H-NMR, DMSO-d₆, 400 MHz |
| | | | | 11,50 (s, 1H), 8,14 (t, 1H), 7,63 (d, 1H), 3,45 (s, 3H), 2,41 (s, 3H) |
| 1-274 | Me | 1,2,3-Triazol-1-yl | SO₂Me | |
| 1-275 | Me | Isobutyl(methyl)carb -amoylamino | SO₂Me | ¹H-NMR, DMSO-d₆, 400 MHz |
| | | | | 11,49 (s, 1H), 8,01 (s, 1H), 7,89 (d, 1H), 7,69 (d, 1H), 3,16 (bs, 5H), 2,98 (s, 3H), 2,39 (s, 3H), 2,21 (s, 3H), 1,95 (m, 1H), 0,87 (d, 6H) |
| 1-276 | Me | 3-Oxo-morpholin-4-yl | SO₂Me | ¹H-NMR, DMSO-d₆, 400 MHz |
| | | | | 11,54 (s, 1H), 8,03 (d, 1H), 7,88 (d, 1H), 4,32 (q, 2H), 4,05-3,89 (m, 2H), 3,70-3,66 (m, 1H), 3,60-3,57 (m, 1H), 3,33 (s, 3H), 2,49 (s, 3H), 2,30 (s, 3H) |
| 1-277 | OMe | [Ethyl(methylsulfony l)amino]methyl | Cl | ¹H-NMR, DMSO-d₆, 400 MHz |
| | | | | 11,21 (s, 1H), 7,66 (d, 1H), 7,43 (d, 1H), 4,52 (s, 2H), 3,85 (s, 3H), 3,10 (q, 2H), 3,01 (s, 3H), 2,39 (s, 3H), 0,98 (t, 3H) |
| 1-278 | F | SO₂Me | CF₃ | |
| 1-279 | OMe | Benzoylamino | Cl | ¹H-NMR, DMSO-d₆, 400 MHz |
| | | | | 11,15 (s, 1H), 10,16 (s, 1H), 8,03 (d, 2H), 7,68-7,49 (m, 5H), 3,82 (s, 3H), 2,38 (s, 3H) |
| 1-280 | OMe | Cyclopropylcarbonyl -amino | Cl | ¹H-NMR, DMSO-d₆, 400 MHz |
| | | | | 11,10 (s, 1H), 10,88 (s, 1H), 7,59 (d, 1H), 7,41 (d, 1H), 3,78 (s, 3H), 2,38 (s, 3H), 1,89 (broad, 1H), 0,79 (m, 4H) |
| 1-281 | OMe | Propionyllamino | Cl | ¹H-NMR, DMSO-d₆, 400 MHz |
| | | | | 11,08 (s, 1H), 9,59 (s, 1H), 7,59 (d, 1H), 7,43 (d, 1H), 3,78 (s, 3H), 2,38 (m, 4H), 1,13 (m, 4H) |
| 1-282 | NO₂ | SO₂Me | SO₂Me | ¹H-NMR, CDCl₃, 400 MHz |
| | | | | 8.75 (s, 1H), 8.6 (d, 1H), 8.15 (d, 1H), 3.68 (s, 3H), 3.58 (s, 3H), 2.45 (s, 3H) |
| 1-283 | NO₂ | SO₂Me | Cl | ¹H-NMR, DMSO-d₆, 400 MHz |
| | | | | 11.9 (s, 1H), 8.32 (d, 1H), 8.22 (d, 1H), 3.58 (s, 3H), 2.35 (s, 3H) |
| 1-284 | NO₂ | SOMe | SO₂Me | ¹H-NMR, MeOD, 400 MHz |
| | | | | 8.5 (d, 1H), 8.22 (d, 1H), 3.55 (s, 3H), 2.6 (s, 3H), 2.38 (s, 3H) |
| 1-285 | NO₂ | SOMe | Br | ¹H-NMR, CDCl_{3.}, 400 MHz |
| | | | | 7.88 (d, 1H), 7.72 (d, 1H), 3.22 (s, 3H), 2.43 (s, 3H) |
| 1-286 | NO₂ | SOMe | Cl | ¹H-NMR, DMSO-d₆, 400 MHz |
| | | | | 11.89 (s, 1H), 8.15 (d, 1H), 8.05 (d, 1H), 3.19 (s, 3H), 2.35 (s, 3H) |
| 1-287 | NO₂ | SMe | SO₂Me | ¹H-NMR, DMSO-d₆, 400 MHz |
| | | | | 11.9 (s, 1H), 8.45 (d, 1H), 8.38 (d, 1H), 3.61 (s, 3H), 2.52 (s, 3H), 2.32 (s, 3H) |
| 1-288 | NO₂ | SMe | Br | ¹H-NMR, CDCl_{3,}, 400 MHz |
| | | | | 8.18 (s, 1H), 7.96 (d, 1H), 7.65 (d, 1H), 2.48 (s, 3H), 2.4 (s, 3H) |
| 1-289 | NO₂ | SMe | Cl | ¹H-NMR, CDCl_{3,}, 400 MHz |
| | | | | 8.3 (s, 1H), 7.76 (d, 1H), 7.72 (d, 1H), 2.49 (s, 3H), 2.42 (s, 3H) |
| 1-290 | Cl | CH₂OCH(CH₃)₂ | SO₂Et | ¹H-NMR, CDCl_{3,}, 400 MHz |
| | | | | 8.55 (s, 1H), 7.98 (d, 1H), 7.71 (d, 1H), 5.03 (s, 2H), 3.82 (m, 1H), 3.42 (q, 2H), 2.52 (s, 3H), 1.25 (d + t, 9H) |
| 1-291 | Cl | CH₂OEt | SO₂Et | ¹H-NMR, CDCl_{3,}, 400 MHz |
| | | | | 8.58 (s, 1H), 7.96 (d, 1H), 7.71 (d, 1H), 5.05 (s, 2H), 3.69 (m, 2H), 3.4 (q, 2H), 2.52 (s, 3H), 1.25 (t, 6H) |
| 1-292 | Cl | CH₂OMe | SO₂Et | ¹H-NMR, CDCl_{3,}, 400 MHz |
| | | | | 8.55 (s, 1H), 7.98 (d, 1H), 7.72 (d, 1H), 5.02 (s, 2H), 3.5 (s, 3H), 3.36 (q, 2H), 2.52 (s, 3H), 1.25 (t, 3H) |
| 1-293 | Cl | CH₂OCH₂C₂F₅ | SO₂Me | ¹H-NMR, CDCl_{3,}, 400 MHz |
| | | | | 8.38 (s, 1H), 8.09 (d, 1H), 7.8 (d, 1H), 5.32 (s, 2H), 4.12 (t, 2H), 3.2 (s, 3H), 2.52 (s, 3H) |
| 1-294 | Cl | CH₂OCH₂CHF₂ | SO₂Me | ¹H-NMR, CDCl_{3,}, 400 MHz |
| | | | | 8.15 (d, 1H), 8.14 (s, 1H), 7.8 (d, 1H), 5.94 (tt, 1H), 5.3 (s, 2H), 3.9 (dt, 2H), 3.25 (s, 3H), 2.52 (s, 3H) |
| 1-295 | Cl | CH₂OCH₂CCH | SO₂Et | ¹H-NMR, CDCl_{3,}, 400 MHz |
| | | | | 8.42 (s, 1H), 8.02 (d, 1H), 7.75 (d, 1H), 5.19 (s, 2H), 4.32 (d, 2H), 3.4 (q, 2H), 2.55 (t, 1 H), 2.52 (s, 3H), 1.28 (t, 3H) |
| 1-296 | Cl | CH₂OC₂H₄OMe | SO₂Me | ¹H-NMR, CDCl_{3,}, 400 MHz |
| | | | | 8.42 (s, 1H), 8.08 (d, 1H), 7.73 (d, 1H), 5.18 (s, 2H), 3.8 (dd, 2H), 3.58 (dd, 2H), 3.32 (s, 3H), 3.3 (s, 3H), 2.52 (s, 3H) |
| 1-297 | Cl | CH₂(OC₂H₄)₂OMe | SO₂Me | ¹H-NMR, CDCl_{3,}, 400 MHz |
| | | | | 8.78 (s, 1H), 8.09 (d, 1H), 7.72 (d, 1H), 5.18 (s, 2H), 3.82 (dd, 2H), 3.66 (dd, 2H), 3.58 (dd, 2H), 3.48 (dd, 2H), 3.31 (s, 3H), 3.29 (s, 3H), 2.51 (s, 3H) |
| 1-298 | Cl | 5-Ethoxymethyl-4,5-dihydro-1,2-oxazol-3 yl | SO₂Et | ¹H-NMR, CDCl₃, 400 MHz |
| | | | | 8.43 (s, 1H), 8.09 (d, 1H), 7.9 (d, 1H), 5.05 (m, 1H), 3.72 (dd, 1H), 3.62 (dd, 1H), 3.6 (q, 2H), 3.46 (dd, 1H), 3.38 (q, 2H), 3.26 (dd, 1H), 2.5 (s, 3H), 1.28 (t, 3H), 1.22 (t, 3H) |
| 1-299 | Cl | 5-Methoxymethyl-4,5-dihydro-1,2-oxazol-3 yl | SO₂Et | ¹H-NMR, CDCl₃, 400 MHz |
| | | | | 8.42 (s, 1H), 8.09 (d, 1H), 7.9 (d, 1H), 5.05 (m, 1H), 3.69 (dd, 1H), 3.58 (dd, 1H), 3.45 (dd, 1H), 3.45 (s, 3H), 3.38 (q, 2H), 3.22 (dd, 1H), 2.5)s, 3H), 1.28 (t, 3H) |
| 1-300 | Et | SOMe | CF₃ | ¹H-NMR, CDCl₃, 400 MHz |
| | | | | 9.52 (s, 1H), 7.68 (s, 2H), 3.43 (m, 1H), 3.35 (m, 1H), 3.0 (s, 3H), 2.5 (s, 3H), 1.2 (t, 3H) |
| 1-301 | iPr | SMe | CF₃ | ¹H-NMR, CDCl₃, 400 MHz |
| | | | | 7.88 (s, 1H), 7.7 (d, 1H), 7.51 (d, 1H), 4.32 (m, 1H), 2.52 (s, 3H), 2.32 (s, 3H), 1.45 (d, 6H) |
| 1-302 | Et | SMe | CF₃ | ¹H-NMR, CDCl₃, 400 MHz |
| | | | | 7.82 (s, 1H), 7.71 (d, 1H), 7.59 (d, 1H), 3.22 (q, 2H), 2.51 (s, 3H), 2.35 (s, 3H), 1.3 (t, 3H) |
| 1-303 | Et | SO₂Me | CF₃ | ¹H-NMR, CDCl₃, 400 MHz |
| | | | | 8.38 (s, 1H), 7.88 (d, 1H), 7.8 (d, 1H), 3.32 (q, 2H), 3.28 (s, 3H), 2.5 (s, 3H), 1.32 (t, 3H) |
| 1-304 | cPr | SOMe | CF₃ | ¹H-NMR, CDCl₃, 400 MHz |
| | | | | 9.92 (s, 1H), 7.7 (m, 2H), 3.15 (s, 3H), 2.82 (m, 1H), 2.52 (s, 3H), 1.2 (m, 1H), 1.1 (m, 1H), 0.98 (m, 1H), 0.7 (m, 1H) |
| 1-305 | CH=CH₂ | SMe | CF₃ | ¹H-NMR, CDCl₃, 400 MHz |
| | | | | 7.78 (m, 1H), 7.68 (d, 1H), 7.68 (s, 1H), 7.4 (dd, 1H), 5.7 (d, 1H), 5.6 (d, 1H), 2.48 (s, 3H), 2.29 (s, 3H) |
| 1-306 | Et | SMe | Cl | ¹H-NMR, CDCl₃, 400 MHz |
| | | | | 7.82 (s, 1H), 7.45 (d, 1H), 7.4 (d, 1H), 3.18 (q, 2H), 2.5 (s, 3H), 2.42 (s, 3H), 1.25 (t, 3H) |
| 1-307 | Et | SO₂Me | Cl | ¹H-NMR, CDCl₃, 400 MHz |
| | | | | 8.53 (s, 1H), 7.6 (d, 1H), 7.5 (d, 1H), 3.3 (s, 3H), 3.3 (q, 2H), 2.5 (s, 3H), 1.3 (t, 3H) |
| 1-308 | Cl | NMe₂ | Cl | ¹H-NMR, CDCl₃, 400 MHz |
| | | | | 8,17 (bs, 1H), 7,45 (d, 1H), 7,40 (d, 1H), 2,92 (s, 6H), 2,49 (s, 3H) |
| 1-309 | CH₂O(CH₂)₂ OMe | NH(CH₂)₃OMe | SO₂Me | ¹H-NMR, CDCl₃, 400 MHz |
| | | | | 7,94 (d, 1H), 7,40 (d, 1H), 4,63 (s, 2H), 3,76 (m, 2H), 3,58 - 3,45 (m, 6H), 3,37 (s, 3H), 3,18 (s, 3H), 3,10 (s, 3H), 2,46 (s, 3H), 1,95 (quin, 2H) |
| 1-313 | Me | SO₂(CH₂)₂OMe | CF₃ | ¹H-NMR, CDCl₃, 400 MHz |
| | | | | 7,85 (d, 1H), 7,77 (d, 1H), 3,90 (t, 2H), 3,52 (t, 2H), 3,27 (s, 3H), 2,78 (s, 3H), 2,51 (s, 3H) |
| 1-314 | Me | SOEt | SO₂Me | ¹H-NMR, CDCl₃, 400 MHz |
| | | | | 8,07 (d, 1H), 7,73 (d, 1H), 3,47 (m, 1H), 3,33 (s, 3H), 3,22 (m, 1H), 2,87 (s, 3H), 2,51 (s, 3H), 1,52 (t, 3H) |
| 1-315 | Me | SO₂Et | SO₂Me | ¹H-NMR, CDCl₃, 400 MHz |
| | | | | 8,36 (d, 1H), 7,83 (d, 1H), 3,66 (q, 2H), 3,56 (s, 3H), 2,85 (s, 3H), 2,52 (s, 3H), 1,53 (t, 3H) |
| 1-316 | Me | SMe | 1,2,4-triazol-1-yl | ¹H-NMR, CDCl₃, 400 MHz |
| | | | | 8,58 (s, 1H), 8,13 (s, 1H), 7,62 (d, 1H), 7,48 (d, 1H), 2,78 (s, 3H), 2,52 (s, 3H), 2,03 (s, 3H) |
| 1-317 | OEt | SMe | CF₃ | ¹H-NMR, CDCl₃, 400 MHz |
| | | | | 8,21 (d, 1H), 7,65 (d, 1H), 4,38 (q, 2H), 2,48 (s + s, 6H), 1,59 (t, 3H) |
| 1-318 | Me | S(CH₂)₂OMe | CF₃ | ¹H-NMR, CDCl₃, 400 MHz |
| | | | | 7,71 (d, 1H), 7,57 (d, 1H), 3,55 (t, 2H), 3,32 (s, 3H), 2,92 (t, 2H), 2,79 (s, 3H), 2,51 (s, 3H) |
| 1-319 | Me | SOMe | 1,2,4-triazol-1-yl | ¹H-NMR, CDCl₃, 400 MHz |
| | | | | 8,41 (s, 1H), 8,10 (s, 1H), 7,71 (d, 1 H), 7,35 (d, 1H), 3,13 (s, 3H), 2,87 (s, 3H), 2,51 (s, 3H) |
| 1-320 | OEt | SOMe | CF₃ | ¹H-NMR, CDCl₃, 400 MHz |
| | | | | 8,32 (d, 1H), 7,72 (d, 1H), 4,64 (m, 1 H), 4,13 (m, 1H), 3,13 (s, 3H), 2,50 (s, 3H), 1,61 (t, 3H) |
| 1-321 | Me | SO(CH₂)₂OMe | CF₃ | ¹H-NMR, CDCl₃, 400 MHz |
| | | | | 7,68 (d, 1H), 7,63 (d, 1H), 3,87 (dt, 1H), 3,73 (m, 1H), 3,49 (dt, 1H), 3,35 (s, 3H), 3,03 (m, 1H), 2,77 (s, 3H), 2,49 (s, 3H) |
| 1-322 | Me | SCH₂CCMe | SO₂Me | ¹H-NMR, CDCl₃, 400 MHz |
| | | | | 8.06 (d, 1H), 7,66 (d, 1H), 3,61 (m, 2H), 3,44 (s, 3H), 2,81 (s, 3H), 2,52 (s, 3H), 1,73 (m, 3H) |
| 1-323 | Me | S-c-Pen | SO₂Me | ¹H-NMR, CDCl₃, 400 MHz |
| | | | | 8.11 (d, 1H), 7,63 (d, 1H), 3,63 (m, 1 H), 3,42 (s, 3H), 2,77 (s, 3H), 2,51 (s, 3H), 1,98 - 1,90 (m, 2H), 1,85 - 1,73 (m, 2H), 1,63 - 1,55 (m, 4H) |
| 1-324 | OMe | SMe | OMe | ¹H-NMR, CDCl₃, 400 MHz |
| | | | | 8.17 (d, 1H), 6,87 (d, 1H), 4,07 (s, 3H), 3,98 (s, 3H), 2,47 (s, 3H), 2,43 (s, 3H) |
| 1-325 | Me | SCH₂CH=CHCH₃ | SO₂Me | ¹H-NMR, CDCl₃, 400 MHz |
| | | | | 7,94 (d, 1H), 7,61 (d, 1H), 5,68 - 5,45 (m, 2H), 3,58 - 3,42 (m, 5H), 2,73 (s, 3H), 2,51 (s, 3H), 1,63 (d, 3H) |
| 1-326 | Me | SOCH₂CCMe | SO₂Me | ¹H-NMR, CDCl₃, 400 MHz |
| | | | | 8.11 (m, 2H), 7,76 (d, 1H), 4,28 - 4,03 (m, 2H), 3,34 (s, 3H), 2,91 (s, 3H), 2,51 (s, 3H), 1,86 (t, 3H) |
| 1-327 | Me | SO₂-c-Pen | SO₂Me | ¹H-NMR, CDCl₃, 400 MHz |
| | | | | 8.40 (d, 1H), 7,83 (d, 1H), 4,45 (quin, 1H), 3,51 (s, 3H), 2,91 (s, 3H), 2,52 (s, 3H), 2,18 (m, 2H), 2,01 (m, 2H), 1,88 (m, 2H), 1,71 (m, 2H) |
| 1-328 | Me | SO-c-Pen | SO₂Me | ¹H-NMR, CDCl₃, 400 MHz |
| | | | | 8.11 (d, 1 H), 7,74 (d, 1 H), 3,98 (m, 1 H), 3,33 (s, 3H), 2,88 (s, 3H), 2,51 (s, 3H), 2,28 (m, 1 H), 2,05 (m, 1 H), 1,91 - 1,51 (m, 6H) |
| 1-329 | Me | S(CH₂)₃Cl | SO₂Me | ¹H-NMR, CDCl₃, 400 MHz |
| | | | | 8.06 (d, 1H), 7,66 (d, 1H), 3,66 (t, 2H), 3,42 (s, 3H), 3,02 (t, 2H), 2,79 (s, 3H), 2,52 (s, 3H), 2,12 (quin, 2H) |
| 1-330 | Me | SCH₂(4-F-Ph) | SO₂Me | ¹H-NMR, CDCl₃, 400 MHz |
| | | | | 8.03 (d, 1H), 7,66 (d, 1H), 7,16 (m, 2H), 6,97 (m, 2H), 4,01 (s, 2H), 3,33 (s, 3H), 2,62 (s, 3H), 2,51 (s, 3H) |
| 1-331 | Me | SO₂CH₂CCMe | SO₂Me | ¹H-NMR, CDCl₃, 400 MHz |
| | | | | 8.36 (d, 1H), 7,87 (d, 1H), 4,60 (m, 2H), 3,51 (s, 3H), 2,98 (s, 3H), 2,52 (s, 3H), 1,83 (t, 3H) |
| 1-332 | Me | SO₂CH₂CH=CHCH₃ | SO₂Me | ¹H-NMR, CDCl₃, 400 MHz |
| | | | | 8,35 (d, 1H), 7,83 (d, 1H), 5,93 (m, 1H), 5,61 (m, 1H), 4,34 (d, 2H), 3,54 (s, 3H), 2,83 (s, 3H), 2,51 (s, 3H), 1,77 (d, 3H) |
| 1-333 | Me | SOCH₂CH=CHCH₃ | SO₂Me | ¹H-NMR, CDCl₃, 400 MHz |
| | | | | 8,02 (m, 1H), 7,73 (m, 1H), 6.07 - 5,87 (m, 1H), 5,75 - 5,60 (m, 1H), 4,10 - 3.83 (m, 2H), 3,32 (s, 3H), 2,86 (s, 3H), 2,51 (s, 3H), 1,78 (d, 3H) |
| 1-334 | Me | SOCH₂-Epoxy-Me | SO₂Me | ¹H-NMR, CDCl₃, 400 MHz |
| | | | | 8,08 (m, 1H), 7,76 (d, 1H), 3,88 - 2,84 (m, 10H), 2,51 (s, 3H), 1,37 (m, 3H) |
| 1-335 | Me | SO₂(CH₂)₃Cl | SO₂Me | ¹H-NMR, CDCl₃, 400 MHz |
| | | | | 8.28 (d, 1H), 7,86 (d, 1H), 3,81 - 3,73 (m, 4H), 3,55 (s, 3H), 2,85 (s, 3H), 2,52 (m, 5H) |
| 1-336 | Me | SO(CH₂)₃Cl | SO₂Me | ¹H-NMR, CDCl₃, 400 MHz |
| | | | | 8.05 (d, 1H), 7,76 (d, 1H), 3,81 - 3,61 (m, 3H), 3,40 - 3,27 (m, 4H), 2,89 (s, 3H), 2,52 (s, 3H), 2,43 (m, 2H) |
| 1-337 | Me | SOCH₂(4-F-Ph) | SO₂Me | ¹H-NMR, DMSO-d₆, 400 MHz |
| | | | | 8.05 (d, 1H), 7,95 (d, 1H), 7,46 (dd, 2H), 7,27 (t, 2H), 4,70 (d, 1H), 4,41 (d, 1H), 3,47 (s, 3H), 2,81 (s, 3H), 2,41 (s, 3H) |
| 1-338 | Me | SO₂CH₂(4-F-Ph | SO₂Me | ¹H-NMR, CDCl₃, 400 MHz |
| | | | | 8.42 (d, 1H), 7,85 (m, 1H), 7,69 (m, 1H), 7,40 (t, 2H), 7,07 (t, 2H), 4,93 (s, 2H), 3,59 (s, 3H), 2,49 (s, 3H), 2,43 (s, 3H) |
| 1-339 | Me | SO₂Me | C₂F₅ | ¹H-NMR, CDCl₃, 400 MHz 7,91 - 7,75 (m, 3H), 3,30 (s, 3H), 2,88 (s, 3H), 2,52 (s, 3H) |
| 1-340 | O(CH₂)₂OMe | SMe | CF₃ | ¹H-NMR, CDCl₃, 400 MHz |
| | | | | 8,32 (d, 1H), 7,66 (d, 1H), 4,55 (m, 2H), 3,78 (m, 2H), 3,34 (s, 3H), 2,48 (s, 3H), 2,44 (s, 3H) |
| 1-341 | O(CH₂)₂OMe | SO₂Me | CF₃ | ¹H-NMR, CDCl₃, 400 MHz |
| | | | | 8,56 (d, 1H), 7,91 (d, 1H), 4,47 (m, 2H), 3,79 (m, 2H), 3,43 (s, 3H), 3,31 (s, 3H), 2,45 (s, 3H) |
| 1-342 | O(CH₂)₂OMe | SOMe | CF₃ | ¹H-NMR, CDCl₃, 400 MHz |
| | | | | 8,49 (d, 1H), 7,73 (d, 1H), 4,91 (dt, 1H), 4,21 (dt, 1H), 3,97 (dt, 1H), 3,60 (dt, 1H), 3,29 (s, 3H), 3,15 (s, 3H), 2,45 (s, 3H) |
| 1-343 | Me | S(CH₂)₂OCH₂CF₃ | SO₂Me | ¹H-NMR, CDCl₃, 400 MHz |
| | | | | 7,91 (d, 1H), 7,63 (d, 1H), 3,89 - 3,80 (m, 4H), 3,43 (s, 3H), 3,08 (m, 2H), 2,76 (s, 3H), 2,52 (s, 3H) |
| 1-344 | Me | SO(CH₂)₂OCH₂CF₃ | SO₂Me | ¹H-NMR, CDCl₃, 400 MHz |
| | | | | 8,06 (d, 1H), 7,75 (d, 1H), 4,23 (m, 1H), 4,13 (m, 1H), 3,93 (q, 2H), 3,68 (m, 1H), 3,50 (m, 1H), 3,36 (s, 3H), 2,84 (s, 3H), 2,52 (s, 3H) |
| 1-345 | Me | SO₂(CH₂)₂OCH₂CF₃ | SO₂Me | ¹H-NMR, CDCl₃, 400 MHz |
| | | | | 8,37 (d, 1H), 7,91 - 7,83 (m, 2H), 4,24 (t, 2H), 3,96 (t, 2H), 3,85 (q, 2H), 3,55 (s, 3H), 2,87 (s, 3H), 2,52 (s, 3H) |
| 1-346 | OEt | SEt | CF₃ | ¹H-NMR, CDCl₃, 400 MHz |
| | | | | 8,22 (d, 1H), 7,66 (d, 1H), 4,37 (q, 2H), 3,03 (q, 2H), 2,50 (s, 3H), 1,59 (t, 3H), 1,22 (t, 3H) |
| 1-347 | O-CH₂-c-Pr | SMe | CF₃ | ¹H-NMR, CDCl₃, 400 MHz |
| | | | | 8,22 (d, 1H), 7,65 (d, 1H), 4,19 (d, 2H), 2,51 (s, 3H), 2,49 (s, 3H), 1,38 (m, 1 H), 0,71 (m, 2H), 0,40 (m, 2H) |
| 1-348 | OMe | SEt | CF₃ | ¹H-NMR, CDCl₃, 400 MHz |
| | | | | 8,23 (d, 1H), 7,68 (d, 1H), 4,15 (s, 3H), 3,01 (q, 2H), 2,50 (s, 3H), 1,22 (t, 3H) |
| 1-349 | OMe | SO₂Et | CF₃ | ¹H-NMR, CDCl₃, 400 MHz |
| | | | | 8,37 (d, 1H), 7,92 (d, 1H), 4,16 (s, 3H), 3,55 (q, 2H), 2,52 (s, 3H), 1,37 (t, 3H) |
| 1-350 | OMe | SOEt | CF₃ | ¹H-NMR, CDCl₃, 400 MHz |
| | | | | 8,33 (d, 1H), 7,74 (d, 1H), 4,19 (s, 3H), 3,64 (m, 1H), 3,23 (m, 1H), 2,51 (s, 3H), 1,34 (t, 3H) |
| 1-351 | OEt | SO₂Et | CF₃ | ¹H-NMR, CDCl₃, 400 MHz 8,37 (d, 1H), 7,91 (d, 1H), 4,34 (q, 2H), 3,58 (q, 2H), 2,51 (s, 3H), 1,61 (t, 3H), 1,36 (t, 3H) |
| 1-352 | OEt | SOEt | CF₃ | ¹H-NMR, CDCl₃, 400 MHz |
| | | | | 8,33 (d, 1H), 7,74 (d, 1H), 4,63 (quin, 1H), 4,13 (quin, 1 H), 3,65 (m, 1 H), 3,25 (m, 1 H), 2,51 (s, 3H), 1,59 (t, 3H), 1,33 (t, 3H) |
| 1-353 | O-CH2-c-Pr | SOMe | CF₃ | ¹H-NMR, CDCl₃, 400 MHz |
| | | | | 8,32 (d, 1H), 7,73 (d, 1H), 4,50 (dd, 1H), 3,97 (dd, 1H), 3,19 (s, 3H), 2,50 (s, 3H), 1,47 (m, 1H), 0,66 (m, 2H), 0,56 (m, 1H), 0,37 (m, 1H) |
| 1-354 | O-CH2-c-Pr | SO₂Me | CF₃ | ¹H-NMR, CDCl₃, 400 MHz |
| | | | | 8,39 (d, 1H), 7,88 (d, 1H), 4,16 (d, 2H), 3,48 (s, 3H), 2,50 (s, 3H), 1,48 (m, 1H), 0,72 (m, 2H), 0,51 (m, 2H) |
| 1-355 | Me | SEt | SO₂Me | ¹H-NMR, CDCl₃, 400 MHz |
| | | | | 7,96 (d, 1H), 7,63 (d, 1H), 3,43 (s, 3H), 2,86 (q, 2H), 2,76 (s, 3H), 2,52 (s, 3H), 1,29 (t, 3H) |

**Tabelle 2: Erfindungsgemäße Verbindungen der allgemeinen Formel (I), worin R für Ethyl steht**

| | | | | |
|---|---|---|---|---|
| 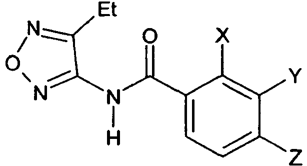 | | | | |

| **Nr.** | **X** | **Y** | **Z** | **Physikalische Daten** |
|---|---|---|---|---|
| 2-1 | CF₃ | OCH₂CON(Me)Et | SO₂Me | |
| 2-2 | CF₃ | OCH₂CON(Me)Et | SO₂Et | |
| 2-4 | CF₃ | 2-(1H-Pyrazol-1-yl)ethoxyl | SO₂Me | |
| 2-5 | CF₃ | 2-(1H-Pyrazol-1-yl)ethoxyl | SO₂Et | |
| 2-6 | CF₃ | Tetrahydrofuran-2-yl-methoxy | SO₂Me | |
| 2-7 | CF₃ | Tetrahydrofuran-2-yl-methoxy | SO₂Et | |
| 2-8 | CF₃ | OH | SO₂Me | |
| 2-9 | CF₃ | OH | SO₂Et | |
| 2-10 | CF₃ | SH | SO₂Me | |
| 2-11 | CF₃ | SH | SO₂Et | |
| 2-15 | CF₃ | SMe | SO₂Me | |
| 2-16 | CF₃ | SMe | SO₂Et | |
| 2-17 | CF₃ | S(O)Me | SO₂Me | |
| 2-24 | CF₃ | S(O)Me | SO₂Et | |
| 2-25 | CF₃ | S(O)₂Me | SO₂Me | |
| 2-26 | CF₃ | S(O)₂Me | SO₂Et | |
| 2-27 | CF₃ | 2-[(Methylsulfonyl) amino]ethoxy | SO₂Me | |
| 2-28 | CF₃ | 2-[(Methylsulfonyl) amino]ethyl}sulfanyl | SO₂Me | |
| 2-29 | CF₃ | 2-[(Methylsulfonyl) amino]ethyl}sulfanyl | SO₂Et | |
| 2-30 | NO₂ | O(CH₂)₂OMe | OMe | |
| 2-31 | NO₂ | OMe | Me | |
| 2-32 | NO₂ | NH₂ | OMe | |
| 2-33 | NO₂ | NH₂ | SO₂Et | |
| 2-34 | NO₂ | NH₂ | Cl | |
| 2-35 | NO₂ | NHMe | Cl | |
| 2-36 | NO₂ | NMe₂ | Cl | |
| 2-37 | NO₂ | NH₂ | Br | |
| 2-38 | NO₂ | NHMe | Br | |
| 2-39 | NO₂ | NMe₂ | Br | |
| 2-40 | NO₂ | NH₂ | F | |
| 2-41 | NO₂ | NHMe | F | |
| 2-42 | NO₂ | NMe₂ | F | |
| 2-43 | NO₂ | NH₂ | SO₂Me | |
| 2-44 | NO₂ | NHMe | SO₂Me | |
| 2-45 | NO₂ | NMe₂ | SO₂Me | |
| 2-46 | NO₂ | NH₂ | 1 H-1,2,4-triazol-1-yl | |
| 2-47 | NO₂ | NHMe | 1 H-1,2,4-triazol-1-yl | |
| 2-48 | NO₂ | NMe₂ | 1 H-1,2,4-triazol-1-yl | |
| 2-49 | Me | F | F | ¹H-NMR, DMSO-d₆, 400 MHz |
| | | | | 11,26 (s, 1H), 7,49 (m, 2H), 2,8 (q, 2H), 2,38 (s, 3H), 1,25 (t, 3H) |
| 2-50 | Me | F | Cl | ¹H-NMR, DMSO-d₆, 400 MHz |
| | | | | 11,32 (s, 1H), 7,61 (t,1H), 7,48 (d, 1H), 2,79 (q, 2H), 2,35 (s,1H),1,26 (t, 3H) |
| 2-51 | Me | SMe | CF₃ | |
| 2-52 | Me | Cl | SO₂Me | ¹H-NMR, DMSO-d₆, 400 MHz |
| | | | | 11,45 (s, 1H), 8,05 (d, 1H), 7,79 (d,1H), 3,44 (s, 3H), 2,80 (q, 2H), 2,48 (s, 3H), 1,28 (t, 3H) |
| 2-53 | Me | SO₂Me | SO₂Me | ¹H-NMR, DMSO-d₆, 400 MHz |
| | | | | 11,50 (s, 1H), 8,27 (d, 1H), 8,08 (d,1H), 3,60 (s, 3H), 3,58 (s, 3H), 2,81 (q, 2H), 2,71 (s, 3H), 1,27 (t, 3H) |
| 2-54 | Me | SO₂Me | CF₃ | ¹H-NMR, CDCl₃, 400 MHz |
| | | | | 8,05 (bs, 1H), 7,88 (d, 1H), 7,78 (d, 1H), 3,20 (s, 3H), 2,90 (q, 2H), 2,75 (s, 3H), 1,40 (t, 3H) |
| 2-55 | Me | Cl | CF₃ | |
| 2-56 | Me | S(O)Me | CF₃ | ¹H-NMR, DMSO-d₆, 400 MHz |
| | | | | 11,44 (s, 1H), 7,88 (m, 2H), 3,05 (s, 3H), 2,87 (s, 3H), 2,82 (q, 2H), 1,28 (t, 3H) |
| 2-57 | Me | SEt | OMe | |
| 2-58 | Me | NMe₂ | SO₂Me | ¹H-NMR, DMSO-d₆, 400 MHz |
| | | | | 11,32 (s, 1H), 7,88 (d, 1H), 7,60 (d,1H), 3,31 (s, 3H), 2,84 (s, 6H), 2,80 (q, 2H), 2,39 (s, 3H), 1,28 (t, 3H) |
| 2-59 | Me | NH(CH₂)₂OMe | SO₂Me | ¹H-NMR, CDCl₃, 400 MHz |
| | | | | 8,84 (bs, 1H), 7,52 (d, 1H), 7,09 (d, 1H), 5,68 (bs, 1H), 3,57 (m, 2H), 3,35 (m, 5H), 3,13 (s, 3H), 2,90 (q, 2H), 2,37 (s, 3H), 1,40 (t, 3H) |
| 2-60 | Me | O(CH₂)₄OMe | SO₂Me | |
| 2-61 | Me | NH₂ | SO₂Me | |
| 2-62 | Me | O(CH₂)₂-O(3,5-Dimethoxypyrimidin-2-yl | SO₂Me | |
| 2-63 | Me | O(CH₂)₂-O-NMe₂ | Cl | |
| 2-64 | Me | O(CH₂)₂-NH(CO)NMe₂ | Cl | |
| 2-65 | Me | O(CH₂)-5-Pyrrolidin-2-on | Br | |
| | | | | |
| 2-66 | Me | O(CH₂)₂-NH(CO)NHCO₂Et | Cl | |
| 2-67 | Me | O(CH₂)-(CO)NEt₂ | Br | |
| 2-68 | Me | O(CH₂)-5-2,4-Dimethyl-2,4-dihydro-3H-1,2,4-triazol-3-on | Cl | |
| 2-69 | Me | O(CH₂)-3,5-Dimethyl-1,2-oxazol-4-yl | Cl | |
| 2-70 | Me | O(CH₂)₂-NHCO₂Me | Cl | |
| 2-71 | Me | 4,5-Dihydro-1,2-oxazol-3 yl | SO₂Me | ¹H-NMR, DMSO-d₆, 400 MHz |
| | | | | 11,46 (s, 1H), 8,04 (d, 1H), 7,90 (d, 1H), 4,50 (t, 2H), 3,35 (t, 2H), 3,27 (s, 3H), 2,82 (q, 2H), 2,35 (s, 3H), 1,27 (t, 3H) |
| 2-72 | Me | Me | SO₂Me | ¹H-NMR, DMSO-d₆, 400 MHz |
| | | | | 11,34 (s, 1H), 7,92 (d, 1H), 7,59 (d, 1H), 3,27 (s, 3H), 2,80 (q, 2H), 2,66 (s, 3H), 2,36 (s,3H),1,27 (t, 3H) |
| 2-73 | Me | OH | SO₂Me | |
| 2-74 | Me | O-CH₂-NHSO₂cPr | Cl | |
| 2-75 | Me | O(CH₂)₂NHSO2Me | SO₂Me | |
| 2-76 | Me | S(O)Me | SO₂Me | |
| 2-77 | Me | SMe | SO₂Me | |
| 2-78 | Me | SMe | OMe | |
| 2-79 | Me | S(O)Me | OMe | |
| 2-80 | Me | SO₂Me | OMe | |
| 2-81 | Me | SMe | Cl | |
| 2-82 | Me | S(O)Me | Cl | |
| 2-83 | Me | SO₂Me | Cl | |
| 2-84 | Me | [1,4]Dioxan-2-yl-methoxy | SO₂Me | |
| 2-85 | Me | [1,4]Dioxan-2-yl-methoxy | SO₂Et | |
| 2-86 | Me | O(CH₂)₄OMe | SO₂Et | |
| 2-87 | Me | O(CH₂)₃OMe | SO₂Me | |
| 2-88 | Me | O(CH₂)₃OMe | SO₂Et | |
| 2-89 | Me | O(CH₂)₂OMe | SO₂Me | |
| 2-90 | Me | O(CH₂)₂OMe | SO₂Et | |
| 2-91 | Me | S(O)Me | SO₂Me | |
| 2-92 | Me | SMe | SO₂Me | |
| 2-93 | Me | SMe | OMe | |
| 2-94 | Me | S(O)Me | OMe | |
| 2-95 | Me | SO₂Me | OMe | |
| 2-96 | Me | SMe | Cl | |
| 2-97 | Me | S(O)Me | Cl | |
| 2-98 | Me | SO₂Me | Cl | |
| 2-99 | Me | SMe | Br | |
| 2-100 | Me | SOMe | Br | |
| 2-101 | Me | SO₂Me | Br | |
| 2-102 | Me | SMe | I | |
| 2-103 | Me | SOMe | I | |
| 2-104 | Me | SO₂Me | I | |
| 2-105 | Me | SEt | Cl | |
| 2-106 | Me | SOEt | Cl | |
| 2-107 | Me | SO₂Et | Cl | |
| 2-108 | Me | SEt | Br | |
| 2-109 | Me | SOEt | Br | |
| 2-110 | Me | SO₂Et | Br | |
| 2-111 | Me | SEt | I | |
| 2-112 | Me | SOEt | I | |
| 2-113 | Me | SO₂Et | I | |
| 2-114 | Me | SEt | F | |
| 2-115 | Me | SOEt | F | |
| 2-116 | Me | SO₂Et | F | |
| 2-117 | Cl | OCH₂(CO)NMe₂ | Cl | ¹H-NMR, DMSO-d₆, 400 MHz |
| | | | | 11,44 (s, 1H), 7,65 (d, 1H), 7,49 (d, 1H), 4,74 (s, 2H), 3,02 (s,3H), 2,87 (s, 3H), 2,81 (q, 2H), 1,27 (t, 3H) |
| 2-118 | Cl | CH₂OCH₂CF₃ | SO₂Me | ¹H-NMR, DMSO-d₆, 400 MHz |
| | | | | 11,60 (s, 1H), 8,12 (d, 1H), 7,98 (d, 1H), 5,27 (s, 2H), 4,30 (q, 2H), 3,38 (s, 3H), 2,83 (q, 2H), 1,30 (t, 3H) |
| 2-119 | Cl | 5-Cyanomethyl- 4,5-dihydro-1,2-oxazol-3 yl | SO₂Et | ¹H-NMR, DMSO-d₆, 400 MHz |
| | | | | 11,62 (s, 1H), 8,11 (m, 2H), 5,20 (m, 1H), 3,60 (m, 1H), 3,44 (q, 2H), 3,15 (m, 1H), 3,02 (m, 2H), 2,81 (q, 2H), 1,28 (t, 3H), 1,16 (t, 3H) |
| 2-120 | Cl | CH₂O-Tetrahydrofuran-3-yl | SO₂Me | ¹H-NMR, CDCl₃, 400 MHz |
| | | | | 8,70 (bs, 1H), 7,94 (d, 1H), 7,72 (d, 1H), |
| | | | | 5,06 (m, 2H), 4,35 (m, 1H), 3,73-3,92 (m, 4H), 3,36 (q, 2H), 2,92 (q, 2H), 2,10 (m, 2H), 1,40 (t, 3H), 1,22 (t, 3H) |
| 2-121 | Cl | CH₂O-Tetrahydrofuran-2-yl | SO₂Me | ¹H-NMR, DMSO-d₆, 400 MHz |
| | | | | 11,55 (s, 1H), 8,14 (d, 1H), 7,93 (d, 1H), 3,58 (s, 3H), 2,81 (q, 2H), 2,48 (s,3H), 1,28 (t, 3H), |
| 2-122 | Cl | SMe | SO₂Me | |
| 2-123 | Cl | F | SMe | |
| 2-124 | Cl | CH₂OCH₂-Tetrahydrofuran-2-yl | SO₂Me | ¹H-NMR, CDCl₃, 400 MHz |
| | | | | 8,84 (bs, 1H), 8,0 (d, 1H), 7,70 (d, 1H), 5,12 (s, 2H), 4,05 (m, 1H), 3,55-3,80 (m, 4H), 3,28 (s, 3H), 2,90 (q, 2H), 1,78-2,0 (m, 3H), 1,48-1,59 (m, 1H), 1,40 (t, 3H) |
| 2-125 | Cl | CH₂OCH₂-Tetrahydrofuran-3-yl | SO₂Et | |
| 2-126 | Cl | O(CH₂)-5-Pyrrolidin-2-on | Cl | |
| 2-127 | Cl | SMe | Cl | ¹H-NMR, DMSO-d₆, 400 MHz |
| | | | | 11,41 (s, 1H), 7,22 (d, 1H), 7,68 (d,1H), 2,80 (q, 2H), 2,43 (s, 3H), 1,28 (t, 3H) |
| 2-128 | Cl | S(O)Me | SO₂Me | |
| 2-129 | Cl | CH₂O-Tetrahydrofuran-3-yl | SO₂Et | |
| 2-130 | Cl | O(CH₂)₂OMe | Cl | |
| 2-131 | Cl | O(CH₂)₂OMe | SO₂Me | |
| 2-132 | Cl | O(CH₂)₄OMe | SO₂Me | |
| 2-133 | Cl | O(CH₂)₄OMe | SO₂Et | |
| 2-134 | Cl | O(CH₂)₃OMe | SO₂Me | |
| 2-135 | Cl | O(CH₂)₃OMe | SO₂Et | |
| 2-136 | Cl | O(CH₂)₂OMe | SO₂Me | |
| 2-137 | Cl | O(CH₂)₂OMe | SO₂Et | |
| 2-138 | Cl | [1,4]Dioxan-2-yl-methoxy | SO₂Me | |
| 2-139 | Cl | [1,4]Dioxan-2-yl-methoxy | SO₂Et | |
| 2-140 | Cl | SO₂Me | Me | |
| 2-141 | Cl | SEt | Me | |
| 2-142 | Cl | SOEt | Me | |
| 2-143 | Cl | SO₂Et | Me | |
| 2-144 | Cl | 4,5-Dihydro-1,2-oxazol-3 yl | SO₂Me | |
| 2-145 | Cl | Cl | SO₂Me | ¹H-NMR, DMSO-d₆, 400 MHz |
| | | | | 11,6 (s, 1H), 8,16 (d, 1H), 7,94 (d, 1H), 3,49 (s, 3H), 3,82 (q, 2H), 1,28 (t, 3H) |
| 2-146 | F | SMe | CF₃ | |
| 2-147 | F | S(O)Me | CF₃ | ¹H-NMR, DMSO-d₆, 400 MHz |
| | | | | 11,75 (bs, 1H), 8,14 (dd, 1H), 7,90 (d, 1H), 2,81 (q, 2H), 1,78-2,0 (m, 3H), 1,28 (t, 3H) |
| 2-148 | OMe | OMe | OMe | |
| 2-149 | OMe | SMe | CF₃ | |
| 2-150 | OMe | S(O)Me | CF₃ | |
| 2-151 | OMe | SO₂Me | CF₃ | |
| 2-152 | Et | NH(CH₂)₂OMe | SO₂Me | |
| 2-153 | Et | F | SO₂Me | |
| 2-154 | Et | SMe | CF₃ | |
| 2-155 | CF₃ | F | SO₂Me | ¹H-NMR, DMSO-d₆, 400 MHz |
| | | | | 11,68 (s, 1H), 8,3 (t, 1H), 7,89 (d, 1H), 3,49 (s, 3H), 2,79 (q, 2H), 1,27 (t, 3H) |
| 2-156 | CF₃ | F | SO₂Et | |
| 2-157 | CF₃ | O(CH₂)₂OMe | SO₂Et | |
| 2-158 | CF₃ | O(CH₂)₃OMe | SO₂Et | |
| 2-159 | CF₃ | O(CH₂)₂OMe | SO₂Me | |
| 2-160 | CF₃ | O(CH₂)₃OMe | SO₂Me | |
| 2-161 | CF₃ | OCH₂CONMe₂ | SO₂Me | ¹H-NMR, CD₃OD, 400 MHz |
| | | | | 8,37 (d, 1H), 7,74 (d, 1H), 5,00 (s, 2H), 3,43 (s, 3H), 3,03 (s, 3H), 2,95 (s, 3H), 2,87 (q, 2H), 1,38 (t, 3H) |
| 2-162 | CF₃ | OCH₂CONMe₂ | SO₂Et | |
| 2-163 | CF₃ | OCH₂CONMe₂ | Cl | |
| 2-164 | CF₃ | OCH₂CONMe₂ | Br | |
| 2-165 | CF₃ | OCH₂CONMe₂ | I | |
| 2-166 | CF₃ | OCH₂CONMe₂ | F | |
| 2-167 | CF₃ | O(CH₂)₂OMe | Cl | |
| 2-168 | CF₃ | O(CH₂)₃OMe | Cl | |
| 2-169 | CF₃ | O(CH₂)₂OMe | Br | |
| 2-170 | CF₃ | O(CH₂)₃OMe | Br | |
| 2-171 | CF₃ | O(CH₂)₂OMe | I | |
| 2-172 | CF₃ | O(CH₂)₃OMe | I | |
| 2-173 | CF₃ | 0(CH₂)₂OMe | F | |
| 2-174 | CF₃ | O(CH₂)₃OMe | F | |
| 2-175 | CF₃ | [1,4]Dioxan-2-yl-methoxy | SO₂Me | |
| 2-176 | CF₃ | [1,4]Dioxan-2-yl-methoxy | SO₂Et | |
| 2-177 | CF₃ | [1,4]Dioxan-2-yl-methoxy | Cl | |
| 2-178 | CF₃ | [1,4]Dioxan-2-yl-methoxy | Br | |
| 2-179 | CF₃ | [1,4]Dioxan-2-yl-methoxy | I | |
| 2-180 | CF₃ | [1,4]Dioxan-2-yl-methoxy | F | |
| 2-181 | Br | OMe | Br | |
| 2-182 | Br | O(CH₂)₂OMe | Br | |
| 2-183 | Br | O(CH₂)₄OMe | SO₂Me | |
| 2-184 | Br | O(CH₂)₄OMe | SO₂Et | |
| 2-185 | Br | O(CH₂)₃OMe | SO₂Me | |
| 2-186 | Br | O(CH₂)₃OMe | SO₂Et | |
| 2-187 | Br | O(CH₂)₂OMe | SO₂Me | |
| 2-188 | Br | O(CH₂)₂OMe | SO₂Et | |
| 2-189 | Br | [1,4]Dioxan-2-yl-methoxy | SO₂Me | |
| 2-190 | Br | [1,4]Dioxan-2-yl-methoxy | SO₂Et | |
| 2-191 | Br | SMe | Me | |
| 2-192 | Br | SOMe | Me | |
| 2-193 | Br | SO₂Me | Me | |
| 2-194 | Br | SEt | Me | |
| 2-195 | Br | SOEt | Me | |
| 2-196 | Br | SO₂Et | Me | |
| 2-197 | I | O(CH₂)₄OMe | SO₂Me | |
| 2-198 | I | O(CH₂)₄OMe | SO₂Et | |
| 2-199 | I | O(CH₂)₃OMe | SO₂Me | |
| 2-200 | I | O(CH₂)₃OMe | SO₂Et | |
| 2-201 | I | O(CH₂)₂OMe | SO₂Me | |
| 2-202 | I | O(CH₂)₂OMe | SO₂Et | |
| 2-203 | I | [1,4]Dioxan-2-yl-methoxy | SO₂Me | |
| 2-204 | I | [1,4]Dioxan-2-yl-methoxy | SO₂Et | |
| 2-205 | I | SMe | Me | |
| 2-206 | I | SOMe | Me | |
| 2-207 | I | SO₂Me | Me | |
| 2-208 | I | SEt | Me | |
| 2-209 | I | SOEt | Me | |
| 2-210 | I | SO₂Et | Me | |
| 2-211 | CH₂SMe | OMe | SO₂Me | |
| 2-212 | CH₂OMe | OMe | SO₂Me | |
| 2-213 | CH₂O(CH₂)₂ OMe | NH(CH₂)₂OEt | SO₂Me | |
| 2-214 | CH₂O(CH₂)₂ OMe | NH(CH₂)₃OEt | SO₂Me | |
| 2-215 | CH₂O(CH₂)₃ OMe | OMe | SO₂Me | |
| 2-216 | CH₂O(CH₂)₂ OMe | NH(CH₂)₂OMe | SO₂Me | |
| 2-217 | CH₂O(CH₂)₂ OMe | NH(CH₂)₃OMe | SO₂Me | |
| 2-218 | SO₂Me | NH₂ | CF₃ | |
| 2-219 | SO₂Me | F | CF₃ | |
| 2-220 | SO₂Me | NHEt | Cl | |
| 2-221 | SMe | SEt | F | |
| 2-222 | SMe | SMe | F | |
| 2-223 | Me | NH₂ | Cl | |
| 2-224 | Me | NH₂ | Br | |
| 2-225 | Me | NHMe | Cl | |
| 2-226 | Me | NHMe | Br | |
| 2-227 | Me | NMe₂ | Cl | |
| 2-228 | Me | NMe₂ | Br | |
| 2-227 | Me | NMe₂ | Cl | |
| 2-228 | Me | NMe₂ | Br | |
| 2-229 | NO₂ | O(CH₂)₂OMe | Me | |
| 2-230 | CF₃ | S(O)₂Et | SO₂Me | |
| 2-231 | CF₃ | S(O)₂Et | SO₂Et | |
| 2-232 | CF₃ | SCH₂CONMe₂ | SO₂Me | |
| 2-233 | CF₃ | SCH₂CONMe₂ | SO₂Et | |
| 2-234 | CF₃ | SCH₂COOH | SO₂Me | |
| 2-235 | CF₃ | SCH₂COOH | SO₂Et | |
| 2-236 | Me | SO₂-CH₂-CH₂-CH=CH₂ | CF₃ | |
| 2-237 | Cl | Me | SO₂Et | |
| 2-238 | CF₃ | SEt | SO₂Me | |
| 2-239 | OMe | NO₂ | Cl | |
| 2-240 | OMe | NH(CO)i-Pr | Cl | |
| 2-241 | OMe | NH(CO)CH2Ph | Cl | |
| 2-242 | CF₃ | SEt | SO₂Et | |
| 2-243 | CF₃ | S(O)Et | SO₂Me | |
| 2-244 | Cl | Me | Cl | |
| 2-245 | Me | 3,5-Dimethylpyrazol-1-yl | SO₂Me | |
| 2-247 | Me | 1,2,3-Triazol-1-yl | SO₂Me | ¹H-NMR, DMSO-d₆, 400 MHz |
| | | | | 11,54 (s, 1H), 8,22 (s, 2H), 8,15 (d, 1H), 8,08 (d, 1H), 3,30 (s, 3H), 2,81 (q, 2H), 1,91 (s, 3H), 1,27 (t, 3H) |
| 2-248 | Me | Me | SMe | |
| 2-249 | Me | Pyrolidin-2-on-1-yl | SO₂Me | |
| 2-250 | CF₃ | S(O)Et | SO₂Et | |
| 2-251 | Cl | Pyrazol-1-yl | SO₂Me | |
| 2-252 | Me | 3-Methyl-pyrazol-1-yl | SO₂Me | |
| 2-253 | Cl | CH₂-N(Et)OMe | SO₂Me | |
| 2-254 | Me | Me | Cl | |
| 2-255 | OH | Cl | Cl | |
| 2-256 | Me | 1,2,4-Triazol-1-yl | SO₂Me | |
| 2-257 | Me | 4-Methoxy-pyrazol-1-yl | SO₂Me | ¹H-NMR, DMSO-d₆, 400 MHz |
| | | | | 11,50 (s, 1H), 8,08 (d, 1H), 7,98 (d, 1H), 7,74 (s, 1H), 7,66 (s, 1H), 3,75 (s, 3H), 3,12 (s,3H), 2,81 (q, 2H), 1,95 (s,3H), 1,27 (t, 3H) |
| 2-258 | Me | 1,2,4-triazol-1-yl | CF₃ | |
| 2-259 | Me | Tetrahydropyrimidin-2(1 H)-one-1-yl | SO₂Me | |
| 2-260 | Me | NH-(CH₂)₂-O(CO)Et | SO₂Me | |
| 2-261 | Me | NH-iPr | SO₂Me | |
| 2-262 | Cl | NH-CH₂-(CO)NHEt | Cl | |
| 2-263 | Me | NH-CH2-(CO)NMe2 | SO₂Me | |
| 2-264 | Me | NH-CH₂-furan-2-yl | SO₂Me | |
| 2-265 | Me | NH-CH₂-(CO)NHEt | SO₂Me | |
| 2-266 | Me | F | SO₂Me | ¹H-NMR, DMSO-d₆, 400 MHz |
| | | | | 11,46 (s, 1H), 7,85 (t, 1H), 7,66 (s, 1H), 3,38 (s, 3H), 2,81 (q, 2H), 2,38 (s,3H), 1,27 (t, 3H) |
| 2-267 | F | SO₂Me | SO₂Me | |
| 2-268 | Cl | (4-Cyclopropyl-3-methyl-5-oxo-4,5-dihydro-1H-1,2,4-triazol-1-yl)-methyl | Cl | |
| 2-269 | Cl | [4-Methyl-5-oxo-3-(2,2,2-trifluorethoxy)-4,5-dihydro-1H-1,2,4-triazol-1-yl]-methyl | Cl | |
| 2-270 | Cl | (3-Isopropoxy-4-methyl-5-oxo-4,5-dihydro-1H-1,2,4-triazol-1-yl]-methyl | Cl | |
| 2-271 | Cl | (4-Methyl-5-oxo-4,5-dihydro-1H-1,2,4-triazol-1-yl)-methyl | Cl | |
| 2-272 | Me | Cl | SO₂Et | ¹H-NMR, DMSO-d₆, 400 MHz |
| | | | | 11,52 (s, 1H), 8,02 (d, 1H), 7,77 (d, 1H), 3,41 (q, 2H), 2,82 (q, 2H), 2,75 (s, 3H),1,28 (t, 3H), 1,14 (t,3H) |
| 2-273 | SO₂Me | F | Cl | |
| 2-274 | Me | 1?2,3-Triazol-1-yl | SO₂Me | ¹H-NMR, DMSO-d₆, 400 MHz |
| | | | | 11,56 (s, 1H), 8,55 (s, 1H), 8,17 (d, 1H), 8,08 (d, 1H), 8,06 (s, 1H), 3,14 (s, 3H), 2,80 (q, 2H), 1,91 (s,3H),1,27 (t, 3H) |
| 2-275 | Me | Isobutyl(methyl)carb -amoylamino | SO₂Me | |
| 2-276 | Me | 3-Oxo-morpholin-4-yl | SO₂Me | |
| 2-277 | OMe | [Ethyl(methylsulfony l)amino]methyl | Cl | |
| 2-278 | F | SO₂Me | CF₃ | |
| 2-279 | OMe | Benzoylamino | Cl | |
| 2-280 | OMe | Cyclopropylcarbonyl -amino | Cl | |
| 2-281 | OMe | Propionyllamino | Cl | |
| 2-282 | NO₂ | SO₂Me | SO₂Me | |
| 2-283 | NO₂ | SO₂Me | Cl | |
| 2-284 | NO₂ | SOMe | SO₂Me | |
| 2-285 | NO₂ | SOMe | Br | |
| 2-286 | NO₂ | SOMe | Cl | |
| 2-287 | NO₂ | SMe | SO₂Me | |
| 2-288 | NO₂ | SMe | Br | |
| 2-289 | NO₂ | SMe | Cl | |
| 2-290 | Cl | CH₂OCH(CH₃)₂ | SO₂Et | |
| 2-291 | Cl | CH₂OEt | SO₂Et | |
| 2-292 | Cl | CH₂OMe | SO₂Et | |
| 2-293 | Cl | CH₂OCH₂C₂F₅ | SO₂Me | |
| 2-294 | Cl | CH₂OCH₂CHF₂ | SO₂Me | |
| 2-295 | Cl | CH₂OCH₂CCH | SO₂Et | |
| 2-296 | Cl | CH₂OC₂H₄OMe | SO₂Me | |
| 2-297 | Cl | CH₂(OC₂H₄)₂OMe | SO₂Me | |
| 2-298 | Cl | 5-Ethoxymethyl-4,5-dihydro-1,2-oxazol-3 yl | SO₂Et | |
| 2-299 | Cl | 5-Methoxymethyl-4,5-dihydro-1,2-oxazol-3 yl | SO₂Et | |
| 2-300 | Et | SOMe | CF₃ | |
| 2-301 | iPr | SMe | CF₃ | |
| 2-302 | Et | SMe | CF₃ | |
| 2-303 | Et | SO₂Me | CF₃ | |
| 2-304 | cPr | SOMe | CF₃ | |
| 2-305 | CH=CH₂ | SMe | CF₃ | |
| 2-306 | Et | SMe | Cl | |
| 2-307 | Et | SO₂Me | Cl | |
| 2-308 | Cl | NMe₂ | Cl | |
| 2-309 | CH₂O(CH₂)₂ OMe | NH(CH₂)₃OMe | SO₂Me | |
| 2-313 | Me | SO₂(CH₂)₂OMe | CF₃ | |
| 2-314 | Me | SOEt | SO₂Me | |
| 2-315 | Me | SO₂Et | SO₂Me | |
| 2-316 | Me | SMe | 1,2,4-triazol-1-yl | |
| 2-317 | OEt | SMe | CF₃ | |
| 2-318 | Me | S(CH₂)₂OMe | CF₃ | |
| 2-319 | Me | SOMe | 1,2,4-triazol-1-yl | |
| 2-320 | OEt | SOMe | CF₃ | |
| 2-321 | Me | SO(CH₂)₂OMe | CF₃ | |
| 2-322 | Me | SCH₂CCMe | SO₂Me | |
| 2-323 | Me | S-c-Pen | SO₂Me | |
| 2-324 | OMe | SMe | OMe | |
| 2-325 | Me | SCH₂CH=CHCH₃ | SO₂Me | |
| 2-326 | Me | SOCH₂CCMe | SO₂Me | |
| 2-327 | Me | SO₂-c-Pen | SO₂Me | |
| 2-328 | Me | SO-c-Pen | SO₂Me | |
| 2-329 | Me | S(CH₂)₃Cl | SO₂Me | |
| 2-330 | Me | SCH₂(4-F-Ph) | SO₂Me | |
| 2-331 | Me | SO₂CH₂CCMe | SO₂Me | |
| 2-332 | Me | SO₂CH₂CH=CHCH₃ | SO₂Me | |
| 2-333 | Me | SOCH₂CH=CHCH₃ | SO₂Me | |
| 2-334 | Me | SOCH₂-Epoxy-Me | SO₂Me | |
| 2-335 | Me | SO₂(CH₂)Cl | SO₂Me | |
| 2-336 | Me | SO(CH₂)₃Cl | SO₂Me | |
| 2-337 | Me | SOCH₂(4-F-Ph) | SO₂Me | |
| 2-338 | Me | SO₂CH₂(4-F-Ph) | SO₂Me | |
| 2-339 | Me | SO₂Me | C₂F₅ | |
| 2-340 | O(CH₂)₂OMe | SMe | CF₃ | |
| 2-341 | O(CH₂)₂OMe | SO₂Me | CF₃ | |
| 2-342 | O(CH₂)₂OMe | SOMe | CF₃ | |
| 2-343 | Me | S(CH₂)₂OCH₂CF₃ | SO₂Me | |
| 2-344 | Me | SO(CH₂)₂OCH₂CF₃ | SO₂Me | |
| 2-345 | Me | SO₂(CH₂)₂OCH₂CF₃ | SO₂Me | |
| 2-346 | OEt | SEt | CF₃ | |
| 2-347 | O-CH₂-c-Pr | SMe | CF₃ | |
| 2-348 | OMe | SEt | CF₃ | |
| 2-349 | OMe | SO₂Et | CF₃ | |
| 2-350 | OMe | SOEt | CF₃ | |
| 2-351 | OEt | SO₂Et | CF₃ | |
| 2-352 | OEt | SOEt | CF₃ | |
| 2-353 | O-CH2-c-Pr | SOMe | CF₃ | |
| 2-354 | O-CH2-c-Pr | SO₂Me | CF₃ | |
| 2-355 | Me | SEt | SO₂Me | |

**Tabelle 3: Erfindungsgemäße Verbindungen der allgemeinen Formel (I), worin R für iso-Propyl steht**

| | | | | |
|---|---|---|---|---|
| 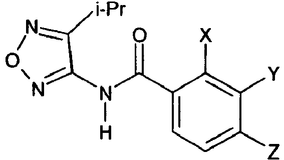 | | | | |

| **Nr.** | **X** | **Y** | **Z** | **Physikalische Daten** |
|---|---|---|---|---|
| 3-1 | CF₃ | OCH₂CON(Me)Et | SO₂Me | |
| 3-2 | CF₃ | OCH₂CON(Me)Et | SO₂Et | |
| 3-4 | CF₃ | 2-(1H-Pyrazol-1-yl)ethoxyl | SO₂Me | |
| 3-5 | CF₃ | 2-(1H-Pyrazol-1-yl)ethoxyl | SO₂Et | |
| 3-6 | CF₃ | Tetrahydrofuran-2-yl-methoxy | SO₂Me | |
| 3-7 | CF₃ | Tetrahydrofuran-2-yl-methoxy | SO₂Et | |
| 3-8 | CF₃ | OH | SO₂Me | |
| 3-9 | CF₃ | OH | SO₂Et | |
| 3-10 | CF₃ | SH | SO₂Me | |
| 3-11 | CF₃ | SH | SO₂Et | |
| 3-15 | CF₃ | SMe | SO₂Me | |
| 3-16 | CF₃ | SMe | SO₂Et | |
| 3-17 | CF₃ | S(O)Me | SO₂Me | |
| 3-24 | CF₃ | S(O)Me | SO₂Et | |
| 3-25 | CF₃ | S(O)₂Me | SO₂Me | |
| 3-26 | CF₃ | S(O)₂Me | SO₂Et | |
| 3-27 | CF₃ | 2-[(Methylsulfonyl) amino]ethoxy | SO₂Me | |
| 3-28 | CF₃ | 2-[(Methylsulfonyl) amino]ethyl}sulfanyl | SO₂Me | |
| 3-29 | CF₃ | 2-[(Methylsulfonyl) amino]ethyl}sulfanyl | SO₂Et | |
| 3-30 | NO₂ | O(CH₂)₂OMe | OMe | |
| 3-31 | NO₂ | OMe | Me | |
| 3-32 | NO₂ | NH₂ | OMe | |
| 3-33 | NO₂ | NH₂ | SO₂Et | |
| 3-34 | NO₂ | NH₂ | Cl | |
| 3-35 | NO₂ | NHMe | Cl | |
| 3-36 | NO₂ | NMe₂ | Cl | |
| 3-37 | NO₂ | NH₂ | Br | |
| 3-38 | NO₂ | NHMe | Br | |
| 3-39 | NO₂ | NMe₂ | Br | |
| 3-40 | NO₂ | NH₂ | F | |
| 3-41 | NO₂ | NHMe | F | |
| 3-42 | NO₂ | NMe₂ | F | |
| 3-43 | NO₂ | NH₂ | SO₂Me | |
| 3-44 | NO₂ | NHMe | SO₂Me | |
| 3-45 | NO₂ | NMe₂ | SO₂Me | |
| 3-46 | NO₂ | NH₂ | 1H-1,2,4-triazol-1-yl | |
| 3-47 | NO₂ | NHMe | 1H-1,2,4-triazol-1-yl | |
| 3-48 | NO₂ | NMe₂ | 1 H-1,2,4-triazol-1-yl | |
| 3-49 | Me | F | F | |
| 3-50 | Me | F | Cl | |
| 3-51 | Me | SMe | CF₃ | |
| 3-52 | Me | Cl | SO₂Me | ¹H-NMR, DMSO-d₆, 400 MHz |
| | | | | 11,38 (s, 1H), 8,06 (d, 1H), 7,77 (d, 1H), 3,44 (s, 3H), 3,22 (m, 1H), 2,48 (s, 3H), 1,31 (d, 6H) |
| 3-53 | Me | SO₂Me | SO₂Me | ¹H-NMR, DMSO-d₆, 400 MHz |
| | | | | 11,40 (s, 1H), 8,29 (d, 1H), 8,08 (d, 1H), 3,60(s, 3H), 3,57 (s, 3H), 3,21 (m, 1H) 2,71 (s, 3H), 1,31 (d, 6H) |
| 3-54 | Me | SO₂Me | CF₃ | ¹H-NMR, CDCl₃, 400 MHz |
| | | | | 7,91 (d, 1H), 7,78 (d, 1H), 3,26 (m, 4H), 2,86 (s, 3H), 1,41 (d, 6H) |
| 3-55 | Me | Cl | CF₃ | |
| 3-56 | Me | S(O)Me | CF₃ | ¹H-NMR, DMSO-d₆, 400 MHz |
| | | | | 11,37 (s, 1H), 7,89 (d, 1H), 7,86 (d, 1H), 3,25 (m, 1H), 3,06 (s, 3H), 2,86 (s, 3H) 1,31 (d, 6H) |
| 3-57 | Me | SEt | OMe | |
| 3-58 | Me | NMe₂ | SO₂Me | |
| 3-59 | Me | NH(CH₂)₂OMe | SO₂Me | |
| 3-60 | Me | O(CH₂)₄OMe | SO₂Me | |
| 3-61 | Me | NH₂ | SO₂Me | |
| 3-62 | Me | O(CH₂)₂-O(3,5-Dimethoxypyrimidin-2-yl | SO₂Me | |
| 3-63 | Me | O(CH₂)₂-O-NMe₂ | Cl | |
| 3-64 | Me | O(CH₂)₂-NH(CO)NMe₂ | Cl | |
| 3-65 | Me | O(CH₂)-5-Pyrrolidin-2-on | Br | |
| 3-66 | Me | O(CH₂)₂-NH(CO)NHCO₂Et | Cl | |
| 3-67 | Me | O(CH₂)-(CO)NEt₂ | Br | |
| | | | | |
| 3-68 | Me | O(CH₂)-5-2,4-Dimethyl-2,4-dihydro-3H-1,2,4-triazol-3-on | Cl | |
| 3-69 | Me | O(CH₂)-3,5-Dimethyl-1,2-oxazol-4-yl | Cl | |
| 3-70 | Me | O(CH₂)₂-NHCO₂Me | Cl | |
| 3-71 | Me | 4,5-Dihydro-1,2-oxazol-3 yl | SO₂Me | |
| 3-72 | Me | Me | SO₂Me | |
| 3-73 | Me | OH | SO₂Me | |
| 3-74 | Me | O-CH₂-NHSO₂cPr | Cl | |
| 3-75 | Me | O(CH₂)₂NHSO2Me | SO₂Me | |
| 3-76 | Me | S(O)Me | SO₂Me | |
| 3-77 | Me | SMe | SO₂Me | |
| 3-78 | Me | SMe | OMe | |
| 3-79 | Me | S(O)Me | OMe | |
| 3-80 | Me | SO₂Me | OMe | |
| 3-81 | Me | SMe | Cl | |
| 3-82 | Me | S(O)Me | Cl | |
| 3-83 | Me | SO₂Me | Cl | |
| 3-84 | Me | [1,4]Dioxan-2-yl-methoxy | SO₂Me | |
| 3-85 | Me | [1,4]Dioxan-2-yl-methoxy | SO₂Et | |
| 3-86 | Me | O(CH₂)₄OMe | SO₂Et | |
| 3-87 | Me | O(CH₂)₃OMe | SO₂Me | |
| 3-88 | Me | O(CH₂)₃OMe | SO₂Et | |
| 3-89 | Me | O(CH₂)₂OMe | SO₂Me | |
| 3-90 | Me | O(CH₂)₂OMe | SO₂Et | |
| 3-91 | Me | S(O)Me | SO₂Me | |
| 3-92 | Me | SMe | SO₂Me | |
| 3-93 | Me | SMe | OMe | |
| 3-94 | Me | S(O)Me | OMe | |
| 3-95 | Me | SO₂Me | OMe | |
| 3-96 | Me | SMe | Cl | |
| 3-97 | Me | S(O)Me | Cl | |
| 3-98 | Me | SO₂Me | Cl | |
| 3-99 | Me | SMe | Br | |
| 3-100 | Me | SOMe | Br | |
| 3-101 | Me | SO₂Me | Br | |
| 3-102 | Me | SMe | I | |
| 3-103 | Me | SOMe | I | |
| 3-104 | Me | SO₂Me | I | |
| 3-105 | Me | SEt | Cl | |
| 3-106 | Me | SOEt | Cl | |
| 3-107 | Me | SO₂Et | Cl | |
| 3-108 | Me | SEt | Br | |
| 3-109 | Me | SOEt | Br | |
| 3-110 | Me | SO₂Et | Br | |
| 3-111 | Me | SEt | I | |
| 3-112 | Me | SOEt | I | |
| 3-113 | Me | SO₂Et | I | |
| 3-114 | Me | SEt | F | |
| 3-115 | Me | SOEt | F | |
| 3-116 | Me | SO₂Et | F | |
| 3-117 | Cl | OCH₂(CO)NMe₂ | Cl | ¹H-NMR, DMSO-d₆, 400 MHz |
| | | | | 11,37 (s, 1H), 7,67 (d, 1H), 7,48 (d, 1H), 4,74 (s, 2H) 3,25 (m, 1H), 3,02 (s, 3H), 2,87 (s, 3H) 1,30 (d, 6H) |
| 3-118 | Cl | Cl | SO₂Me | |
| 3-119 | Cl | CH₂OCH₂CF₃ | SO₂Me | ¹H-NMR, CDCl₃, 400 MHz |
| | | | | 8,41 (bs, 1H), 8,04 (d, 1H), 7,77 (d, 1H), 5,32 (s, 2H), 4,05 (q, 2H), 3,29 (m, 1H), 3,20 (s, 3H), 1,43 (d, 6H) |
| 3-120 | Cl | 5-Cyanomethyl- 4,5-dihydro-1,2-oxazol-3 yl | SO₂Et | ¹H-NMR, CDCl₃, 400 MHz |
| | | | | 8,40 (bs, 1H), 8,10 (d, 1H), 7,94 (d, 1H), 5,16 (m, 1H), 3,72 (m, 1H), 3,35 (q, 2H), 3,25 (m, 2H), 2,90 (m, 2H), 1,42 (d, 6H), 1,29 (t, 3H) |
| 3-121 | Cl | CH₂O-Tetrahydrofuran-3-yl | SO₂Me | |
| 3-122 | Cl | CH₂O-Tetrahydrofuran-2-yl | SO₂Me | |
| 3-123 | Cl | SMe | SO₂Me | |
| 3-124 | Cl | F | SMe | |
| 3-125 | Cl | CH₂OCH₂-Tetrahydrofuran-2-yl | SO₂Me | ¹H-NMR, DMSO-d₆, 400 MHz |
| | | | | 11,51 (s, 1H), 8,11 (d, 1H), 7,92 (d, 1H), 5,1 (m, 2H) 3,99 (m, 1H), 3,71 (q, 1H), 3,66-3,53 (m, 3H), 3,39 (s, 3H), 3,25 (q, 1H), 1,93-1,71 (m, 3H), 1,54 (m, 1H), 1,31 (d, 6H) |
| 3-126 | Cl | CH₂OCH₂-Tetrahydrofuran-3-yl | SO₂Et | |
| 3-127 | Cl | O(CH₂)-5-Pyrrolidin-2-on | Cl | |
| 3-128 | Cl | SMe | Cl | |
| 3-129 | Cl | S(O)Me | SO₂Me | |
| 3-130 | Cl | CH₂O-Tetrahydrofuran-3-yl | SO₂Et | |
| 3-131 | Cl | O(CH₂)₂OMe | Cl | |
| 3-132 | Cl | O(CH₂)₂OMe | SO₂Me | |
| 3-133 | Cl | O(CH₂)₄OMe | SO₂Me | |
| 3-134 | Cl | O(CH₂)₄OMe | SO₂Et | |
| 3-135 | Cl | O(CH₂)₃OMe | SO₂Me | |
| 3-136 | Cl | O(CH₂)₃OMe | SO₂Et | |
| 3-137 | Cl | O(CH₂)₂OMe | SO₂Me | |
| 3-138 | Cl | O(CH₂)₂OMe | SO₂Et | |
| 3-139 | Cl | [1,4]Dioxan-2-yl-methoxy | SO₂Me | |
| 3-140 | Cl | [1,4]Dioxan-2-yl-methoxy | SO₂Et | |
| 3-141 | Cl | SO₂Me | Me | |
| 3-142 | Cl | SEt | Me | |
| 3-143 | Cl | SOEt | Me | |
| 3-144 | Cl | SO₂Et | Me | |
| 2-145 | Cl | 4,5-Dihydro-1,3-oxazol-3 yl | SO₂Me | |
| 3-146 | Cl | Cl | SO₂Me | |
| 3-147 | F | SMe | CF₃ | |
| 3-148 | F | S(O)Me | CF₃ | |
| 3-149 | OMe | SMe | CF₃ | |
| 3-150 | OMe | S(O)Me | CF₃ | |
| 3-151 | OMe | SO₂Me | CF₃ | |
| 3-152 | Et | NH(CH₂)₂OMe | SO₂Me | |
| 3-153 | Et | F | SO₂Me | |
| 3-154 | Et | SMe | CF₃ | |
| 3-155 | CF₃ | F | SO₂Me | |
| 3-156 | CF₃ | F | SO₂Et | |
| 3-157 | CF₃ | O(CH₂)₂OMe | SO₂Et | |
| 3-158 | CF₃ | O(CH₂)₃OMe | SO₂Et | |
| 3-159 | CF₃ | O(CH₂)₂OMe | SO₂Me | |
| 3-160 | CF₃ | O(CH₂)₃OMe | SO₂Me | |
| 3-161 | CF₃ | OCH₂CONMe₂ | SO₂Me | |
| 3-162 | CF₃ | OCH₂CONMe₂ | SO₂Et | |
| 3-163 | CF₃ | OCH₂CONMe₂ | Cl | |
| 3-164 | CF₃ | OCH₂CONMe₂ | Br | |
| 3-165 | CF₃ | OCH₂CONMe₂ | I | |
| 3-166 | CF₃ | OCH₂CONMe₂ | F | |
| 3-167 | CF₃ | O(CH₂)₂OMe | Cl | |
| 3-168 | CF₃ | O(CH₂)₃OMe | Cl | |
| 3-169 | CF₃ | O(CH₂)₂OMe | Br | |
| 3-170 | CF₃ | O(CH₂)₃OMe | Br | |
| 3-171 | CF₃ | O(CH₂)₂OMe | I | |
| 3-172 | CF₃ | O(CH₂)₃OMe | I | |
| 3-173 | CF₃ | O(CH₂)₂OMe | F | |
| 3-174 | CF₃ | O(CH₂)₃OMe | F | |
| 3-175 | CF₃ | [1,4]Dioxan-2-yl-methoxy | SO₂Me | |
| 3-176 | CF₃ | [1,4]Dioxan-2-yl-methoxy | SO₂Et | |
| 3-177 | CF₃ | [1,4]Dioxan-2-yl-methoxy | Cl | |
| 2-178 | CF₃ | [1,4]Dioxan-2-yl-methoxy | Br | |
| 3-179 | CF₃ | [1,4]Dioxan-2-yl-methoxy | I | |
| 3-180 | CF₃ | [1,4]Dioxan-2-yl-methoxy | F | |
| 3-181 | Br | OMe | Br | |
| 3-182 | Br | O(CH₂)₂OMe | Br | |
| 3-183 | Br | O(CH₂)₄OMe | SO₂Me | |
| 3-184 | Br | O(CH₂)₄OMe | SO₂Et | |
| 3-185 | Br | O(CH₂)₃OMe | SO₂Me | |
| 3-186 | Br | O(CH₂)₃OMe | SO₂Et | |
| 3-187 | Br | O(CH₂)₂OMe | SO₂Me | |
| 3-188 | Br | O(CH₂)₂OMe | SO₂Et | |
| 3-189 | Br | [1,4]Dioxan-2-yl-methoxy | SO₂Me | |
| 3-190 | Br | [1,4]Dioxan-2-yl-methoxy | SO₂Et | |
| 3-191 | Br | SMe | Me | |
| 3-192 | Br | SOMe | Me | |
| 3-193 | Br | SO₂Me | Me | |
| 3-194 | Br | SEt | Me | |
| 3-195 | Br | SOEt | Me | |
| 3-196 | Br | SO₂Et | Me | |
| 3-197 | I | O(CH₂)₄OMe | SO₂Me | |
| 3-198 | I | O(CH₂)₄OMe | SO₂Et | |
| 3-199 | I | O(CH₂)₃OMe | SO₂Me | |
| 3-200 | I | O(CH₂)₃OMe | SO₂Et | |
| 3-201 | I | O(CH₂)₂OMe | SO₂Me | |
| 3-202 | I | O(CH₂)₂OMe | SO₂Et | |
| 3-203 | I | [1,4]Dioxan-2-yl-methoxy | SO₂Me | |
| 3-204 | I | [1,4]Dioxan-2-yl-methoxy | SO₂Et | |
| 3-205 | I | SMe | Me | |
| 3-206 | I | SOMe | Me | |
| 3-207 | I | SO₂Me | Me | |
| 3-208 | I | SEt | Me | |
| 3-209 | I | SOEt | Me | |
| 3-210 | I | SO₂Et | Me | |
| 3-211 | CH₂SMe | OMe | SO₂Me | |
| 3-212 | CH₂OMe | OMe | SO₂Me | |
| 3-213 | CH₂O(C H₂)₂OMe | NH(CH₂)₂OEt | SO₂Me | |
| 3-214 | CH₂O(C H₂)₂OMe | NH(CH₂)₃OEt | SO₂Me | |
| 3-215 | CH₂O(C H₂)₃OMe | OMe | SO₂Me | |
| 3-216 | CH₂O(C H₂)₂OMe | NH(CH₂)₂OMe | SO₂Me | |
| 3-217 | CH₂O(C H₂)₂OMe | NH(CH₂)₃OMe | SO₂Me | |
| 3-218 | SO₂Me | NH₂ | CF₃ | |
| 3-219 | SO₂Me | F | CF₃ | |
| 3-220 | SO₂Me | NHEt | Cl | |
| 3-221 | SMe | SEt | F | |
| 3-222 | SMe | SMe | F | |
| 3-223 | Me | NH₂ | Cl | |
| 3-224 | Me | NH₂ | Br | |
| 3-225 | Me | NHMe | Cl | |
| 3-226 | Me | NHMe | Br | |
| 3-227 | Me | NMe₂ | Cl | |
| 3-228 | Me | NMe₂ | Br | |
| 3-227 | Me | NMe₂ | Cl | |
| 3-228 | Me | NMe₂ | Br | |
| 3-229 | NO₂ | O(CH₂)₂OMe | Me | |
| 3-230 | CF₃ | S(O)₂Et | SO₂Me | |
| 3-231 | CF₃ | S(O)₂Et | SO₂Et | |
| 3-232 | CF₃ | SCH₂CONMe₂ | SO₂Me | |
| 3-233 | CF₃ | SCH₂CONMe₂ | SO₂Et | |
| 3-234 | CF₃ | SCH₂COOH | SO₂Me | |
| 3-235 | CF₃ | SCH₂COOH | SO₂Et | |
| 3-236 | Me | SO₂-CH₂-CH₂-CH=CH₂ | CF₃ | |
| 3-237 | Cl | Me | SO₂Et | ¹H-NMR, DMSO-d₆, 400 MHz |
| | | | | 11,45 (s, 1H), 8,02 (d, 1H), 7,76 (d,1H), 3,43 (q,2H) 3,25 (m, 1H), 2,75 (s, 3H) 1,31 (d, 6H),1,15 (t, 3H) |
| 3-238 | CF₃ | SEt | SO₂Me | |
| 3-239 | OMe | NO2 | Cl | |
| 3-240 | OMe | NH(CO)i-Pr | Cl | |
| 3-241 | OMe | NH(CO)CH2Ph | Cl | |
| 3-242 | CF₃ | SEt | SO₂Et | |
| 3-243 | CF₃ | S(O)Et | SO₂Me | |
| 3-244 | Cl | Me | Cl | |
| 3-245 | Me | 3,5-Dimethyl-pyrazol-1-yl | SO₂Me | |
| 3-247 | Me | 1,2,3-Triazol-1-yl | SO₂Me | |
| 3-248 | Me | Me | SMe | |
| 3-249 | Me | Pyrolidin-2-on-1-yl | SO₂Me | |
| 3-250 | CF₃ | S(O)Et | SO₂Et | |
| 3-251 | Cl | Pyrazol-1-yl | SO₂Me | |
| 3-252 | Me | 3-Methyl-pyrazol-1-yl | SO₂Me | |
| 3-253 | Cl | CH₂-N(Et)OMe | SO₂Me | |
| 3-254 | Me | Me | Cl | |
| 3-255 | OH | Cl | Cl | |
| 3-256 | Me | 1,2,4-Triazol-1-yl | SO₂Me | |
| 3-257 | Me | 4-Methoxy-pyrazol-1-yl | SO₂Me | |
| 3-258 | Me | 1,2,4-triazol-1-yl | CF₃ | |
| 3-259 | Me | Tetrahydro-pyrimidin-2(1 H)-one-1-yl | SO₂Me | |
| 3-260 | Me | NH-(CH₂)₂-O(CO)Et | SO₂Me | |
| 3-261 | Me | NH-iPr | SO₂Me | |
| 3-262 | Cl | NH-CH₂-(CO)NHEt | Cl | |
| 3-263 | Me | NH-CH₂₋(CO)NMe2 | SO₂Me | |
| 3-264 | Me | NH-CH₂-furan-2-yl | SO₂Me | |
| 3-265 | Me | NH-CH₂-(CO)NHEt | SO₂Me | |
| 3-266 | Me | F | SO₂Me | |
| 3-267 | F | SO₂Me | SO₂Me | |
| 3-268 | Cl | (4-Cyclopropyl-3-methyl-5-oxo-4,5-dihydro-1H-1,2,4-triazol-1-yl)-methyl | Cl | |
| 3-269 | Cl | [4-Methyl-5-oxo-3-(2,2,2-trifluorethoxy)-4,5-dihydro-1H-1,2,4-triazol-1-yl]-methyl | Cl | |
| 3-270 | Cl | (3-Isopropoxy-4-methyl-5-oxo-4,5-dihydro-1H-1,2,4-triazol-1-yl]-methyl | Cl | |
| 3-271 | Cl | (4-Methyl-5-oxo-4,5-dihydro-1H-1,2,4-triazol-1-yl)-methyl | Cl | |
| 3-272 | Me | Cl | SO₂Et | |
| 3-273 | SO₂Me | F | Cl | |
| 3-274 | Me | 1,2,3-Triazol-1-yl | SO₂Me | |
| 3-275 | Me | Isobutyl(methyl)carbamoylamino | SO₂Me | |
| 3-276 | Me | 3-Oxo-morpholin-4-yl | SO₂Me | |
| 3-277 | OMe | [Ethyl(methylsulfonyl)a mino]methyl | Cl | |
| 3-278 | F | SO₂Me | CF₃ | |
| 3-279 | OMe | Benzoylamino | Cl | |
| 3-280 | OMe | Cyclopropylcarbonylamino | Cl | |
| 3-281 | OMe | Propionyllamino | Cl | |
| 3-282 | NO₂ | SO₂Me | SO₂Me | |
| 3-283 | NO₂ | SO₂Me | Cl | |
| 3-284 | NO₂ | SOMe | SO₂Me | |
| 3-285 | NO₂ | SOMe | Br | |
| 3-286 | NO₂ | SOMe | Cl | |
| 3-287 | NO₂ | SMe | SO₂Me | |
| 3-288 | NO₂ | SMe | Br | |
| 3-289 | NO₂ | SMe | Cl | |
| 3-290 | Cl | CH₂OCH(CH₃)₂ | SO₂Et | |
| 3-291 | Cl | CH₂OEt | SO₂Et | |
| 3-292 | Cl | CH₂OMe | SO₂Et | |
| 3-293 | Cl | CH₂OCH₂C₂F₅ | SO₂Me | |
| 3-294 | Cl | CH₂OCH₂CHF | SO₂Me | |
| 3-295 | Cl | CH₂OCH₂CCH | SO₂Et | |
| 3-296 | Cl | CH₂₀OC₂H₄OMe | SO₂Me | |
| 3-297 | Cl | CH₂(OC₂H₄)₂OMe | SO₂Me | |
| 3-298 | Cl | 5-Ethoxymethyl-4,5-dihydro-1,2-oxazol-3yl | SO₂Et | |
| 3-299 | Cl | 5-Methoxymethyl-4,5-dihydro-1,2-oxazol-3yl | SO₂Et | |
| 3-300 | Et | SOMe | CF₃ | |
| 3-301 | iPr | SMe | CF₃ | |
| 3-302 | Et | SMe | CF₃ | |
| 3-303 | Et | SO₂Me | CF₃ | |
| 3-304 | cPr | SOMe | CF₃ | |
| 3-305 | CH=CH₂ | SMe | CF₃ | |
| 3-306 | Et | SMe | Cl | |
| 3-307 | Et | SO₂Me | Cl | |
| 3-308 | Cl | NMe₂ | Cl | |
| 3-309 | CH₂O(C H₂)₂OMe | NH(CH₂)₃OMe | SO₂Me | |
| 3-313 | Me | SO₂(CH₂)₂OMe | CF₃ | |
| 3-314 | Me | SOEt | SO₂Me | |
| 3-315 | Me | SO₂Et | SO₂Me | |
| 3-316 | Me | SMe | 1,2,4-triazol-1-yl | |
| 3-317 | OEt | SMe | CF₃ | |
| 3-318 | Me | S(CH₂)₂OMe | CF₃ | |
| 3-319 | Me | SOMe | 1,2,4-triazol-1-yl | |
| 3-320 | OEt | SOMe | CF₃ | |
| 3-321 | Me | SO(CH₂)₂OMe | CF₃ | |
| 3-322 | Me | SCH₂CCMe | SO₂Me | |
| 3-323 | Me | S-c-Pen | SO₂Me | |
| 3-324 | OMe | SMe | OMe | |
| 3-325 | Me | SCH₂CH=CHCH₃ | SO₂Me | |
| 3-326 | Me | SOCH₂CCMe | SO₂Me | |
| 3-327 | Me | SO₃₋c-Pen | SO₂Me | |
| 3-328 | Me | SO-c-Pen | SO₂Me | |
| 3-329 | Me | S(CH₂)₃Cl | SO₂Me | |
| 3-330 | Me | SCH₂(4-F-Ph) | SO₂Me | |
| 3-331 | Me | SO₂CH₂CCMe | SO₂Me | |
| 3-332 | Me | SO₂CH₂CH=CHCH₃ | SO₂Me | |
| 3-333 | Me | SOCH₂CH=CHCH₃ | SO₂Me | |
| 3-334 | Me | SOCH₂-Epoxy-Me | SO₂Me | |
| 3-335 | Me | SO₂(CH₂)₃Cl | SO₂Me | |
| 3-336 | Me | SO(CH₂)₃Cl | SO₂Me | |
| 3-337 | Me | SOCH₂(4-F-Ph) | SO₂Me | |
| 3-338 | Me | SO₂CH₂(4-F-Ph) | SO₂Me | |
| 3-339 | Me | SO₂Me | C₂F₅ | |
| 3-340 | O(CH₂)₂ OMe | SMe | CF₃ | |
| 3-341 | O(CH₂)₂ OMe | SO₂Me | CF₃ | |
| 3-342 | O(CH₂)₂ OMe | SOMe | CF₃ | |
| 3-343 | Me | S(CH₂)₂OCH₂CF₃ | SO₂Me | |
| 3-344 | Me | SO(CH₂)₂OCH₂CF₃ | SO₂Me | |
| 3-345 | Me | SO₂(CH₂)₂OCH₂CF₃ | SO₂Me | |
| 3-346 | OEt | SEt | CF₃ | |
| 3-347 | O-CH₂-c-Pr | SMe | CF₃ | |
| 3-348 | OMe | SEt | CF₃ | |
| 3-349 | OMe | SO₂Et | CF₃ | |
| 3-350 | OMe | SOEt | CF₃ | |
| 3-351 | OEt | SO₂Et | CF₃ | |
| 3-352 | OEt | SOEt | CF₃ | |
| 3-353 | O-CH₂₋c-Pr | SOMe | CF₃ | |
| 3-354 | O-CH₂₋c-Pr | SO₂Me | CF₃ | |
| 3-355 | Me | SEt | SO₂Me | |

**Tabelle 4: Erfindungsgemäße Verbindungen der allgemeinen Formel (I), worin R für Propyl steht**

| | | | | |
|---|---|---|---|---|
| 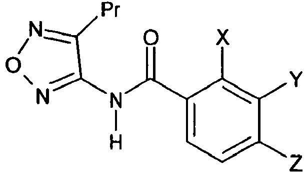 | | | | |

| **Nr.** | **X** | **Y** | **Z** | **Physikalische Daten** |
|---|---|---|---|---|
| 4-1 | CF₃ | OCH₂CON(Me)Et | SO₂Me | |
| 4-2 | CF₃ | OCH₂CON(Me)Et | SO₂Et | |
| 4-4 | CF₃ | 2-(1 H-Pyrazol-1-yl)ethoxyl | SO₂Me | |
| 4-5 | CF₃ | 2-(1H-Pyrazol-1-yl)ethoxyl | SO₂Et | |
| 4-6 | CF₃ | Tetrahydrofuran-2-yl-methoxy | SO₂Me | |
| 4-7 | CF₃ | Tetrahydrofuran-2-yl-methoxy | SO₂Et | |
| 4-8 | CF₃ | OH | SO₂Me | |
| 4-9 | CF₃ | OH | SO₂Et | |
| 4-10 | CF₃ | SH | SO₂Me | |
| 4-11 | CF₃ | SH | SO₂Et | |
| 4-15 | CF₃ | SMe | SO₂Me | |
| 4-16 | CF₃ | SMe | SO₂Et | |
| 4-17 | CF₃ | S(O)Me | SO₂Me | |
| 4-24 | CF₃ | S(O)Me | SO₂Et | |
| 4-25 | CF₃ | S(O)₂Me | SO₂Me | |
| 4-26 | CF₃ | S(O)₂Me | SO₂Et | |
| 4-27 | CF₃ | 2-[(Methylsulfonyl) amino]ethoxy | SO₂Me | |
| 4-28 | CF₃ | 2-[(Methylsulfonyl) amino]ethyl}sulfanyl | SO₂Me | |
| 4-29 | CF₃ | 2-[(Methylsulfonyl) amino]ethyl}sulfanyl | SO₂Et | |
| 4-30 | NO₂ | O(CH₂)₂OMe | OMe | |
| 4-31 | NO₂ | OMe | Me | |
| 4-32 | NO₂ | NH₂ | OMe | |
| 4-33 | NO₂ | NH₂ | SO₂Et | |
| 4-34 | NO₂ | NH₂ | Cl | |
| 4-35 | NO₂ | NHMe | Cl | |
| 4-36 | NO₂ | NMe₂ | Cl | |
| 4-37 | NO₂ | NH₂ | Br | |
| 4-38 | NO₂ | NHMe | Br | |
| 4-39 | NO₂ | NMe₂ | Br | |
| 4-40 | NO₂ | NH₂ | F | |
| 4-41 | NO₂ | NHMe | F | |
| 4-42 | NO₂ | NMe₂ | F | |
| 4-43 | NO₂ | NH₂ | SO₂Me | |
| 4-44 | NO₂ | NHMe | SO₂Me | |
| 4-45 | NO₂ | NMe₂ | SO₂Me | |
| 4-46 | NO₂ | NH₂ | 1H-1,2,4-triazol-1-yl | |
| 4-47 | NO₂ | NHMe | 1H-1,2,4-triazol-1-yl | |
| 4-48 | NO₂ | NMe₂ | 1H-1,2,4-triazol-1-yl | |
| 4-49 | Me | F | F | |
| 4-50 | Me | F | Cl | |
| 4-51 | Me | SMe | CF₃ | |
| 4-52 | Me | Cl | SO₂Me | |
| 4-53 | Me | SO₂Me | SO₂Me | ¹H-NMR, DMSO-d₆, 400 MHz |
| | | | | 11,48 (s, 1H), 8,28 (d, 1H), 8,08 (d, 1H), 3,61 (s, 3H), 3,58 (s, 3H), 2,79 (t, 2H), 2,70 (s, 3H), 1,71 (m, 2H), 0,94 (t, 3H) |
| 4-54 | Me | SO₂Me | CF₃ | ¹H-NMR, CDCl₃, 400 MHz |
| | | | | 8,59 (bs, 1H), 7,87 (d, 1H), 7,76 (d, 1H), 3,20 (s, 3H), 2,83 (t, 2H), 2,78 (s, 3H), 1,83 (m, 2H), 1,05 (t, 3H) |
| 4-55 | Me | Cl | CF₃ | |
| 4-56 | Me | S(O)Me | CF₃ | |
| 4-57 | Me | SEt | OMe | |
| 4-58 | Me | NMe₂ | SO₂Me | |
| 4-59 | Me | NH(CH₂)₂OMe | SO₂Me | |
| 4-60 | Me | O(CH₂)₄OMe | SO₂Me | |
| 4-61 | Me | NH₂ | SO₂Me | |
| 4-62 | Me | O(CH₂)₂-O(3,5-Di-methoxypyrimidin-2-yl | SO₂Me | |
| 4-63 | Me | O(CH₂)₂-O-NMe₂ | Cl | |
| 4-64 | Me | O(CH₂)₂-NH(CO)NMe₂ | Cl | |
| 4-65 | Me | O(CH₂)-5-Pyrrolidin-2-on | Br | |
| 4-66 | Me | O(CH₂)₂-NH(CO)NHCO₂Et | Cl | |
| 4-67 | Me | O(CH₂)-(CO)NEt₂ | Br | |
| 4-68 | Me | O(CH₂)-5-2,4-Dimethyl-2,4-dihydro-3H-1,2,4-triazol-3-on | Cl | |
| 4-69 | Me | O(CH₂)-3,5-Dimethyl-1,2-oxazol-4-yl | Cl | |
| 4-70 | Me | O(CH₂)₂-NHCO₂Me | Cl | |
| 4-71 | Me | 4,5-Dihydro-1,2-oxazol-3 yl | SO₂Me | |
| 4-72 | Me | Me | SO₂Me | |
| 4-73 | Me | OH | SO₂Me | |
| 4-74 | Me | O-CH₂-NHSO₂cPr | Cl | |
| 4-75 | Me | O(CH₂)₂NHSO2Me | SO₂Me | |
| 4-76 | Me | S(O)Me | SO₂Me | |
| 4-77 | Me | SMe | SO₂Me | |
| 4-78 | Me | SMe | OMe | |
| 4-79 | Me | S(O)Me | OMe | |
| 4-80 | Me | SO₂Me | OMe | |
| 4-81 | Me | SMe | Cl | |
| 4-82 | Me | S(O)Me | Cl | |
| 4-83 | Me | SO₂Me | Cl | |
| 4-84 | Me | [1,4]Dioxan-2-yl-methoxy | SO₂Me | |
| 4-85 | Me | [1,4]Dioxan-2-yl-methoxy | SO₂Et | |
| 4-86 | Me | O(CH₂)₄OMe | SO₂Et | |
| 4-87 | Me | O(CH₂)₃OMe | SO₂Me | |
| 4-88 | Me | O(CH₂)₃OMe | SO₂Et | |
| 4-89 | Me | O(CH₂)₂OMe | SO₂Me | |
| 4-90 | Me | O(CH₂)₂OMe | SO₂Et | |
| 4-91 | Me | S(O)Me | SO₂Me | |
| 4-92 | Me | SMe | SO₂Me | |
| 4-93 | Me | SMe | OMe | |
| 4-94 | Me | S(O)Me | OMe | |
| 4-95 | Me | SO₂Me | OMe | |
| 4-96 | Me | SMe | Cl | |
| 4-97 | Me | S(O)Me | Cl | |
| 4-98 | Me | SO₂Me | Cl | |
| 4-99 | Me | SMe | Br | |
| 4-100 | Me | SOMe | Br | |
| 4-101 | Me | SO₂Me | Br | |
| 4-102 | Me | SMe | I | |
| 4-103 | Me | SOMe | I | |
| 4-104 | Me | SO₂Me | I | |
| 4-105 | Me | SEt | Cl | |
| 4-106 | Me | SOEt | Cl | |
| 4-107 | Me | SO₂Et | Cl | |
| 4-108 | Me | SEt | Br | |
| 4-109 | Me | SOEt | Br | |
| 4-110 | Me | SO₂Et | Br | |
| 4-111 | Me | SEt | I | |
| 4-112 | Me | SOEt | I | |
| 4-113 | Me | SO₂Et | I | |
| 4-114 | Me | SEt | F | |
| 4-115 | Me | SOEt | F | |
| 4-116 | Me | SO₂Et | F | |
| 4-117 | Cl | OCH₂(CO)NMe₂ | Cl | |
| 4-118 | Cl | Cl | SO₂Me | |
| 4-119 | Cl | CH₂OCH₂CF₃ | SO₂Me | ¹H-NMR, CDCl₃, 400 MHz |
| | | | | 8,40 (bs, 1H), 8,05 (d, 1H), 7,76 (d, 1 H), 5,33 (s, 2H), 4,05 (q, 2H), 3,22 (s, 3H), 2,86 (t, 2H), 1,84 (m, 2H), 1,05 (t, 3H) |
| 4-120 | Cl | 5-Cyanomethyl- 4,5-dihydro-1,2-oxazol-3yl | SO₂Et | ¹H-NMR, CDCl₃, 400 MHz |
| | | | | 8,19 (bs, 1H), 8.13 (d. 1H), 7,95 (d, 1H), 5,17 (m, 1H), 3,73 (m, 1H), 3,36 (q, 2H), 3,25 (m, 1H), 2,86 (m, 4H), 1,82 (m, 2H), 1,30 (t, 3H), 1,05 (t, 3H) |
| 4-121 | Cl | CH₂O-Tetrahydrofuran-3-yl | SO₂Me | |
| 4-122 | Cl | CH₂O-Tetrahydrofuran-2-yl | SO₂Me | |
| 4-123 | Cl | SMe | SO₂Me | |
| 4-124 | Cl | F | SMe | |
| 4-125 | Cl | CH₂OCH₂-Tetrahydrofuran-2-yl | SO₂Me | |
| 4-126 | Cl | CH₂OCH₂-Tetrahydrofuran-3-yl | SO₂Et | |
| 4-127 | Cl | O(CH₂)-5-Pyrrolidin-2-on | Cl | |
| 4-128 | Cl | SMe | Cl | |
| 4-129 | Cl | S(O)Me | SO₂Me | |
| 4-130 | Cl | CH₂O-Tetrahydrofuran-3-yl | SO₂Et | |
| 4-131 | Cl | O(CH₂)₂OMe | Cl | |
| 4-132 | Cl | O(CH₂)₂OMe | SO₂Me | |
| 4-133 | Cl | O(CH₂)₄OMe | SO₂Me | |
| 4-134 | Cl | O(CH₂)₄OMe | SO₂Et | |
| 4-135 | Cl | O(CH₂)₃OMe | SO₂Me | |
| 4-136 | Cl | O(CH₂)₃OMe | SO₂Et | |
| 4-137 | Cl | O(CH₂)₂OMe | SO₂Me | |
| 4-138 | Cl | O(CH₂)₂OMe | SO₂Et | |
| 4-139 | Cl | [1,4]Dioxan-2-yl-methoxy | SO₂Me | |
| 4-140 | Cl | [1,4]Dioxan-2-yl-methoxy | SO₂Et | |
| 4-141 | Cl | SO₂Me | Me | |
| 4-142 | Cl | SEt | Me | |
| 4-143 | Cl | SOEt | Me | |
| 4-144 | Cl | SO₂Et | Me | |
| 2-145 | Cl | 4,5-Dihydro-1,4-oxazol-3 yl | SO₂Me | |
| 4-146 | Cl | Cl | SO₂Me | |
| 4-147 | F | SMe | CF₃ | |
| 4-148 | F | S(O)Me | CF₃ | |
| 4-149 | OMe | SMe | CF₃ | |
| 4-150 | OMe | S(O)Me | CF₃ | |
| 4-151 | OMe | SO₂Me | CF₃ | |
| 4-152 | Et | NH(CH₂)₂OMe | SO₂Me | |
| 4-153 | Et | F | SO₂Me | |
| 4-154 | Et | SMe | CF₃ | |
| 4-155 | CF₃ | F | SO₂Me | |
| 4-156 | CF₃ | F | SO₂Et | |
| 4-157 | CF₃ | O(CH₂)₂OMe | SO₂Et | |
| 4-158 | CF₃ | O(CH₂)₃OMe | SO₂Et | |
| 4-159 | CF₃ | O(CH₂)₂OMe | SO₂Me | |
| 4-160 | CF₃ | O(CH₂)₃OMe | SO₂Me | |
| 4-161 | CF₃ | OCH₂CONMe₂ | SO₂Me | |
| 4-162 | CF₃ | OCH₂CONMe₂ | SO₂Et | |
| 4-163 | CF₃ | OCH₂CONMe₂ | Cl | |
| 4-164 | CF₃ | OCH₂CONMe₂ | Br | |
| 4-165 | CF₃ | OCH₂CONMe₂ | I | |
| 4-166 | CF₃ | OCH₂CONMe₂ | F | |
| 4-167 | CF₃ | O(CH₂)₂OMe | Cl | |
| 4-168 | CF₃ | O(CH₂)₃OMe | Cl | |
| 4-169 | CF₃ | O(CH₂)₂OMe | Br | |
| 4-170 | CF₃ | O(CH₂)₃OMe | Br | |
| 4-171 | CF₃ | O(CH₂)₂OMe | I | |
| 4-172 | CF₃ | O(CH₂)₃OMe | I | |
| 4-173 | CF₃ | O(CH₂)₂OMe | F | |
| 4-174 | CF₃ | O(CH₂)₃OMe | F | |
| 4-175 | CF₃ | [1,4]Dioxan-2-yl-methoxy | SO₂Me | |
| 4-176 | CF₃ | [1,4]Dioxan-2-yl-methoxy | SO₂Et | |
| 4-177 | CF₃ | [1,4]Dioxan-2-yl-methoxy | Cl | |
| 2-178 | CF₃ | [1,4]Dioxan-2-yl-methoxy | Br | |
| 4-179 | CF₃ | [1,4]Dioxan-2-yl-methoxy | I | |
| 4-180 | CF₃ | [1,4]Dioxan-2-yl-methoxy | F | |
| 4-181 | Br | OMe | Br | |
| 4-182 | Br | O(CH₂)₂OMe | Br | |
| 4-183 | Br | O(CH₂)₄OMe | SO₂Me | |
| 4-184 | Br | O(CH₂)₄OMe | SO₂Et | |
| 4-185 | Br | O(CH₂)₃OMe | SO₂Me | |
| 4-186 | Br | O(CH₂)₃OMe | SO₂Et | |
| 4-187 | Br | O(CH₂)₂OMe | SO₂Me | |
| 4-188 | Br | O(CH₂)₂OMe | SO₂Et | |
| 4-189 | Br | [1,4]Dioxan-2-yl-methoxy | SO₂Me | |
| 4-190 | Br | [1,4]Dioxan-2-yl-methoxy | SO₂Et | |
| 4-191 | Br | SMe | Me | |
| 4-192 | Br | SOMe | Me | |
| 4-193 | Br | SO₂Me | Me | |
| 4-194 | Br | SEt | Me | |
| 4-195 | Br | SOEt | Me | |
| 4-196 | Br | SO₂Et | Me | |
| 4-197 | I | O(CH₂)₄OMe | SO₂Me | |
| 4-198 | I | O(CH₂)₄OMe | SO₂Et | |
| 4-199 | I | O(CH₂)₃OMe | SO₂Me | |
| 4-200 | I | O(CH₂)₃OMe | SO₂Et | |
| 4-201 | I | O(CH₂)₂OMe | SO₂Me | |
| 4-202 | I | O(CH₂)₂OMe | SO₂Et | |
| 4-203 | I | [1,4]Dioxan-2-yl-methoxy | SO₂Me | |
| 4-204 | I | [1,4]Dioxan-2-yl-methoxy | SO₂Et | |
| 4-205 | I | SMe | Me | |
| 4-206 | I | SOMe | Me | |
| 4-207 | I | SO₂Me | Me | |
| 4-208 | I | SEt | Me | |
| 4-209 | I | SOEt | Me | |
| 4-210 | I | SO₂Et | Me | |
| 4-211 | CH₂SMe | OMe | SO₂Me | |
| 4-212 | CH₂OMe | OMe | SO₂Me | |
| 4-213 | CH₂O(C H₂)₂OMe | NH(CH₂)₂OEt | SO₂Me | |
| 4-214 | CH₂O(C H₂)₂OMe | NH(CH₂)₃OEt | SO₂Me | |
| 4-215 | CH₂O(C H₂)₃OMe | OMe | SO₂Me | |
| 4-216 | CH₂O(C H₂)₂OMe | NH(CH₂)₂OMe | SO₂Me | |
| 4-217 | CH₂O(C H₂)₂OMe | NH(CH₂)₃OMe | SO₂Me | |
| 4-218 | SO₂Me | NH₂ | CF₃ | |
| 4-219 | SO₂Me | F | CF₃ | |
| 4-220 | SO₂Me | NHEt | Cl | |
| 4-221 | SMe | SEt | F | |
| 4-222 | SMe | SMe | F | |
| 4-223 | Me | NH₂ | Cl | |
| 4-224 | Me | NH₂ | Br | |
| 4-225 | Me | NHMe | Cl | |
| 4-226 | Me | NHMe | Br | |
| 4-227 | Me | NMe₂ | Cl | |
| 4-228 | Me | NMe₂ | Br | |
| 4-227 | Me | NMe₂ | Cl | |
| 4-228 | Me | NMe₂ | Br | |
| 4-229 | NO₂ | O(CH₂)₂OMe | Me | |
| 4-230 | CF₃ | S(O)₂Et | SO₂Me | |
| 4-231 | CF₃ | S(O)₂Et | SO₂Et | |
| 4-232 | CF₃ | SCH₂CONMe₂ | SO₂Me | |
| 4-233 | CF₃ | SCH₂CONMe₂ | SO₂Et | |
| 4-234 | CF₃ | SCH₂COOH | SO₂Me | |
| 4-235 | CF₃ | SCH₂COOH | SO₂Et | |
| 4-236 | Me | SO₂-CH₂-CH₂-CH=CH₂ | CF₃ | |
| 4-237 | Cl | Me | SO₂Et | |
| 4-238 | CF₃ | SEt | SO₂Me | |
| 4-239 | OMe | NO2 | Cl | |
| 4-240 | OMe | NH(CO)i-Pr | Cl | |
| 4-241 | OMe | NH(CO)CH2Ph | Cl | |
| 4-242 | CF₃ | SEt | SO₂Et | |
| 4-243 | CF₃ | S(O)Et | SO₂Me | |
| 4-244 | Cl | Me | Cl | |
| 4-245 | Me | 3,5-Dimethyl-pyrazol-1-yl | SO₂Me | |
| 4-247 | Me | 1,2,3-Triazol-1-yl | SO₂Me | |
| 4-248 | Me | Me | SMe | |
| 4-249 | Me | Pyrolidin-2-on-1-yl | SO₂Me | |
| 4-250 | CF₃ | S(O)Et | SO₂Et | |
| 4-251 | Cl | Pyrazol-1-yl | SO₂Me | |
| 4-252 | Me | 3-Methyl-pyrazol-1-yl | SO₂Me | |
| 4-253 | Cl | CH₂-N(Et)OMe | SO₂Me | |
| 4-254 | Me | Me | Cl | |
| 4-255 | OH | Cl | Cl | |
| 4-256 | Me | 1,2,4-Triazol-1-yl | SO₂Me | |
| 4-257 | Me | 4-Methoxy-pyrazol-1-yl | SO₂Me | |
| 4-258 | Me | 1,2,4-triazol-1-yl | CF₃ | |
| 4-259 | Me | Tetrahydro-pyrimidin-2(1 H)-one-1-yl | SO₂Me | |
| 4-260 | Me | NH-(CH₂)₂-O(CO)Et | SO₂Me | |
| 4-261 | Me | NH-iPr | SO₂Me | |
| 4-262 | Cl | NH-CH₂-(CO)NHEt | Cl | |
| 4-263 | Me | NH-CH₂-(CO)NMe2 | SO₂Me | |
| 4-264 | Me | NH-CH₂-furan-2-yl | SO₂Me | |
| 4-265 | Me | NH-CH₂-(CO)NHEt | SO₂Me | |
| 4-266 | Me | F | SO₂Me | |
| 4-267 | F | SO₂Me | SO₂Me | |
| 4-268 | Cl | (4-Cyclopropyl-3-methyl-5-oxo-4,5-dihydro-1H-1,2,4-triazol-1-yl)-methyl | Cl | |
| 4-269 | Cl | [4-Methyl-5-oxo-3-(2,2,2-trifluorethoxy)-4,5-dihydro-1H-1,2,4-triazol-1-yl]-methyl | Cl | |
| 4-270 | Cl | (3-Isopropoxy-4-methyl-5-oxo-4,5-dihydro-1H-1,2,4-triazol-1-yl]-methyl | Cl | |
| 4-271 | Cl | (4-Methyl-5-oxo-4,5-dihydro-1H-1,2,4-triazol-1-yl)-methyl | Cl | |
| 4-272 | Me | Cl | SO₂Et | |
| 4-273 | SO₂Me | F | Cl | |
| 4-274 | Me | 1,2,3-Triazol-1-yl | SO₂Me | |
| 4-275 | Me | Isobutyl(methyl)carbamoylamino | SO₂Me | |
| 4-276 | Me | 3-Oxo-morpholin-4-yl | SO₂Me | |
| 4-277 | OMe | [Ethyl(methylsulfonyl)a mino]methyl | Cl | |
| 4-278 | F | SO₂Me | CF₃ | |
| 4-279 | OMe | Benzoylamino | Cl | |
| 4-280 | OMe | Cyclopropylcarbonylamino | Cl | |
| 4-281 | OMe | Propionyllamino | Cl | |
| 4-282 | NO₂ | SO₂Me | SO₂Me | |
| 4-283 | NO₂ | SO₂Me | Cl | |
| 4-284 | NO₂ | SOMe | SO₂Me | |
| 4-285 | NO₂ | SOMe | Br | |
| 4-286 | NO₂ | SOMe | Cl | |
| 4-287 | NO₂ | SMe | SO₂Me | |
| 4-288 | NO₂ | SMe | Br | |
| 4-289 | NO₂ | SMe | Cl | |
| 4-290 | Cl | CH₂OCH(CH₃)₂ | SO₂Et | |
| 4-291 | Cl | CH₂OEt | SO₂Et | |
| 4-292 | Cl | CH₂OMe | SO₂Et | |
| 4-293 | Cl | CH₂OCH₂C₂F₅ | SO₂Me | |
| 4-294 | Cl | CH₂OCH₂CHF₂ | SO₂Me | |
| 4-295 | Cl | CH₂OCH₂CCH | SO₂Et | |
| 4-296 | Cl | CH₂OC₂H₄OMe | SO₂Me | |
| 4-297 | Cl | CH₂(OC₂H₄)₂OMe | SO₂Me | |
| 4-298 | Cl | 5-Ethoxymethyl- 4,5-dihydro-1,2-oxazol-3 yl | SO₂Et | |
| 4-299 | Cl | 5-Methoxymethyl- 4,5-dihydro-1,2-oxazol-3 yl | SO₂Et | |
| 4-300 | Et | SOMe | CF₃ | |
| 4-301 | iPr | SMe | CF₃ | |
| 4-302 | Et | SMe | CF₃ | |
| 4-303 | Et | SO₂Me | CF₃ | |
| 4-304 | cPr | SOMe | CF₃ | |
| 4-305 | CH=CH₂ | SMe | CF₃ | |
| 4-306 | Et | SMe | Cl | |
| 4-307 | Et | SO₂Me | Cl | |
| 4-308 | Cl | NMe₂ | Cl | |
| 4-309 | CH₂O(C H₂)₂OMe | NH(CH₂)₃OMe | SO₂Me | |
| 4-313 | Me | SO₂(CH₂)₂OMe | CF₃ | |
| 4-314 | Me | SOEt | SO₂Me | |
| 4-315 | Me | SO₂Et | SO₂Me | |
| 4-316 | Me | SMe | 1,2,4-triazol-1-yl | |
| 4-317 | OEt | SMe | CF₃ | |
| 4-318 | Me | S(CH₂)₂OMe | CF₃ | |
| 4-319 | Me | SOMe | 1,2,4-triazol-1-yl | |
| 4-320 | OEt | SOMe | CF₃ | |
| 4-321 | Me | SO(CH₂)₂OMe | CF₃ | |
| 4-322 | Me | SCH₂CCMe | SO₂Me | |
| 4-323 | Me | S-c-Pen | SO₂Me | |
| 4-324 | OMe | SMe | OMe | |
| 4-325 | Me | SCH₂CH=CHCH₃ | SO₂Me | |
| 4-326 | Me | SOCH₂CCMe | SO₂Me | |
| 4-327 | Me | SO₄₋c-Pen | SO₂Me | |
| 4-328 | Me | SO-c-Pen | SO₂Me | |
| 4-329 | Me | S(CH₂)₃Cl | SO₂Me | |
| 4-330 | Me | SCH₂(4-F-Ph) | SO₂Me | |
| 4-331 | Me | SO₂CH₂CCMe | SO₂Me | |
| 4-332 | Me | SO₂CH₂CH=CHCH₃ | SO₂Me | |
| 4-333 | Me | SOCH₂CH=CHCH₃ | SO₂Me | |
| 4-334 | Me | SOCH₂-Epoxy-Me | SO₂Me | |
| 4-335 | Me | SO₂(CH₂)₃Cl | SO₂Me | |
| 4-336 | Me | SO(CH₂)₃Cl | SO₂Me | |
| 4-337 | Me | SOCH₂(4-F-Ph) | SO₂Me | |
| 4-338 | Me | SO₂CH₂(4-F-Ph) | SO₂Me | |
| 4-339 | Me | SO₂Me | C₂F₅ | |
| 4-340 | O(CH₂)₂ OMe | SMe | CF₃ | |
| 4-341 | O(CH₂)₂ OMe | SO₂Me | CF₃ | |
| 4-342 | O(CH₂)₂ OMe | SOMe | CF₃ | |
| 4-343 | Me | S(CH₂)₂OCH₂CF₃ | SO₂Me | |
| 4-344 | Me | SO(CH₂)₂OCH₂CF₃ | SO₂Me | |
| 4-345 | Me | SO₂(CH₂)₂OCH₂CF₃ | SO₂Me | |
| 4-346 | OEt | SEt | CF₃ | |
| 4-347 | O-CH₂-c-Pr | SMe | CF₃ | |
| 4-348 | OMe | SEt | CF₃ | |
| 4-349 | OMe | SO₂Et | CF₃ | |
| 4-350 | OMe | SOEt | CF₃ | |
| 4-351 | OEt | SO₂Et | CF₃ | |
| 4-352 | OEt | SOEt | CF₃ | |
| 4-353 | O-CH₂₋c-Pr | SOMe | CF₃ | |
| 4-354 | O-CH₂₋c-Pr | SO₂Me | CF₃ | |
| 4-355 | Me | SEt | SO₂Me | |

**Tabelle 5: Erfindungsgemäße Verbindungen der allgemeinen Formel (I), worin R für Chlormethyl steht**

| | | | | |
|---|---|---|---|---|
| 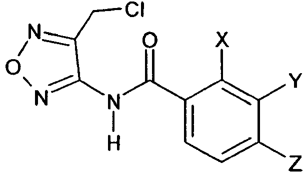 | | | | |

| **Nr.** | **X** | **Y** | **Z** | **Physikalische Daten** |
|---|---|---|---|---|
| 5-1 | CF₃ | OCH₂CON(Me)Et | SO₂Me | |
| 5-2 | CF₃ | OCH₂CON(Me)Et | SO₂Et | |
| 5-4 | CF₃ | 2-(1H-Pyrazol-1-yl)ethoxyl | SO₂Me | |
| 5-5 | CF₃ | 2-(1 H-Pyrazol-1-yl)ethoxyl | SO₂Et | |
| 5-6 | CF₃ | Tetrahydrofuran-2-yl-methoxy | SO₂Me | |
| 5-7 | CF₃ | Tetrahydrofuran-2-yl-methoxy | SO₂Et | |
| 5-8 | CF₃ | OH | SO₂Me | |
| 5-9 | CF₃ | OH | SO₂Et | |
| 5-10 | CF₃ | SH | SO₂Me | |
| 5-11 | CF₃ | SH | SO₂Et | |
| 5-15 | CF₃ | SMe | SO₂Me | |
| 5-16 | CF₃ | SMe | SO₂Et | |
| 5-17 | CF₃ | S(O)Me | SO₂Me | |
| 5-24 | CF₃ | S(O)Me | SO₂Et | |
| 5-25 | CF₃ | S(O)₂Me | SO₂Me | |
| 5-26 | CF₃ | S(O)₂Me | SO₂Et | |
| 5-27 | CF₃ | 2-[(Methylsulfonyl) amino]ethoxy | SO₂Me | |
| 5-28 | CF₃ | 2-[(Methylsulfonyl) amino]ethyl}sulfanyl | SO₂Me | |
| 5-29 | CF₃ | 2-[(Methylsulfonyl) amino]ethyl}sulfanyl | SO₂Et | |
| 5-30 | NO₂ | O(CH₂)₂OMe | OMe | |
| 5-31 | NO₂ | OMe | Me | |
| 5-32 | NO₂ | NH₂ | OMe | |
| 5-33 | NO₂ | NH₂ | SO₂Et | |
| 5-34 | NO₂ | NH₂ | Cl | |
| 5-35 | NO₂ | NHMe | Cl | |
| 5-36 | NO₂ | NMe₂ | Cl | |
| 5-37 | NO₂ | NH₂ | Br | |
| 5-38 | NO₂ | NHMe | Br | |
| 5-39 | NO₂ | NMe₂ | Br | |
| 5-40 | NO₂ | NH₂ | F | |
| 5-41 | NO₂ | NHMe | F | |
| 5-42 | NO₂ | NMe₂ | F | |
| 5-43 | NO₂ | NH₂ | SO₂Me | |
| 5-44 | NO₂ | NHMe | SO₂Me | |
| 5-45 | NO₂ | NMe₂ | SO₂Me | |
| 5-46 | NO₂ | NH₂ | 1H-1,2,4-triazol-1-yl | |
| 5-47 | NO₂ | NHMe | 1H-1,2,4-triazol-1-yl | |
| 5-48 | NO₂ | NMe₂ | 1H-1,2,4-triazol-1-yl | |
| 5-49 | Me | F | F | |
| 5-50 | Me | F | Cl | |
| 5-51 | Me | SMe | CF₃ | |
| 5-52 | Me | Cl | SO₂Me | |
| 5-53 | Me | SO₂Me | SO₂Me | ¹H-NMR, DMSO-d₆, 400 MHz |
| | | | | 11,89 (s, 1H), 8,29 (d, 1H), 8,08 (d, 1H), 5,08 (s, 2H), 3,61 (s, 3H), 3,57 (s, 3H), 2,71 (s, 3H) |
| 5-54 | Me | SO₂Me | CF₃ | ¹H-NMR, DMSO-d₆, 400 MHz |
| | | | | 11,89 (s, 1H), 8,08 (d, 1H), 8,01 (d, 1H), 5,09 (s, 2H), 3,43 (s, 3H), 2,73 (s, 3H) |
| 5-55 | Me | Cl | CF₃ | |
| 5-56 | Me | S(O)Me | CF₃ | ¹H-NMR, DMSO-d₆, 400 MHz |
| | | | | 11,86 (s, 1H), 7,91 (d, 1H), 7,87 (d, 1H), 5,09 (s, 2H), 3,05 (s, 3H), 2,87 (s, 3H) |
| 5-57 | Me | SEt | OMe | |
| 5-58 | Me | NMe₂ | SO₂Me | |
| 5-59 | Me | NH(CH₂)₂OMe | SO₂Me | |
| 5-60 | Me | O(CH₂)₄OMe | SO₂Me | |
| 5-61 | Me | NH₂ | SO₂Me | |
| 5-62 | Me | O(CH₂)₂-O(3,5-Dimethoxypyrimidin-2-yl | SO₂Me | |
| 5-63 | Me | O(CH₂)₂-O-NMe₂ | Cl | |
| 5-64 | Me | O(CH₂)₂-NH(CO)NMe₂ | Cl | |
| | | | | |
| 5-65 | Me | O(CH₂)-5-Pyrrolidin-2-on | Br | |
| 5-66 | Me | O(CH₂)₂-NH(CO)NHCO₂Et | Cl | |
| 5-67 | Me | O(CH₂)- (CO)NEt₂ | Br | |
| 5-68 | Me | O(CH₂) -5-2,4-Dimethyl-2,4-dihydro-3H-1,2,4-triazol-3-on | Cl | |
| 5-69 | Me | O(CH₂)-3,5-Dimethyl-1,2-oxazol-4-yl | Cl | |
| 5-70 | Me | O(CH₂)₂-NHCO₂Me | Cl | |
| 5-71 | Me | 4,5-Dihydro-1,2-oxazol-3 yl | SO₂Me | |
| 5-72 | Me | Me | SO₂Me | |
| 5-73 | Me | OH | SO₂Me | |
| 5-74 | Me | O-CH₂-NHSO₂cPr | Cl | |
| 5-75 | Me | O(CH₂)₂NHSO2Me | SO₂Me | |
| 5-76 | Me | S(O)Me | SO₂Me | |
| 5-77 | Me | SMe | SO₂Me | |
| 5-78 | Me | SMe | OMe | |
| 5-79 | Me | S(O)Me | OMe | |
| 5-80 | Me | SO₂Me | OMe | |
| 5-81 | Me | SMe | Cl | |
| 5-82 | Me | S(O)Me | Cl | |
| 5-83 | Me | SO₂Me | Cl | |
| 5-84 | Me | [1,4]Dioxan-2-yl-methoxy | SO₂Me | |
| 5-85 | Me | [1,4]Dioxan-2-yl-methoxy | SO₂Et | |
| 5-86 | Me | O(CH₂)₄OMe | SO₂Et | |
| 5-87 | Me | O(CH₂)₃OMe | SO₂Me | |
| 5-88 | Me | O(CH₂)₃OMe | SO₂Et | |
| 5-89 | Me | O(CH₂)₂OMe | SO₂Me | |
| 5-90 | Me | O(CH₂)₂OMe | SO₂Et | |
| 5-91 | Me | S(O)Me | SO₂Me | |
| 5-92 | Me | SMe | SO₂Me | |
| 5-93 | Me | SMe | OMe | |
| 5-94 | Me | S(O)Me | OMe | |
| 5-95 | Me | SO₂Me | OMe | |
| 5-96 | Me | SMe | Cl | |
| 5-97 | Me | S(O)Me | Cl | |
| 5-98 | Me | SO₂Me | Cl | |
| 5-99 | Me | SMe | Br | |
| 5-100 | Me | SOMe | Br | |
| 5-101 | Me | SO₂Me | Br | |
| 5-102 | Me | SMe | I | |
| 5-103 | Me | SOMe | I | |
| 5-104 | Me | SO₂Me | I | |
| 5-105 | Me | SEt | Cl | |
| 5-106 | Me | SOEt | Cl | |
| 5-107 | Me | SO₂Et | Cl | |
| 5-108 | Me | SEt | Br | |
| 5-109 | Me | SOEt | Br | |
| 5-110 | Me | SO₂Et | Br | |
| 5-111 | Me | SEt | I | |
| 5-112 | Me | SOEt | I | |
| 5-113 | Me | SO₂Et | I | |
| 5-114 | Me | SEt | F | |
| 5-115 | Me | SOEt | F | |
| 5-116 | Me | SO₂Et | F | |
| 5-117 | Cl | OCH₂(CO)NMe₂ | Cl | |
| 5-118 | Cl | Cl | SO₂Me | |
| 5-119 | Cl | CH₂OCH₂CF₃ | SO₂Me | ¹H-NMR, CDCl₃, 400 MHz |
| | | | | 8,60 (bs, 1H), 8,10 (d, 1H), 7,83 (d, 1 H), 5,35 (s, 2H), 5,0 (s, 2H), 4,06 (q, 2H), 3,23 (s, 3H) |
| 5-120 | Cl | 5-Cyanomethyl- 4,5-dihydro-1,2-oxazol-3 yl | SO₂Et | ¹H-NMR, DMSO-d₆, 400 MHz |
| | | | | 12,03 (s, 1H), 8,16 (d, 1H), 8,10 (d, 1H), 5,20 (m, 1H), 5,08 (s, 2H), 3,60 (dd, 1H), 3,44 (q, 2H), 3,17 (dd, 1 H), 3,05 (m, 2H), 1,16 (t, 3H) |
| 5-121 | Cl | CH₂O-Tetrahydrofuran-3-yl | SO₂Me | |
| 5-122 | Cl | CH₂O-Tetrahydrofuran-2-yl | SO₂Me | |
| 5-123 | Cl | SMe | SO₂Me | |
| 5-124 | Cl | F | SMe | |
| 5-125 | Cl | CH₂OCH₂-Tetrahydrofuran-2-yl | SO₂Me | |
| 5-126 | Cl | CH₂OCH₂-Tetrahydrofuran-3-yl | SO₂Et | |
| 5-127 | Cl | O(CH₂)-5-Pyrrolidin-2-on | Cl | |
| 5-128 | Cl | SMe | Cl | ¹H-NMR, CDCl₃, 400 MHz |
| | | | | 12,19 (bs, 1H), 7,70 (d, 1H), 7,62 (d, 1 H), 6,30 (s, 2H), 2,21 (s, 3H), |
| 5-129 | Cl | S(O)Me | SO₂Me | |
| 5-130 | Cl | CH₂O-Tetrahydrofuran-yl | SO₂Et | ¹H-NMR, CDCl₃, 400 MHz |
| | | | | 9,05 (bs, 1H), 7,97 (d, 1H), 7,75 (d, 1H), 5,07 (s, 2H), 5,02 (s, 2H), 4,34 (m, 1H), 3,72-3,92 (m, 4H), 3,37 (q, 2H), 2,08 (m, 2H), 1,25 (t, 3H) |
| 5-131 | Cl | O(CH₂)₂OMe | Cl | |
| 5-132 | Cl | O(CH₂)₂OMe | SO₂Me | |
| 5-133 | Cl | O(CH₂)₄OMe | SO₂Me | |
| 5-134 | Cl | O(CH₂)₄OMe | SO₂Et | |
| 5-135 | Cl | O(CH₂)₃OMe | SO₂Me | |
| 5-136 | Cl | O(CH₂)₃OMe | SO₂Et | |
| 5-137 | Cl | O(CH₂)₂OMe | SO₂Me | |
| 5-138 | Cl | O(CH₂)₂OMe | SO₂Et | |
| 5-139 | Cl | [1,4]Dioxan-2-yl-methoxy | SO₂Me | |
| 5-140 | Cl | [1,4]Dioxan-2-yl-methoxy | SO₂Et | |
| 5-141 | Cl | SO₂Me | Me | |
| 5-142 | Cl | SEt | Me | |
| 5-143 | Cl | SOEt | Me | |
| 5-144 | Cl | SO₂Et | Me | |
| 2-145 | Cl | 4,5-Dihydro-1,5-oxazol-3 yl | SO₂Me | |
| 5-146 | Cl | Cl | SO₂Me | |
| 5-147 | F | SMe | CF₃ | |
| 5-148 | F | S(O)Me | CF₃ | ¹H-NMR, CDCl₃, 400 MHz |
| | | | | 12,05 (bs, 1H), 8,13 (dd, 1H), 7,92 (d, 1H), 5,09 (s, 2H), 3,18 (s, 3H), |
| 5-149 | OMe | SMe | CF₃ | |
| 5-150 | OMe | S(O)Me | CF₃ | |
| 5-151 | OMe | SO₂Me | CF₃ | |
| 5-152 | Et | NH(CH₂)₂OMe | SO₂Me | |
| 5-153 | Et | F | SO₂Me | |
| 5-154 | Et | SMe | CF₃ | |
| 5-155 | CF₃ | F | SO₂Me | |
| 5-156 | CF₃ | F | SO₂Et | |
| 5-157 | CF₃ | O(CH₂)₂OMe | SO₂Et | |
| 5-158 | CF₃ | O(CH₂)₃OMe | SO₂Et | |
| 5-159 | CF₃ | O(CH₂)₂OMe | SO₂Me | |
| 5-160 | CF₃ | O(CH₂)₃OMe | SO₂Me | |
| 5-161 | CF₃ | OCH₂CONMe₂ | SO₂Me | |
| 5-162 | CF₃ | OCH₂CONMe₂ | SO₂Et | |
| 5-163 | CF₃ | OCH₂CONMe₂ | Cl | |
| 5-164 | CF₃ | OCH₂CONMe₂ | Br | |
| 5-165 | CF₃ | OCH₂CONMe₂ | I | |
| 5-166 | CF₃ | OCH₂CONMe₂ | F | |
| 5-167 | CF₃ | O(CH₂)₂OMe | Cl | |
| 5-168 | CF₃ | O(CH₂)₃OMe | Cl | |
| 5-169 | CF₃ | O(CH₂)₂OMe | Br | |
| 5-170 | CF₃ | O(CH₂)₃OMe | Br | |
| 5-171 | CF₃ | O(CH₂)₂OMe | I | |
| 5-172 | CF₃ | O(CH₂)₃OMe | I | |
| 5-173 | CF₃ | O(CH₂)₂OMe | F | |
| 5-174 | CF₃ | O(CH₂)₃OMe | F | |
| 5-175 | CF₃ | [1,4]Dioxan-2-yl-methoxy | SO₂Me | |
| 5-176 | CF₃ | [1,4]Dioxan-2-yl-methoxy | SO₂Et | |
| 5-177 | CF₃ | [1,4]Dioxan-2-yl-methoxy | Cl | |
| 2-178 | CF₃ | [1,4]Dioxan-2-yl-methoxy | Br | |
| 5-179 | CF₃ | [1,4]Dioxan-2-yl-methoxy | I | |
| 5-180 | CF₃ | [1,4]Dioxan-2-yl-methoxy | F | |
| 5-181 | Br | OMe | Br | |
| 5-182 | Br | O(CH₂)₂OMe | Br | |
| 5-183 | Br | O(CH₂)₄OMe | SO₂Me | |
| 5-184 | Br | O(CH₂)₄OMe | SO₂Et | |
| 5-185 | Br | O(CH₂)₃OMe | SO₂Me | |
| 5-186 | Br | O(CH₂)₃OMe | SO₂Et | |
| 5-187 | Br | O(CH₂)₂OMe | SO₂Me | |
| 5-188 | Br | O(CH₂)₂OMe | SO₂Et | |
| 5-189 | Br | [1,4]Dioxan-2-yl-methoxy | SO₂Me | |
| 5-190 | Br | [1,4]Dioxan-2-yl-methoxy | SO₂Et | |
| 5-191 | Br | SMe | Me | |
| 5-192 | Br | SOMe | Me | |
| 5-193 | Br | SO₂Me | Me | |
| 5-194 | Br | SEt | Me | |
| 5-195 | Br | SOEt | Me | |
| 5-196 | Br | SO₂Et | Me | |
| 5-197 | I | O(CH₂)₄OMe | SO₂Me | |
| 5-198 | I | O(CH₂)₄OMe | SO₂Et | |
| 5-199 | I | O(CH₂)₃OMe | SO₂Me | |
| 5-200 | I | O(CH₂)₃OMe | SO₂Et | |
| 5-201 | I | O(CH₂)₂OMe | SO₂Me | |
| 5-202 | I | O(CH₂)₂OMe | SO₂Et | |
| 5-203 | I | [1,4]Dioxan-2-yl-methoxy | SO₂Me | |
| 5-204 | I | [1,4]Dioxan-2-yl-methoxy | SO₂Et | |
| 5-205 | I | SMe | Me | |
| 5-206 | I | SOMe | Me | |
| 5-207 | I | SO₂Me | Me | |
| 5-208 | I | SEt | Me | |
| 5-209 | I | SOEt | Me | |
| 5-210 | I | SO₂Et | Me | |
| 5-211 | CH₂SMe | OMe | SO₂Me | |
| 5-212 | CH₂OMe | OMe | SO₂Me | |
| 5-213 | CH₂O(C H₂)₂OMe | NH(CH₂)₂OEt | SO₂Me | |
| 5-214 | CH₂O(C H₂)₂OMe | NH(CH₂)₃OEt | SO₂Me | |
| 5-215 | CH₂O(C H₂)₃OMe | OMe | SO₂Me | |
| 5-216 | CH₂O(C H₂)₂OMe | NH(CH₂)₂OMe | SO₂Me | |
| 5-217 | CH₂O(C H₂)₂OMe | NH(CH₂)₃OMe | SO₂Me | |
| 5-218 | SO₂Me | NH₂ | CF₃ | |
| **Nr.** | **X** | **Y** | **Z** | **Physikalische Daten** |
| 5-219 | SO₂Me | F | CF₃ | |
| 5-220 | SO₂Me | NHEt | Cl | |
| 5-221 | SMe | SEt | F | |
| 5-222 | SMe | SMe | F | |
| 5-223 | Me | NH₂ | Cl | |
| 5-224 | Me | NH₂ | Br | |
| 5-225 | Me | NHMe | Cl | |
| 5-226 | Me | NHMe | Br | |
| 5-227 | Me | NMe₂ | Cl | |
| 5-228 | Me | NMe₂ | Br | |
| 5-227 | Me | NMe₂ | Cl | |
| 5-228 | Me | NMe₂ | Br | |
| 5-229 | NO₂ | O(CH₂)₂OMe | Me | |
| 5-230 | CF₃ | S(O)₂Et | SO₂Me | |
| 5-231 | CF₃ | S(O)₂Et | SO₂Et | |
| 5-232 | CF₃ | SCH₂CONMe₂ | SO₂Me | |
| 5-233 | CF₃ | SCH₂CONMe₂ | SO₂Et | |
| 5-234 | CF₃ | SCH₂COOH | SO₂Me | |
| 5-235 | CF₃ | SCH₂COOH | SO₂Et | |
| 5-236 | Me | SO₂-CH₂-CH₂-CH=CH₂ | CF₃ | |
| 5-237 | Cl | Me | SO₂Et | |
| 5-238 | CF₃ | SEt | SO₂Me | |
| 5-239 | OMe | NO2 | Cl | |
| 5-240 | OMe | NH(CO)i-Pr | Cl | |
| 5-241 | OMe | NH(CO)CH2Ph | Cl | |
| 5-242 | CF₃ | SEt | SO₂Et | |
| 5-243 | CF₃ | S(O)Et | SO₂Me | |
| 5-244 | Cl | Me | Cl | |
| 5-245 | Me | 3,5-Dimethyl-pyrazol-1-yl | SO₂Me | |
| 5-247 | Me | 1,2,3-Triazol-1-yl | SO₂Me | ¹H-NMR, DMSO-d₆, 400 MHz |
| | | | | 12,65 (s, 1H), 8,57 (s, 1H), 8,12 (d, 1H), 8,08 (d, 1H), 8,05 (s, 1H), 4,61 (s, 2H), 3,11 (s, 3H), |
| 5-248 | Me | Me | SMe | ¹H-NMR, DMSO-d₆, 400 MHz |
| | | | | 11,46 (s, 1H), 7,49 (d, 1H), 7,21 (d, 1 H), 5,08 (s, 2H), 2,32 (s, 3H), 2,24 (s, 3H) |
| 5-249 | Me | Pyrolidin-2-on-1-yl | SO₂Me | |
| 5-250 | CF₃ | S(O)Et | SO₂Et | |
| 5-251 | Cl | Pyrazol-1-yl | SO₂Me | |
| 5-252 | Me | 3-Methyl-pyrazol-1-yl | SO₂Me | |
| 5-253 | Cl | CH₂-N(Et)OMe | SO₂Me | |
| 5-254 | Me | Me | Cl | |
| 5-255 | OH | Cl | Cl | |
| 5-256 | Me | 1,2,4-Triazol-1-yl | SO₂Me | |
| 5-257 | Me | 4-Methoxy-pyrazol-1-yl | SO₂Me | |
| 5-258 | Me | 1,2,4-triazol-1-yl | CF₃ | |
| 5-259 | Me | Tetrahydro-pyrimidin-2(1H)-one-1-yl | SO₂Me | |
| 5-260 | Me | NH-(CH₂)₂-O(CO)Et | SO₂Me | |
| 5-261 | Me | NH-iPr | SO₂Me | |
| 5-262 | Cl | NH-CH₂-(CO)NHEt | Cl | |
| 5-263 | Me | NH-CH₂-(CO)NMe2 | SO₂Me | |
| 5-264 | Me | NH-CH₂-furan-2-yl | SO₂Me | |
| 5-265 | Me | NH-CH₂-(CO)NHEt | SO₂Me | |
| 5-266 | Me | F | SO₂Me | |
| 5-267 | F | SO₂Me | SO₂Me | |
| 5-268 | Cl | (4-Cyclopropyl-3-methyl-5-oxo-4,5-dihydro-1H-1,2,4-triazol-1-yl)-methyl | Cl | |
| 5-269 | Cl | [4-Methyl-5-oxo-3-(2,2,2-trifluorethoxy)-4,5-dihydro-1H-1,2,4-triazol-1-yl]-methyl | Cl | |
| 5-270 | Cl | (3-Isopropoxy- 4-methyl-5-oxo-4,5-dihydro-1H-1,2,4-triazol-1-yl]-methyl | Cl | |
| 5-271 | Cl | (4-Methyl-5-oxo-4,5-dihydro-1H-1,2,4-triazol-1-yl)-methyl | Cl | |
| 5-272 | Me | Cl | SO₂Et | |
| 5-273 | SO₂Me | F | Cl | |
| 5-274 | Me | 1,2,3-Triazol-1-yl | SO₂Me | |
| 5-275 | Me | Isobutyl(methyl)carbamoylamino | SO₂Me | |
| 5-276 | Me | 3-Oxo-morpholin-4-yl | SO₂Me | |
| 5-277 | OMe | [Ethyl(methylsulfonyl)a mino]methyl | Cl | |
| 5-278 | F | SO₂Me | CF₃ | |
| 5-279 | OMe | Benzoylamino | Cl | |
| 5-280 | OMe | Cyclopropylcarbonylamino | Cl | |
| 5-281 | OMe | Propionyllamino | Cl | |
| 5-282 | NO₂ | SO₂Me | SO₂Me | |
| 5-283 | NO₂ | SO₂Me | Cl | |
| 5-284 | NO₂ | SOMe | SO₂Me | |
| 5-285 | NO₂ | SOMe | Br | |
| 5-286 | NO₂ | SOMe | Cl | |
| 5-287 | NO₂ | SMe | SO₂Me | |
| 5-288 | NO₂ | SMe | Br | |
| 5-289 | NO₂ | SMe | Cl | |
| 5-290 | Cl | CH₂OCH(CH₃)₂ | SO₂Et | |
| 5-291 | Cl | CH₂OEt | SO₂Et | |
| 5-292 | Cl | CH₂OMe | SO₂Et | |
| 5-293 | Cl | CH₂OCH₂C₂F₅ | SO₂Me | |
| 5-294 | Cl | CH₂OCH₂CHF₂ | SO₂Me | |
| 5-295 | Cl | CH₂OCH₂CCH | SO₂Et | |
| 5-296 | Cl | CH₂OC₂H₄OMe | SO₂Me | |
| 5-297 | Cl | CH₂(OC₂H₄)₂OMe | SO₂Me | |
| 5-298 | Cl | 5-Ethoxymethyl- 4,5-dihydro-1,2-oxazol-3 yl | SO₂Et | |
| 5-299 | Cl | 5-Methoxymethyl- 4,5-dihydro-1,2-oxazol-3 yl | SO₂Et | |
| 5-300 | Et | SOMe | CF₃ | |
| 5-301 | iPr | SMe | CF₃ | |
| 5-302 | Et | SMe | CF₃ | |
| 5-303 | Et | SO₂Me | CF₃ | |
| 5-304 | cPr | SOMe | CF₃ | |
| 5-305 | CH=CH₂ | SMe | CF₃ | |
| 5-306 | Et | SMe | Cl | |
| 5-307 | Et | SO₂Me | Cl | |
| 5-308 | Cl | NMe₂ | Cl | |
| 5-309 | CH₂O(C H₂)₂OMe | NH(CH₂)₃OMe | SO₂Me | |
| 5-313 | Me | SO₂(CH₂)₂OMe | CF₃ | |
| 5-314 | Me | SOEt | SO₂Me | |
| 5-315 | Me | SO₂Et | SO₂Me | |
| 5-316 | Me | SMe | 1,2,4-triazol-1-yl | |
| 5-317 | OEt | SMe | CF₃ | |
| 5-318 | Me | S(CH₂)₂OMe | CF₃ | |
| 5-319 | Me | SOMe | 1,2,4-triazol-1-yl | |
| 5-320 | OEt | SOMe | CF₃ | |
| 5-321 | Me | SO(CH₂)₂OMe | CF₃ | |
| 5-322 | Me | SCH₂CCMe | SO₂Me | |
| 5-323 | Me | S-c-Pen | SO₂Me | |
| 5-324 | OMe | SMe | OMe | |
| 5-325 | Me | SCH₂CH=CHCH₃ | SO₂Me | |
| 5-326 | Me | SOCH₂CCMe | SO₂Me | |
| 5-327 | Me | SO₅₋c-Pen | SO₂Me | |
| 5-328 | Me | SO-c-Pen | SO₂Me | |
| 5-329 | Me | S(CH₂)₃Cl | SO₂Me | |
| 5-330 | Me | SCH₂(4-F-Ph) | SO₂Me | |
| 5-331 | Me | SO₂CH₂CCMe | SO₂Me | |
| 5-332 | Me | SO₂CH₂CH=CHCH₃ | SO₂Me | |
| 5-333 | Me | SOCH₂CH=CHCH₃ | SO₂Me | |
| 5-334 | Me | SOCH₂-Epoxy-Me | SO₂Me | |
| 5-335 | Me | SO₂(CH₂)₃Cl | SO₂Me | |
| 5-336 | Me | SO(CH₂)₃Cl | SO₂Me | |
| 5-337 | Me | SOCH₂(4-F-Ph) | SO₂Me | |
| 5-338 | Me | SO₂CH₂(4-F-Ph) | SO₂Me | |
| 5-339 | Me | SO₂Me | C₂F₅ | |
| 5-340 | O(CH₂)₂ OMe | SMe | CF₃ | |
| 5-341 | O(CH₂)₂ OMe | SO₂Me | CF₃ | |
| 5-342 | O(CH₂)₂ OMe | SOMe | CF₃ | |
| 5-343 | Me | S(CH₂)₂OCH₂CF₃ | SO₂Me | |
| 5-344 | Me | SO(CH₂)₂OCH₂CF₃ | SO₂Me | |
| 5-345 | Me | SO₂(CH₂)₂OCH₂CF₃ | SO₂Me | |
| 5-346 | OEt | SEt | CF₃ | |
| 5-347 | O-CH₂-c-Pr | SMe | CF₃ | |
| 5-348 | OMe | SEt | CF₃ | |
| 5-349 | OMe | SO₂Et | CF₃ | |
| 5-350 | OMe | SOEt | CF₃ | |
| 5-351 | OEt | SO₂Et | CF₃ | |
| 5-352 | OEt | SOEt | CF₃ | |
| 5-353 | O-CH₂₋c-Pr | SOMe | CF₃ | |
| 5-354 | O-CH₂₋c-Pr | SO₂Me | CF₃ | |
| 5-355 | Me | SEt | SO₂Me | |

**Tabelle 6: Erfindungsgemäße Verbindungen der allgemeinen Formel (I), worin R für tertiär-Butyl steht**

| | | | | |
|---|---|---|---|---|
| 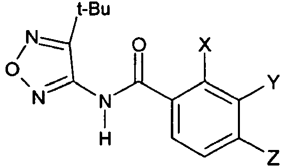 | | | | |

| **Nr.** | **X** | **Y** | **Z** | **Physikalische Daten** |
|---|---|---|---|---|
| 6-1 | CF₃ | OCH₂CON(Me)Et | SO₂Me | |
| 6-2 | CF₃ | OCH₂CON(Me)Et | SO₂Et | |
| 6-4 | CF₃ | 2-(1H-Pyrazo)-1-yl)ethoxyl | SO₂Me | |
| 6-5 | CF₃ | 2-(1H-Pyrazol-1-yl)ethoxyl | SO₂Et | |
| 6-6 | CF₃ | Tetrahydrofuran-2-yl-methoxy | SO₂Me | |
| 6-7 | CF₃ | Tetrahydrofuran-2-yl-methoxy | SO₂Et | |
| 6-8 | CF₃ | OH | SO₂Me | |
| 6-9 | CF₃ | OH | SO₂Et | |
| 6-10 | CF₃ | SH | SO₂Me | |
| 6-11 | CF₃ | SH | SO₂Et | |
| 6-15 | CF₃ | SMe | SO₂Me | |
| 6-16 | CF₃ | SMe | SO₂Et | |
| 6-17 | CF₃ | S(O)Me | SO₂Me | |
| 6-24 | CF₃ | S(O)Me | SO₂Et | |
| 6-25 | CF₃ | S(O)₂Me | SO₂Me | |
| 6-26 | CF₃ | S(O)₂Me | SO₂Et | |
| 6-27 | CF₃ | 2-[(Methylsulfonyl) amino]ethoxy | SO₂Me | |
| 628 | CF₃ | 2-[(Mmethylsulfonyl) amino]ethyl}sulfanyl | SO₂Me | |
| 6-29 | CF₃ | 2-[(Methylsulfonyl) | SO₂Et | |
| | | amino]ethyl}sulfanyl | | |
| 6-30 | Me | H | NO₂ | |
| 6-31 | NO₂ | OMe | Me | |
| 6-32 | NO₂ | NH₂ | OMe | |
| 6-33 | NO₂ | NH₂ | SO₂Et | |
| 6-34 | NO₂ | NH₂ | Cl | |
| 6-35 | NO₂ | NHMe | Cl | |
| 6-36 | NO₂ | NMe₂ | Cl | |
| 6-37 | NO₂ | NH₂ | Br | ¹H-NMR, CDCl₃, 400 MHz |
| | | | | 9.70 (s, 2H), 8.42 (d, 1H), 8.92 (d, 1H), 1.55 (s, 9H) |
| 6-38 | NO₂ | NHMe | Br | |
| 6-39 | NO₂ | NMe₂ | Br | |
| 6-40 | NO₂ | NH₂ | F | |
| 6-41 | NO₂ | NHMe | F | |
| 6-42 | NO₂ | NMe₂ | F | |
| 6-43 | NO₂ | NH₂ | SO₂Me | |
| 6-44 | NO₂ | NHMe | SO₂Me | |
| 6-45 | NO₂ | NMe₂ | SO₂Me | |
| 6-46 | NO₂ | NH₂ | 1H-1,2,4-Triazol-5-yl | |
| 6-47 | NO₂ | NHMe | 1H-1,2,4-Triazol-5-yl | |
| 6-48 | NO₂ | NMe₂ | 1H-1,2,4-Triazol-5-yl | |
| 6-49 | Me | F | F | |
| 6-50 | Me | F | Cl | |
| 6-51 | Me | SMe | CF₃ | |
| 6-52 | Me | Cl | SO₂Me | |
| 6-53 | Me | SO₂Me | SO₂Me | ¹H-NMR, DMSO-d₆, 400 MHz |
| | | | | 11,04 (s, 1H), 8,29 (d, 1H), 7,99 (d, 1H), 3,60 (s, 3H), 3,58 (s, 3H), 2,73 (s, 3H), 1,40 (s, 9H) |
| 6-54 | Me | SO₂Me | CF₃ | ¹H-NMR, CDCl₃, 400 MHz 7,85-7,89 (m, 2H), 7,75 (bs, 1H), 5,15 (m, 1H), 3,20 (s, 3H), 2,81 (s, 3H), 1,47 (s, 9H) |
| 6-55 | Me | Cl | CF₃ | |
| 6-56 | Me | S(O)Me | CF₃ | |
| 6-57 | Me | SEt | OMe | |
| 6-58 | Me | NMe₂ | SO₂Me | |
| 6-59 | Me | NH(CH₂)₂OMe | SO₂Me | |
| 6-60 | Me | O(CH₂)₄OMe | SO₂Me | |
| 6-61 | Me | NH₂ | SO₂Me | |
| 6-62 | Me | O(CH₂)₂-O(3,5-Dimethoxypyrimidin-2-yl | SO₂Me | |
| 6-63 | Me | O(CH₂)₂-O)-NMe₂ | Cl | |
| 6-64 | Me | O(CH₂)₂-NH(CO)NMe₂ | Cl | |
| 6-65 | Me | O(CH₂)-5-Pyrrolidin-2-on | Br | |
| 6-66 | Me | O(CH₂)₂-NH(CO)NHCO₂Et | Cl | |
| 6-67 | Me | O(CH₂)- (CO)NEt₂ | Br | |
| 6-68 | Me | O(CH₂) -5-2,4-Dimethyl-2,4-dihydro-3H-1,2,4-triazol-3-on | Cl | |
| 6-69 | Me | O(CH₂)-3,5-Dimethyl-1,2-oxazol-4-yl | Cl | |
| 6-70 | Me | O(CH₂)₂-NHCO₂Me | Cl | |
| 6-71 | Me | 4,5-Dihydro-1,2-oxazol-3 yl | SO₂Me | |
| 6-72 | Me | Me | SO₂Me | |
| 6-73 | Me | OH | SO₂Me | |
| 6-74 | Me | O-CH₂-NHSO₂cPr | Cl | |
| 6-75 | Me | O(CH₂)₂NHSO2Me | SO₂Me | |
| 6-76 | Me | S(O)Me | SO₂Me | |
| 6-77 | Me | SMe | SO₂Me | |
| 6-78 | Me | SMe | OMe | |
| 6-79 | Me | S(O)Me | OMe | |
| 6-80 | Me | SO₂Me | OMe | |
| 6-81 | Me | SMe | Cl | |
| 6-82 | Me | S(O)Me | Cl | |
| 6-83 | Me | SO₂Me | Cl | |
| 6-84 | Me | [1,4]Dioxan-2-yl-methoxy | SO₂Me | |
| 6-85 | Me | [1,4]Dioxan-2-yl-methoxy | SO₂Et | |
| 6-86 | Me | O(CH₂)₄OMe | SO₂Et | |
| 6-87 | Me | O(CH₂)₃OMe | SO₂Me | |
| 6-88 | Me | O(CH₂)₃OMe | SO₂Et | |
| 6-89 | Me | O(CH₂)₂OMe | SO₂Me | |
| 6-90 | Me | O(CH₂)₂OMe | SO₂Et | |
| 6-91 | Me | S(O)Me | SO₂Me | |
| 6-92 | Me | SMe | SO₂Me | |
| 6-93 | Me | SMe | OMe | |
| 6-94 | Me | S(O)Me | OMe | |
| 6-95 | Me | SO₂Me | OMe | |
| 6-96 | Me | SMe | Cl | |
| 6-97 | Me | S(O)Me | Cl | |
| 6-98 | Me | SO₂Me | Cl | |
| 6-99 | Me | SMe | Br | |
| 6-100 | Me | SOMe | Br | |
| 6-101 | Me | SO₂Me | Br | |
| 6-102 | Me | SMe | I | |
| 6-103 | Me | SOMe | I | |
| 6-104 | Me | SO₂Me | I | |
| 6-105 | Me | SEt | Cl | |
| 6-106 | Me | SOEt | Cl | |
| 6-107 | Me | SO₂Et | Cl | |
| 6-108 | Me | SEt | Br | |
| 6-109 | Me | SOEt | Br | |
| 6-110 | Me | SO₂Et | Br | |
| 6-111 | Me | SEt | I | |
| 6-112 | Me | SOEt | I | |
| 6-113 | Me | SO₂Et | I | |
| 6-114 | Me | SEt | F | |
| 6-115 | Me | SOEt | F | |
| 6-116 | Me | SO₂Et | F | |
| 6-117 | Cl | OCH₂(CO)NMe₂ | Cl | |
| 6-118 | Cl | Cl | SO₂Me | |
| 6-119 | Cl | CH₂OCH₂CF₃ | SO₂Me | ¹H-NMR, CDCl₃, 400 MHz |
| | | | | 8,05 (d, 2H), 7,78 (d, 1H), 5,30 (s, 2H), 4,04 (q, 2H), 3,20 (s, 3H), 1,49 (s, 9H) |
| 6-120 | Cl | 5-Cyanomethyl- 4,5-dihydro-1,2-oxazol-3 yl | SO₂Et | ¹H-NMR, CDCl₃, 400 MHz |
| | | | | 8,10 (d, 1H), 7,94 (bs, 2H), 5,15 (m, 1H), 3,73 (m, 1H), 3,35 (m, 2H), 3,25 (m, 1H), 2,90 (m, 2H), 1,48 (s, 9H), 1,28 (t, 3H) |
| 6-121 | Cl | CH₂O-Tetra-hydrofuran-3-yl | SO₂Me | |
| 6-122 | Cl | CH₂O-Tetrahydrofuran-2-yl | SO₂Me | |
| 6-123 | Cl | SMe | SO₂Me | |
| 6-124 | Cl | F | SMe | |
| 6-125 | Cl | CH₂OCH₂-Tetrahydrofuran-2-yl | SO₂Me | |
| 6-126 | Cl | CH₂OCH₂-Tetrahydrofuran-3-yl | SO₂Et | |
| 6-127 | Cl | O(CH₂)-5-Pyrrolidin-2-on | Cl | |
| 6-128 | Cl | SMe | Cl | |
| 6-129 | Cl | S(O)Me | SO₂Me | |
| 6-130 | Cl | CH2O-Tetrahydrofuran-3-yl | SO₂Et | |
| 6-131 | Cl | O(CH₂)₂OMe | Cl | |
| 6-132 | Cl | O(CH₂)₂OMe | SO₂Me | |
| 6-133 | Cl | O(CH₂)₄OMe | SO₂Me | |
| 6-134 | Cl | O(CH₂)₄OMe | SO₂Et | |
| 6-135 | Cl | O(CH₂)₃OMe | SO₂Me | |
| 6-136 | Cl | O(CH₂)₃OMe | SO₂Et | |
| 6-137 | Cl | O(CH₂)₂OMe | SO₂Me | |
| 6-138 | Cl | O(CH₂)₂OMe | SO₂Et | |
| 6-139 | Cl | [1,4]Dioxan-2-yl-methoxy | SO₂Me | |
| 6-140 | Cl | [1,4]Dioxan-2-yl-methoxy | SO₂Et | |
| 6-141 | Cl | SO₂Me | Me | |
| 6-142 | Cl | SEt | Me | |
| 6-143 | Cl | SOEt | Me | |
| 6-144 | Cl | SO₂Et | Me | |
| 6-145 | Cl | 4,5-Dihydro-1,2-oxazol-3 yl | SO₂Me | |
| 6-146 | Cl | Cl | SO₂Me | |
| 6-147 | F | SMe | CF₃ | |
| 6-148 | F | S(O)Me | CF₃ | |
| 6-149 | OMe | SMe | CF₃ | |
| 6-150 | OMe | S(O)Me | CF₃ | |
| 6-151 | OMe | SO₂Me | CF₃ | |
| 6-152 | Et | NH(CH₂)₂OMe | SO₂Me | |
| 6-153 | Et | F | SO₂Me | |
| 6-154 | Et | SMe | CF₃ | |
| 6-155 | CF₃ | F | SO₂Me | |
| 6-156 | CF₃ | F | SO₂Et | |
| 6-157 | CF₃ | O(CH₂)₂OMe | SO₂Et | |
| 6-158 | CF₃ | O(CH₂)₃OMe | SO₂Et | |
| 6-159 | CF₃ | O(CH₂)₂OMe | SO₂Me | |
| 6-160 | CF₃ | O(CH₂)₃OMe | SO₂Me | |
| 6-161 | CF₃ | OCH₂CONMe₂ | SO₂Me | |
| 6-162 | CF₃ | OCH₂CONMe₂ | SO₂Et | |
| 6-163 | CF₃ | OCH₂CONMe₂ | Cl | |
| 6-164 | CF₃ | OCH₂CONMe₂ | Br | |
| 6-165 | CF₃ | OCH₂CONMe₂ | I | |
| 6-166 | CF₃ | OCH₂CONMe₂ | F | |
| 6-167 | CF₃ | O(CH₂)₂OMe | Cl | |
| 6-168 | CF₃ | O(CH₂)₃OMe | Cl | |
| 6-169 | CF₃ | O(CH₂)₂OMe | Br | |
| 6-170 | CF₃ | O(CH₂)₃OMe | Br | |
| 6-171 | CF₃ | O(CH₂)₂OMe | I | |
| 6-172 | CF₃ | O(CH₂)₃OMe | I | |
| 6-173 | CF₃ | O(CH₂)₂OMe | F | |
| 6-174 | CF₃ | O(CH₂)₃OMe | F | |
| 6-175 | CF₃ | [1,4]Dioxan-2-yl-methoxy | SO₂Me | |
| 6-176 | CF₃ | [1,4]Dioxan-2-yl-methoxy | SO₂Et | |
| 6-177 | CF₃ | [1,4]Dioxan-2-yl-methoxy | Cl | |
| 6-178 | CF₃ | [1,4]Dioxan-2-yl-methoxy | Br | |
| 6-179 | CF₃ | [1,4]Dioxan-2-yl-methoxy | I | |
| 6-180 | CF₃ | [1,4]Dioxan-2-yl-methoxy | F | |
| 6-181 | Br | OMe | Br | |
| 6-182 | Br | O(CH₂)₂OMe | Br | |
| 6-183 | Br | O(CH₂)₄OMe | SO₂Me | |
| 6-184 | Br | O(CH₂)₄OMe | SO₂Et | |
| 6-185 | Br | O(CH₂)₃OMe | SO₂Me | |
| 6-186 | Br | O(CH₂)₃OMe | SO₂Et | |
| 6-187 | Br | O(CH₂)₂OMe | SO₂Me | |
| 6-188 | Br | O(CH₂)₂OMe | SO₂Et | |
| 6-189 | Br | [1,4]Dioxan-2-yl-methoxy | SO₂Me | |
| 6-190 | Br | [1,4]Dioxan-2-yl-methoxy | SO₂Et | |
| 6-191 | Br | SMe | Me | |
| 6-192 | Br | SOMe | Me | |
| 6-193 | Br | SO₂Me | Me | |
| 6-194 | Br | SEt | Me | |
| 6-195 | Br | SOEt | Me | |
| 6-196 | Br | SO₂Et | Me | |
| 6-197 | I | O(CH₂)₄OMe | SO₂Me | |
| 6-198 | I | O(CH₂)₄OMe | SO₂Et | |
| 6-199 | I | O(CH₂)₃OMe | SO₂Me | |
| 6-200 | I | O(CH₂)₃OMe | SO₂Et | |
| 6-201 | I | O(CH₂)₂OMe | SO₂Me | |
| 6-202 | I | O(CH₂)₂OMe | SO₂Et | |
| 6-203 | I | [1,4]Dioxan-2-yl-methoxy | SO₂Me | |
| 6-204 | I | [1,4]Dioxan-2-yl-methoxy | SO₂Et | |
| 6-205 | I | SMe | Me | |
| 6-206 | I | SOMe | Me | |
| 6-207 | I | SO₂Me | Me | |
| 6-208 | I | SEt | Me | |
| 6-209 | I | SOEt | Me | |
| 6-210 | I | SO₂Et | Me | |
| 6-211 | CH₂SMe | OMe | SO₂Me | |
| 6-212 | CH₂OMe | OMe | SO₂Me | |
| 6-213 | CH₂O(CH₂)₂ OMe | NH(CH₂)₂OEt | SO₂Me | |
| 6-214 | CH₂O(CH₂)₂ OMe | NH(CH₂)₃OEt | SO₂Me | |
| 6-215 | CH₂O(CH₂)₃ OMe | OMe | SO₂Me | |
| 6-216 | CH₂O(CH₂)₂ OMe | NH(CH₂)₂OMe | SO₂Me | |
| 6-217 | CH₂O(CH₂)₂ OMe | NH(CH₂)₃OMe | SO₂Me | |
| 6-218 | SO₂Me | NH₂ | CF₃ | |
| 6-219 | SO₂Me | F | CF₃ | |
| 6-220 | SO₂Me | NHEt | Cl | |
| 6-221 | SMe | SEt | F | |
| 6-222 | SMe | SMe | F | |
| 6-223 | Me | NH₂ | Cl | |
| 6-224 | Me | NH₂ | Br | |
| 6-225 | Me | NHMe | Cl | |
| 6-226 | Me | NHMe | Br | |
| 6-227 | Me | NMe₂ | Cl | |
| 6-228 | Me | NMe₂ | Br | |
| 6-229 | NO₂ | O(CH₂)₂OMe | Me | |
| 6-230 | CF₃ | S(O)₂Et | SO₂Me | |
| 6-231 | CF₃ | S(O)₂Et | SO₂Et | |
| 6-232 | CF₃ | SCH₂CONMe₂ | SO₂Me | |
| 6-233 | CF₃ | SCH₂CONMe₂ | SO₂Et | |
| 6-234 | CF₃ | SCH₂COOH | SO₂Me | |
| 6-235 | CF₃ | SCH₂COOH | SO₂Et | |
| 6-236 | Me | SO2-CH2-CH2-CH=CH2 | CF₃ | |
| 6-237 | Cl | Me | SO₂Et | |
| 6-238 | CF₃ | SEt | SO₂Me | |
| 6-239 | OMe | N02 | Cl | |
| 6-240 | OMe | NH(CO)i-Pr | Cl | |
| 6-241 | OMe | NH(CO)CH2Ph | Cl | |
| 6-242 | CF₃ | SEt | SO₂Et | |
| 6-243 | CF₃ | S(O)Et | SO₂Me | |
| 6-244 | Cl | Me | Cl | |
| 6-245 | Me | 3,5-Dimethyl-pyrazol-6-yl | SO₂Me | |
| 6-246 | SMe | H | CF₃ | |
| 6-247 | Me | 1,2,3-Triazol-6-yl | SO₂Me | |
| 6-248 | Me | Me | SMe | |
| 6-249 | Me | Pyrolidin-2-on-6-yl | SO₂Me | |
| 6-250 | CF₃ | S(O)Et | SO₂Et | |
| 6-251 | Cl | Pyrazol-6-yl | SO₂Me | |
| 6-252 | Me | 3-Methyl-pyrazol-6-yl | SO₂Me | |
| 6-253 | Cl | CH₂-N(Et)OMe | SO₂Me | |
| 6-254 | Me | Me | Cl | |
| 6-255 | OH | Cl | Cl | |
| 6-256 | Me | 1,2,4-Triazol-1-yl | SO₂Me | |
| 6-257 | Me | 4-Methoxy-pyrazol-6-yl | SO₂Me | |
| 6-258 | Me | 1,2,4-Triazol-1-yl | CF₃ | |
| 6-259 | Me | Tetrahydro-pyrimidin-2(1H)-on-6-yl | SO₂Me | |
| 6-260 | Me | NH-(CH2)2-O(CO)Et | SO₂Me | |
| 6-261 | Me | NH-iPr | SO₂Me | |
| 6-262 | Cl | NH-CH2-(CO)NHEt | Cl | |
| 6-263 | Me | NH-CH2-(CO)NMe2 | SO₂Me | |
| 6-264 | Me | NH-CH2-furan-2-yl | SO₂Me | |
| 6-265 | Me | NH-CH2-(CO)NHEt | SO₂Me | |
| 6-266 | Me | F | SO₂Me | |
| 6-267 | F | SO₂Me | SO₂Me | |
| 6-268 | Cl | (4-Cyclopropyl-3-methyl-5-oxo-4,5-dihydro-1H-1,2,4-triazol-6-yl)-methyl | Cl | |
| 6-269 | Cl | [4-Methyl-5-oxo-3-(2,2,2-trifluorethoxy)-4,5-dihydro-1H-1,2,4-triazol-6-yl]-methyl | Cl | |
| 6-270 | Cl | (3-Isopropoxy- 4-methyl-5-oxo-4,5-dihydro-1H-1,2,4-triazol-6-yl]-methyl | Cl | |
| 6-271 | Cl | (4-Methyl-5-oxo-4,5-dihydro-1H-1,2,4-triazol-6-yl)-methyl | Cl | |
| 6-272 | Me | Cl | SO₂Et | |
| 6-273 | SO₂Me | F | Cl | |
| 6-274 | Cl | | Morpholin-4-yl | |
| 6-275 | Me | Isobutyl(methyl)carba moyl-amino | SO₂Me | |
| 6-276 | Me | 3-oxo-morpholin-4-yl | SO₂Me | |
| 6-277 | OMe | [Ethyl(methylsulfonyl) amino]methyl | Cl | |
| 6-278 | Cl | | (4-Methyl-3-trifluormethy I-5-oxo-4,5-dihydro-1H- 1,2,4-triazol-6-yl)- | |
| 6-279 | OMe | Benzoylamino | Cl | |
| 6-280 | OMe | Cyclopropylcarbonyla mino | Cl | |
| 6-281 | OMe | Propionyllamino | Cl | |
| 6-282 | NO₂ | SO₂Me | SO₂Me | ¹H-NMR, MeOD 400 MHz |
| | | | | 8.66 (d, 1H), 8.38 (d, 1H), 3.69 (s, 3H), 3.60 (s, 3H), 1.45 (s, 9H) |
| 6-283 | NO₂ | SOMe | SO₂Me | ¹H-NMR, MeOD 400 MHz |
| | | | | 8.36 (d, 1H), 8.25 (d, 1H), 3.41 (s, 3H), 3.40 (s, 3H), 1.42 (s, 9H) |
| 6-284 | NO₂ | SO₂Me | Cl | ¹H-NMR, MeOD 400 MHz |
| | | | | 8.15 (d, 1H), 8.03 (d, 1H), 3.48 (s, 3H), 1.45 (s, 9H) |
| 6-285 | NO₂ | SOMe | Br | ¹H-NMR, DMSO-d₆, 400 MHz |
| | | | | 11.32 (s, 1H), 8.2 (d, 1H), 7.95 (d, 1 H), 3.18 (s, 3H), 1.38 (s, 9H) |
| 6-286 | NO₂ | SOMe | Cl | ¹H-NMR, MeOD 400 MHz |
| | | | | 8.02 (d, 1H), 7.92 (d, 1H), 3.28 (s, 3H), 1.45 (s, 9H) |
| 6-287 | NO₂ | SMe | SO₂Me | ¹H-NMR, MeOD, 400 MHz |
| | | | | 8.51 (d, 1H), 8.2 (d, 1H), 3.55 (s, 3H), 2.6 (s, 3H), 1.43 (s, 9H) |
| 6-288 | NO₂ | SMe | Br | ¹H-NMR, DMSO-d₆, 400 MHz |
| | | | | 11.25 (s, 1H), 8.25 (d, 1H), 7.92 (d, 1H), 2.45 (s, 3H), 1.35 (s, 9H) |
| 6-289 | NO₂ | SMe | Cl | ¹H-NMR, CDCl₃. 400 MHz |
| | | | | 7.75 (s, 2H), 2.48 (s, 3H), 1.42 (s, 9H) |
| 6-290 | Cl | CH₂OEt | SO₂Et | ¹H-NMR, CDCl_{3,}, 400 MHz |
| | | | | 8.03 (d, 1H), 7.85 (s, 1H), 7.78 (d, 1H), 5.08 (s, 2H), 3.69 (q, 2H), 3.4 (q, 2H), 1.48 (s, 9H), 1.25 (t, 3H), 1.24 (t, 3H) |
| 6-291 | Cl | CH₂OC₂H₄OC₂H₄OM e | SO₂Me | ¹H-NMR, CDCl_{3,}, 400 MHz |
| | | | | 8.08 (d, 1H), 7.96 (s, 1H), 7.75 (d, 1H), 5.19 (s, 2H), 3.82 (dd, 2H), 3.68 (dd, 2H), 3.58 (dd, 2H), 3.48 (dd, 2H), 3.31 (s, 3H), 3.29 (s, 3H), 1.49 (s, 9H) |
| 6-292 | Cl | CH₂OMe | SO₂Et | ¹H-NMR, CDCl_{3,}, 400 MHz |
| | | | | 8.01 (d, 1H), 7.92 (s, 1H), 7.77 (d, 1H), 5.03 (s, 2H), 3.5 (s, 3H), 3.38 (q, 2H), 1.48 (s, 9H), 1.22 (t, 3H) |
| 6-293 | Cl | 5-Methoxymethyl-5-methyl- 4,5-dihydro-1,2-oxazol-3 yl | SO₂Et | ¹H-NMR, CDCl₃, 400 MHz |
| | | | | 8.09 (d, 1H), 7.91 (d, 1H), 7.82 (s, 1H), 3.53 (q, 2H), 3.48 (d, 1H), 3.45 (s, 3H), 3.4 (q, 2H), 3,09 (d, 1H), 1.55 (s, 3H), 1.48 (s, 9H), 1.28 (t, 3H) |
| 6-294 | Cl | 5-Methoxymethyl-4,5-dihydro-1,2-oxazol-3 yl | SO₂Et | ¹H-NMR, CDCl₃, 400 MHz 8.09 (d, 1H), 7.91 (d, 1H), 7.89 (s, 1 H), 5.05 (m, 1H), 3.69 (dd, 1H), 3.6 (dd, 1H), 3.47 (dd, 1H), 3.45 (s, 3H), 3.38 (q, 2H), 3.25 (dd, 1H), 1.48 (s, 9H), 1.28 (t, 3H) |
| 6-295 | Et | SOMe | CF₃ | ¹H-NMR, CDCl₃, 400 MHz |
| | | | | 8.4 (s, br, 1H), 7.69 (d, 1H), 7.62 (d, 1H), 3.48 (q, 2H), 3.0 (s, 3H), 1.48 (s, 9H), 1.28 (t, 3H) |
| 6-296 | Cl | 5-Ethoxymethyl- 4,5-dihydro-1,2-oxazol-3 yl | SO₂Et | ¹H-NMR, CDCl₃, 400 MHz |
| | | | | 8.12 (d, 1H), 7.95 (d, 1H), 7.75 (s, 1H), 5.05 (m, 1H), 3.73 (dd, 1H), 3.65 (dd, 1H), 3.62 (q, 2H), 3.5 (dd, 1H), 3.38 (q, 2H), 3.29 (dd, 1H), 1.48 (s, 9H), 1.29 (t, 3H), 1.22 (t, 3H) |
| 6-308 | Cl | NMe₂ | Cl | |
| 6-309 | CH₂O(CH₂)₂ OMe | NH(CH₂)₃OMe | SO₂Me | |
| 6-313 | Me | SO₂(CH₂)₂OMe | CF₃ | |
| 6-314 | Me | SOEt | SO₂Me | |
| 6-315 | Me | SO₂Et | SO₂Me | |
| 6-316 | Me | SMe | 1,2,4-Triazol-1-yl | |
| 6-317 | OEt | SMe | CF₃ | |
| 6-318 | Me | S(CH₂)₂OMe | CF₃ | |
| 6-319 | Me | SOMe | 1,2,4-triazol-1-yl | |
| 6-320 | OEt | SOMe | CF₃ | |
| 6-321 | Me | SO(CH₂)₂OMe | CF₃ | |
| 6-322 | Me | SCH₂CCMe | SO₂Me | |
| 6-323 | Me | S-c-Pen | SO₂Me | |
| 6-324 | OMe | SMe | OMe | |
| 6-325 | Me | SCH₂CH=CHCH₃ | SO₂Me | |
| 6-326 | Me | SOCH2CCMe | SO₂Me | |
| 6-327 | Me | SO₂-c-Pen | SO₂Me | |
| 6-328 | Me | SO-c-Pen | SO₂Me | |
| 6-329 | Me | S(CH2)3Cl | SO₂Me | |
| 6-330 | Me | SCH₂(4-F-Ph) | SO₂Me | |
| 6-331 | Me | SO₂CH₂CCMe | SO₂Me | |
| 6-332 | Me | SO₂CH₂CH=CHCH₃ | SO₂Me | |
| 6-333 | Me | SOCH₂CH=CHCH₃ | SO₂Me | |
| 6-334 | Me | SOCH₂-Epoxy-CH₃ | SO₂Me | |
| 6-335 | Me | SO₂(CH₂)3Cl | SO₂Me | |
| 6-336 | Me | SO(CH₂)3Cl | SO₂Me | |
| 6-337 | Me | SOCH₂(4-F-Ph) | SO₂Me | |
| 6-338 | Me | SO₂CH₂(4-F-Ph) | SO₂Me | |
| 6-339 | Me | SO₂Me | C₂F₅ | |
| 6-340 | O(CH₂)₂OMe | SMe | CF₃ | |
| 6-341 | O(CH₂)₂OMe | SO₂Me | CF₃ | |
| 6-342 | O(CH₂)₂OMe | SOMe | CF₃ | |
| 6-343 | Me | S(CH₂)₂OCH₂CF₃ | SO₂Me | |
| 6-344 | Me | SO(CH₂)₂OCH₂CF₃ | SO₂Me | |
| 6-345 | Me | SO₂(CH₂)₂OCH₂CF₃ | SO₂Me | |
| 6-346 | OEt | SEt | CF₃ | |
| 6-347 | O-CH₂-c-Pr | SMe | CF₃ | |
| 6-348 | OMe | SEt | CF₃ | |
| 6-349 | OMe | SO₂Et | CF₃ | |
| 6-350 | OMe | SOEt | CF₃ | |
| 6-351 | OEt | SO₂Et | CF₃ | |
| 6-352 | OEt | SOEt | CF₃ | |
| 6-353 | O-CH₂-c-Pr | SOMe | CF₃ | |
| 6-354 | O-CH₂-c-Pr | SO₂Me | CF₃ | |
| 6-355 | Me | SEt | SO₂Me | |

**Tabelle 7: Erfindungsgemäße Verbindungen der allgemeinen Formel (I), worin R für Trifluormethyl steht**

| | | | | |
|---|---|---|---|---|
| 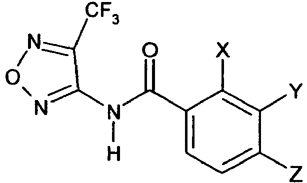 | | | | |

| **Nr.** | **X** | **Y** | **Z** | **Physikalische Daten** |
|---|---|---|---|---|
| 7-1 | CF₃ | OCH₂CON(Me)Et | SO₂Me | |
| 7-2 | CF₃ | OCH₂CON(Me)Et | SO₂Et | |
| 7-4 | CF₃ | 2-(1H-Pyrazol-1-yl)ethoxyl | SO₂Me | |
| 7-5 | CF₃ | 2-(1H-Pyrazol-1-yl)ethoxyl | SO₂Et | |
| 7-6 | CF₃ | Tetrahydrofuran-2-yl-methoxy | SO₂Me | |
| 7-7 | CF₃ | Tetrahydrofuran-2-yl-methoxy | SO₂Et | |
| 7-8 | CF₃ | OH | SO₂Me | |
| 7-9 | CF₃ | OH | SO₂Et | |
| 7-10 | CF₃ | SH | SO₂Me | |
| 7-11 | CF₃ | SH | SO₂Et | |
| 7-15 | CF₃ | SMe | SO₂Me | |
| 7-16 | CF₃ | SMe | SO₂Et | |
| 7-17 | CF₃ | S(O)Me | SO₂Me | |
| 7-24 | CF₃ | S(O)Me | SO₂Et | |
| 7-25 | CF₃ | S(O)₂Me | SO₂Me | |
| 7-26 | CF₃ | S(O)₂Me | SO₂Et | |
| 7-27 | CF₃ | 2-[(Methylsulfonyl) amino]ethoxy | SO₂Me | |
| 7-28 | CF₃ | 2-[(Methylsulfonyl) amino]ethyl}sulfanyl | SO₂Me | |
| 7-29 | CF₃ | 2-[(Methylsulfonyl) amino]ethyl}sulfanyl | SO₂Et | |
| 7-30 | NO₂ | O(CH₂)₂OMe | OMe | |
| 7-31 | NO₂ | OMe | Me | |
| 7-32 | NO₂ | NH₂ | OMe | |
| 7-33 | NO₂ | NH₂ | SO₂Et | |
| 7-34 | NO₂ | NH₂ | Cl | |
| 7-35 | NO₂ | NHMe | Cl | |
| 7-36 | NO₂ | NMe₂ | Cl | |
| 7-37 | NO₂ | NH₂ | Br | |
| 7-38 | NO₂ | NHMe | Br | |
| 7-39 | NO₂ | NMe₂ | Br | |
| 7-40 | NO₂ | NH₂ | F | |
| 7-41 | NO₂ | NHMe | F | |
| 7-42 | NO₂ | NMe₂ | F | |
| 7-43 | NO₂ | NH₂ | SO₂Me | |
| 7-44 | NO₂ | NHMe | SO₂Me | |
| 7-45 | NO₂ | NMe₂ | SO₂Me | |
| 7-46 | NO₂ | NH₂ | 1H-1,2,4-triazol-1-yl | |
| 7-47 | NO₂ | NHMe | 1H-1,2,4-triazol-1-yl | |
| 7-48 | NO₂ | NMe₂ | 1 H-1,2,4-triazol-1-yl | |
| 7-49 | Me | F | F | ¹H-NMR, DMSO-d₆, 400 MHz |
| | | | | 11,26 (s, 1H), 7,49 (m, 2H), 2,8 (q, 2H), 2,38 (s, 3H), 1,25 (t, 3H) |
| 7-50 | Me | F | Cl | ¹H-NMR, DMSO-d₆, 400 MHz |
| | | | | 11,32 (s, 1H), 7,61 (t,1H), 7,48 (d, 1H), 2,79 (q, 2H), 2,35 (s,1H),1,26 (t, 3H) |
| 7-51 | Me | SMe | CF₃ | |
| 7-52 | Me | Cl | SO₂Me | ¹H-NMR, DMSO-d₆, 400 MHz |
| | | | | 11,45 (s, 1H), 8,05 (d, 1H), 7,79 (d,1H), 3,44 (s, 3H), 2,80 (q, 2H), 2,48 (s, 3H), 1,28 (t, 3H) |
| 7-53 | Me | SO₂Me | SO₂Me | ¹H-NMR, DMSO-d₆, 400 MHz |
| | | | | 11,50 (s, 1H), 8,27 (d, 1H), 8,08 (d,1H), 3,60 (s, 3H), 3,58 (s, 3H), 2,81 (q, 2H), 2,71 (s, 3H), 1,27 (t, 3H) |
| 7-54 | Me | SO₂Me | CF₃ | ¹H-NMR, CDCl₃, 400 MHz |
| | | | | 8,05 (bs, 1H), 7,88 (d, 1H), 7,78 (d, 1H), 3,20 (s, 3H), 2,90 (q, 2H), 2,75 (s, 3H), 1,40 (t, 3H) |
| 7-55 | Me | Cl | CF₃ | |
| 7-56 | Me | S(O)Me | CF₃ | ¹H-NMR, DMSO-d₆, 400 MHz |
| | | | | 11,44 (s, 1H), 7,88 (m, 2H), 3,05 (s, 3H), 2,87 (s, 3H), 2,82 (q, 2H), 1,28 (t, 3H) |
| 7-57 | Me | SEt | OMe | |
| 7-58 | Me | NMe₂ | SO₂Me | ¹H-NMR, DMSO-d₆, 400 MHz |
| | | | | 11,32 (s, 1H), 7,88 (d, 1H), 7,60 (d,1H), 3,31 (s, 3H), 2,84 (s, 6H), 2,80 (q, 2H), 2,39 (s, 3H), 1,28 (t, 3H) |
| 7-59 | Me | NH(CH₂)₂OMe | SO₂Me | ¹H-NMR, CDCl₃, 400 MHz |
| | | | | 8,84 (bs, 1H), 7,52 (d, 1H), 7,09 (d, 1H), 5,68 (bs, 1H), 3,57 (m, 2H), 3,35 (m, 5H), 3,13 (s, 3H), 2,90 (q, 2H), 2,37 (s, 3H), 1,40 (t, 3H) |
| 7-60 | Me | O(CH₂)₄OMe | SO₂Me | |
| 7-61 | Me | NH₂ | SO₂Me | |
| 7-62 | Me | O(CH₂)₂-O(3,5-Dimethoxypyrimidin-2-yl | SO₂Me | |
| 7-63 | Me | O(CH₂)₂-O-NMe₂ | Cl | |
| 7-64 | Me | O(CH₂)₂-NH(CO)NMe₂ | Cl | |
| 7-65 | Me | O(CH₂)-5-Pyrrolidin-2-on | Br | |
| 7-66 | Me | O(CH₂)₂-NH(CO)NHCO₂Et | Cl | |
| 7-67 | Me | O(CH₂)- (CO)NEt₂ | Br | |
| 7-68 | Me | O(CH₂) -5-2,4-Dimethyl-2,4-dihydro-3H-1,2,4-triazol-3-on | Cl | |
| 7-69 | Me | O(CH₂)-3,5-Dimethyl-1,2-oxazol-4-yl | Cl | |
| 7-70 | Me | O(CH₂)₂-NHCO₂Me | Cl | |
| 7-71 | Me | 4,5-Dihydro-1,2-oxazol-3 yl | SO₂Me | ¹H-NMR, DMSO-d₆, 400 MHz |
| | | | | 11,46 (s, 1H), 8,04 (d, 1H), 7,90 (d, 1H), 4,50 (t, 2H), 3,35 (t, 2H), 3,27 (s, 3H), 2,82 (q, 2H), 2,35 (s, 3H), 1,27 (t, 3H) |
| 7-72 | Me | Me | SO₂Me | ¹H-NMR, DMSO-d₆, 400 MHz |
| | | | | 11,34 (s,1H), 7,92 (d, 1H), 7,59 (d, 1H), 3,27 (s, 3H), 2,80 (q, 2H), 2,66 (s, 3H), 2,36 (s,3H),1,27 (t, 3H) |
| 7-73 | Me | OH | SO₂Me | |
| 7-74 | Me | O-CH₂-NHSO₂cPr | Cl | |
| 7-75 | Me | O(CH₂)₂NHSO2Me | SO₂Me | |
| 7-76 | Me | S(O)Me | SO₂Me | |
| 7-77 | Me | SMe | SO₂Me | |
| 7-78 | Me | SMe | OMe | |
| 7-79 | Me | S(O)Me | OMe | |
| 7-80 | Me | SO₂Me | OMe | |
| 7-81 | Me | SMe | Cl | |
| 7-82 | Me | S(O)Me | Cl | |
| 7-83 | Me | SO₂Me | Cl | |
| 7-84 | Me | [1,4]Dioxan-2-yl-methoxy | SO₂Me | |
| 7-85 | Me | [1,4]Dioxan-2-yl-methoxy | SO₂Et | |
| 7-86 | Me | O(CH₂)₄OMe | SO₂Et | |
| 7-87 | Me | O(CH₂)₃OMe | SO₂Me | |
| 7-88 | Me | O(CH₂)₃OMe | SO₂Et | |
| 7-89 | Me | O(CH₂)₂OMe | SO₂Me | |
| 7-90 | Me | O(CH₂)₂OMe | SO₂Et | |
| 7-91 | Me | S(O)Me | SO₂Me | |
| 7-92 | Me | SMe | SO₂Me | |
| 7-93 | Me | SMe | OMe | |
| 7-94 | Me | S(O)Me | OMe | |
| 7-95 | Me | SO₂Me | OMe | |
| 7-96 | Me | SMe | Cl | |
| 7-97 | Me | S(O)Me | Cl | |
| 7-98 | Me | SO₂Me | Cl | |
| 7-99 | Me | SMe | Br | |
| 7-100 | Me | SOMe | Br | |
| 7-101 | Me | SO₂Me | Br | |
| 7-102 | Me | SMe | I | |
| 7-103 | Me | SOMe | I | |
| 7-104 | Me | SO₂Me | I | |
| 7-105 | Me | SEt | Cl | |
| 7-106 | Me | SOEt | Cl | |
| 7-107 | Me | SO₂Et | Cl | |
| 7-108 | Me | SEt | Br | |
| 7-109 | Me | SOEt | Br | |
| 7-110 | Me | SO₂Et | Br | |
| 7-111 | Me | SEt | I | |
| 7-112 | Me | SOEt | I | |
| 7-113 | Me | SO₂Et | I | |
| 7-114 | Me | SEt | F | |
| 7-115 | Me | SOEt | F | |
| 7-116 | Me | SO₂Et | F | |
| 7-117 | Cl | OCH₂(CO)NMe₂ | Cl | ¹H-NMR, DMSO-d₆, 400 MHz |
| | | | | 11,44 (s, 1H), 7,65 (d, 1H), 7,49 (d, 1H), 4,74 (s, 2H), 3,02 (s,3H), 2,87 (s, 3H), 2,81 (q, 2H), 1,27 (t, 3H) |
| 7-118 | Cl | Cl | SO₂Me | |
| 7-119 | Cl | CH₂OCH₂CF₃ | SO₂Me | ¹H-NMR, DMSO-d₆, 400 MHz |
| | | | | 11,60 (s, 1H), 8,12 (d, 1H), 7,98 (d, 1H), 5,27 (s, 2H), 4,30 (q, 2H), 3,38 (s, 3H), 2,83 (q, 2H), 1,30 (t, 3H) |
| 7-120 | Cl | 5-Cyanomethyl- 4,5-dihydro-1,2-oxazol-3yl | SO₂Et | ¹H-NMR, DMSO-d₆, 400 MHz |
| | | | | 11,62 (s, 1H), 8,11 (m, 2H), 5,20 (m, 1H), 3,60 (m, 1H), 3,44 (q, 2H), 3,15 (m, 1 H), 3,02 (m, 2H), 2,81 (q, 2H), 1,28 (t, 3H), 1,16 (t, 3H) |
| 7-121 | Cl | CH₂O-Tetrahydrofuran-3-yl | SO₂Me | ¹H-NMR, CDCl₃, 400 MHz |
| | | | | 8,70 (bs, 1H), 7,94 (d, 1H), 7,72 (d, 1H), 5,06 (m, 2H), 4,35 (m, 1H), 3,73-3,92 (m, 4H), 3,36 (q, 2H), 2,92 (q, 2H), 2,10 (m, 2H), 1,40 (t, 3H), 1,22 (t, 3H) |
| 7-122 | Cl | CH₂O-Tetrahydrofuran-2-yl | SO₂Me | ¹H-NMR, DMSO-d₆, 400 MHz |
| | | | | 11,55 (s, 1H), 8,14 (d, 1H), 7,93 (d, 1H), 3,58 (s, 3H), 2,81 (q, 2H), 2,48 (s,3H), 1,28 (t, 3H), |
| 7-123 | Cl | SMe | SO₂Me | |
| 7-124 | Cl | F | SMe | |
| 7-125 | Cl | CH₂OCH₂-Tetrahydrofuran-2-yl | SO₂Me | ¹H-NMR, CDCl₃, 400 MHz |
| | | | | 8,84 (bs, 1H), 8,0 (d, 1H), 7,70 (d, 1H), 5,12 (s, 2H), 4,05 (m, 1H), 3,55-3,80 (m, 4H), 3,28 (s, 3H), 2,90 (q, 2H), 1,78-2,0 (m, 3H), 1,48-1,59 (m, 1H), 1,40 (t, 3H) |
| 7-126 | Cl | CH₂OCH₂-Tetrahydrofuran-3-yl | SO₂Et | |
| 7-127 | Cl | O(CH₂)-5-Pyrrolidin-2-on | Cl | |
| 7-128 | Cl | SMe | Cl | ¹H-NMR, DMSO-d₆, 400 MHz |
| | | | | 11,41 (s, 1H), 7,22 (d, 1H), 7,68 (d,1H), 2,80 (q, 2H), 2,43 (s, 3H), 1,28 (t, 3H) |
| 7-129 | Cl | S(O)Me | SO₂Me | |
| 7-130 | Cl | CH₂O-Tetrahydrofuran-3-yl | SO₂Et | |
| 7-131 | Cl | O(CH₂)₂OMe | Cl | |
| 7-132 | Cl | O(CH₂)₂OMe | SO₂Me | |
| 7-133 | Cl | O(CH₂)₄OMe | SO₂Me | |
| 7-134 | Cl | O(CH₂)₄OMe | SO₂Et | |
| 7-135 | Cl | O(CH₂)₃OMe | SO₂Me | |
| 7-136 | Cl | O(CH₂)₃OMe | SO₂Et | |
| 7-137 | Cl | O(CH₂)₂OMe | SO₂Me | |
| 7-138 | Cl | O(CH₂)₂OMe | SO₂Et | |
| 7-139 | Cl | [1,4]Dioxan-2-yl-methoxy | SO₂Me | |
| 7-140 | Cl | [1,4]Dioxan-2-yl-methoxy | SO₂Et | |
| 7-141 | Cl | SO₂Me | Me | |
| 7-142 | Cl | SEt | Me | |
| 7-143 | Cl | SOEt | Me | |
| 7-144 | Cl | SO₂Et | Me | |
| 2-145 | Cl | 4,5-Dihydro-1,7-oxazol-3 yl | SO₂Me | |
| 7-146 | Cl | Cl | SO₂Me | ¹H-NMR, DMSO-d₆, 400 MHz |
| | | | | 11,6 (s, 1H), 8,16 (d, 1H), 7,94 (d, 1H), 3,49 (s, 3H), 3,82 (q, 2H), 1,28 (t, 3H) |
| 7-147 | F | SMe | CF₃ | |
| 7-148 | F | S(O)Me | CF₃ | ¹H-NMR, DMSO-d₆, 400 MHz |
| | | | | 11,75 (bs, 1H), 8,14 (dd, 1H), 7,90 (d, 1H), 2,81 (q, 2H), 1,78-2,0 (m, 3H), 1,28 (t, 3H) |
| 7-149 | OMe | SMe | CF₃ | |
| 7-150 | OMe | S(O)Me | CF₃ | |
| 7-151 | OMe | SO₂Me | CF₃ | |
| 7-152 | Et | NH(CH₂)₂OMe | SO₂Me | |
| 7-153 | Et | F | SO₂Me | |
| 7-154 | Et | SMe | CF₃ | |
| 7-155 | CF₃ | F | SO₂Me | |
| 7-156 | CF₃ | F | SO₂Et | ¹H-NMR, DMSO-d₆, 400 MHz |
| | | | | 11,68 (s, 1H), 8,3 (t, 1H), 7,89 (d, 1H), 3,49 (s, 3H), 2,79 (q, 2H), 1,27 (t, 3H) |
| 7-157 | CF₃ | O(CH₂)₂OMe | SO₂Et | |
| 7-158 | CF₃ | O(CH₂)₃OMe | SO₂Et | |
| 7-159 | CF₃ | O(CH₂)₂OMe | SO₂Me | |
| 7-160 | CF₃ | O(CH₂)₃OMe | SO₂Me | |
| 7-161 | CF₃ | OCH₂CONMe₂ | SO₂Me | |
| 7-162 | CF₃ | OCH₂CONMe₂ | SO₂Et | |
| 7-163 | CF₃ | OCH₂CONMe₂ | Cl | |
| 7-164 | CF₃ | OCH₂CONMe₂ | Br | |
| 7-165 | CF₃ | OCH₂CONMe₂ | I | |
| 7-166 | CF₃ | OCH₂CONMe₂ | F | |
| 7-167 | CF₃ | O(CH₂)₂OMe | Cl | |
| 7-168 | CF₃ | O(CH₂)₃OMe | Cl | |
| 7-169 | CF₃ | O(CH₂)₂OMe | Br | |
| 7-170 | CF₃ | O(CH₂)₃OMe | Br | |
| 7-171 | CF₃ | O(CH₂)₂OMe | I | |
| 7-172 | CF₃ | O(CH₂)₃OMe | I | |
| 7-173 | CF₃ | O(CH₂)₂OMe | F | |
| 7-174 | CF₃ | O(CH₂)₃OMe | F | |
| 7-175 | CF₃ | [1,4]Dioxan-2-yl-methoxy | SO₂Me | |
| 7-176 | CF₃ | [1,4]Dioxan-2-yl-methoxy | SO₂Et | |
| 7-177 | CF₃ | [1,4]Dioxan-2-yl-methoxy | Cl | |
| 2-178 | CF₃ | [1,4]Dioxan-2-yl-methoxy | Br | |
| 7-179 | CF₃ | [1,4]Dioxan-2-yl-methoxy | I | |
| 7-180 | CF₃ | [1,4]Dioxan-2-yl-methoxy | F | |
| 7-181 | Br | OMe | Br | |
| 7-182 | Br | O(CH₂)₂OMe | Br | |
| 7-183 | Br | O(CH₂)₄OMe | SO₂Me | |
| 7-184 | Br | O(CH₂)₄OMe | SO₂Et | |
| 7-185 | Br | O(CH₂)₃OMe | SO₂Me | |
| 7-186 | Br | O(CH₂)₃OMe | SO₂Et | |
| 7-187 | Br | O(CH₂)₂OMe | SO₂Me | |
| 7-188 | Br | O(CH₂)₂OMe | SO₂Et | |
| 7-189 | Br | [1,4]Dioxan-2-yl-methoxy | SO₂Me | |
| 7-190 | Br | [1,4]Dioxan-2-yl-methoxy | SO₂Et | |
| 7-191 | Br | SMe | Me | |
| 7-192 | Br | SOMe | Me | |
| 7-193 | Br | SO₂Me | Me | |
| 7-194 | Br | SEt | Me | |
| 7-195 | Br | SOEt | Me | |
| 7-196 | Br | SO₂Et | Me | |
| 7-197 | I | O(CH₂)₄OMe | SO₂Me | |
| 7-198 | I | O(CH₂)₄OMe | SO₂Et | |
| 7-199 | I | O(CH₂)₃OMe | SO₂Me | |
| 7-200 | I | O(CH₂)₃OMe | SO₂Et | |
| 7-201 | I | O(CH₂)₂OMe | SO₂Me | |
| 7-202 | I | O(CH₂)₂OMe | SO₂Et | |
| 7-203 | I | [1,4]Dioxan-2-yl-methoxy | SO₂Me | |
| 7-204 | I | [1,4]Dioxan-2-yl-methoxy | SO₂Et | |
| 7-205 | I | SMe | Me | |
| 7-206 | I | SOMe | Me | |
| 7-207 | I | SO₂Me | Me | |
| 7-208 | I | SEt | Me | |
| 7-209 | I | SOEt | Me | |
| 7-210 | I | SO₂Et | Me | |
| 7-211 | CH₂SMe | OMe | SO₂Me | |
| 7-212 | CH₂OMe | OMe | SO₂Me | |
| 7-213 | CH₂O(C H₂)₂OMe | NH(CH₂)₂OEt | SO₂Me | |
| 7-214 | CH₂O(C H₂)₂OMe | NH(CH₂)₃OEt | SO₂Me | |
| 7-215 | CH₂O(C H₂)₃OMe | OMe | SO₂Me | |
| 7-216 | CH₂O(C H₂)₂OMe | NH(CH₂)₂OMe | SO₂Me | |
| 7-217 | CH₂O(C H₂)₂OMe | NH(CH₂)₃OMe | SO₂Me | |
| 7-218 | SO₂Me | NH₂ | CF₃ | |
| 7-219 | SO₂Me | F | CF₃ | |
| 7-220 | SO₂Me | NHEt | Cl | |
| 7-221 | SMe | SEt | F | |
| 7-222 | SMe | SMe | F | |
| 7-223 | Me | NH₂ | Cl | |
| 7-224 | Me | NH₂ | Br | |
| 7-225 | Me | NHMe | Cl | |
| 7-226 | Me | NHMe | Br | |
| 7-227 | Me | NMe₂ | Cl | |
| 7-228 | Me | NMe₂ | Br | |
| 7-227 | Me | NMe₂ | Cl | |
| 7-228 | Me | NMe₂ | Br | |
| 7-229 | NO₂ | O(CH₂)₂OMe | Me | |
| 7-230 | CF₃ | S(O)₂Et | SO₂Me | |
| 7-231 | CF₃ | S(O)₂Et | SO₂Et | |
| 7-232 | CF₃ | SCH₂CONMe₂ | SO₂Me | |
| 7-233 | CF₃ | SCH₂CONMe₂ | SO₂Et | |
| 7-234 | CF₃ | SCH₂COOH | SO₂Me | |
| 7-235 | CF₃ | SCH₂COOH | SO₂Et | |
| 7-236 | Me | SO₂-CH₂-CH₂-CH₌CH₂ | CF₃ | |
| 7-237 | Cl | Me | SO₂Et | |
| 7-238 | CF₃ | SEt | SO₂Me | |
| 7-239 | OMe | NO2 | Cl | |
| 7-240 | OMe | NH(CO)i-Pr | Cl | |
| 7-241 | OMe | NH(CO)CH2Ph | Cl | |
| 7-242 | CF₃ | SEt | SO₂Et | |
| 7-243 | CF₃ | S(O)Et | SO₂Me | |
| 7-244 | Cl | Me | Cl | |
| 7-245 | Me | 3,5-Dimethyl-pyrazol-1-yl | SO₂Me | |
| 7-247 | Me | 1,2,3-Triazol-1-yl | SO₂Me | |
| 7-248 | Me | Me | SMe | |
| 7-249 | Me | Pyrolidin-2-on-1-yl | SO₂Me | |
| 7-250 | CF₃ | S(O)Et | SO₂Et | |
| 7-251 | Cl | Pyrazol-1-yl | SO₂Me | |
| 7-252 | Me | 3-Methyl-pyrazol-1-yl | SO₂Me | |
| 7-253 | Cl | CH₂-N(Et)OMe | SO₂Me | |
| 7-254 | Me | Me | Cl | |
| 7-255 | OH | Cl | Cl | |
| 7-256 | Me | 1,2,4-Triazol-1-yl | SO₂Me | |
| 7-257 | Me | 4-Methoxy-pyrazol-1-yl | SO₂Me | |
| 7-258 | Me | 1,2,4-triazol-1-yl | CF₃ | |
| 7-259 | Me | Tetrahydro-pyrimidin-2(1H)-one-1-yl | SO₂Me | |
| 7-260 | Me | NH-(CH₂)₂-O(CO)Et | SO₂Me | |
| 7-261 | Me | NH-iPr | SO₂Me | |
| 7-262 | Cl | NH-CH₂-(CO)NHEt | Cl | |
| 7-263 | Me | NH-CH₂-(CO)NMe2 | SO₂Me | |
| 7-264 | Me | NH-CH₂-furan-2-yl | SO₂Me | |
| 7-265 | Me | NH-CH₂-(CO)NHEt | SO₂Me | |
| 7-266 | Me | F | SO₂Me | |
| 7-267 | F | SO₂Me | SO₂Me | |
| 7-268 | Cl | (4-Cyclopropyl-3-methyl-5-oxo-4,5-dihydro-1H-1,2,4-triazol-1-yl)-methyl | Cl | |
| 7-269 | Cl | [4-Methyl-5-oxo-3-(2,2,2-trifluorethoxy)-4,5-dihydro-1H-1,2,4-triazol-1-yl]-methyl | Cl | |
| 7-270 | Cl | (3-Isopropoxy-4-methyl-5-oxo-4,5-dihydro-1H-1,2,4-triazol-1-yl]-methyl | Cl | |
| 7-271 | Cl | (4-Methyl-5-oxo-4,5-dihydro-1H-1,2,4-triazol-1-yl)-methyl | Cl | |
| 7-272 | Me | Cl | SO₂Et | |
| 7-273 | SO₂Me | F | Cl | |
| 7-274 | Me | 1,2,3-Triazol-1-yl | SO₂Me | |
| 7-275 | Me | Isobutyl(methyl)carbamoylamino | SO₂Me | |
| 7-276 | Me | 3-Oxo-morpholin-4-yl | SO₂Me | |
| 7-277 | OMe | [Ethyl(methylsulfonyl)a mino]methyl | Cl | |
| 7-278 | F | SO₂Me | CF₃ | |
| 7-279 | OMe | Benzoylamino | Cl | |
| 7-280 | OMe | Cyclopropylcarbonylamino | Cl | |
| 7-281 | OMe | Propionyllamino | Cl | |
| 7-282 | NO₂ | SO₂Me | SO₂Me | |
| 7-283 | NO₂ | SO₂Me | Cl | |
| 7-284 | NO₂ | SOMe | SO₂Me | |
| 7-285 | NO₂ | SOMe | Br | |
| 7-286 | NO₂ | SOMe | Cl | |
| 7-287 | NO₂ | SMe | SO₂Me | |
| 7-288 | NO₂ | SMe | Br | |
| 7-289 | NO₂ | SMe | Cl | |
| 7-290 | Cl | CH₂OCH(CH₃)₂ | SO₂Et | |
| 7-291 | Cl | CH₂OEt | SO₂Et | |
| 7-292 | Cl | CH₂OMe | SO₂Et | |
| 7-293 | Cl | CH₂OCH₂C₂F₅ | SO₂Me | |
| 7-294 | Cl | CH₂OCH₂CHF₂ | SO₂Me | |
| 7-295 | Cl | CH₂OCH₂CCH | SO₂Et | |
| 7-296 | Cl | CH₂OC₂H₄OMe | SO₂Me | |
| 7-297 | Cl | CH₂(OC₂H₄)₂OMe | SO₂Me | |
| 7-298 | Cl | 5-Ethoxymethyl- 4,5-dihydro-1,2-oxazol-3yl | SO₂Et | |
| 7-299 | Cl | 5-Methoxymethyl- 4,5-dihydro-1,2-oxazol-3yl | SO₂Et | |
| 7-300 | Et | SOMe | CF₃ | |
| 7-301 | iPr | SMe | CF₃ | |
| 7-302 | Et | SMe | CF₃ | |
| 7-303 | Et | SO₂Me | CF₃ | |
| 7-304 | cPr | SOMe | CF₃ | |
| 7-305 | CH=CH₂ | SMe | CF₃ | |
| 7-306 | Et | SMe | Cl | |
| 7-307 | Et | SO₂Me | Cl | |
| 7-308 | Cl | NMe₂ | Cl | |
| 7-309 | CH₂O(CH₂)₂OMe | NH(CH₂)₃OMe | SO₂Me | |
| 7-313 | Me | SO₂(CH₂)₂OMe | CF₃ | |
| 7-314 | Me | SOEt | SO₂Me | |
| 7-315 | Me | SO₂Et | SO₂Me | |
| 7-316 | Me | SMe | 1,2,4- triazol-1-yl | |
| 7-317 | OEt | SMe | CF₃ | |
| 7-318 | Me | S(CH₂)₂OMe | CF₃ | |
| 7-319 | Me | SOMe | 1,2,4-triazol-1-yl | |
| 7-320 | OEt | SOMe | CF₃ | |
| 7-321 | Me | SO(CH₂)₂OMe | CF₃ | |
| 7-322 | Me | SCH₂CCMe | SO₂Me | |
| 7-323 | Me | S-c-Pen | SO₂Me | |
| 7-324 | OMe | SMe | OMe | |
| 7-325 | Me | SCH₂CH=CHCH₃ | SO₂Me | |
| 7-326 | Me | SOCH₂CCMe | SO₂Me | |
| 7-327 | Me | SO₇₋c-Pen | SO₂Me | |
| 7-328 | Me | SO-c-Pen | SO₂Me | |
| 7-329 | Me | S(CH₂)₃Cl | SO₂Me | |
| 7-330 | Me | SCH₂(4-F-Ph) | SO₂Me | |
| 7-331 | Me | SO₂CH₂CCMe | SO₂Me | |
| 7-332 | Me | SO₂CH₂CH=CHCH₃ | SO₂Me | |
| 7-333 | Me | SOCH₂CH=CHCH₃ | SO₂Me | |
| 7-334 | Me | SOCH₂-Epoxy-Me | SO₂Me | |
| 7-335 | Me | SO₂(CH₂)₃Cl | SO₂Me | |
| 7-336 | Me | SO(CH₂)₃Cl | SO₂Me | |
| 7-337 | Me | SOCH₂(4-F-Ph) | SO₂Me | |
| 7-338 | Me | SO₂CH₂(4-F-Ph) | SO₂Me | |
| 7-339 | Me | SO₂Me | C₂F₅ | |
| 7-340 | O(CH₂)₂ OMe | SMe | CF₃ | |
| 7-341 | O(CH₂)₂ OMe | SO₂Me | CF₃ | |
| 7-342 | O(CH₂)₂ OMe | SOMe | CF₃ | |
| 7-343 | Me | S(CH₂)₂OCH₂CF₃ | SO₂Me | |
| 7-344 | Me | SO(CH₂)₂OCH₂CF₃ | SO₂Me | |
| 7-345 | Me | SO₂(CH₂)₂OCH₂CF₃ | SO₂Me | |
| 7-346 | OEt | SEt | CF₃ | |
| 7-347 | O-CH₂-c-Pr | SMe | CF₃ | |
| 7-348 | OMe | SEt | CF₃ | |
| 7-349 | OMe | SO₂Et | CF₃ | |
| 7-350 | OMe | SOEt | CF₃ | |
| 7-351 | OEt | SO₂Et | CF₃ | |
| 7-352 | OEt | SOEt | CF₃ | |
| 7-353 | O-CH₂₋c-Pr | SOMe | CF₃ | |
| 7-354 | O-CH₂₋c-Pr | SO₂Me | CF₃ | |
| 7-355 | Me | SEt | SO₂Me | |

**Tabelle 8: Erfindungsgemäße Verbindungen der allgemeinen Formel (I), worin R für Cyano steht**

| | | | | |
|---|---|---|---|---|
| 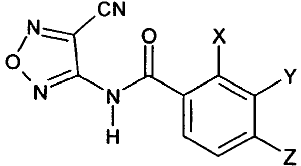 | | | | |

| **Nr.** | **X** | **Y** | **Z** | **Physikalische Daten** |
|---|---|---|---|---|
| 8-1 | CF₃ | OCH₂CON(Me)Et | SO₂Me | |
| 8-2 | CF₃ | OCH₂CON(Me)Et | SO₂Et | |
| 8-4 | CF₃ | 2-(1H-Pyrazol-1-yl)ethoxyl | SO₂Me | |
| 8-5 | CF₃ | 2-(1H-Pyrazol-1-yl)ethoxyl | SO₂Et | |
| 8-6 | CF₃ | Tetrahydrofuran-2-yl-methoxy | SO₂Me | |
| 8-7 | CF₃ | Tetrahydrofuran-2-yl-methoxy | SO₂Et | |
| 8-8 | CF₃ | OH | SO₂Me | |
| 8-9 | CF₃ | OH | SO₂Et | |
| 8-10 | CF₃ | SH | SO₂Me | |
| 8-11 | CF₃ | SH | SO₂Et | |
| 8-15 | CF₃ | SMe | SO₂Me | |
| 8-16 | CF₃ | SMe | SO₂Et | |
| 8-17 | CF₃ | S(O)Me | SO₂Me | |
| 8-24 | CF₃ | S(O)Me | SO₂Et | |
| 8-25 | CF₃ | S(O)₂Me | SO₂Me | |
| 8-26 | CF₃ | S(O)₂Me | SO₂Et | |
| 8-27 | CF₃ | 2-[(Methylsulfonyl) amino]ethoxy | SO₂Me | |
| 8-28 | CF₃ | 2-[(Methylsulfonyl) amino]ethyl}sulfanyl | SO₂Me | |
| 8-29 | CF₃ | 2-[(Methylsulfonyl) amino]ethyl}sulfanyl | SO₂Et | |
| 8-30 | NO₂ | O(CH₂)₂OMe | OMe | |
| 8-31 | NO₂ | OMe | Me | |
| 8-32 | NO₂ | NH₂ | OMe | |
| 8-33 | NO₂ | NH₂ | SO₂Et | |
| 8-34 | NO₂ | NH₂ | Cl | |
| 8-35 | NO₂ | NHMe | Cl | |
| 8-36 | NO₂ | NMe₂ | Cl | |
| 8-37 | NO₂ | NH₂ | Br | |
| 8-38 | NO₂ | NHMe | Br | |
| 8-39 | NO₂ | NMe₂ | Br | |
| 8-40 | NO₂ | NH₂ | F | |
| 8-41 | NO₂ | NHMe | F | |
| 8-42 | NO₂ | NMe₂ | F | |
| 8-43 | NO₂ | NH₂ | SO₂Me | |
| 8-44 | NO₂ | NHMe | SO₂Me | |
| 8-45 | NO₂ | NMe₂ | SO₂Me | |
| 8-46 | NO₂ | NH₂ | 1 H-1,2,4-triazol-1-yl | |
| 8-47 | NO₂ | NHMe | 1 H-1,2,4-triazol-1-yl | |
| 8-48 | NO₂ | NMe₂ | 1H-1,2,4-triazol-1-yl | |
| 8-49 | Me | F | F | |
| 8-50 | Me | F | Cl | |
| 8-51 | Me | SMe | CF₃ | |
| 8-52 | Me | Cl | SO₂Me | ¹H-NMR, DMSO-d₆, 400 MHz |
| | | | | 12,67 (s, 1H), 8,08 (d, 1H), 7,80 (d, 1H), 3,45 (s, 3H) |
| 8-53 | Me | SO₂Me | SO₂Me | ¹H-NMR, DMSO-d₆, 400 MHz |
| | | | | 12,68 (bs, 1H), 8,29 (d, 1H), 8,07 (d, 1H), 3,61 (s, 3H), 3,59 (s, 3H), 2,72 (s, 3H) |
| 8-54 | Me | SO₂Me | CF₃ | ¹H-NMR, DMSO-d₆, 400 MHz |
| | | | | 12,64 (bs, 1H), 8,09 (d, 1H), 8,02 (d, 1 H), 3,43 (s, 3H), 2,77 (s, 3H) |
| 8-55 | Me | Cl | CF₃ | |
| 8-56 | Me | S(O)Me | CF₃ | ¹H-NMR, DMSO-d₆, 400 MHz |
| | | | | 12,63 (s, 1H), 7,92 (d, 1H), 7,88 (d, 1H), 3,06 (s, 3H), 2,88 (s, 3H) |
| 8-57 | Me | SEt | OMe | |
| 8-58 | Me | NMe₂ | SO₂Me | |
| 8-59 | Me | NH(CH₂)₂OMe | SO₂Me | |
| 8-60 | Me | O(CH₂)₄OMe | SO₂Me | |
| 8-61 | Me | NH₂ | SO₂Me | |
| 8-62 | Me | O(CH₂)₂-O(3,5-Dimethoxypyrimidin-2-yl | SO₂Me | |
| 8-63 | Me | O(CH2)2-O-NMe₂ | Cl | |
| 8-64 | Me | O(CH₂)₂-NH(CO)NMe₂ | Cl | |
| 8-65 | Me | O(CH₂)-5-Pyrrolidin-2-on | Br | |
| 8-66 | Me | O(CH₂)₂-NH(CO)NHCO₂Et | Cl | |
| 8-67 | Me | O(CH₂)-(CO)NEt₂ | Br | |
| 8-68 | Me | O(CH₂)-5-2,4-Dimethyl-2,4-dihydro-3H-1,2,4-triazol-3-on | Cl | |
| 8-69 | Me | O(CH₂)-3,5-Dimethyl-1,2-oxazol-4-yl | Cl | |
| 8-70 | Me | O(CH₂)₂-NHCO₂Me | Cl | |
| 8-71 | Me | 4,5-Dihydro-1,2-oxazol-3 yl | SO₂Me | |
| 8-72 | Me | Me | SO₂Me | ¹H-NMR, DMSO-d₆, 600 MHz |
| | | | | 12,55 (s, 1H), 7,93 (d, 1H), 7,62 (d, 1H), 3,29 (s, 3H), 2,64 (s,3H), 2,38 (s,3H) |
| 8-73 | Me | OH | SO₂Me | |
| 8-74 | Me | O-CH₂-NHSO₂cPr | Cl | |
| 8-75 | Me | O(CH₂)₂NHSO2Me | SO₂Me | |
| 8-76 | Me | S(O)Me | SO₂Me | |
| 8-77 | Me | SMe | SO₂Me | |
| 8-78 | Me | SMe | OMe | |
| 8-79 | Me | S(O)Me | OMe | |
| 8-80 | Me | SO₂Me | OMe | |
| 8-81 | Me | SMe | Cl | |
| 8-82 | Me | S(O)Me | Cl | |
| 8-83 | Me | SO₂Me | Cl | |
| 8-84 | Me | [1,4]Dioxan-2-yl-methoxy | SO₂Me | |
| 8-85 | Me | [1,4]Dioxan-2-yl-methoxy | SO₂Et | |
| 8-86 | Me | O(CH₂)₄OMe | SO₂Et | |
| 8-87 | Me | O(CH₂)₃OMe | SO₂Me | |
| 8-88 | Me | O(CH₂)₃OMe | SO₂Et | |
| 8-89 | Me | O(CH₂)₂OMe | SO₂Me | |
| 8-90 | Me | O(CH₂)₂OMe | SO₂Et | |
| 8-91 | Me | S(O)Me | SO₂Me | |
| 8-92 | Me | SMe | SO₂Me | |
| 8-93 | Me | SMe | OMe | |
| 8-94 | Me | S(O)Me | OMe | |
| 8-95 | Me | SO₂Me | OMe | |
| 8-96 | Me | SMe | Cl | |
| 8-97 | Me | S(O)Me | Cl | |
| 8-98 | Me | SO₂Me | Cl | |
| 8-99 | Me | SMe | Br | |
| 8-100 | Me | SOMe | Br | |
| 8-101 | Me | SO₂Me | Br | |
| 8-102 | Me | SMe | I | |
| 8-103 | Me | SOMe | I | |
| 8-104 | Me | SO₂Me | I | |
| 8-105 | Me | SEt | Cl | |
| 8-106 | Me | SOEt | Cl | |
| 8-107 | Me | SO₂Et | Cl | |
| 8-108 | Me | SEt | Br | |
| 8-109 | Me | SOEt | Br | |
| 8-110 | Me | SO₂Et | Br | |
| 8-111 | Me | SEt | I | |
| 8-112 | Me | SOEt | I | |
| 8-113 | Me | SO₂Et | I | |
| 8-114 | Me | SEt | F | |
| 8-115 | Me | SOEt | F | |
| 8-116 | Me | SO₂Et | F | |
| 8-117 | Cl | OCH₂(CO)NMe₂ | Cl | |
| 8-118 | Cl | Cl | SO₂Me | ¹H-NMR, DMSO-d₆, 400 MHz |
| | | | | 12,82 (s, 1H), 8,21 (d, 1H), 7,92 (d, 1H), 3,50 (s, 3H) |
| 8-119 | Cl | CH₂OCH₂CF₃ | SO₂Me | ¹H-NMR, DMSO-d₆, 400 MHz |
| | | | | 12,80 (s, 1H), 8,15 (d, 1H), 7,95 (d, 1H), 5,25 (s, 2H), 4,30 (q, 2H), 3,37 (s, 3H) |
| 8-120 | Cl | 5-Cyanomethyl-4,5-dihydro-1,2-oxazol-3yl | SO₂Et | ¹H-NMR, DMSO-d₆, 400 MHz |
| | | | | 12,80 (s, 1H), 8,18 (d, 1H), 8,09 (d, 1H), 5,19 (m, 1H), 3,61 (m, 1H), 3,45 (q, 2H), 3,16 (m, 1H), 3,02 (m, 2H), 1,16 (t, 3H) |
| 8-121 | Cl | CH₂O-Tetrahydrofuran-3-yl | SO₂Me | |
| 8-122 | Cl | CH₂O-Tetrahydrofuran-2-yl | SO₂Me | ¹H-NMR, CDCl₃, 400 MHz |
| | | | | 10,92 (s, 1H), 7,78 (d, 1H), 7,70 (d, 1H), 5,0 (m, 2H), 4,32 (m, 1H), 3,70-3,88 (m, 4H), 3,36 (q, 2H), 2,06 (m, 2H), 1,22 (t, 3H) |
| 8-123 | Cl | SMe | SO₂Me | |
| 8-124 | Cl | F | SMe | |
| 8-125 | Cl | CH₂OCH₂-Tetrahydrofuran-2-yl | SO₂Me | ¹H-NMR, DMSO-d₆, 400 MHz |
| | | | | 12,78 (s, 1H), 8,16 (d, 1H), 7,92 (d, 1H), 5,11 (m, 2H), 3,99 (m, 1H), 3,71 (q,1H), 3,63-3,54 (m,3H), 3,39 (s,3H), 1,95-1,72 (m, 3H), 1,54 (m,1H) |
| 8-126 | Cl | CH₂OCH₂-Tetrahydrofuran-3-yl | SO₂Et | |
| 8-127 | Cl | O(CH₂)-5-Pyrrolidin-2-on | Cl | |
| 8-128 | Cl | SMe | Cl | ¹H-NMR, DMSO-d₆, 400 MHz |
| | | | | 12,62 (bs, 1H), 7,77 (d, 1H), 7,67 (d, 1H), 2,42 (s, 3H), |
| 8-129 | Cl | S(O)Me | SO₂Me | |
| 8-130 | Cl | CH₂O-Tetrahydrofuran-3-yl | SO₂Et | |
| 8-131 | Cl | O(CH₂)₂OMe | Cl | |
| 8-132 | Cl | O(CH₂)₂OMe | SO₂Me | |
| 8-133 | Cl | O(CH₂)₄OMe | SO₂Me | |
| 8-134 | Cl | O(CH₂)₄OMe | SO₂Et | |
| 8-135 | Cl | O(CH₂)₃OMe | SO₂Me | |
| 8-136 | Cl | O(CH₂)₃OMe | SO₂Et | |
| 8-137 | Cl | O(CH₂)₂OMe | SO₂Me | |
| 8-138 | Cl | O(CH₂)₂OMe | SO₂Et | |
| 8-139 | Cl | [1,4]Dioxan-2-yl-methoxy | SO₂Me | |
| 8-140 | Cl | [1,4]Dioxan-2-yl-methoxy | SO₂Et | |
| 8-141 | Cl | SO₂Me | Me | |
| 8-142 | Cl | SEt | Me | |
| 8-143 | Cl | SOEt | Me | |
| 8-144 | Cl | SO₂Et | Me | |
| 2-145 | Cl | 4,5-Dihydro-1,8-oxazol-3 yl | SO₂Me | |
| 8-146 | Cl | Cl | SO₂Me | |
| 8-147 | F | SMe | CF₃ | |
| 8-148 | F | S(O)Me | CF₃ | ¹H-NMR, CDCl₃, 400 MHz |
| | | | | 11,10 (bs, 1H), 8,22 (dd, 1H), 7,71 (dd, 1H), 3,21 (s, 3H) |
| 8-149 | OMe | SMe | CF₃ | |
| 8-150 | OMe | S(O)Me | CF₃ | |
| 8-151 | OMe | SO₂Me | CF₃ | |
| 8-152 | Et | NH(CH₂)₂OMe | SO₂Me | |
| 8-153 | Et | F | SO₂Me | ¹H-NMR, DMSO-d₆, 600 MHz |
| | | | | 12,72 (s, 1H), 7,89 (t, 1H), 7,68 (d, 1H), 3,41 (s, 3H), 2,84 (q, 2H), 1,21 (t, 3H) |
| 8-154 | Et | SMe | CF₃ | |
| 8-155 | CF₃ | F | SO₂Me | |
| 8-156 | CF₃ | F | SO₂Et | |
| 8-157 | CF₃ | O(CH₂)₂OMe | SO₂Et | |
| 8-158 | CF₃ | O(CH₂)₃OMe | SO₂Et | |
| 8-159 | CF₃ | O(CH₂)₂OMe | SO₂Me | |
| 8-160 | CF₃ | O(CH₂)₃OMe | SO₂Me | |
| 8-161 | CF₃ | OCH₂CONMe₂ | SO₂Me | |
| 8-162 | CF₃ | OCH₂CONMe₂ | SO₂Et | |
| 8-163 | CF₃ | OCH₂CONMe₂ | Cl | |
| 8-164 | CF₃ | OCH₂CONMe₂ | Br | |
| 8-165 | CF₃ | OCH₂CONMe₂ | I | |
| 8-166 | CF₃ | OCH₂CONMe₂ | F | |
| 8-167 | CF₃ | O(CH₂)₂OMe | Cl | |
| 8-168 | CF₃ | O(CH₂)₃OMe | Cl | |
| 8-169 | CF₃ | O(CH₂)₂OMe | Br | |
| 8-170 | CF₃ | O(CH₂)₃OMe | Br | |
| 8-171 | CF₃ | O(CH₂)₂OMe | I | |
| 8-172 | CF₃ | O(CH₂)₃OMe | I | |
| 8-173 | CF₃ | O(CH₂)₂OMe | F | |
| 8-174 | CF₃ | O(CH₂)₃OMe | F | |
| 8-175 | CF₃ | [1,4]Dioxan-2-yl-methoxy | SO₂Me | |
| 8-176 | CF₃ | [1,4]Dioxan-2-yl-methoxy | SO₂Et | |
| 8-177 | CF₃ | [1,4]Dioxan-2-yl-methoxy | Cl | |
| 2-178 | CF₃ | [1,4]Dioxan-2-yl-methoxy | Br | |
| 8-179 | CF₃ | [1,4]Dioxan-2-yl-methoxy | I | |
| 8-180 | CF₃ | [1,4]Dioxan-2-yl-methoxy | F | |
| 8-181 | Br | OMe | Br | |
| 8-182 | Br | O(CH₂)₂OMe | Br | |
| 8-183 | Br | O(CH₂)₄OMe | SO₂Me | |
| 8-184 | Br | O(CH₂)₄OMe | SO₂Et | |
| 8-185 | Br | O(CH₂)₃OMe | SO₂Me | |
| 8-186 | Br | O(CH₂)₃OMe | SO₂Et | |
| 8-187 | Br | O(CH₂)₂OMe | SO₂Me | |
| 8-188 | Br | O(CH₂)₂OMe | SO₂Et | |
| 8-189 | Br | [1,4]Dioxan-2-yl-methoxy | SO₂Me | |
| 8-190 | Br | [1,4]Dioxan-2-yl-methoxy | SO₂Et | |
| 8-191 | Br | SMe | Me | |
| 8-192 | Br | SOMe | Me | |
| 8-193 | Br | SO₂Me | Me | |
| 8-194 | Br | SEt | Me | |
| 8-195 | Br | SOEt | Me | |
| 8-196 | Br | SO₂Et | Me | |
| 8-197 | I | O(CH₂)₄OMe | SO₂Me | |
| 8-198 | I | O(CH₂)₄OMe | SO₂Et | |
| 8-199 | I | O(CH₂)₃OMe | SO₂Me | |
| 8-200 | I | O(CH₂)₃OMe | SO₂Et | |
| 8-201 | I | O(CH₂)₂OMe | SO₂Me | |
| 8-202 | I | O(CH₂)₂OMe | SO₂Et | |
| 8-203 | I | [1,4]Dioxan-2-yl-methoxy | SO₂Me | |
| 8-204 | I | [1,4]Dioxan-2-yl-methoxy | SO₂Et | |
| 8-205 | I | SMe | Me | |
| 8-206 | I | SOMe | Me | |
| 8-207 | I | SO₂Me | Me | |
| 8-208 | I | SEt | Me | |
| 8-209 | I | SOEt | Me | |
| 8-210 | I | SO₂Et | Me | |
| 8-211 | CH₂SMe | OMe | SO₂Me | |
| 8-212 | CH₂OMe | OMe | SO₂Me | |
| 8-213 | CH₂O(C H₂)₂OMe | NH(CH₂)₂OEt | SO₂Me | |
| 8-214 | CH₂O(C H₂)₂OMe | NH(CH₂)₃OEt | SO₂Me | |
| 8-215 | CH₂O(C H₂)₃OMe | OMe | SO₂Me | |
| 8-216 | CH₂O(C H₂)₂OMe | NH(CH₂)₂OMe | SO₂Me | |
| 8-217 | CH₂O(C H₂)₂OMe | NH(CH₂)₃OMe | SO₂Me | |
| 8-218 | SO₂Me | NH₂ | CF₃ | |
| 8-219 | SO₂Me | F | CF₃ | |
| 8-220 | SO₂Me | NHEt | Cl | |
| 8-221 | SMe | SEt | F | ¹H-NMR, CDCl₃, 400 MHz |
| | | | | 12,41 (bs, 1H), 7,58 (dd, 1H), 7,46 (dd, 1H), 3,02 (q,2H), 2,41 (s, 3H), 1,17 (t, 3H) |
| 8-222 | SMe | SMe | F | ¹H-NMR, DMSO-d₆, 400 MHz |
| | | | | 12,40 (s, 1H), 7,53 (dd, 1H), 7,45 (bd, 1H), |
| 8-223 | Me | NH₂ | Cl | |
| 8-224 | Me | NH₂ | Br | |
| 8-225 | Me | NHMe | Cl | |
| 8-226 | Me | NHMe | Br | |
| 8-227 | Me | NMe₂ | Cl | |
| 8-228 | Me | NMe₂ | Br | |
| 8-227 | Me | NMe₂ | Cl | |
| 8-228 | Me | NMe₂ | Br | |
| 8-229 | NO₂ | O(CH₂)₂OMe | Me | |
| 8-230 | CF₃ | S(O)₂Et | SO₂Me | |
| 8-231 | CF₃ | S(O)₂Et | SO₂Et | |
| 8-232 | CF₃ | SCH₂CONMe₂ | SO₂Me | |
| 8-233 | CF₃ | SCH₂CONMe₂ | SO₂Et | |
| 8-234 | CF₃ | SCH₂COOH | SO₂Me | |
| 8-235 | CF₃ | SCH₂COOH | SO₂Et | |
| 8-236 | Me | SO₂-CH₂-CH₂-CH=CH₂ | CF₃ | |
| 8-237 | Cl | Me | SO₂Et | ¹H-NMR, DMSO-d₆, 400 MHz |
| | | | | 12,70 (s, 1H), 8,05 (d, 1H), 7,78 (d, 1H), 3,43 (q, 2H), 2,75 (s, 3H), 1,12 (t, 3H) |
| 8-238 | CF₃ | SEt | SO₂Me | |
| 8-239 | OMe | NO₂ | Cl | |
| 8-240 | OMe | NH(CO)i-Pr | Cl | |
| 8-241 | OMe | NH(CO)CH2Ph | Cl | |
| 8-242 | CF₃ | SEt | SO₂Et | |
| 8-243 | CF₃ | S(O)Et | SO₂Me | |
| 8-244 | Cl | Me | Cl | |
| 8-245 | Me | 3,5-Dimethyl-pyrazol-1-yl | SO₂Me | |
| 8-246 | SMe | H | CF₃ | |
| 8-247 | Me | 1,2,3-Triazol-1-yl | SO₂Me | ¹H-NMR, DMSO-d₆, 400 MHz |
| | | | | 12,77 (s, 1H), 8,56 (s, 1H), 8,21 (d, 1H), 8,11 (d, 1H), 8,06 (s, 1H), 3,15 (s, 3H), 1,94 (s, 3H) |
| 8-248 | Me | Me | SMe | ¹H-NMR, DMSO-d₆, 400 MHz |
| | | | | 12,30 (s, 1H), 7,43 (d, 1H), 7,19 (d,1H), 2,32 (s, 3H), 2,22 (s,3H) |
| 8-249 | Me | Pyrolidin-2-on-1-yl | SO₂Me | |
| 8-250 | CF₃ | S(O)Et | SO₂Et | |
| 8-251 | Cl | Pyrazol-1-yl | SO₂Me | |
| 8-252 | Me | 3-Methyl-pyrazol-1-yl | SO₂Me | |
| 8-253 | Cl | CH₂-N(Et)OMe | SO₂Me | |
| 8-254 | Me | Me | Cl | |
| 8-255 | OH | Cl | Cl | |
| 8-256 | Me | 1,2,4-Triazol-1-yl | SO₂Me | |
| 8-257 | Me | 4-Methoxy-pyrazol-1-yl | SO₂Me | |
| 8-258 | Me | 1,2,4-triazol-1-yl | CF₃ | |
| 8-259 | Me | Tetrahydro-pyrimidin-2(1H)-one-1-yl | SO₂Me | |
| 8-260 | Me | NH-(CH₂)₂-O(CO)Et | SO₂Me | |
| 8-261 | Me | NH-iPr | SO₂Me | |
| 8-262 | Cl | NH-CH₂-(CO)NHEt | Cl | |
| 8-263 | Me | NH-CH₂-(CO)NMe2 | SO₂Me | |
| 8-264 | Me | NH-CH₂-furan-2-yl | SO₂Me | |
| 8-265 | Me | NH-CH₂-(CO)NHEt | SO₂Me | |
| 8-266 | Me | F | SO₂Me | |
| 8-267 | F | SO₂Me | SO₂Me | |
| 8-268 | Cl | (4-Cyclopropyl-3-methyl-5-oxo-4,5-dihydro-1H-1,2,4-triazol-1-yl)-methyl | Cl | |
| 8-269 | Cl | [4-Methyl-5-oxo-3-(2,2,2-trifluorethoxy)-4,5-dihydro-1H-1,2,4-triazol-1-yl]-methyl | Cl | |
| 8-270 | Cl | (3-Isopropoxy-4-methyl-5-oxo-4,5-dihydro-1H-1,2,4-triazol-1-yl]-methyl | Cl | |
| 8-271 | Cl | (4-Methyl-5-oxo-4,5-dihydro-1H-1,2,4-triazol-1-yl)-methyl | Cl | |
| 8-272 | Me | Cl | SO₂Et | |
| 8-273 | SO₂Me | F | Cl | |
| 8-274 | Me | 1,2,3-Triazol-1-yl | SO₂Me | |
| 8-275 | Me | Isobutyl(methyl)carbamoylamino | SO₂Me | |
| 8-276 | Me | 3-Oxo-morpholin-4-yl | SO₂Me | |
| 8-277 | OMe | [Ethyl(methylsulfonyl)a mino]methyl | Cl | |
| 8-278 | F | SO₂Me | CF₃ | |
| 8-279 | OMe | Benzoylamino | Cl | |
| 8-280 | OMe | Cyclopropylcarbonylamino | Cl | |
| 8-281 | OMe | Propionyllamino | Cl | |
| 8-282 | NO₂ | SO₂Me | SO₂Me | |
| 8-283 | NO₂ | SO₂Me | Cl | |
| 8-284 | NO₂ | SOMe | SO₂Me | |
| 8-285 | NO₂ | SOMe | Br | |
| 8-286 | NO₂ | SOMe | Cl | |
| 8-287 | NO₂ | SMe | SO₂Me | |
| 8-288 | NO₂ | SMe | Br | |
| 8-289 | NO₂ | SMe | Cl | |
| 8-290 | Cl | CH₂OCH(CH₃)₂ | SO₂Et | |
| 8-291 | Cl | CH₂OEt | SO₂Et | |
| 8-292 | Cl | CH₂OMe | SO₂Et | |
| 8-293 | Cl | CH₂OCH₂C₂F₅ | SO₂Me | |
| 8-294 | Cl | CH₂OCH₂CHF₂ | SO₂Me | |
| 8-295 | Cl | CH₂OCH₂CCH | SO₂Et | |
| 8-296 | Cl | CH₂OC₂H₄OMe | SO₂Me | |
| 8-297 | Cl | CH₂(OC₂H₄)₂OMe | SO₂Me | |
| 8-298 | Cl | 5-Ethoxymethyl- 4,5-dihydro-1,2-oxazol-3 yl | SO₂Et | |
| 8-299 | Cl | 5-Methoxymethyl- 4,5-dihydro-1,2-oxazol-3 yl | SO₂Et | |
| 8-300 | Et | SOMe | CF₃ | |
| 8-301 | iPr | SMe | CF₃ | |
| 8-302 | Et | SMe | CF₃ | |
| 8-303 | Et | SO₂Me | CF₃ | |
| 8-304 | cPr | SOMe | CF₃ | |
| 8-305 | CH=CH₂ | SMe | CF₃ | |
| 8-306 | Et | SMe | Cl | |
| 8-307 | Et | SO₂Me | Cl | |
| 8-308 | Cl | NMe₂ | Cl | |
| 8-309 | CH₂O(C H₂)₂OMe | NH(CH₂)₃OMe | SO₂Me | |
| 8-313 | Me | SO₂(CH₂)₂OMe | CF₃ | |
| 8-314 | Me | SOEt | SO₂Me | |
| 8-315 | Me | SO₂Et | SO₂Me | |
| 8-316 | Me | SMe | 1,2,4-triazol-1-yl | |
| 8-317 | OEt | SMe | CF₃ | |
| 8-318 | Me | S(CH₂)₂OMe | CF₃ | |
| 8-319 | Me | SOMe | 1,2,4-triazol-1-yl | |
| 8-320 | OEt | SOMe | CF₃ | |
| 8-321 | Me | SO(CH₂)₂OMe | CF₃ | |
| 8-322 | Me | SCH₂CCMe | SO₂Me | |
| 8-323 | Me | S-c-Pen | SO₂Me | |
| 8-324 | OMe | SMe | OMe | |
| 8-325 | Me | SCH₂CH=CHCH₃ | SO₂Me | |
| 8-326 | Me | SOCH₂CCMe | SO₂Me | |
| 8-327 | Me | SO₈₋c-Pen | SO₂Me | |
| 8-328 | Me | SO-c-Pen | SO₂Me | |
| 8-329 | Me | S(CH₂)₃Cl | SO₂Me | |
| 8-330 | Me | SCH₂(4-F-Ph) | SO₂Me | |
| 8-331 | Me | SO₂CH₂CCMe | SO₂Me | |
| 8-332 | Me | SO₂CH₂CH=CHCH₃ | SO₂Me | |
| 8-333 | Me | SOCH₂CH=CHCH₃ | SO₂Me | |
| 8-334 | Me | SOCH₂-Epoxy-Me | SO₂Me | |
| 8-335 | Me | SO₂(CH₂)₃Cl | SO₂Me | |
| 8-336 | Me | SO(CH₂)₃Cl | SO₂Me | |
| 8-337 | Me | SOCH₂(4-F-Ph) | SO₂Me | |
| 8-338 | Me | SO₂CH₂(4-F-Ph) | SO₂Me | |
| 8-339 | Me | SO₂Me | C₂F₅ | |
| 8-340 | O(CH₂)₂ OMe | SMe | CF₃ | |
| 8-341 | O(CH₂)₂ OMe | SO₂Me | CF₃ | |
| 8-342 | O(CH₂)₂ OMe | SOMe | CF₃ | |
| 8-343 | Me | S(CH₂)₂OCH₂CF₃ | SO₂Me | |
| 8-344 | Me | SO(CH₂)₂OCH₂CF₃ | SO₂Me | |
| 8-345 | Me | SO₂(CH₂)₂OCH₂CF₃ | SO₂Me | |
| 8-346 | OEt | SEt | CF₃ | |
| 8-347 | O-CH₂-c-Pr | SMe | CF₃ | |
| 8-348 | OMe | SEt | CF₃ | |
| 8-349 | OMe | SO₂Et | CF₃ | |
| 8-350 | OMe | SOEt | CF₃ | |
| 8-351 | OEt | SO₂Et | CF₃ | |
| 8-352 | OEt | SOEt | CF₃ | |
| 8-353 | O-CH₂₋c-Pr | SOMe | CF₃ | |
| 8-354 | O-CH₂₋c-Pr | SO₂Me | CF₃ | |
| 8-355 | Me | SEt | SO₂Me | |

**Tabelle 9: Erfindungsgemäße Verbindungen der allgemeinen Formel (I), worin R für einen Rest OR* steht**

| | | | | | |
|---|---|---|---|---|---|
| 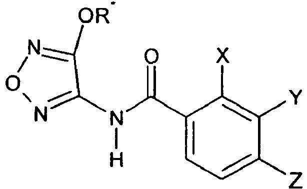 | | | | | |

| **Nr.** | **R*** | **X** | **Y** | **Z** | **Physikalische Daten** |
|---|---|---|---|---|---|
| 9-50 | Me | NO₂ | O(CH₂)₂OMe | OMe | |
| 9-51 | Me | NO₂ | OMe | Me | |
| 9-52 | Me | NO₂ | NH₂ | OMe | |
| 9-53 | Me | NO₂ | NH₂ | SO₂Me | |
| 9-54 | Me | NO₂ | NH₂ | Cl | |
| 9-55 | Me | NO₂ | NHMe | Cl | |
| 9-56 | Me | NO₂ | NMe₂ | Cl | |
| 9-57 | Me | NO₂ | NH₂ | Br | |
| 9-58 | Me | NO₂ | NHMe | Br | |
| 9-59 | Me | NO₂ | NMe₂ | Br | |
| 9-60 | Me | NO₂ | NH₂ | F | |
| 9-61 | Me | NO₂ | NHMe | F | |
| 9-62 | Me | NO₂ | NMe₂ | F | |
| 9-63 | Me | NO₂ | NH₂ | SO₂Me | |
| 9-64 | Me | NO₂ | NHMe | SO₂Me | |
| 9-65 | Me | NO₂ | NMe₂ | SO₂Me | |
| 9-66 | Me | NO₂ | NH₂ | 1H-1,2,4-triazol-1-yl | |
| 9-67 | Me | NO₂ | NHMe | 1H-1,2,4-triazol-1-yl | |
| 9-68 | Me | NO₂ | NMe₂ | 1H-1,2,4-triazol-1-yl | |
| 9-69 | Me | Me | F | F | ¹H-NMR, DMSO-d₆, 400 MHz |
| | | | | | 11,22 (s, 1H), 7,45-7,40 (m, 2H), 4,09 (s, 3H), 2,34 (d, 3H) |
| 9-70 | Me | Me | F | Cl | |
| 9-71 | Me | Me | SMe | CF₃ | |
| 9-72 | Me | Me | Cl | SO₂Me | ¹H-NMR, DMSO-d₆, 400 MHz |
| | | | | | 11,49 (s, 1H), 8,03 (d, 1H), 7,71 (d, 1H), 4,10 (s, 3H), 3,43 (s, 3H), 2,46 (s, 3H) |
| 9-73 | Me | Me | SO₂Me | SO₂Me | ¹H-NMR, DMSO-d₆, 400 MHz |
| | | | | | 11,51 (s, 1H), 8,24 (d, 1H), 7,99 (d, 1H), 4,11 (s, 3H), 3,60 (s, 3H), 3,57 (s, 3H), 2,67 (s, 3H) |
| 9-74 | Et | Me | SO₂Me | SO₂Me | |
| 9-75 | n-Pr | Me | SO₂Me | SO₂Me | |
| 9-76 | i-Pr | Me | SO₂Me | SO₂Me | |
| 9-77 | t-Bu | Me | SO₂Me | SO₂Me | |
| 9-78 | Ph | Me | SO₂Me | SO₂Me | ¹H-NMR, DMSO-d₆, 400 MHz |
| | | | | | 11,80 (s, 1H), 8,25 (d, 1H), 7,96 (d, 1H), 7,49 (m, 2H), 7,39 (d, 2H), 7,30 (t, 1H), 3,59 (s, 3H), 3,56 (s, 3H), 2,69 (s, 3H) |
| 9-79 | Me | Me | SO₂Me | CF₃ | ¹H-NMR, DMSO-d₆, 400 MHz |
| | | | | | 11,50 (s, 1H), 8,02 (d, 1H), 7,92 (d, 1H), 4,11 (s, 3H), 3,31 (s, 3H), 2,70 (s, 3H) |
| 9-80 | Et | Me | SO₂Me | CF₃ | ¹H-NMR, DMSO-d₆, 400 MHz |
| | | | | | 11,47 (s, 1H), 8,01 (d, 1H), 7,94 (d, 1H), 4,43 (q, 2H), 3,42 (s, 3H), 2,72(s, 3H), 1,40 (t, 3H) |
| 9-81 | n-Pr | Me | SO₂Me | CF₃ | ¹H-NMR, DMSO-d₆, 400 MHz |
| | | | | | 11,46 (s, 1H), 8,02 (d, 1H), 7,95 (d, 1H), 4,34 (t, 2H), 3,42 (s, 3H), 2,73 (s, 3H), 1,80 (m, 2H), 0,98 (t, 3H) |
| 9-82 | i-Pr | Me | SO₂Me | CF₃ | |
| 9-83 | t-Bu | Me | SO₂Me | CF₃ | |
| 9-84 | Ph | Me | SO₂Me | CF₃ | ¹H-NMR, DMSO-d₆, 400 MHz |
| | | | | | 11,78 (s, 1H), 8,02 (d, 1H), 7,91 (d, 1H), 7,50 (m, 2H), 7,40 (d, 2H), 7,31 (t, 1H), 3,42 (s, 3H), 2,72 (s, 3H) |
| 9-85 | Me | Me | Cl | CF₃ | |
| 9-86 | Me | Me | S(O)Me | CF₃ | ¹H-NMR, DMSO-d₆, 400 MHz |
| | | | | | 11,48 (s, 1H), 7,85 (d, 1H), 7,80 (d, 1H), 4,10 (s, 3H), 3,04 (s, 3H), 2,83 (s, 3H) |
| 9-87 | Me | Me | SEt | OMe | |
| 9-88 | Me | Me | NMe₂ | SO₂Me | ¹H-NMR, DMSO-d₆, 400 MHz |
| | | | | | 11,33 (s, 1H), 7,84 (d, 1H), 7,52 (d, 1H), 4,09 (s, 3H), 3,33 (s, 3H), 2,83 (s, 6H), 2,36 (s, 3H) |
| 9-89 | Et | Me | NMe₂ | SO₂Me | |
| 9-90 | n-Pr | Me | NMe₂ | SO₂Me | |
| 9-91 | i-Pr | Me | NMe₂ | SO₂Me | |
| 9-92 | t-Bu | Me | NMe₂ | SO₂Me | |
| 9-93 | Ph | Me | NMe₂ | SO₂Me | |
| 9-94 | Me | Me | NH(CH₂)₂OMe | SO₂Me | |
| 9-95 | Et | Me | NH(CH₂)₂OMe | SO₂Me | |
| 9-96 | n-Pr | Me | NH(CH₂)₂OMe | SO₂Me | |
| 9-97 | i-Pr | Me | NH(CH₂)₂OMe | SO₂Me | |
| 9-98 | t-Bu | Me | NH(CH₂)₂OMe | SO₂Me | |
| 9-99 | Ph | Me | NH(CH₂)₂OMe | SO₂Me | |
| 9-100 | Me | Me | O(CH₂)₄OMe | SO₂Me | |
| 9-101 | Me | Me | NH₂ | SO₂Me | |
| 9-102 | Me | Me | O(CH₂)₂-O(3,5-Dimethoxypyrimi din-2-yl | SO₂Me | |
| 9-103 | Me | Me | O(CH₂)₂-O-NMe₂ | Cl | |
| 9-104 | Me | Me | O(CH₂)₂-NH(CO)NMe₂ | Cl | |
| 9-105 | Me | Me | O(CH₂)-5-Pyrrolidin-2-on | Br | |
| 9-106 | Me | Me | O(CH₂)₂-NH (CO)NHCO₂Et | Cl | |
| 9-107 | Me | Me | O(CH₂)-(CO)NEt₂ | Br | |
| 9-108 | Me | Me | O(CH₂) -5-2,4-Dimethyl-2,4-dihydro-3H-1,2,4-triazol-3-on | Cl | |
| 9-109 | Me | Me | O(CH₂)-3,5-Dimethyl-1,2-oxazol-4-yl | Cl | |
| 9-110 | Me | Me | O(CH₂)₂-NHCO₂Me | Cl | |
| 9-111 | Me | Me | 4,5-Dihydro-1,2-oxazol-3 yl | SO₂Me | |
| 9-112 | Et | Me | 4,5-Dihydro-1,2-oxazol-3 yl | SO₂Me | |
| 9-113 | n-Pr | Me | 4,5-Dihydro-1,2-oxazol-3 yl | SO₂Me | |
| 9-114 | i-Pr | Me | 4,5-Dihydro-1,2-oxazol-3 yl | SO₂Me | |
| 9-115 | t-Bu | Me | 4,5-Dihydro-1,2-oxazol-3 yl | SO₂Me | |
| 9-116 | Ph | Me | 4,5-Dihydro-1,2-oxazol-3 yl | SO₂Me | |
| 9-117 | Me | Me | Me | SO₂Me | ¹H-NMR, DMSO-d₆, 400 MHz |
| | | | | | 11,34 (s, 1H), 7,98 (d, 1H), 7,51 (d, 1H), 4,10 (s, 3H), 3,24 (s, 3H), 2,61 (s, 3H), 2,31 (s, 3H) |
| 9-118 | Me | Me | OH | SO₂Me | |
| 9-119 | Me | Me | O-CH₂-NHSO₂cPr | Cl | |
| 9-120 | Me | Me | O-CH₂-NHSO₂Me | SO₂Me | |
| 9-121 | Et | Me | O-CH₂-NHSO₂Me | SO₂Me | |
| 9-122 | n-Pr | Me | O-CH₂-NHSO₂Me | SO₂Me | |
| 9-123 | i-Pr | Me | O-CH₂-NHSO₂Me | SO₂Me | |
| 9-124 | t-Bu | Me | O-CH₂-NHSO₂Me | SO₂Me | |
| 9-125 | Ph | Me | O-CH₂-NHSO₂Me | SO₂Me | |
| 9-126 | Me | Me | SMe | Cl | |
| 9-127 | Me | Me | SOMe | Cl | |
| 9-128 | Et | Me | SO₂Me | Cl | |
| 9-129 | n-Pr | Me | SO₂Me | Cl | |
| 9-130 | i-Pr | Me | SO₂Me | Cl | |
| 9-131 | t-Bu | Me | SO₂Me | Cl | |
| 9-132 | Ph | Me | SO₂Me | Cl | |
| 9-133 | Me | Me | SO₂Me | Cl | |
| 9-134 | Me | Me | SMe | Br | |
| 9-135 | Me | Me | SOMe | Br | |
| 9-136 | Me | Me | SO₂Me | Br | |
| 9-137 | Me | Me | SMe | I | |
| 9-138 | Me | Me | SOMe | I | |
| 9-139 | Me | Me | SO₂Me | I | |
| 9-140 | Me | Me | SEt | Cl | |
| 9-141 | Me | Me | SOEt | Cl | |
| 9-142 | Me | Me | SO₂Et | Cl | |
| 9-143 | Me | Me | SEt | Br | |
| 9-144 | Me | Me | SOEt | Br | |
| 9-145 | Me | Me | SO₂Et | Br | |
| 9-146 | Me | Me | SEt | I | |
| 9-147 | Me | Me | SOEt | I | |
| 9-148 | Me | Me | SO₂Et | I | |
| 9-149 | Me | Me | SEt | F | |
| 9-150 | Me | Me | SOEt | F | |
| 9-151 | Me | Me | SO₂Et | F | |
| 9-152 | Me | Me | S(O)Me | SO₂Me | |
| 9-153 | Me | Me | SMe | SO₂Me | |
| 9-154 | Me | Me | SMe | OMe | |
| 9-155 | Me | Me | S(O)Me | OMe | |
| 9-156 | Me | Me | SO₂Me | OMe | |
| 9-157 | Me | Me | SMe | Cl | |
| 9-158 | Me | Me | S(O)Me | Cl | |
| 9-159 | Me | Me | SO₂Me | Cl | |
| 9-160 | Me | Me | [1,4]Dioxan-2-yl-methoxy | SO₂Me | |
| 9-161 | Me | Me | [1,4]Dioxan-2-yl-methoxy | SO₂Et | |
| 9-162 | Me | Me | O(CH₂)₄OMe | SO₂Et | |
| 9-163 | Me | Me | O(CH₂)₃OMe | SO₂Me | |
| 9-164 | Me | Me | O(CH₂)₃OMe | SO₂Et | |
| 9-165 | Me | Me | O(CH₂)₂OMe | SO₂Me | |
| 9-166 | Me | Me | O(CH₂)₂OMe | SO₂Et | |
| 9-167 | Me | Me | S(O)Me | SO₂Me | |
| 9-168 | Me | Me | SMe | SO₂Me | |
| 9-169 | Me | Me | SMe | OMe | |
| 9-170 | Me | Me | S(O)Me | OMe | |
| 9-171 | Me | Me | SO₂Me | OMe | |
| 9-172 | Me | Cl | OCH₂(CO)NMe₂ | Cl | |
| 9-173 | Me | Cl | Cl | SO₂Me | ¹H-NMR, DMSO-d₆, 400 MHz |
| | | | | | 11,72 (s, 1H), 8,12 (d, 1H), 7,87 (d, 1H), 4,10 (s, 3H), 3,48 (s, 3H), 2,46 (s, 3H) |
| 9-174 | Me | Cl | CH₂OCH₂CF₃ | SO₂Me | ¹H-NMR, CDCl₃, 400 MHz |
| | | | | | 8,15 (d, 1 H), 8,0 (bs, 1H), 7,81 (d, 1H), 5,35 (s, 2H), 4,18 (s, 3H), 4,05 (q, 2H), 3,21 (s, 3H) |
| 9-175 | Et | Cl | CH₂OCH₂CF₃ | SO₂Me | |
| 9-176 | n-Pr | Cl | CH₂OCH₂CF₃ | SO₂Me | ¹H-NMR, DMSO-d₆, 400 MHz |
| | | | | | 11,57 (s, 1H), 8,11 (d, 1H), 7,98 (d, 1H), 5,25 (s, 2H), 4,33 (q, 2H), 4,31 (q, 2H), 3,37 (s, 3H), 1,78 |
| | | | | | (m, 2H), 0,98 (t, 3H) |
| 9-177 | i-Pr | Cl | CH₂OCH₂CF₃ | SO₂Me | |
| 9-178 | t-Bu | Cl | CH₂OCH₂CF₃ | SO₂Me | |
| 9-179 | Ph | Cl | CH₂OCH₂CF₃ | SO₂Me | ¹H-NMR, DMSO-d₆, 400 MHz |
| | | | | | 11,91 (s, 1H), 8,11 (d, 1H), 7,86 (d, 1H), 7,50 (m, 2H), 7,40 (d, 2H), 7,31 (t, 1H), 5,25 (s, 2H), 4,29 (q, 2H), 3,36 (s, 3H) |
| 9-180 | Me | Cl | 5-Cyanomethyl-4,5-dihydro-1,2-oxazol-3 yl | SO₂Et | ¹H-NMR, CDCl₃, 400 MHz |
| | | | | | 8,15 (bs, 1H), 8,10 (d, 1H), 7,95 (bs, 1H), 5,17 (m, 1H), 4,18 (s, 3H), 3,72 (m, 1H), 3,37 (q, 2H), 3,25 (m, 1H), 2,80-2,98 (m, 2H), 1,30 (t, 3H) |
| 9-181 | Et | Cl | 5-Cyanomethyl-4,5-dihydro-1,2-oxazol-3 yl | SO₂Et | |
| 9-182 | n-Pr | Cl | 5-Cyanomethyl-4,5-dihydro-1,2-oxazol-3 yl | SO₂Et | ¹H-NMR, DMSO-d₆, 400 MHz |
| | | | | | 11,60 (s, 1H), 8,11 (d, 1H), 8,05 (d, 1H), 5,19 (m, 1H), 4,32 (m, 2H), 3,59 (m, 1H), 3,42 (q, 2H), 3,15 (dd, 1H), 3,01 (m, 2H), 1,77 (m, 2H), 1,15 (t, 3H), 0,95 (t, 3H) |
| 9-183 | i-Pr | Cl | 5-Cyanomethyl-4,5-dihydro-1,2-oxazol-3 yl | SO₂Et | |
| 9-184 | t-Bu | Cl | 5-Cyanomethyl-4,5-dihydro-1,2-oxazol-3 yl | SO₂Et | |
| 9-185 | Ph | Cl | 5-Cyanomethyl-4,5-dihydro-1,2-oxazol-3 yl | SO₂Et | ¹H-NMR, DMSO-d₆, 400 MHz |
| | | | | | 11,93 (s, 1H), 8,11 (d, 1H), 8,00 (d, 1H), 7,50 (m, 2H), 7,41 (d, 2H9, 7,32 (t, 1H), 5,19 (m, 1H), 3,50 (dd, 2H), 3,44 (q, 2H), 3,31 (s, 1H), 3,15 (dd, 2H), 3,02 (m, 2H), 1,16 (t, 3H) |
| 9-186 | Me | Cl | CH₂O-Tetrahydrofuran-3-yl | SO₂Me | |
| 9-187 | Et | Cl | CH₂O-Tetrahydrofuran-3-yl | SO₂Me | |
| 9-188 | n-Pr | Cl | CH₂O-Tetrahydrofuran-3-yl | SO₂Me | |
| 9-189 | i-Pr | Cl | CH₂O-Tetrahydrofuran-3-yl | SO₂Me | |
| 9-190 | t-Bu | Cl | CH₂O-Tetrahydrofuran-3-yl | SO₂Me | |
| 9-191 | Ph | Cl | CH₂O-Tetrahydrofuran-2-yl | SO₂Me | |
| 9-192 | Me | Cl | SMe | SO₂Me | |
| 9-193 | Me | Cl | F | SMe | |
| 9-194 | n-Pr | Cl | CH₂OCH₂-Tetrahydrofuran -2-yl | SO₂Me | ¹H-NMR, DMSO-d₆, 400 MHz |
| | | | | | 11,58 (bs, 1H), 8,09 (d, 1H), 7,85 (d, 1H), 5,08 (m, 2H), 4,32 (t, 2H), 3,97 (m, 1H), 3,71 (dd, 1H), 3,65 - 3,51 (m, 3H), 3,38 (s, 3H), 1,90-1,70 (m, 5H), 1,53 (m, 1H), 0,95 (t, 3H) |
| 9-195 | Me | Cl | CH₂OCH₂-Tetrahydrofuran -3-yl | SO₂Et | |
| 9-196 | n-Pr | Cl | O(CH₂)-5-Pyrrolidin-2-on | Cl | |
| 9-197 | Me | Cl | O(CH₂)-5-Pyrrolidin-2-on | Cl | |
| 9-198 | Me | Cl | O(CH₂)-5-Pyrrolidin-2-on | Cl | |
| 9-199 | Me | Cl | S(O)Me | SO₂Me | |
| 9-200 | Me | Cl | CH₂O-Tetrahydrofuran-3-yl | SO₂Et | ¹H-NMR, CDCl₃, 400 MHz |
| | | | | | 8,37 (bs, 1H), 8,0 (d, 1H), 7,73 (d, 1H), 5,07 (m, 2H), 4,34 (m, 1H), 4,18 (s, 3H), 3,73-3,94 (m, 4H), 3,36 (q, 2H), 2,08 (m, 2H), 1,25 (t, 3H) |
| 9-201 | Me | Cl | SMe | Cl | ¹H-NMR, CDCl₃, 400 MHz |
| | | | | | 8,03 (s, 1H), 7,66 (d, 1H), 4,19 (s, 3H), 2,47 (s, 3H) |
| 9-202 | Me | Cl | O(CH₂)₂OMe | Cl | |
| 9-203 | Et | Cl | O(CH₂)₂OMe | Cl | |
| 9-204 | n-Pr | Cl | O(CH₂)₂OMe | Cl | |
| 9-205 | i-Pr | Cl | O(CH₂)₂OMe | Cl | |
| 9-206 | t-Bu | Cl | O(CH₂)₂OMe | Cl | |
| 9-207 | Ph | Cl | O(CH₂)₂OMe | Cl | |
| 9-208 | Me | Cl | O(CH₂)₄OMe | SO₂Me | |
| 9-209 | Me | Cl | O(CH₂)₄OMe | SO₂Et | |
| 9-210 | Me | Cl | O(CH₂)₃OMe | SO₂Me | |
| 9-211 | Me | Cl | O(CH₂)₃OMe | SO₂Et | |
| 9-212 | Me | Cl | O(CH₂)₂OMe | SO₂Me | |
| 9-213 | Me | Cl | O(CH₂)₂OMe | SO₂Et | |
| 9-214 | Me | Cl | [1,4]Dioxan-2-yl-methoxy | SO₂Me | |
| 9-215 | Me | Cl | [1,4]Dioxan-2-yl-methoxy | SO₂Et | |
| 9-216 | Me | Cl | SO₂Me | Me | |
| 9-217 | Me | Cl | SEt | Me | |
| 9-218 | Me | Cl | SOEt | Me | |
| 9-219 | Me | Cl | SO₂Et | Me | |
| 9-220 | Me | Cl | 4,5-Dihydro-1,2-oxazol-3 yl | SO₂Me | |
| 9-221 | Me | Cl | Cl | SO₂Me | |
| 9-222 | Me | F | SMe | CF₃ | |
| 9-223 | n.Pr | F | SMe | CF₃ | |
| 9-224 | n-Pr | F | S(O)Me | CF₃ | ¹H-NMR, CDCl₃, 400 MHz |
| | | | | | 8,73 (bd, 1H), 8,47 (dd, 1H), 7,74 (bd, 1H), 4,41 (t, 2H), 3,14 (s, 3H), 1,79 (m, 2H), 1,03 (t, 3H) |
| 9-225 | Me | F | S(O)Me | CF₃ | ¹H-NMR, CDCl₃, 400 MHz |
| | | | | | 8,85 (b, 1H), 8,49 (dd, 1H), 7,74 (dd, 1H), 7,69 (bs, 1H), 4,18 (s, 3H), 3,13 (s, 3H) |
| 9-226 | | F | S(O)Me | CF₃ | |
| 9-227 | Me | F | SO₂Me | CF₃ | |
| 9-228 | n.Pr | F | SO₂Me | CF₃ | |
| 9-229 | Me | SMe | SEt | F | ¹H-NMR, CDCl₃, 400 MHz |
| | | | | | 10,02 (bs, 1H), 8,49 (b, 1H), 8,20 (dd, 1H), 7,96 (b, 1H), 7,22 (dd, 1H), 4,40 (t, 3H), 3,05 (q, 2H), 2,49 (s, 3H), 1,88 (m, 2H), 1,26 (t, 2H), 1,05 (t, 3H) |
| 9-230 | n-Pr | SMe | SEt | F | ¹H-NMR, CDCl₃, 400 MHz |
| | | | | | 9,75 (bs, 1H), 8,49 (b, 1H), 8,20 (dd, 1H), 7,94 (b, 1H), 7,18 (dd, 1H), 4,19 (s, 3H), 3,00 (s, 3H), 2,49 (s, 3H), 1,28 (t, 3H) |
| 9-231 | Me | OMe | SMe | CF₃ | |
| 9-232 | Me | OMe | S(O)Me | CF₃ | |
| 9-233 | Me | OMe | SO₂Me | CF₃ | |
| 9-234 | Me | Et | NH(CH₂)₂OMe | SO₂Me | |
| 9-235 | Me | Et | F | SO₂Me | |
| 9-236 | Me | Et | SMe | CF₃ | |
| 9-237 | Me | CF₃ | F | SO₂Me | ¹H-NMR, DMSO-d₆, 400 MHz |
| | | | | | 11,76 (s, 1H), 8,27 (t, 1H), 7,79 (d, 1H), 4,11 (s, 3H), 3,48 (s, 3H) |
| 9-238 | Me | CF₃ | F | SO₂Et | |
| 9-239 | Me | CF₃ | O(CH₂)₂OMe | SO₂Et | |
| 9-240 | Me | CF₃ | O(CH₂)₃OMe | SO₂Et | |
| 9-241 | Me | CF₃ | O(CH₂)₂OMe | SO₂Me | |
| 9-242 | Me | CF₃ | O(CH₂)₃OMe | SO₂Me | |
| 9-243 | Me | CF₃ | OCH₂CONMe₂ | SO₂Me | |
| 9-244 | Me | CF₃ | OCH₂CONMe₂ | SO₂Et | |
| 9-245 | Me | CF₃ | OCH₂CONMe₂ | Cl | |
| 9-246 | Me | CF₃ | OCH₂CONMe₂ | Br | |
| 9-247 | Me | CF₃ | OCH₂CONMe₂ | I | |
| 9-248 | Me | CF₃ | OCH₂CONMe₂ | F | |
| 9-249 | Me | CF₃ | O(CH₂)₂OMe | Cl | |
| 9-250 | Me | CF₃ | O(CH₂)₃OMe | Cl | |
| 9-251 | Me | CF₃ | O(CH₂)₂OMe | Br | |
| 9-252 | Me | CF₃ | O(CH₂)₃OMe | Br | |
| 9-253 | Me | CF₃ | O(CH₂)₂OMe | I | |
| 9-254 | Me | CF₃ | O(CH₂)₃OMe | I | |
| 9-255 | Me | CF₃ | O(CH₂)₂OMe | F | |
| 9-256 | Me | CF₃ | O(CH₂)₃OMe | F | |
| 9-257 | Me | CF₃ | [1,4]Dioxan-2-yl-methoxy | SO₂Me | |
| 9-258 | Me | CF₃ | [1,4]Dioxan-2-yl-methoxy | SO₂Et | |
| 9-259 | Me | CF₃ | [1,4]Dioxan-2-yl-methoxy | Cl | |
| 9-260 | Me | CF₃ | [1,4]Dioxan-2-yl-methoxy | Br | |
| 9-261 | Me | CF₃ | [1,4]Dioxan-2-yl-methoxy | I | |
| 9-262 | Me | CF₃ | [1,4]Dioxan-2-yl-methoxy | F | |
| 9-263 | Me | Br | OMe | Br | |
| 9-264 | Me | Br | O(CH₂)₂OMe | Br | |
| 9-265 | Me | Br | O(CH₂)₄OMe | SO₂Me | |
| 9-266 | Me | Br | O(CH₂)₄OMe | SO₂Et | |
| 9-267 | Me | Br | O(CH₂)₃OMe | SO₂Me | |
| 9-268 | Me | Br | O(CH₂)₃OMe | SO₂Et | |
| 9-269 | Me | Br | O(CH₂)₂OMe | SO₂Me | |
| 9-270 | Me | Br | O(CH₂)₂OMe | SO₂Et | |
| 9-271 | Me | Br | [1,4]dioxan-2-yl-methoxy | SO₂Me | |
| 9-272 | Me | Br | [1,4]Dioxan-2-yl-methoxy | SO₂Et | |
| 9-273 | Me | Br | SMe | Me | |
| 9-274 | Me | Br | SOMe | Me | |
| 9-275 | Me | Br | SO₂Me | Me | |
| 9-276 | Me | Br | SEt | Me | |
| 9-277 | Me | Br | SOEt | Me | |
| 9-278 | Me | Br | SO₂Et | Me | |
| 9-279 | Me | I | O(CH₂)₄OMe | SO₂Me | |
| 9-280 | Me | I | O(CH₂)₄OMe | SO₂Et | |
| 9-281 | Me | I | O(CH₂)₃OMe | SO₂Me | |
| 9-282 | Me | I | O(CH₂)₃OMe | SO₂Et | |
| 9-283 | Me | I | O(CH₂)₂OMe | SO₂Me | |
| 9-284 | Me | I | O(CH₂)₂OMe | SO₂Et | |
| 9-285 | Me | I | [1,4]Dioxan-2-yl-methoxy | SO₂Me | |
| 9-286 | Me | I | [1,4]Dioxan-2-yl-methoxy | SO₂Et | |
| 9-287 | Me | I | SMe | Me | |
| 9-288 | Me | I | SOMe | Me | |
| 9-289 | Me | I | SO₂Me | Me | |
| 9-290 | Me | I | SEt | Me | |
| 9-291 | Me | I | SOEt | Me | |
| 9-292 | Me | I | SO₂Et | Me | |
| 9-293 | Me | CH₂SMe | OMe | SO₂Me | |
| 9-294 | Me | CH₂OMe | OMe | SO₂Me | |
| 9-295 | Me | CH₂O(CH₂) ₂OMe | NH(CH₂)₂OEt | SO₂Me | |
| 9-296 | Me | CH₂O(CH₂) ₂OMe | NH(CH₂)₃OEt | SO₂Me | |
| 9-297 | Me | CH₂O(CH₂) ₃OMe | OMe | SO₂Me | |
| 9-298 | Me | CH₂O(CH₂) ₂OMe | NH(CH₂)₂OMe | SO₂Me | |
| 9-299 | Me | CH₂O(CH₂) ₂OMe | NH(CH₂)₃OMe | SO₂Me | |
| 9-300 | Me | SO₂Me | NH₂ | CF₃ | |
| 9-301 | Me | SO₂Me | F | CF₃ | ¹H-NMR, DMSO-d₆, 400 MHz |
| | | | | | 11,57 (s, 1H), 8,28 (t, 1H), 7,70 (d, 1H), 4,10 (s, 3H), 3,45 (s, 3H) |
| 9-302 | Me | SO₂Me | NHEt | Cl | |
| 9-303 | Me | SMe | SEt | F | |
| 9-304 | Me | SMe | SMe | F | ¹H-NMR, CDCl₃, 400 MHz |
| | | | | | 9,85 (bs, 1H), 7,91 (bs, 1H), 7,20 (dd, 1H), 4,19 (s, 3H), 2,56 (s, 3H), 2,49 (s, 3H) |
| 9-305 | n-Pr | SMe | SMe | F | ¹H-NMR, CDCl₃, 400 MHz |
| | | | | | 10,00 (bs, 1H), 7,96 (b, 1H), 7,20 (dd, 1 H), 4,39 (t, 2H), 2,56 (s, 3H), 2,49 (s, 3H), 1,88 (m, 2H), 1,04 (t, 3H). |
| 9-306 | Benzyl | Me | SO₂Me | CF₃ | |
| 9-307 | Me | Cl | Me | SO₂Et | ¹H-NMR, DMSO-d₆, 400 MHz |
| | | | | | 11,60 (s, 1H), 7,97 (d, 1H), 7,69 (d, 1H), 4,10 (s, 3H), 3,41 (q, 2H), 2,72 (s, 3H), 1,12 (t, 3H) |
| 9-308 | Benzyl | Me | SO₂Me | CF3 | |
| 9-309 | Benzyl | Me | SO₂Me | SO₂Me | |
| 9-310 | Benzyl | Me | S(O)Me | CF₃ | ¹H-NMR, DMSO-d₆, 400 MHz |
| | | | | | 11,50 (s, 1H), 7,84 (d, 1H), 7,80 (d, 1 H), 7,52 (d, 2H), 7,40 (m, 3H), 7,30 (t, 1H), 5,45 (s, 2H), 3,03 (s, 3H), 2,80 (s, 3H) |
| 9-311 | Me | Cl | 1H-Pyrazol-1-yl | SO₂Me | ¹H-NMR, DMSO-d₆, 400 MHz |
| | | | | | 11,76 (s, 1H), 8,19 (d, 1H), 8,06 (d, 1 H), 8,01 (s, 1H), 7,87 (s, 1 H), 4,10 (s, 3H), 3,19 (s, 3H) |
| 9-312 | Me | Me | F | SO₂Me | ¹H-NMR, DMSO-d₆, 400 MHz |
| | | | | | 11,28 (s, 1 H), 7,57 (t, 1 H), 7,41 (d, 1 H), 4,09 (s, 3H), 2,33 (d, 3H) |
| 9-313 | Me | Me | 1 H-1,2,3-Triazol-1-yl | SO₂Me | ¹H-NMR, DMSO-d₆, 400 MHz |
| | | | | | 11,62 (s, 1H), 8,53 (s, 1H), 8,14 (d, 1H), 8,06 (s, 1H), 8,03 (d, 1H), 4,10 (s, 3H), 3,14 (s, 3H), 1,89 (s, 3H) |
| 9-314 | Me | Cl | Me | Cl | ¹H-NMR, DMSO-d₆, 400 MHz |
| | | | | | 11,39 (s, 1H), 7,57 (d, 1H), 7,45 (d, 1H), 4,09 (s, 3H), 2,47 (s, 3H) |
| 9-315 | Me | Cl | CH2OCH2-2-Tetrahydrofuran -2-yl | SO2Me | ¹H-NMR, DMSO-d₆, 400 MHz |
| | | | | | 11,65 (s, 1H), 8,08 (d, 1H), 7,85 (d, 1H), 5,08 (s, 2H), 4,10 (s, 3H), 3,97 (m, 1H), 3,70 (q, 1H), 3,65-3,52 (m, 3H), 3,38 (s, 3H), 1,94-1,72 (m, 3H), 1,55 (m, 1H) |
| 9-316 | Me | Me | Me | SMe | ¹H-NMR, DMSO-d₆, 400 MHz |
| | | | | | 10,98 (s, 1H), 7,33 (d, 1H), 7,15 (d, 1H), 4,08 (s, 3H), 2,25 (s, 3H), 2,23 (s, 3H) |
| 9-317 | Ph | Me | S(O)Me | CF₃ | ¹H-NMR, DMSO-d₆, 400 MHz |
| | | | | | 11,73 (s, 1H), 7,86 (d, 1H), 7,76 (d, 1H), 7,49 (m, 2H), 7,41 (d, 2H), 7,31 (t, 1 H), 3,04 (s, 3H), 2,84 (s, 3H) |
| 9-318 | Me | Cl | S(O)Me | Cl | ¹H-NMR, DMSO-d₆, 400 MHz |
| | | | | | 11,58 (s, 1H), 7,85 (d, 1H), 7,81 (d, 1H), 4,10 (s, 3H), 3,51 (s, 3H) |
| 9-319 | Me | Cl | CH2N(OMe)Et | SO2Me | ¹H-NMR, DMSO-d₆, 400 MHz |
| | | | | | 11,59 (s, 1 H), 8,11 (d, 1H), 7,82 (d, 1H), 4,52 (s, 2H), 4,10 (s, 3H), 3,57 (s, 3H), 3,17 (s, 3H), 2,85 (q, 2H), 1,14 (t, 3H) |
| 9-320 | n-Pr | Cl | Me | SO₂Et | ¹H-NMR, DMSO-d₆, 400 MHz |
| | | | | | 11,49 (s, 1H), 7,99 (d, 1H), 7,70 (d, 1H), 4,33 (t, 2H), 3,42 (q, 2H), 2,73 (s, §H), 1,79 (m, 2H), 1,14 (t, 3H), 0,98 (t, 3H) |
| 9-321 | Me | Me | 4-Methoxy-1H-pyrazol-1-yl | SO₂Me | ¹H-NMR, DMSO-d₆, 400 MHz |
| | | | | | 11,54(s, 1H), 8,05 (d, 1H), 7,91 (d, 1H), 7,73 (s, 1H), 7,66 (s, 1H), 4,09 (s, 3H), 3,75 (s, 3H), 3,10 (s, 3H), 1,92 (s, 3H) |
| 9-322 | Ph | Me | Cl | SO₂Me | ¹H-NMR, DMSO-d₆, 400 MHz |
| | | | | | 11,73 (s, 1H), 8,03 (d, 1H), 7,67 (d, 1H), 7,49 (m, 2H), 7,38 (d, 2H), 7,30 (t, 1H), 3,43 (s, 3H), 2,45 (s, 3H) |
| 9-323 | n-Pr | Me | S(O)Me | CF₃ | ¹H-NMR, DMSO-d₆, 400 MHz |
| | | | | | 11,42 (s, 1H), 7,86 (d, 1H), 7,81 (d, 1H), 4,34 (t, 2H), 3,05 (s, 3H), 2,85 (s, 3H), 1,79 (m, 2H), 0,98 (t, 3H) |

**Tabelle 10: Erfindungsgemäße Verbindungen der allgemeinen Formel (I),**

| | | | | | |
|---|---|---|---|---|---|
| 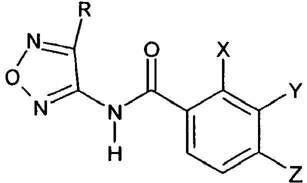 | | | | | |

| **Nr.** | **R** | **X** | **Y** | **Z** | **Physikalische Daten** |
|---|---|---|---|---|---|
| 10-46 | Ph | NO₂ | O(CH₂)₂OMe | OMe | |
| 10-47 | Ph | NO₂ | OMe | Me | |
| 10-48 | Ph | NO₂ | NH₂ | OMe | |
| 10-49 | Ph | NO₂ | NH₂ | SO₂Et | |
| 10-50 | Ph | NO₂ | NH₂ | Cl | |
| 10-51 | Ph | NO₂ | NHMe | Cl | |
| 10-52 | Ph | NO₂ | NMe₂ | Cl | |
| 10-53 | Ph | NO₂ | NH₂ | Br | |
| 10-54 | Ph | NO₂ | NHMe | Br | |
| 10-55 | Ph | NO₂ | NMe₂ | Br | |
| 10-56 | Ph | NO₂ | NH₂ | F | |
| 10-57 | Ph | NO₂ | NHMe | F | |
| 10-58 | Ph | NO₂ | NMe₂ | F | |
| 10-59 | Ph | NO₂ | NH₂ | SO₂Me | |
| 10-60 | Ph | NO₂ | NHMe | SO₂Me | |
| 10-61 | Ph | NO₂ | NMe₂ | SO₂Me | |
| 10-62 | Ph | NO₂ | NH₂ | 1H-1,2,4-Triazol-1-yl | |
| 10-63 | Ph | NO₂ | NHMe | 1H-1,2,4-Triazol-1-yl | |
| 10-64 | Ph | NO₂ | NMe₂ | 1H-1,2,4-Triazol-1-yl | |
| 10-65 | Ph | Me | F | F | |
| 10-66 | Ph | Me | F | Cl | |
| 10-67 | Ph | Me | SMe | CF₃ | |
| 10-68 | Ph | Me | Cl | SO₂Me | ¹H-NMR, DMSO-d₆, 400 MHz |
| | | | | | 11,58 (s, 1H), 8,05 (d, 1H), 7,78 (m, 3H), 7,58 (m, 3H), 7,59 (m, 3H), |
| | | | | | 3,42 (s, 3H), 2,39 (s, 3H) |
| 10-69 | Ph | Me | SO₂Me | SO₂Me | |
| 10-70 | 4-Cl-Ph | Me | SO₂Me | SO₂Me | |
| 10-71 | 1-Ethyl-benzimidazol-2-yl | Me | SO₂Me | SO₂Me | |
| 10-72 | 1,2,4-Triazol-1-yl | Me | SO₂Me | SO₂Me | |
| 10-73 | Benzoxazol-2-yl | Me | SO₂Me | SO₂Me | ¹H-NMR, DMSO-d₆, 400 MHz |
| | | | | | 11,90 (s, 1H), 9,39 (s, 1H), 8,47 (s, 1H), 8,28 (d, 1H), 8,06 (d, 1H), 3,59 (s, 3H), 3,56 (s, 3H), 2,69 (s, 3H) |
| 10-74 | Ph | Me | SO₂Me | CF₃ | ¹H-NMR, DMSO-d₆, 400 MHz |
| | | | | | 11,60 (s, 1H), 8,03 (d, 1H), 7,99 (d, 1H), 7,79 (m, 2H), 7,69 (m, 3H), 3,41 (s, 3H), 2,66 (s, 3H) |
| 10-75 | 4-Cl-Ph | Me | SO₂Me | CF₃ | ¹H-NMR, DMSO-d₆, 400 MHz |
| | | | | | 11,63 (s, 1H), 8,05 (d, 1H), 8,01 (d, 1H), 7,81 (m, 2H), 7,67 (m, 2H), 3,41 (s, 3H), 2,67 (s, 3H) |
| 10-76 | 1-Ethyl-benzimidazol-2-yl | Me | SO₂Me | CF₃ | ¹H-NMR, DMSO-d₆, 400 MHz |
| | | | | | 11,85 (s, 1H), 8,18 (d, 1H), 8,14 (d, 1H), 7,84 (d, 1H), 7,80 (d, 1H), 7,46 (t, 1H), 7,37 (t, 1H), 4,68 (q, 2H), 3,45 (s, 3H), 2,82 (s, 3H), 1,43 (t, 3H) |
| 10-77 | 1,2,4-Triazol-1-yl | Me | SO₂Me | CF₃ | ¹H-NMR, DMSO-d₆, 400 MHz |
| | | | | | 11,90 (s, 1H), 9,39 (s, 1H), 8,47 (s, 1H), 8,06 (d, 1H), 8,00 (d, 1H), 3,42 (s, 3H), 2,73 (s, 3H) |
| 10-78 | Benzoxazol-2-yl | Me | SO₂Me | CF₃ | ¹H-NMR, DMSO-d₆, 400 MHz |
| | | | | | 11,95 (s, 1H), 8,13 (s, 2H), 7,96 (d, 1H), 7,94 (d, 1H), 7,61 (t, 1H), 7,55 (t, 1H), 3,44 (s, 3H), 2,82 (s, 3H) |
| 10-79 | Ph | Me | Cl | CF₃ | |
| 10-80 | Ph | Me | S(O)Me | CF₃ | |
| 10-81 | Ph | Me | SEt | OMe | |
| 10-82 | Ph | Me | NMe₂ | SO₂Me | |
| 10-83 | 4-Cl-Ph | Me | NMe₂ | SO₂Me | |
| 10-84 | 1-Ethyl-benzimidazol-2-yl | Me | NMe₂ | SO₂Me | |
| 10-85 | 1,2,4-Triazol-1-yl | Me | NMe₂ | SO₂Me | |
| 10-86 | Benzoxazol-2-yl | Me | NMe₂ | SO₂Me | |
| 10-87 | Ph | Me | NH(CH₂)₂OMe | SO₂Me | |
| 10-88 | 4-Cl-Ph | Me | NH(CH₂)₂OMe | SO₂Me | |
| 10-89 | 1-Ethyl-benzimidazol-2-yl | Me | NH(CH₂)₂OMe | SO₂Me | |
| 10-90 | 1,2,4-Triazol-1-yl | Me | NH(CH₂)₂OMe | SO₂Me | |
| 10-91 | Benzoxazol-2-yl | Me | NH(CH₂)₂OMe | SO₂Me | |
| 10-92 | Ph | Me | O(CH₂)₄OMe | SO₂Me | |
| 10-93 | Ph | Me | NH₂ | SO₂Me | |
| 10-94 | Ph | Me | O(CH₂)₂-O(3,5-dimethoxypyri midin-2-yl | SO₂Me | |
| 10-95 | Ph | Me | O(CH₂)₂-O-NMe₂ | Cl | |
| 10-96 | Ph | Me | O(CH₂)₂-NH(CO)NMe₂ | Cl | |
| 10-97 | Ph | Me | O(CH₂)-5-pyrrolidin-2-on | Br | |
| 10-98 | Ph | Me | O(CH₂)₂-NH(CO)NHCO ₂Et | Cl | |
| 10-99 | Ph | Me | O(CH₂)-(CO)NEt₂ | Br | |
| 10-100 | Ph | Me | O(CH₂) -5-2,4-dimethyl-2,4-dihydro-3H-1,2,4-triazol-3-on | Cl | |
| 10-101 | Ph | Me | O(CH₂)-3,5-dimethyl-1,2-oxazol-4-yl | Cl | |
| 10-102 | Ph | Me | O(CH₂)₂-NHCO₂Me | Cl | |
| 10-103 | Ph | Me | 4,5-dihydro-1,2-oxazol-3 yl | SO₂Me | |
| 10-104 | 4-Cl-Ph | Me | 4,5-dihydro-1,2-oxazol-3 yl | SO₂Me | |
| 10-105 | 1-Ethylbenzimidazol-2-yl | Me | 4,5-dihydro-1,2-oxazol-3 yl | SO₂Me | |
| 10-106 | 1,2,4-Triazol-1-yl | Me | 4,5-dihydro-1,2-oxazol-3 yl | SO₂Me | |
| 10-107 | Benzoxazol-2-yl | Me | 4,5-dihydro-1,2-oxazol-3 yl | SO₂Me | |
| 10-108 | Ph | Me | Me | SO₂Me | |
| 10-109 | Ph | Me | OH | SO₂Me | |
| 10-110 | Ph | Me | O-CH₂-NHSO₂cPr | Cl | |
| 10-111 | Ph | Me | O-CH₂-NHSO₂Me | SO₂Me | |
| 10-112 | 4-Cl-Ph | Me | O-CH₂-NHSO₂Me | SO₂Me | |
| 10-113 | 1-Ethylbenzimidazol-2-yl | Me | O-CH₂-NHSO₂Me | SO₂Me | |
| 10-114 | 1,2,4-Triazol-1-yl | Me | O-CH₂-NHSO₂Me | SO₂Me | |
| 10-115 | Benzoxazol-2-yl | Me | O-CH₂-NHSO₂Me | SO₂Me | |
| 10-116 | Ph | Me | SMe | Cl | |
| 10-117 | Ph | Me | SOMe | Cl | |
| 10-118 | 4-Cl-Ph | Me | SO₂Me | Cl | |
| 10-119 | 1-Ethylbenzimidazol-2-yl | Me | SO₂Me | Cl | |
| 10-120 | 1,2,4-Triazol-1-yl | Me | SO₂Me | Cl | |
| 10-121 | Benzoxazol-2-yl | Me | SO₂Me | Cl | |
| 10-122 | Ph | Me | S(O)Me | SO₂Me | |
| 10-123 | Ph | Me | SMe | SO₂Me | |
| 10-124 | Ph | Me | SMe | OMe | |
| 10-125 | Ph | Me | S(O)Me | OMe | |
| 10-126 | Ph | Me | SO₂Me | OMe | |
| 10-127 | Ph | Me | SMe | Cl | |
| 10-128 | Ph | Me | S(O)Me | Cl | |
| 10-129 | Ph | Me | SO₂Me | Cl | |
| 10-130 | Ph | Me | [1,4]dioxan-2-yl-methoxy | SO₂Me | |
| 10-131 | Ph | Me | [1,4]dioxan-2-yl-methoxy | SO₂Et | |
| 10-132 | Ph | Me | O(CH₂)₄OMe | SO₂Et | |
| 10-133 | Ph | Me | O(CH₂)₃OMe | SO₂Me | |
| 10-134 | Ph | Me | O(CH₂)₃OMe | SO₂Et | |
| 10-135 | Ph | Me | O(CH₂)₂OMe | SO₂Me | |
| 10-136 | Ph | Me | O(CH₂)₂OMe | SO₂Et | |
| 10-137 | Ph | Me | S(O)Me | SO₂Me | |
| 10-138 | Ph | Me | SMe | SO₂Me | |
| 10-139 | Ph | Me | SMe | OMe | |
| 10-140 | Ph | Me | S(O)Me | OMe | |
| 10-141 | Ph | Me | SO₂Me | OMe | |
| 10-142 | Ph | Me | SMe | Cl | |
| 10-143 | Ph | Me | S(O)Me | Cl | |
| 10-144 | Ph | Me | SO₂Me | Cl | |
| 10-145 | Ph | Me | SMe | Br | |
| 10-146 | Ph | Me | SOMe | Br | |
| 10-147 | Ph | Me | SO₂Me | Br | |
| 10-148 | Ph | Me | SMe | I | |
| 10-149 | Ph | Me | SOMe | I | |
| 10-150 | Ph | Me | SO₂Me | I | |
| 10-151 | Ph | Me | SEt | Cl | |
| 10-152 | Ph | Me | SOEt | Cl | |
| 10-153 | Ph | Me | SO₂Et | Cl | |
| 10-154 | Ph | Me | SEt | Br | |
| 10-155 | Ph | Me | SOEt | Br | |
| 10-156 | Ph | Me | SO₂Et | Br | |
| 10-157 | Ph | Me | SEt | I | |
| 10-158 | Ph | Me | SOEt | I | |
| 10-159 | Ph | Me | SO₂Et | I | |
| 10-160 | Ph | Me | SEt | F | |
| 10-161 | Ph | Me | SOEt | F | |
| 10-162 | Ph | Me | SO₂Et | F | |
| 10-163 | Ph | Cl | OCH₂(CO)NM e₂ | Cl | |
| 10-164 | Ph | Cl | Cl | SO₂Me | |
| 10-165 | Ph | Cl | CH₂OCH₂CF₃ | SO₂Me | ¹H-NMR, DMSO-d₆, 400 MHz |
| | | | | | 11,72 (s, 1H), 8,12 (d, 1H), 7,94 (d, 1H), 7,82 (m, 2H), 7,59 (m, 3H), 5,23 (s, 2H), 4,29 (q, |
| | | | | | 2H), 3,38 (s, 3H) |
| 10-166 | 4-Cl-Ph | Cl | CH₂OCH₂CF₃ | SO₂Me | ¹H-NMR, DMSO-d₆, 400 MHz |
| | | | | | 11,76 (s, 1H), 8,11 (d, 1H), 7,95 (d, 1H), 7,83 (d, 2H), 7,65 (d, 2H), 5,25 (s, 2H), 4,28 (q, 2H), 3,36 (s, 3H) |
| 10-167 | 1-Ethylbenzimidazol -2-yl | Cl | CH₂OCH₂CF₃ | SO₂Me | |
| 10-168 | 1,2,4-Triazol-1-yl | Cl | CH₂OCH₂CF₃ | SO₂Me | ¹H-NMR, DMSO-d₆, 400 MHz |
| | | | | | 11,99 (s, 1H), 9,34 (s, 1H), 8,47 (s, 1H), 8,14 (d, 1H), 7,95 (d, 1H), 5,24 (s, 2H), 4,29 (q, 2H), 3,38 (s, 3H) |
| 10-169 | Benzoxazol-2-yl | Cl | CH₂OCH₂CF₃ | SO₂Me | ¹H-NMR, DMSO-d₆, 400 MHz |
| | | | | | 11,96 (s, 1H), 8,23 (d, 1H), 8,08 (d, 1H), 8,02 (d, 1H), 7,95 (d, 1 H),7,62 (dd, 1H), 7,54 (dd, 1H), 5,27 (s, 2H), 4,30 (q, 2H), 3,38 (s, 3H) |
| 10-170 | Ph | Cl | 5-Cyanomethyl-4,5-dihydro-1,2-oxazol-3 yl | SO₂Et | |
| 10-171 | 4-Cl-Ph | Cl | 5-Cyanomethyl- 4,5-dihydro-1,2-oxazol-3 yl | SO₂Et | ¹H-NMR, CDCl₃, 400 MHz |
| | | | | | 8,51 (bs, 1H), 8,08 (d, 1H), 7,70 (m, 3H), 7,51 (d, 2H), 5,15 (s, 2H), 4,03 (m, 1H), 3,81 - 3,57 (m, 4H), 3,26 (s, 3H), 2,00-1,80 (m, 3H), 1,58 (m, 1H) |
| 10-172 | 1-Ethyl-benzimidazol-2-yl | Cl | 5-Cyanomethyl- 4,5-dihydro-1,2-oxazol-3 yl | SO₂Et | ¹H-NMR, DMSO-d₆, 400 MHz |
| | | | | | 11,92 (bs, 1H), 8,26 (s, 2H), 7,84 (m, 2H), 7,46 (dd, 1H), 7,37 (dd, 1H), 5,20 (m, 1H), 4,68 (m, 2H), 3,61 (dd, 1H), 3,46 (q, 2H), 3,19 (dd, 1H), 3,01 (m, 2H), 1,42 (t, 3H), 1,18 (t, 3H) |
| 10-173 | 1,2,4-Triazol-1-yl | Cl | 5-Cyanomethyl- 4,5-dihydro-1,2-oxazol-3 yl | SO₂Et | ¹H-NMR, DMSO-d₆, 400 MHz |
| | | | | | 12,01 (s, 1H), 9,35 (s, 1H), 8,47 (s, 1H), 8,15 (d, 1H), 8,08 (d, 1H), ), 5,19 (m, 1H), 3,60 (dd, 1H), 3,41 (q, 2H), 3,17 (dd, 1H), 3,00 (m, 2H), 1,16 (t, 3H) |
| 10-174 | Benzoxazol-2-yl | Cl | 5-cyanomethyl- 4,5-dihydro-1,2-oxazol-3 yl | SO₂Et | ¹H-NMR, DMSO-d₆, 400 MHz |
| | | | | | 12,08 (bs, 1H), 8,23 (m, 2H), 8,00 (m, 2H), 7,49 (m, 2H), 5,20 (m, 1H), 3,70-2,90 (m, 6H), 1,19 (t, 3H) |
| 10-175 | Ph | Cl | CH₂O-Tetrahydrofuran-3-yl | SO₂Me | |
| 10-176 | 4-Cl-Ph | Cl | CH₂O-Tetrahydrofuran-3-yl | SO₂Me | |
| 10-177 | 1-Ethylbenzimidazol -2-yl | Cl | CH₂O-Tetrahydrofuran-3-yl | SO₂Me | |
| 10-178 | 1,2,4-Triazol-1-yl | Cl | CH₂O-Tetrahydrofuran-3-yl | SO₂Me | |
| 10-179 | Benzoxazol-2-yl | Cl | CH₂O-Tetrahydrofuran-3-yl | SO₂Me | |
| 10-180 | Ph | Cl | CH₂O-Tetrahydrofuran-2-yl | SO₂Me | |
| 10-181 | Ph | Cl | SMe | SO₂Me | |
| 10-182 | Ph | Cl | F | SMe | |
| 10-183 | 1,2,4-Triazol-1-yl | Cl | CH₂OCH₂-Tetrahydrofuran-2-yl | SO₂Me | ¹H-NMR, DMSO-d₆, 400 MHz |
| | | | | | 11,95 (bs, 1H), 9,34 (s, 1H), 8,49 (s, 1H), 8,12 (d, 1H), 7,91 (d, 1H), 5,18 (s, 2H), 3,97 (m, 1H), 3,71 (dd, 1H), 3,68 - 3,51 (m, 3H), 3,40 (s, 3H), 1,94-1,43 (m, 4 H) |
| 10-184 | 1-Ethylbenzimidazol -2-yl | Cl | CH₂OCH₂-Tetrahydrofuran-2-yl | SO₂Me | ¹H-NMR, DMSO-d₆, 400 MHz11,92 (bs, 1H), 8,21 (d, 1H), 8,10 (d, 1H), 7,82 (dd, 2H), 7,46 (dd, |
| | | | | | 1 H), 7,35 (dd, 1H), 5,21 (s, 2H), 4,47 (q, 2H), 3,98 (m, 1H), 3,72 (dd, 1H), 3,66 - 3,52 (m, 3H), 3,42 (s, 3H), 1,90 (m, 1H), 1,77 (m, 2H), 1,55 (m, 1H), 1,42 (t, 3H) |
| 10-185 | Ph | Cl | CH₂OCH₂-Tetrahydrofura n-3-yl | SO₂Me | |
| 10-186 | Ph | Cl | O(CH₂)-5-Pyrrolidin-2-on | Cl | |
| 10-187 | Ph | Cl | SMe | Cl | |
| 10-188 | Ph | Cl | S(O)Me | Cl | |
| 10-189 | Ph | Cl | SO₂Me | Cl | |
| 10-190 | Ph | Cl | CH₂O-Tetrahydrofuran-3-yl | SO₂Et | |
| 10-191 | Ph | Cl | O(CH₂)₂OMe | Cl | |
| 10-192 | 4-Cl-Ph | Cl | O(CH₂)₂OMe | Cl | |
| 10-193 | 1-Ethyl-benzimidazol-2-yl | Cl | O(CH₂)₂OMe | Cl | |
| 10-194 | 1, 2,4-Triazol-1-yl | Cl | O(CH₂)₂OMe | Cl | |
| 10-195 | Benzoxazol-2-yl | Cl | O(CH₂)₂OMe | Cl | |
| 10-196 | Ph | Cl | O(CH₂)₄OMe | SO₂Me | |
| 10-197 | Ph | Cl | O(CH₂)₄OMe | SO₂Et | |
| 10-198 | Ph | Cl | O(CH₂)₃OMe | SO₂Me | |
| 10-199 | Ph | Cl | O(CH₂)₃OMe | SO₂Et | |
| 10-200 | Ph | Cl | O(CH₂)₂OMe | SO₂Me | |
| 10-201 | Ph | Cl | O(CH₂)₂OMe | SO₂Et | |
| 10-202 | Ph | Cl | [1,4]Dioxan-2-yl-methoxy | SO₂Me | |
| 10-203 | Ph | Cl | [1,4]Dioxan-2-yl-methoxy | SO₂Et | |
| 10-204 | Ph | Cl | SO₂Me | Me | |
| 10-205 | Ph | Cl | SEt | Me | |
| 10-206 | Ph | Cl | SOEt | Me | |
| 10-207 | Ph | Cl | SO₂Et | Me | |
| 10-208 | Ph | Cl | 4,5-Ddihydro-1,2-oxazol-3 yl | SO₂Me | |
| 10-209 | Ph | Cl | Cl | SO₂Me | |
| 10-210 | Ph | F | SMe | CF₃ | |
| 10-211 | 4-Cl-Ph | F | S(O)Me | CF₃ | ¹H-NMR, CDCl₃, 400 MHz |
| | | | | | 9,12 (s, 1H), 8,76 (dd, 1H), 7,75 (dd, 1H), 7,67 (d, 2H), 7,49 (d, 2H), 3,12 (s, 3H) |
| 10-212 | Ph | F | S(O)Me | CF₃ | ¹H-NMR, CDCl₃, 400 MHz |
| | | | | | 9,19 (bs, 1H), 8,26 (dd, 1H), 7,71 (d, 1H), 7,67 (d, 2H), 7,50 (d, 2H), 3,13 (s, 3H) |
| 10-213 | Ph | OMe | SMe | CF₃ | |
| 10-214 | Ph | OMe | S(O)Me | CF₃ | |
| 10-215 | Ph | OMe | SO₂Me | CF₃ | |
| 10-216 | Ph | Et | NH(CH₂)₂OMe | SO₂Me | |
| 10-217 | Ph | Et | F | SO₂Me | |
| 10-218 | Ph | Et | SMe | CF₃ | |
| 10-219 | Ph | CF₃ | F | SO₂Me | |
| 10-220 | Ph | CF₃ | F | SO₂Et | |
| 10-221 | Ph | CF₃ | O(CH₂)₂OMe | SO₂Et | |
| 10-222 | Ph | CF₃ | O(CH₂)₃OMe | SO₂Et | |
| 10-223 | Ph | CF₃ | O(CH₂)₂OMe | SO₂Me | |
| 10-224 | Ph | CF₃ | O(CH₂)₃OMe | SO₂Me | |
| 10-225 | Ph | CF₃ | OCH₂CONMe₂ | SO₂Me | |
| 10-226 | Ph | CF₃ | OCH₂CONMe₂ | SO₂Et | |
| 10-227 | Ph | CF₃ | OCH₂CONMe₂ | Cl | |
| 10-228 | Ph | CF₃ | OCH₂CONMe₂ | Br | |
| 10-229 | Ph | CF₃ | OCH₂CONMe₂ | I | |
| 10-230 | Ph | CF₃ | OCH₂CONMe₂ | F | |
| 10-231 | Ph | CF₃ | O(CH₂)₂OMe | Cl | |
| 10-232 | Ph | CF₃ | O(CH₂)₃OMe | Cl | |
| 10-233 | Ph | CF₃ | O(CH₂)₂OMe | Br | |
| 10-234 | Ph | CF₃ | O(CH₂)₃OMe | Br | |
| 10-235 | Ph | CF₃ | O(CH₂)₂OMe | I | |
| 10-236 | Ph | CF₃ | O(CH₂)₃OMe | I | |
| 10-237 | Ph | CF₃ | O(CH₂)₂OMe | F | |
| 10-238 | Ph | CF₃ | O(CH₂)₃OMe | F | |
| 10-239 | Ph | CF₃ | [1,4]Dioxan-2-yl-methoxy | SO₂Me | |
| 10-240 | Ph | CF₃ | [1,4]Dioxan-2-yl-methoxy | SO₂Et | |
| 10-241 | Ph | CF₃ | [1,4]Dioxan-2-yl-methoxy | Cl | |
| 10-242 | Ph | CF₃ | [1,4]Dioxan-2-yl-methoxy | Br | |
| 10-243 | Ph | CF₃ | [1,4]Dioxan-2-yl-methoxy | I | |
| 10-244 | Ph | CF₃ | [1,4]Dioxan-2-yl-methoxy | F | |
| 10-245 | Ph | Br | OMe | Br | |
| 10-246 | Ph | Br | O(CH₂)₂OMe | Br | |
| 10-247 | Ph | Br | O(CH₂)₄OMe | SO₂Me | |
| 10-248 | Ph | Br | O(CH₂)₄OMe | SO₂Et | |
| 10-249 | Ph | Br | O(CH₂)₃OMe | SO₂Me | |
| 10-250 | Ph | Br | O(CH₂)₃OMe | SO₂Et | |
| 10-251 | Ph | Br | O(CH₂)₂OMe | SO₂Me | |
| 10-252 | Ph | Br | O(CH₂)₂OMe | SO₂Et | |
| 10-253 | Ph | Br | [1,4]dioxan-2-yl-methoxy | SO₂Me | |
| 10-254 | Ph | Br | [1,4]dioxan-2-yl-methoxy | SO₂Et | |
| 10-255 | Ph | Br | SMe | Me | |
| 10-256 | Ph | Br | SOMe | Me | |
| 10-257 | Ph | Br | SO₂Me | Me | |
| 10-258 | Ph | Br | SEt | Me | |
| 10-259 | Ph | Br | SOEt | Me | |
| 10-260 | Ph | Br | SO₂Et | Me | |
| 10-261 | Ph | I | O(CH₂)₄OMe | SO₂Me | |
| 10-262 | Ph | I | O(CH₂)₄OMe | SO₂Et | |
| 10-263 | Ph | I | O(CH₂)₃OMe | SO₂Me | |
| 10-264 | Ph | I | O(CH₂)₃OMe | SO₂Et | |
| 10-265 | Ph | I | O(CH₂)₂OMe | SO₂Me | |
| 10-266 | Ph | I | O(CH₂)₂OMe | SO₂Et | |
| 10-267 | Ph | I | [1,4]dioxan-2-yl-methoxy | SO₂Me | |
| 10-268 | Ph | I | [1,4]dioxan-2-yl-methoxy | SO₂Et | |
| 10-269 | Ph | I | SMe | Me | |
| 10-270 | Ph | I | SOMe | Me | |
| 10-271 | Ph | I | SO₂Me | Me | |
| 10-272 | Ph | I | SEt | Me | |
| 10-273 | Ph | I | SOEt | Me | |
| 10-274 | Ph | I | SO₂Et | Me | |
| 10-275 | Ph | CH₂SMe | OMe | SO₂Me | |
| 10-276 | Ph | CH₂OMe | OMe | SO₂Me | |
| 10-277 | Ph | CH₂O(CH₂) ₂OMe | NH(CH₂)₂OEt | SO₂Me | |
| 10-278 | Ph | CH₂O(CH₂) ₂OMe | NH(CH₂)₃OEt | SO₂Me | |
| 10-279 | Ph | CH₂O(CH₂) ₃OMe | OMe | SO₂Me | |
| 10-280 | Ph | CH₂O(CH₂) ₂OMe | NH(CH₂)₂OMe | SO₂Me | |
| 10-281 | Ph | CH₂O(CH₂) ₂OMe | NH(CH₂)₃OMe | SO₂Me | |
| 10-282 | Ph | SO₂Me | NH₂ | CF₃ | |
| 10-283 | Ph | SO₂Me | F | CF₃ | |
| 10-284 | Ph | SO₂Me | NHEt | Cl | |
| 10-285 | Ph | SMe | SEt | F | |
| 10-286 | Ph | SMe | SMe | F | |
| 10-287 | 5-phenyl-1 H-1,2,3-triazol-1-yl | Me | SO₂Me | CF₃ | ¹H-NMR, DMSO-d₆, 400 MHz |
| | | | | | 12,21 (s, 1H), 8,32 (s, 1H), 8,02 (d, 1H), 7,70 (d, 1H), 7,53 (m, 5H), 3,40 (s, 3H), 2,58 (s, 3H). |
| 10-288 | 5-phenyl-1 H-1,2,3-triazol-1-yl | Me | SO₂Me | SO₂Me | |
| 10-289 | 4-OMe-phenyl | Me | SO₂Me | CF₃ | ¹H-NMR, DMSO-d₆, 400 MHz |
| | | | | | 11,51 (s, 1H), 8,04 (d, 1H), 7,99 (d, 1H), 7,76 (d, 2H), 7,14 (m, 2H), 3, 83 (s, 3H), 3,41 (s, 3H), 2,28 (s, 3H) |
| 10-290 | 4-OMe-phenyl | Me | SO₂Me | SO₂Me | ¹H-NMR, DMSO-d₆, 400 MHz |
| | | | | | 11,50 (s, 1H), 8,26 d, 1H), 8,05 (d, 1H), 7,72 (d, 2H), 7,13 (d, 2H), 3,83 (s, 3H), 3,51 (s, 3H), 3,47 (s, 3H), 2,64 (s, 3H) |
| 10-291 | 4-OMe-phenyl | Me | S(O)Me | SO₂Me | ¹H-NMR, DMSO-d₆, 400 MHz |
| | | | | | 11,49 (s, 1H), 7,87 (broad, 2H), 7,94 (d, 1H), 7,75 (m, 2H), 7,15 (m, 2H), 3,83 (s, 3H), |
| | | | | | 3,04 (s, 3H), 2,80 (s, 3H) |
| 10-292 | 4-OMe-phenyl | Cl | 5-cyanomethyl- 4,5-dihydro-1,2-oxazol-3 yl | SO₂Et | ¹H-NMR, DMSO-d₆, 400 MHz |
| | | | | | 11,67 (s, 1H), 8,13 (d, 1H), 8,08 (d, 1H), 7,75 (m, 2H), 7,77 (m, 2H), 7,14 (m, 2H), 5,20 (m, 1 H), 3,84 (s, 3H), 3,61 (dd, 1H), 3,43 (q, 2H), 3,17 (dd, 1H), 1,17 (t, 3H) |
| 10-293 | 4-OMe-Phenyl | Cl | CH₂OCH₂CF₃ | SO₂Me | ¹H-NMR, DMSO-d₆, 400 MHz |
| | | | | | 11,63 (s, 1H), 8,13 (d, 1H), 7,95 (d, 1H), 7,78 (m, 2H), 7,13 (m, 2H), 5,25 (s, 2H), 4,29 (q, 2H), 3,84 (s, 3H), 3,36 (s, 3H) |
| 10-293 | 4-OMe-Phenyl | Cl | Me | SO₂Me | ¹H-NMR, DMSO-d₆, 400 MHz |
| | | | | | 11,50 (s, 1H), 8,06 (d, 1H), 7,80 (d, 1H), 7,75 (m, 2H), 7,14 (m, 2H), 3,83 (s, 3H), 3,43 (s, 3H), 2,41 (s, 3H) |
| 10-294 | 4-Cl-Ph | Cl | CH₂OCH₂-Tetrahydrofura n-2-yl | SO₂Me | ¹H-NMR, CDCl₃, 400 MHz |
| | | | | | 8,06 (d, 1H), 7,71 (m, 3H), 7,50 (m, 2H), 5,16 (s, 2H), 4,02 (m, 1 H), 3,81-3,57 (m, 4H), 3,25 (s, 3H), 1,99-1,83 m, 3H). |
| 10-295 | 4-Cl-Ph | Me | Cl | SO₂Me | ¹H-NMR, DMSO-d₆, 400 MHz |
| | | | | | 11,62 (s, 1H), 8,05 (d, 1H), 7,80 (m, 3H), 7,66 (m, 2H), 7,14 (m, 2H), 3,43 (s, 3H), 2,40 (s, 3H) |
| 10-296 | 4-Cl-Ph | Me | S(O)Me | CF₃ | ¹H-NMR, DMSO-d₆, 400 MHz |
| | | | | | 11,62 (s, 1H), 7,87 (s, 2H), 7,82 (d, 2H), 7,66 (d, 2H), 3,04 (s, 3H), 2,79 (s, 3H) |
| 10-297 | 4-F-Ph | Me | SO₂Me | CF₃ | ¹H-NMR, DMSO-d₆, 400 MHz |
| | | | | | 11,59 (s, 1H), 8,04 (d, |
| | | | | | 1H), 7,98 (d, 1H), 7,85 (m, 2H), 7,45 (m, 2H), 3,40 (s, 3H), 2,64 (s, 3H) |
| 10-298 | 4-F-Ph | Me | SO₂Me | SO₂Me | ¹H-NMR, DMSO-d₆, 400 MHz |
| | | | | | 11,60 (s, 1H), 8,28 (d, 1H), 8,08 (d, 1H), 7,85 (m, 2H), 7,44 (m, 2H), 3,59 (s, 3H), 3,47 (s, 3H), 2,62 (s, 3H) |
| 10-299 | 4-F-Ph | Cl | CH₂OCH₂-tetrahydrofura n-2-yl | SO₂Me | ¹H-NMR, DMSO-d₆, 400 MHz |
| | | | | | 11,70 (s, 1H), 8,09 (d, 1 H), 7,86 (m, 3H), 7,44 (t, 2H), 5,07 (s, 2H), 3,97 (m, 1H), 3,58 (dd, 1 H), 3,65-3,51 (m, 3H), 3,39 (s, 3H), 1,95-1,72 (m, 3H), 1,53 (m, 1 H) |
| 10-300 | 4-F-Ph | Cl | CH₂OCH₂CF₃ | SO₂Me | ¹H-NMR, DMSO-d₆, 400 MHz |
| | | | | | 11,71 (s, 1H), 8,11 (d, 1 H), 7,93 (d, 1H), 7,86 (dd, 2H), 7,43 (dd, 2H), 5,24 (s, 2H), 4,29 (q, 2H), 3,36 (s, 3H) |
| 10-301 | 4-F-Ph | Me | Me | SO₂Me | ¹H-NMR, DMSO-d₆, 400 MHz |
| | | | | | 11,45 (s, 1H), 7,92 (d, 1 H), 7,85 (m, 2H), 7,61 (d, 1H), 7,43 (dd, 2H), 3,26 (s, 3H), 2,61 (s, 3H), 2,27 (s, 3H) |
| 10-302 | 4-F-Ph | Cl | 5-Cyanomethyl- 4,5-dihydro-1,2-oxazol-3 yl | SO₂Et | ¹H-NMR, DMSO-d₆, 400 MHz |
| | | | | | 11,78 (s, 1H), 8,10 (dd, 2H), 7,87 (m, 2H), 7,44 (dd, 2H), 5,19 (m, 1H), 3,59 (dd, 1 H), 3,43 (q, 2H), 3,15 (dd, 1 H), 3,01 (m, 2H), 1,16 (t, 3H) |
| 10-304 | 4-F-Ph | Me | S(O)Me | CF₃ | ¹H-NMR, DMSO-d₆, 400 MHz |
| | | | | | 11,58 (s, 1H), 7,85 (m, 4H), 7,44 (m, 2H), 3,04 (s, 3H), 2,78 (s, 3H) |
| 10-305 | Ph | Cl | Me | SO₂Et | ¹H-NMR, DMSO-d₆, 400 MHz |
| | | | | | 11,64 (s, 1H), 8,01 (d, 1H), 7,80 (m, 2H), 7,75 (d, 1H), 7,59 (m, 3H), 3,42 (q, 2H), 2,73 (s, 3H), 1,13 (t, 3H) |
| 10-306 | Ph | Me | 1,2,3-1H-triazol-1-yl | SO₂Me | ¹H-NMR, DMSO-d₆, 400 MHz |
| | | | | | 11,72 (s, 1H), 8,53 (s, 1H), 8,17 (d, 1H), 8,12 (d, 1 H), 8,05 (s, 1H), 7,79 (m, 2H), 7,57 (m, 2H), 3,13 (s, 3H), 1,81 (s, 3H) |
| 10-307 | 1,2,4-triazol-1-yl | Me | S(O)Me | CF₃ | ¹H-NMR, DMSO-d₆, 400 M Hz |
| | | | | | 11,87 (s, 1H), 9,39 (s, 1 H), 8,48 (s, 1H), 7,90 (d, 1H), 7,88 (d, 1H), 3,05 (s, 3H), 2,85 (s, 3H) |
| 10-308 | 4-Methyl-1,3-thiazol-2-yl | Me | SO₂Me | CF₃ | ¹H-NMR, DMSO-d₆, 400 MHz |
| | | | | | 11,65 (s, 1H), 8,10 (2d, 2H), 7,71 (s, 1H), 3,44 (s, 3H), 2,82 (s, 3H) |
| 10-309 | Thiophen-2-yl | Me | SO₂Me | CF₃ | |
| 10-310 | 1,2,4-Oxadiazol-3-yl | Me | SO₂Me | CF₃ | |
| 10-311 | 5-Methoxymethyl-1,2,4-oxadiazol-3-yl | Me | SO₂Me | CF₃ | |
| 10-312 | 1-Methyl-5-ethylsulfonyl-1,3,4-triazol-2-yl | Me | SO₂Me | CF₃ | |
| 10-313 | 4-Methyl-1,3-thiazol-2-yl | Me | SO₂Me | SO₂Me | |
| 10-314 | Thiophen-2-yl | Me | SO₂Me | SO₂Me | ¹H-NMR, DMSO-d₆, 400 MHz |
| | | | | | 11,60 (s, 1H), 8,30 (d, 1H), 8,15 (d, 1H), 7,94 (d, 1H), 7,77 (d, 1H), 7,31 (dd, 1H), 3,61 (s, 3H), 3,57 (s, 1H), 2,71 (s, 3H) |
| 10-315 | 1,2,4-Oxadiazol-3-yl | Me | SO₂Me | SO₂Me | |
| 10-316 | 5-Methoxymethyl-1,2,4-oxadiazol-3-yl | Me | SO₂Me | SO₂Me | |
| 10-317 | 1-Methyl-5-methylsulfonyl-1,3,4-triazol-2-yl | Me | SO₂Me | SO₂Me | ¹H-NMR, DMSO-d₆, 400 MHz 11,89 (s, 1H), 8,08 (d, 1H), 7,99 (d 1H), 4,08 (s, 3H), 3,66 (s, 3H), 3,42 (s, 3H), 2,72 (s, 3H) |
| 10-318 | Pyridin-2-yl | Me | SO₂Me | CF₃ | ¹H-NMR, DMSO-d₆, 400 MHz |
| | | | | | 11,62 (s, 1H), 8,74 (d, 1H), 8,13-8,05 (m, 4H), 7,63 (dd, 1H), 3,43 (s, 1H), 2,78 (s, 3H) |
| 10-319 | Pyridin-2-yl | Me | S(O)Me | CF₃ | |
| 10-320 | Pyridin-2-yl | Me | SO₂Me | SO₂Me | |
| 10-321 | Pyridin-3-yl | Me | SO₂Me | CF₃ | ¹H-NMR, DMSO-d₆, 400 MHz |
| | | | | | 11,74 (s, 1H), 8,98 (bs, 1H), 8,78 (bd, 1H), 8,21 (dd, 1H), 8,04 (d, 1 H), 8,02 (d, 1H), 7,63 (dd, 1H), 3,41 (s, 3H), 2,66 (s, 3H) |
| 10-322 | Pyridin-3-yl | Me | S(O)Me | CF₃ | |
| 10-323 | Pyridin-3yl | Me | SO₂Me | SO₂Me | ¹H-NMR, DMSO-d₆, 400 MHz |
| | | | | | 11,74 (s, 1H), 8,96 (d, 1H), 8,77 (dd, 1H), 8,27 (d, 1H), 8,21 (dt, 1H), 8,07 (d, 1H), 7,62 (dd, 1H), 3,59 (s, 3H), 3,54 (s, 3H), 2,62 (s, 3H) |
| 10-324 | Pyridin-4-yl | Me | SO₂Me | CF₃ | |
| 10-325 | Pyridin-4-yl | Me | S(O)Me | CF3 | ¹H-NMR, DMSO-d₆, 400 MHz |
| | | | | | 11,82 (s, 1H), 8,85 (m, 2H), 7,88 (m, 4H), 3,04 (s, 3H), 2,79 (s, 3H) |
| 10-326 | Pyridin-4-yl | Me | SO₂Me | SO₂Me | |
| 10-327 | 4-(5-allylsulfanyl)-4-methyl-4H-1,2,4-triazol-3-yl | Me | SO₂Me | CF₃ | ¹H-NMR, DMSO-d₆, 400 MHz |
| | | | | | 11,52 (s, 1H), 8,09 (d, 1H), 8,03 (d 1H), 5,95 (m, 1H), 5,24 (dd, 1 H), 5,10 (dd, 1H), 3,88 (d, 2H), 3,43 (s, 3H), 2,74 (s, 3H) |
| 10-328 | 4-(5-allylsulfanyl)-4-methyl-4H-1,2,4-triazol-3-yl | Me | S(O)Me | CF₃ | |
| 10-329 | 4-(5-allylsulfanyl)-4-methyl-4H-1,2,4-triazol-3-yl | Me | SO₂Me | SO₂Me | |

**Tabelle 11: Erfindungsgemäße Verbindungen der allgemeinen Formel (I)**

| | | | | | |
|---|---|---|---|---|---|
| 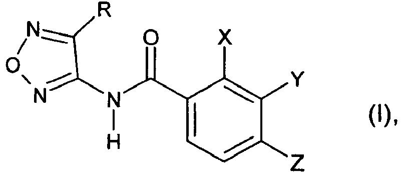 | | | | | |

| **Nr.** | **R** | **X** | **Y** | **Z** | **Physikalische Daten** |
|---|---|---|---|---|---|
| 11-2 | Bz | CF₃ | SMe | SO₂Me | |
| 11-3 | Bz | CF₃ | OCH₂(CO)NMe₂ | SO₂Me | |
| 11-4 | Bz | Cl | Pyrazol-1-yl | SO₂Me | |
| 11-5 | Bz | Cl | CH₂OCH₂CF₃ | SO₂Me | ¹H-NMR, CDCl₃, 400 MHz |
| | | | | | 10,13 (s, 1H), 8,44 (d, 2H), 8,25 (d, 1H), 7,90 (d, 1H), 7,76 (t, 1H), 7,60 (t, 2H), 5,42 (s, 2H), 4,08 (q, 2H), 3,26 (s, 3H) |
| 11-6 | Bz | Cl | CH₂O-Tetrahydrofuran-3-yl | SO₂Me | |
| 11-7 | Bz | Cl | 5-Cyano-methyl-4,5-dihydro-1,2-oxazol-3 yl | SO₂Et | |
| 11-8 | Bz | Me | 4,5-Dihydro-1,2-oxazol-3 yl | SO₂Me | |
| 11-9 | Bz | Me | SO₂Me | Cl | |
| 11-10 | Bz | Me | SO₂Me | SO₂Me | |
| 11-11 | Bz | Me | SO₂Me | CF₃ | |
| 11-12 | Bz | Me | NMe₂ | SO₂Me | |
| 11-13 | Bz | Cl | O(CH₂)₂OMe | Cl | |
| 11-14 | Bz | Me | NH(CH₂)₂OMe | SO₂Me | |
| 11-15 | Bz | Me | O(CH₂)₂NHSO₂Me | SO₂Me | |
| 11-16 | CO₂Me | CF₃ | SMe | SO₂CH₃ | |
| 11-17 | CO₂Me | CF₃ | OCH₂(CO)NMe₂ | SO₂Me | |
| 11-18 | CO₂Me | Cl | Pyrazol-1-yl | SO₂Me | |
| 11-19 | CO₂Me | F | SO₂Me | CF₃ | |
| 11-20 | CO₂Me | Cl | CH₂OCH₂CF₃ | SO₂Me | ¹H-NMR, CDCl₃, 400 MHz |
| | | | | | 9,55 (bs, 1H), 8,22 (d, 1H), 7,88 (d, 1H), 5,39 (s, 2H), 4,08 (m, 5H), 3,25 (s, 3H) |
| 11-21 | CO₂Me | Cl | CH₂O-Tetrahydrofuran-3-yl | SO₂Me | |
| 11-22 | CO₂Me | Cl | 5-Cyano-methyl-4,5-dihydro-1,2-oxazol-3 yl | SO₂Et | ¹H-NMR, CDCl₃, 400 MHz |
| | | | | | 9,68 (bs, 1H), 8,18 (d, 1H), 8,03 (d, 1H), 5,19 (m, 1H), 4,10 (s, 3H), 3,75 (m, 1H), 3,39 (q, 2H), 3,30 (m, 1H), 2,82-3,02 (m, 2H), 1,32 (t, 3H) |
| 11-23 | CO₂Me | Me | 4,5-Dihydro-1,2-oxazol-3 yl | SO₂Me | |
| 11-24 | CO₂Me | Me | SO₂Me | Cl | |
| 11-25 | CO₂Me | Me | SO₂Me | SO₂Me | |
| 11-26 | CO₂Me | Me | SO₂Me | CF₃ | ¹H-NMR, DMSO-d₆, 400 MHz |
| | | | | | 12,13 (s, 1H), 8,06 (d, 1H), 7,97 (d, 1H), 3,95 (s, 3H), 3,41 (s, 3H), 2,75 (s, 3H) |
| 11-27 | CO₂Me | Me | NMe₂ | SO₂Me | |
| 11-28 | CO₂Me | Cl | O(CH₂)₂OMe | Cl | |
| 11-29 | CO₂Me | Me | NH(CH₂)₂OMe | SO₂Me | |
| 11-30 | CO₂Me | Me | O(CH₂)₂NHSO₂Me | SO₂Me | |
| 11-31 | NHAc | CF3 | SMe | SO₂CH₃ | |
| 11-32 | NHAc | CF3 | OCH₂(CO)NMe₂ | SO₂Me | |
| 11-33 | NHAc | Cl | Pyrazol-1-yl | SO₂Me | |
| 11-34 | NHAc | F | SO₂Me | CF₃ | |
| 11-35 | NHAc | Cl | CH₂OCH₂CF₃ | SO₂Me | |
| 11-36 | NHAc | Cl | CH₂O-Tetrahydrofuran-3-yl | SO₂Me | |
| 11-37 | NHAc | Cl | 5-Cyanomethyl-4,5-dihydro-1,2-oxazol-3 yl | SO₂Et | |
| 11-38 | NHAc | Me | 4,5-Dihydro-1,2-oxazol-3 yl | SO₂Me | |
| 11-39 | NHAc | Me | SO₂Me | Cl | |
| 11-40 | NHAc | Me | SO₂Me | SO₂Me | ¹H-NMR, DMSO-d₆, 400 MHz |
| | | | | | 11,41 (s, 1H), 10,85 (s, 1H), 8,28 (d, 1H), 8,03 (d, 1H), 6,14 (bs, 2H), 3,60 (s, 3H), 3,57 (s, 3H), 2,70 (s, 3H), 2,09 (s, 3H) |
| 11-41 | NHAc | Me | SO₂Me | CF₃ | ¹H-NMR, DMSO-d₆, 400 MHz |
| | | | | | 11,40 (s, 1H), 10,85 (s, 1H), 8,07 (d, 1H), 7,99 (d, 1H), 3,42 (s, 3H), 2,76 (s, 3H), 2,09 (s, 3H) |
| 11-42 | NHAc | Me | NMe₂ | SO₂Me | |
| 11-43 | NHAc | Cl | O(CH₂)₂OMe | Cl | |
| 11-44 | NHAc | Me | NH(CH₂)₂OMe | SO₂Me | |
| 11-45 | NHAc | Me | O(CH₂)₂NHSO₂Me | SO₂Me | |
| 11-46 | Ac | CF3 | SMe | SO₂CH₃ | |
| 11-47 | Ac | CF3 | OCH2(CO)NMe2 | SO₂Me | |
| 11-48 | Ac | Cl | Pyrazol-1-yl | SO₂Me | |
| 11-49 | Ac | F | SO₂Me | CF₃ | |
| 11-50 | Ac | Cl | CH₂OCH₂CF₃ | SO₂Me | |
| 11-51 | Ac | Cl | CH₂O-Tetrahydrofuran-3-yl | SO₂Me | |
| 11-52 | Ac | Cl | 5-Cyano-methyl-4,5-dihydro-1,2-oxazol-3 yl | SO₂Et | |
| 11-53 | Ac | Me | 4,5-Dihydro-1,2-oxazol-3 yl | SO₂Me | |
| 11-54 | Ac | Me | SO₂Me | Cl | |
| 11-55 | Ac | Me | SO₂Me | SO₂Me | |
| 11-56 | Ac | Me | SO₂Me | CF₃ | ¹H-NMR, DMSO-d₆, 400 MHz |
| | | | | | 11,76 (s, 1H), 8,04 (d, 1H), 7,95 (d, 1H), 3,43 (s, 3H), 2,74 (s, 3H), 2,71 (s, 3H) |
| 11-57 | Ac | Me | NMe₂ | SO₂Me | |
| 11-58 | Ac | Cl | O(CH₂)₂OMe | Cl | |
| 11-59 | Ac | Me | NH(CH₂)₂OMe | SO₂Me | |
| 11-60 | Ac | Me | O(CH₂)₂NHSO₂Me | SO₂Me | |
| 11-61 | Piperidin -1-yl | CF3 | SMe | SO₂CH₃ | |
| 11-62 | Piperidin -1-yl | CF3 | OCH₂(CO)NMe₂ | SO₂Me | |
| 11-63 | Piperidin -1-yl | Cl | Pyrazol-1-yl | SO₂Me | |
| 11-64 | Piperidin -1-yl | F | SO₂Me | CF₃ | |
| 11-65 | Piperidin -1-yl | Cl | CH₂OCH₂CF₃ | SO₂Me | ¹H-NMR, DMSO-d₆, 400 MHz |
| | | | | | 11,31 (s, 1H), 8,11 (d, 1H), 7,89 (d, 1H), 5,24 (s, 2H), 4,29 (q, 2H), 3,36 (s, 3H), 1,59 (bs, 6H) |
| 11-66 | Piperidin-1-yl | Cl | CH₂O-Tetrahydrofuran-3-yl | SO₂Me | |
| 11-67 | Piperidin -1-yl | Cl | 5-Cyanomethyl-4,5-dihydro-1,2-oxazol-3 yl | SO₂Et | ¹H-NMR, CDCl₃, 400 MHz |
| | | | | | 8,13 (broad d, 1H), 7,95 (broad, 1 H), 5,17 (m, 2H), 3,71 (m, 1 H), 3,39-3,20 (m, 6H), 2,90 (m, 2H), 1,68 (*broad*, 6H), 1,27 (t, 3H) |
| 11-68 | Piperidin -1-yl | Me | 4,5-Dihydro-1,2-oxazol-3 yl | SO₂Me | |
| 11-69 | Piperidin -1-yl | Me | SO₂Me | Cl | |
| 11-70 | Piperidin -1-yl | Me | SO₂Me | SO₂Me | ¹H-NMR, DMSO-d₆, 400 MHz |
| | | | | | 11,20 (s, 1H), 8,29 (d, 1H), 7,99 (d, 1H), 3,60 (s, 3H), 3,57 (s, 3H), 3,31 (broad, 4H), 2,70 (s, 3H), 1,61 (bs, 6H) |
| 11-71 | Piperidin -1-yl | Me | SO₂Me | CF₃ | ¹H-NMR, DMSO-d₆, 400 MHz |
| | | | | | 11,19 (s, 1H), 8,04 (d, 1H), 7,93 (d, 1H), 3,43 (s, 3H), 3,29 (m, 4H), 2,74 (s, 3H), 1,60 (broad, 6H) |
| 11-72 | Piperidin -1-yl | Me | NMe₂ | SO₂Me | |
| 11-73 | Piperidin -1-yl | Cl | O(CH₂)₂OMe | Cl | |
| 11-74 | SMe | CF3 | SMe | SO₂CH₃ | |
| 11-75 | SMe | CF3 | OCH₂(CO)NMe₂ | SO₂Me | |
| 11-76 | SMe | Cl | Pyrazol-1-yl | SO₂Me | |
| 11-77 | SMe | F | SO₂Me | CF₃ | |
| 11-78 | SMe | Cl | CH₂OCH₂CF₃ | SO₂Me | |
| 11-79 | SMe | Cl | CH₂O-Ttetrahydrofuran-3-yl | SO₂Me | |
| 11-80 | SMe | Cl | 5-Cyano-methyl-4,5-dihydro-1,2-oxazol-3 yl | SO₂Et | |
| 11-81 | SMe | Me | 4,5-Dihydro-1,2-oxazol-3 yl | SO₂Me | |
| 11-82 | SMe | Me | SO₂Me | Cl | |
| 11-83 | SMe | Me | SO₂Me | SO₂Me | |
| 11-84 | SMe | Me | SO₂Me | CF₃ | ¹H-NMR, DMSO-d₆, 400 MHz |
| | | | | | 11,80 (s, 1H), 8,05 (d, 1H), 7,96 (d, 1H), 3,42 (s, 3H), 2,76 (s, 3H), 2,66 (s, 3H) |
| 11-85 | SMe | Me | NMe₂ | SO₂Me | |
| 11-86 | SMe | Cl | O(CH₂)₂OMe | Cl | |
| 11-87 | SMe | Me | NH(CH₂)₂OMe | SO₂Me | |
| 11-88 | SMe | Me | O(CH₂)₂NHSO₂Me | SO₂Me | |
| 11-89 | Cl | CF3 | SMe | SO₂CH₃ | |
| 11-90 | Cl | CF3 | OCH₂(CO)NMe₂ | SO₂Me | |
| 11-91 | Cl | Cl | Pyrazol-1-yl | SO₂Me | |
| 11-92 | Cl | F | SO₂Me | CF₃ | |
| 11-93 | Cl | Cl | CH₂OCH₂CF₃ | SO₂Me | |
| 11-94 | Cl | Cl | CH₂O-Tetrahydrofuran-3-yl | SO₂Me | |
| 11-95 | Cl | Cl | 5-Cyanomethyldihydro-1,2-oxazol-3 yl | SO₂Et | |
| 11-96 | Cl | Me | 4,5-Dihydro-1,2-oxazol-3 yl | SO₂Me | |
| 11-97 | Cl | Me | SO₂Me | Cl | |
| 11-98 | Cl | Me | SO₂Me | SO₂Me | |
| 11-99 | Cl | Me | SO₂Me | CF₃ | |
| 11-100 | Cl | Me | NMe₂ | SO₂Me | |
| 11-101 | Cl | Cl | O(CH₂)₂OMe | Cl | |
| 11-102 | Cl | Me | NH(CH₂)₂OMe | SO₂Me | |
| 11-103 | Cl | Me | O(CH₂)₂NHSO₂Me | SO₂Me | |
| 11-104 | Br | CF3 | SMe | SO₂CH₃ | |
| 11-105 | Br | CF3 | OCH2(CO)NMe2 | SO₂Me | |
| 11-106 | Br | Cl | Pyrazol-1-yl | SO₂Me | |
| 11-107 | Br | F | SO₂Me | CF₃ | |
| 11-108 | Br | Cl | CH₂OCH₂CF₃ | SO₂Me | ¹H-NMR, DMSO-d₆, 400 MHz |
| | | | | | 11,86 (s, 1H), 8,13 (d, 1H), 7,94 (d, 1H), 5,26 (s, 2H), 4,30 (q, 2H), 3,37 (s, 3H) |
| 11-109 | Br | Cl | CH₂O-Tetrahydrofuran-3-yl | SO₂Me | |
| 11-110 | Br | Cl | 5-Cyanomethyldihydro-1,2-oxazol-3 yl | SO₂Et | |
| 11-111 | Br | Me | 4,5-Dihydro-1,2-oxazol-3 yl | SO₂Me | |
| 11-112 | Br | Me | SO₂Me | Cl | |
| 11-113 | Br | Me | SO₂Me | SO₂Me | ¹H-NMR, DMSO-d₆, 400 MHz |
| | | | | | 11,76 (s, 1H), 8,27 (d, 1H), 7,05 (d, 1H), 3,60 (s, 3H), 3,57 (s, 3H), 2,73 (s, 3H) |
| 11-114 | Br | Me | SO₂Me | CF₃ | ¹H-NMR, DMSO-d₆, 400 MHz |
| | | | | | 11,74 (s, 1H), 8,05 (d, 1H), 7,99 (d, 1H), 3,43 (s, 3H), 2,76 (s, 3H) |
| 11-115 | Br | Me | NMe₂ | SO₂Me | |
| 11-116 | Br | Cl | O(CH₂)₂OMe | Cl | |
| 11-117 | Br | Me | NH(CH₂)₂OMe | SO₂Me | |
| 11-118 | Br | Me | O(CH₂)₂NHSO₂Me | SO₂Me | |
| 11-119 | I | CF3 | SMe | SO₂CH₃ | |
| 11-120 | I | CF3 | OCH₂(CO)NMe₂ | SO₂Me | |
| 11-121 | I | Cl | Pyrazol-1-yl | SO₂Me | |
| 11-122 | I | F | SO₂Me | CF₃ | |
| 11-123 | I | Cl | CH₂OCH₂CF₃ | SO₂Me | |
| 11-124 | I | Cl | CH₂O-Tetrahydrofuran-3-yl | SO₂Me | |
| 11-125 | I | Cl | 5-Cyano-methyl-4,5-dihydro-1,2-oxazol-3 yl | SO₂Et | |
| 11-126 | I | Me | 4,5-Dihydro-1,2-oxazol-3 yl | SO₂Me | |
| 11-127 | I | Me | SO₂Me | Cl | |
| 11-128 | I | Me | SO₂Me | SO₂Me | |
| 11-129 | I | Me | SO₂Me | CF₃ | |
| 11-130 | I | Me | NMe₂ | SO₂Me | |
| 11-131 | I | Cl | O(CH₂)₂OMe | Cl | |
| 11-132 | I | Me | NH(CH₂)₂OMe | SO₂Me | |
| 11-133 | I | Me | O(CH₂)₂NHSO₂Me | SO₂Me | |
| 11-134 | C(O)NH 2 | CF3 | SMe | SO₂CH₃ | |
| 11-135 | C(O)NH 2 | CF3 | OCH₂(CO)NMe₂ | SO₂Me | |
| 11-136 | C(O)NH 2 | Cl | Pyrazol-1-yl | SO₂Me | |
| 11-137 | C(O)NH 2 | F | SO₂Me | CF₃ | |
| 11-138 | C(O)NH 2 | Cl | CH₂OCH₂CF₃ | SO₂Me | |
| 11-139 | C(O)NH 2 | Cl | CH₂O-Tetrahydrofuran-3-yl | SO₂Me | |
| 11-140 | C(O)NH 2 | Cl | 5-Cyanomethyl-4,5-dihydro-1,2-oxazol-3 yl | SO₂Et | |
| 11-141 | C(O)NH 2 | Me | 4,5-Dihydro-1,2-oxazol-3 yl | SO₂Me | |
| 11-142 | CH₂OMe | Me | SO₂Me | Cl | |
| 11-143 | CH₂OMe | Me | SO₂Me | SO₂Me | |
| 11-144 | CH₂OMe | Me | SO₂Me | CF₃ | |
| 11-145 | CH₂OMe | Me | NMe₂ | SO₂Me | |
| 11-146 | CH₂OMe | Cl | O(CH₂)₂OMe | Cl | |
| 11-147 | CH₂OMe | Me | NH(CH₂)₂OMe | SO₂Me | |
| 11-148 | CH₂OMe | Me | O(CH₂)₂NHSO₂Me | SO₂Me | |
| 11-149 | NH₂ | CF3 | SMe | SO₂CH₃ | |
| 11-150 | NH₂ | CF3 | OCH₂ (CO)NMe₂ | SO₂Me | |
| 11-151 | NH₂ | Cl | Pyrazol-1-yl | SO₂Me | |
| 11-152 | NH₂ | Me | 1,2,3-triazol-2-yl | SO₂Me | |
| 11-153 | NH₂ | Cl | CH₂OCH₂CF₃ | SO₂Me | ¹H-NMR, DMSO-d₆, 400 MHz |
| | | | | | 11,32 (s, 1H), 8,13 (d, 1H), 8,05 (d, 1H), 6,13 (bs, 2H), 5,25 (s, 2H), 4,30 (q, 2H), 3,36 (s, 3H) |
| 11-154 | NH₂ | Cl | CH₂O-Tetrahydrofuran-3-yl | SO₂Me | |
| 11-155 | NH₂ | Cl | 5-Cyano-methyl-4,5-dihydro-1,2-oxazol-3 yl | SO₂Et | ¹H-NMR, DMSO-d₆, 400 MHz |
| | | | | | 11,35 (s, 1H), 8,18 (d, 1H), 8,15 (d, 1H), 6,14 (bs, 2H), 5,20 (m, 1H), 3,60 (dd, 1 H), 3,42 (q, 2H), 3,17 (dd, 1 H), 3,02 (m, 2H), 1,17 (t, 3H) |
| 11-156 | NH₂ | Me | 4,5-Dihydro-1,2-oxazol-3 yl | SO₂Me | |
| 11-157 | NH₂ | Me | SO₂Me | Cl | |
| 11-158 | NH₂ | Me | SO₂Me | SO₂Me | ¹H-NMR, DMSO-d₆, 400 MHz |
| | | | | | 11,02 (s, 1H), 8,14 (d, 1H), 7,89 (d, 1H), 6,12 (bs, 2H), 3,42 (s, 3H), 2,73 (s, 3H) |
| 11-159 | NH₂ | Me | SO₂Me | CF₃ | ¹H-NMR, DMSO-d₆, 400 MHz |
| | | | | | 11,20 (s, 1H), 8,10 (d, 1H), 8,06 (d, 1H), 6,15 (bs, 2H), 3,60 (s, 3H), 3,58 (s, 3H), 2,67 (s, 3H) |
| 11-160 | NH₂ | Me | NMe₂ | SO₂Me | |
| 11-161 | NH₂ | Cl | O(CH₂)₂OMe | Cl | |
| 11-162 | NH₂ | Me | NH(CH₂)₂OMe | SO₂Me | |
| 11-163 | NH₂ | Me | O(CH₂)₂NHSO2Me | SO₂Me | |
| 11-164 | H | CF3 | SMe | SO₂CH₃ | |
| 11-165 | H | CF3 | OCH2(CO)NMe2 | SO₂Me | |
| 11-166 | H | Cl | Pyrazol-1-yl | SO₂Me | |
| 11-167 | H | F | SO2Me | CF₃ | |
| 11-168 | H | Cl | CH₂OCH₂CF₃ | SO₂Me | |
| 11-169 | H | Cl | CH₂O-Tetrahydrofuran-3-yl | SO₂Me | |
| 11-170 | H | Cl | 5-Cyanomethyl-4,5-dihydro-1,2-oxazol-3 yl | SO₂Et | |
| 11-171 | H | Me | 4,5-Dihydro-1,2-oxazol-3 yl | SO₂Me | |
| 11-172 | H | Me | SO₂Me | Cl | |
| 11-173 | H | Me | SO₂Me | SO₂Me | |
| 11-174 | H | Me | SO₂Me | CF₃ | |
| 11-175 | H | Me | NMe₂ | SO₂Me | |
| 11-176 | H | Cl | O(CH₂)₂OMe | Cl | |
| 11-177 | H | Me | NH(CH₂)₂OMe | SO₂Me | |
| 11-178 | H | Me | O(CH₂)₂NHSO2Me | SO₂Me | |
| 11-179 | NO₂ | CF3 | SMe | SO₂CH₃ | |
| 11-180 | NO₂ | CF3 | OCH₂(CO)NMe₂ | SO₂Me | |
| 11-181 | NO₂ | Cl | Pyrazol-1-yl | SO₂Me | |
| 11-182 | NO₂ | F | SO₂Me | CF₃ | |
| 11-183 | NO₂ | Cl | CH₂OCH₂CF₃ | SO₂Me | ¹H-NMR, CDCl₃, 400 MHz |
| | | | | | 9,48 (broad s, 1H), 8,13 (d, 1H), 7,87 (d, 1H), 5,34 (s, 2H), 4,08 (q, 2H), 3,32 (s, 3H) |
| 11-184 | NO₂ | Cl | CH₂O-Tetrahydrofuran-3-yl | SO₂Me | |
| 11-185 | NO₂ | Cl | 5-Cyano-methyl-4,5-dihydro-1,2-oxazol-3 yl | SO₂Et | ¹H-NMR, DMSO-d₆, 400 MHz |
| | | | | | 12,26 (s, 1H), 8,15 (d, 1H), 8,01 (d, 1H), 5,20 (m, 1H), 3,61 (dd, 1H), 3,44 (q, 2H), 3,17 (dd, 1H), 3,03 (m, 2H),1,18 (t, 3H) |
| 11-186 | NO₂ | Me | 4,5-Dihydro-1,2-oxazol-3 yl | SO₂Me | |
| 11-187 | NO₂ | Me | SO₂Me | Cl | |
| 11-188 | NO₂ | Me | SO₂Me | SO₂Me | ¹H-NMR, DMSO-d₆, 400 MHz |
| | | | | | 12,28 (s, 1H), 8,29 (d, 1H), 8,02 (d, 1H), 3,61 (s, 3H), 3,58 (s, 3H), 2,70 (s, 3H) |
| 11-189 | NO₂ | Me | SO₂Me | CF₃ | ¹H-NMR, DMSO-d₆, 400 MHz |
| | | | | | 12,30 (s, 1H), 8,09 (d, 1H), 7,93 |
| | | | | | (d, 1H), 3,44 (s, 3H), 2,72 (s, 3H) |
| 11-190 | NO₂ | Me | NMe₂ | SO₂Me | |
| 11-191 | NO₂ | Cl | O(CH₂)₂OMe | Cl | |
| 11-192 | NO₂ | Me | NH(CH₂)₂OMe | SO₂Me | |
| 11-193 | NO₂ | Me | O(CH₂)₂NHSO2Me | SO₂Me | |
| 11-194 | c-Pr | CF3 | SMe | SO₂CH₃ | |
| 11-195 | c-Pr | CF3 | OCH2(CO)NMe2 | SO₂Me | |
| 11-196 | c-Pr | Cl | Pyrazol-1-yl | SO₂Me | |
| 11-197 | c-Pr | F | SO₂Me | CF₃ | |
| 11-198 | c-Pr | Cl | CH₂OCH₂CF₃ | SO₂Me | ¹H-NMR, DMSO-d₆, 400 MHz |
| | | | | | 11,64 (s, 1H), 8,13 (d, 1H), 7,99 (d, 1H), 5,24 (s, 2H), 4,30 (q, 2H), 3,37 (s, 3H), 2,09 (m, 1H), 1,14 (m, 2H), 0,97 (m, 2H) |
| 11-199 | c-Pr | Cl | CH₂O-Tetrahydrofuran-3-yl | SO₂Me | |
| 11-200 | c-Pr | Cl | 5-Cyanomethyl-4,5-dihydro-1,2-oxazol-3 yl | SO₂Et | |
| 11-201 | c-Pr | Me | 4,5-Dihydro-1,2-oxazol-3 yl | SO₂Me | |
| 11-202 | c-Pr | Me | SO₂Me | Cl | |
| 11-203 | c-Pr | Me | SO₂Me | SO₂Me | ¹H-NMR, DMSO-d₆, 400 MHz |
| | | | | | 11,55 (s, 1H), 8,28 (d, 1H), 8,08 (d, 1H), 3,60 (s, 3H), 3,57 (s, 3H), 2,72 (s, 3H), 2,06 (m, 1H), 1,13 (m, 2H), 0,95 (m, 2H) |
| 11-204 | c-Pr | Me | SO₂Me | CF₃ | ¹H-NMR, DMSO-d₆, 400 MHz |
| | | | | | 11,53 (s, 1H), 8,04 (s, 2H), 3,43 (s, 3H), 2,76(s, 3H), 2,07 (m, 1H), 1,12 (m, 2H), 0,96 (m, 2H) |
| 11-205 | c-Pr | Me | NMe₂ | SO₂Me | |
| 11-206 | c-Pr | Cl | O(CH₂)₂OMe | Cl | |
| 11-207 | c-Pr | Me | NH(CH₂)₂OMe | SO₂Me | |
| 11-208 | c-Pr | Me | O(CH₂)₂NHSO₂Me | SO₂Me | |
| 11-209 | Piperidin -1-yl | Cl | SMe | SO₂Me | |
| 11-210 | Piperidin | F | S(O)Me | CF₃ | ¹H-NMR, CDCl₃, 400 MHz |
| | -1-yl | | | | 8,78 (broad, 1H), 8,35 (bs, 1H), 7,75 (d, 1H), 3,29 (bs, 4H), 3,14 (s, 1H), 1,75-1,61 (m, 6H) |
| 11-211 | Piperidin | Cl | (Tetrahydrofuran-2-yl-methoxy)methyl | SO₂Me | ¹H-NMR, CDCl₃, 400 MHz |
| | -1-yl | | | | 8,11 (d, 1H), 7,60 (d, 1H), 5,14 |
| | | | | | (m, 2H), 4,09 (m, 1H), 3,86-3,68 (m, 2H), 3,65-3,54 (m, 2H), 3,30 (s, 3H), 2,02-1,81 (m, 4H), 1,74-1,50 (m, 4H), 1,69 (m, 3H), 1,58 (m, 2H) |
| 11-212 | Piperidin -1-yl | Cl | Me | SO₂Et | ¹H-NMR, DMSO-d₆, 400 MHz |
| | | | | | 11,22 (s, 1H), 8,03 (d, 1H), 7,68 (d, 1H), 3,41 (q,2H), 3,31 (m, 4H), 2,72 (s, 3H), 1,59 (broad, 6H), 1,12 /t, 3H) |
| 11-213 | Piperidin -1-yl | Me | Me | SO₂Me | ¹H-NMR, DMSO-d₆, 400 MHz |
| | | | | | 11,04 (s, 1H), 7,94 (d, 1H), 7,53 (d, 1H), 3,29 (s,3H), 2,63 (s, 3H), 2,36 (s, 3H) |
| 11-214 | Piperidin -1-yl | Cl | Me | SO₂Me | ¹H-NMR, DMSO-d₆, 400 MHz |
| | | | | | 11,15 (s, 1H), 8,05 (d, 1H), 7,72 (d, 1H), 3,43 (s,3H), 3,28 (broad, 4H), 1,59 (bs, 6H) |
| 11-215 | Piperidin -1-yl | Me | Cl | SO₂Me | ¹H-NMR, DMSO-d₆, 400 MHz |
| | | | | SO₂Me | 11,10 (s, 1H), 8,06 (d, 1H), 7,84 (d, 1H), 3,42 (s,3H), 3,31 (broad, 4H) |
| 11-216 | Piperidin -1-yl | Me | S(O)Me | CF₃ | ¹H-NMR, DMSO-d₆, 400 MHz |
| | | | | | 11,15 (s, 1H), 7,88 (d, 1H), 7,79 (d, 1H), 3,29 (m, 4H), 3,05 (s, 3H).2,86 (s, 3H), 1,60 (broad, 6H). |
| 11-217 | Piperidin -1-yl | Cl | Cl | SO₂Me | ¹H-NMR, DMSO-d₆, 400 MHz |
| | | | | | 11,32 (s, 1H), 8,15 (d, 1H), 7,87 (d, 1H), 3,48 (s,3H), 3,29 (m, 4H), 1,59 (broad, 6H). |
| 11-218 | NO₂ | Me | S(O)Me | CF₃ | ¹H-NMR, DMSO-d₆, 400 MHz |
| | | | | | 12,29 (s, 1H), 7,91 (d, 1H), 7,82 (d, 1H), 3,06 (s,3H), 2,87 (s, 3H) |
| 11-219 | NH2 | Cl | (Tetrahydrofuran-2-yl-methoxy)methyl | SO₂Me | ¹H-NMR, DMSO-d₆, 400 MHz |
| | | | | | 11,31 (s, 1H), 8,12 (d, 1H), 8,00 (d, 1H), 6,12 (bs, 2H), 5,08 (m, 2H), 3,99 (m, 1H), 3,70 (q, 2H), 3,68-3,52 (m, 3H), 3,39 (s, 3H), 1,97-1,70 (m, 3H), 1,53 (m, 1H). |
| 11-220 | NH2 | Me | 1, 2, 3-Triazol-1-yl | SO₂Me | ¹H-NMR, DMSO-d₆, 400 MHz |
| | | | | | 12,24 (s, 1H), 8,55 (s, 1 H), 8,18 (d, 1H), 8,16 (d, 1H), 6,16 (bs, 2H), 3,14 (s, 3H), 1,90 (s, 3H), |
| 11-221 | CO₂Me | F | S(O)Me | CF₃ | |
| 11-222 | CO₂Me | Cl | (Tetrahydrofuran-2-yl-methoxy)methyl | SO₂Me | ¹H-NMR, DMSO-d₆, 400 MHz |
| | | | | | 12,20 (s, 1H), 8,13 (d, 1H), 7,87 |
| | | | | | (d, 1H), 5,10 (s, 2H), 3,98 (m, 1H), 3,92 (s,3H), 3,72 (q, 2H), 3,65-3,53 (m, 3H), 3,39 (s, 3H), 1,96-1,72 (m, 3H), 1,55 (m, 1H). |
| 11-223 | CO₂Me | Me | Cl | SO₂Me | ¹H-NMR, DMSO-d₆, 400 MHz |
| | | | | | 12,09 (s, 1H), 8,07 (d, 1H), 7,75 (d, 1H), 3,94 (s,3H), 3,44 (s, 3H), 2,49 (s, 3H) |
| 11-224 | CO₂Me | Me | S(O)Me | CF₃ | ¹H-NMR, DMSO-d₆, 400 MHz |
| | | | | | 12,09 (s, 1H), 7,90 (d, 1H), 7,83 (d, 1H), 3,95 (s,3H), 3,06 (s, 3H), 2,87 (s, 3H) |
| 11-225 | CO₂Me | Cl | 1H-pyrazol-1-yl | SO₂Me | ¹H-NMR, DMSO-d₆, 400 MHz |
| | | | | | 12,25 (s, 1H), 8,24 (d, 1H), 8,08 (d, 1H), 8,04 (d, 1H), 7,88 (d, 1 H), 6,60 (t, 1 H), 3,93(s,3H), 3,17 (s, 3H). |
| 11-226 | CO₂Me | Me | F | SO₂Me | ¹H-NMR, DMSO-d₆, 400 MHz |
| | | | | | 12,08 (bs, 1H), 7,86 (t, 1H), 7,64 (d, 1H), 3,93 (s,3H), 3,31 (s, 3H), 2,29 (d, 3H). |
| 11-227 | CO₂Me | Me | 1,2,4-Triazol-1-yl | SO₂Me | ¹H-NMR, DMSO-d₆, 400 MHz |
| | | | | | 12,20 (s, 1H), 8,55 (s, 1H), 8,20 (d, 1H), 8,07 (d, 1H), 8,05 (s, 1H), 3,92 (s,3H), 3,15 (s, 3H), 1,93 (s, 3H) |
| 11-228 | CO₂Me | Cl | Cl | SO₂Me | ¹H-NMR, DMSO-d₆, 400 MHz |
| | | | | | 12,17 (s, 1H), 8,18 (d, 1H), 7,87 (d, 1H), 3,94 (s,3H), 3,48 (s, 3H). |
| 11-229 | CO₂Me | Me | Me | SMe | ¹H-NMR, DMSO-d₆, 400 MHz |
| | | | | | 11,62 (s, 1H), 7,39 (d, 1H), 7,20 (d, 1H), 3,90 (s,3H), 2,32 (s, 3H), 2,25 (s, 3H). |
| 11-230 | CO₂Me | Cl | Me | Cl | ¹H-NMR, DMSO-d₆, 400 MHz |
| | | | | | 11,96 (s, 1H), 7,64 (d, 1H), 7,48 (d, 1 H), 3,92 (s,3H), 2,49 (s, 3H) |
| 11-231 | CO₂Me | Cl | F | Me | ¹H-NMR, DMSO-d₆, 400 MHz |
| | | | | | 11,89 (s, 1H), 7,62 (t, 1 H), 7,45 (d, 1H), 3,92 (s,3H), 2,36 (d, 3H). |
| 11-232 | CO₂Me | Cl | SMe | SO₂Me | ¹H-NMR, DMSO-d₆, 400 MHz |
| | | | | | 12,14 (s, 1H), 8,16 (d, 1H), 7,87 (d, 1H), 3,94 (s,3H), 3,58 (s, 3H), 3,31 (s, 3H) |
| 11-233 | CO₂Et | Me | SO₂Me | CF₃ | ¹H-NMR, DMSO-d₆, 400 MHz |
| | | | | | 12,14 (s, 1H), 8,07 (d, 1 H), 7,96 1H), 4,42 (q, 2H), 3,43 (s, |
| | | | | | 3H), 3,75 (s, 3H), 1,34 (t, 3H). |
| 11-234 | CO₂H | Me | SO₂Me | CF₃ | ¹H-NMR, DMSO-d₆, 400 MHz |
| | | | | | 11,87 (s, 1H), 8,06 (d, 1H), 7,96 (d, 1H), 3,43 (s, 3H), 2,75 (s, 3H) |
| 11-235 | Piperidin -1-yl-carbonyl | Cl | 5-Cyanomethyl-4,5-dihydro-1,2-oxazol-3 yl | SO₂Et | ¹H-NMR, DMSO-d₆, 400 MHz |
| | | | | | 12,31 (s, 1H), 8,13 (d, 1H), 7,98 (d, 1H), 5,10 (m, 1H), 3,61 (dd, 1H), 3,44 (q, 2H), 3,05 (dd, 1H), 1,16 (t, 3H) |
| 11-236 | Piperidin -1-yl-carbonyl | Cl | CH₂OCH₂CF₃ | SO₂Me | ¹H-NMR, DMSO-d₆. 400 MHz |
| | | | | | 12,31 (s, 1H), 8,13 (d, 1H), 7,84 (d, 1H), 5,22 (s, 2H), 4,29 (q, 2H), 3,59 (m, 2H), 3,40 (m, 2H), 3,37 (s, 3H), 1,6 (m, 6H) |
| 11-237 | Piperidin -1-yl-carbonyl | Me | SO₂Me | CF₃ | ¹H-NMR, DMSO-d₆, 400 MHz |
| | | | | | 12,24 (s, 1H), 8,04 (d, 1H), 7,93 (d, 1H), 3,60 (m, 2H), 3,48 (m, 2H), 3,43 (s, 3H), 2,72 (s, 3H), 1,59 (m, 6H) |
| 11-238 | Piperidin -1-yl-carbonyl | Me | Cl | SO₂Me | ¹H-NMR, DMSO-d₆, 400 MHz |
| | | | | | 12,21 (s, 1H), 8,05 (d, 1H), 7,71 (d, 1H), 3,60 (m, 2H), 3,46 (m, 2H), 3,43 (s,3H), 1,68-1,52 (m, 6H) |
| 11-239 | SEt | Me | SO₂Me | CF₃ | ¹H-NMR, DMSO-d₆, 400 MHz |
| | | | | | 11,75 (bs, 1H), 7,99 (d, 1H), 7,89 (d, 1H), 3,42 (s, 3H), 3,15 (q, 2H), 2,76 (s, 3H), 1,37(t, 3H) |
| 11-240 | SPh | Me | SO₂Me | CF₃ | ¹H-NMR, DMSO-d₆, 400 MHz |
| | | | | | 11,90 (s, 1H), 8,03 (d, 1H), 7,88 (d, 1H), 7,62 (m, 2H), 7,46 (m, 3H), 3,40 (s, 3H), 2,73 (s, 3H) |
| 11-241 | SO₂Me | Me | SO₂Me | CF₃ | ¹H-NMR, DMSO-d₆, 400 MHz |
| | | | | | 12,00 (s, 1H), 8,08 (d, 1H), 7,95 (d, 1H), 3,69 (s, 3H), 3,43 (s, 3H), 2,77 (s, 3H) |
| 11-242 | Br | Me | Me | SO₂Me | ¹H-NMR, DMSO-d₆, 400 MHz |
| | | | | | 11,61 (s, 1H), 7,93 (d, 1H), 7,58 (d, 1H), 3,28 (s, 3H), 2,65 (s, 3H), 2,39 (s, 3H) |
| 11-243 | S(O)Me | Me | SO₂Me | CF₃ | |
| 11-244 | S(O)Me | Me | Me | SO₂Me | |
| 11-245 | c-Pr | Me | S(O)Me | CF₃ | ¹H-NMR, DMSO-d₆, 400 MHz |
| | | | | | 11,50 (bs, 1H), 7,89 (s, 2H), 3,06 (s, 3H), 2,88 (s, 3H), 2,08 (m, 1H), 1,12 (m, 2H), 0,95 (m, 2H) |
| 11-246 | c-Pr | Cl | (Tetrahydrofuran-2-yl-methoxy)methyl | SO₂Me | ¹H-NMR, DMSO-d₆, 400 MHz |
| | | | | | 11,63 (s, 1H), 8,11 (d, 1H), 7,94 (d, 1H), 5,11 (m, 2H), 3,98 (m, 1H), 3,71 (dd, 1H), 3,60 (m, 2H), 3,39 (s, 3H), 2,08 (m, 1H), 1,95 - 1,70 (m, 3H), 1,55 (m, 1 H), 1,12 (mt, 2H), 0,96 (m, 2H) |
| 11-247 | c-Pr | Cl | Cl | SO₂Me | ¹H-NMR, DMSO-d₆, 400 MHz |
| | | | | | 11,65 (s, 1H), 8,16 (d, 1H), 7,95 (d, 1H), 3,48 (s, 3H), 2,07 (m, 1H), 1,13 (m, 2H), 0,95 (m, 2H) |
| 11-248 | c-Pr | Me | Me | SO₂Me | ¹H-NMR, DMSO-d₆, 400 MHz |
| | | | | | 11,38 (s, 1H), 7,92 (d, 1H), 7,61 (d, 1H), 3,28 (s, 3H), 2,65 (s, 3H), 2,38 (s, 3H), 2,05 (m, 1H), 1,13 (m, 2H), 0,96 (m, 2H) |
| 11-249 | Bz | Me | SMe | CF₃ | |
| 11-250 | Bz | Me | S(O)Me | CF₃ | |
| 11-251 | Bz | Me | SO₂Me | CF₃ | |
| 11-252 | Bz | Me | SMe | SO₂CH₃ | |
| 11-253 | Bz | Me | S(O)Me | SO₂CH₃ | |
| 11-254 | Bz | Me | SO₂Me | SO₂CH₃ | |
| 11-255 | Bz | F | SMe | CF₃ | |
| 11-256 | Bz | F | S(O)Me | CF₃ | |
| 11-257 | Bz | F | SO₂Me | CF₃ | |

B. Formulierungsbeispiele
a) Ein Stäubemittel wird erhalten, indem man 10 Gew.-Teile einer Verbindung der Formel (I) und/oder deren Salze und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I) und/oder deren Salze, 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gew.-Teile einer Verbindung der Formel (I) und/oder deren Salze mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I) und/oder deren Salze, 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
   75 Gew.-Teile einer Verbindung der Formel (I) und/oder deren Salze,
   10 Gew.-Teile ligninsulfonsaures Calcium,
   5 Gew.-Teile Natriumlaurylsulfat,
   3 Gew.-Teile Polyvinylalkohol und
   7 Gew.-Teile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gew.-Teile einer Verbindung der Formel (I) und/oder deren Salze,
   5 Gew.-Teile 2,2'-dinaphthylmethan-6,6'-disulfonsaures Natrium
   2 Gew.-Teile oleoylmethyltaurinsaures Natrium,
   1 Gew.-Teil Polyvinylalkohol,
   17 Gew.-Teile Calciumcarbonat und
   50 Gew.-Teile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Herbizide Wirkung gegen Schadpflanzen im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 I/ha unter Zusatz von 0,2% Netzmittel auf die Oberfläche der Abdeckerde appliziert. Nach der Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen). Dabei zeigen beispielsweise die Verbindungen Nr. 1-119, 1-53, 1-148, 1-54, 1-12, 1-71, 2-56, 9-180 sowie 9-174 bei einer Aufwandmenge von 80 g/ha jeweils eine mindestens 80 %-ige Wirkung gegen Echinochloa crus galli und Veronica persica auf. Die Verbindungen Nr. 1-51, 1-123, 1-148, 1-149, 1-54, 2-119 sowie 2-71 zeigen bei einer Aufwandmenge von 80 g/ha jeweils eine mindestens 80 %-ige Wirkung gegen Abutilon theophrasti und Amaranthus retroflexus. Die Verbindungen Nr. 6-120, 3-120 sowie 6-3 zeigen bei einer Aufwandmenge von 80 g/ha jeweils eine mindestens 80 %-ige Wirkung gegen Matricaria inodora und Veronica persica.

### 2. Herbizide Wirkung gegen Schadpflanzen im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 I/ha unter Zusatz von 0,2% Netzmittel auf die grünen Pflanzenteile gesprüht.

Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen). Dabei zeigen beispielsweise die Verbindungen Nr. 1-119, 1-51, 1-53, 1-123, 1-148, 1-54, 2-119, 1-127, 9-180, 9-174, 3-119, 6-120, 4-120, 3-120 sowie 9-200 bei einer Aufwandmenge von 80 g/ha jeweils eine mindestens 80 %-ige Wirkung gegen Amaranthus retroflexus und Viola tricolor. Die Verbindungen Nr. 1-147, 1-71, 2-71, sowie 6-3 zeigen bei einer Aufwandmenge von 80 g/ha jeweils eine mindestens 80 %-ige Wirkung gegen Abutilon theophrasti und Veronica persica.

## Patentansprüche

1. Verbindungen der Formel (I) oder deren Salze 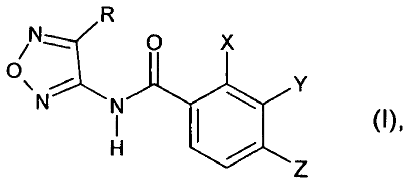 worin
R bedeutet Wasserstoff, (C₁-C₆)-Alkyl, (C₃-C₇)-cyclo-Alkyl, Halogen-(C₁-C₆)-alkyl, (C₁-C₆)-Alkoxy, Halogen-(C₁-C₆)-alkoxy, (C₂-C₆)-Alkenyl, (C₂-C₆)-Alkenyloxy, (C₂-C₆)-Halogenalkenyl, (C₂-C₆)-Alkinyl, (C₂-C₆)-Alkinyloxy, (C₂-C₆)-Halogenalkinyl, Cyano, Nitro, Methylsulfenyl, Methylsulfinyl, Methylsulfonyl, Acetylamino, Benzoylamino, Methoxycarbonyl, Ethoxycarbonyl, Methoxycarbonylmethyl, Ethoxycarbonylmethyl, Benzoyl, Methylcarbonyl, Piperidinylcarbonyl, Trifluormethylcarbonyl, Halogen, Amino, Aminocarbonyl, Methylaminocarbonyl, Dimethylaminocarbonyl, Methoxymethyl, oder
jeweils durch s Reste aus der Gruppe Methyl, Ethyl, Methoxy, Trifluormethyl und Halogen substituiertes Heteroaryl, Heterocyclyl oder Phenyl,
X und Z bedeuten unabhängig voneinander jeweils Nitro, Halogen, Cyano, Formyl, Rhodano, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₃-C₆)-Halogenalkinyl, (C₃-C₆)-Cycloalkyl, (C₃-C₆)-Halogencycloalkyl, (C₃-C₆)-Cycloalkyl-(C₁-C₆)-alkyl, (C₃-C₆)-Halogencycloalkyl-(C₁-C₆)-alkyl, COR¹, OR¹, OCOR¹, OSO₂R², S(O)ₙR², SO₂=OR¹, SO₂N(R¹)₂, NR¹SO₂R² , NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-COOR¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², NR₁R₂, P(O)(OR⁵)₂, oder
jeweils durch s Reste aus der Gruppe Methyl, Ethyl, Methoxy, Nitro, Trifluormethyl und Halogen substituiertes Heteroaryl, Heterocyclyl oder Phenyl,
Y bedeutet Nitro, Halogen, Cyano, Rhodano, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₃-C₆)-Halogenalkinyl, (C₃-C₆)-Cycloalkyl, (C₃-C₆)-Halogencycloalkyl, (C₃-C₆)-Cycloalkyl-(C₁-C₆)-alkyl, (C₃-C₆)-Halogencycloalkyl-(C₁-C₆)-alkyl, COR¹,CO₂R¹, OR¹, OCOR¹, OSO₂R², S(O)ₙR², SO₂OR¹, SO₂N(R¹)₂, NR¹SO₂R², NR¹COR¹, (C₁-C₆)-Alkyl-Heteroaryl, O-(C₁-C₆)-Alkyl-Heterocyclyl, O-(C₁-C₆)-Alkyl-Heteroaryl, (C₁-C₆)-Alkyl-Heterocyclyl, (C₁-C₆)-Alkyl-S(O)ₙR², (C₁-C₆)-Alkyl-OR¹, (C₁-C₆)-Alkyl-OCOR¹,(C₁-C₆)-Alkyl-OSO₂R², (C₁-C₆)-Alkyl-COOR¹, (C₁-C₆)-Alkyl-CN, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², NR¹R², P(O)(OR⁵)₂, Tetrahydrofuranyloxymethyl, Tetrahydrofuranylmethoxymethyl, O(CH₂)-3,5-dimethyl-1,2-oxazol-4-yl, O(CH₂)₂-O(3,5-dimethoxypyrimidin-2-yl, O(CH₂)-5-pyrrolidin-2-on, O(CH₂)-5-2,4-dimethyl-2,4-dihydro-3H-1,2,4-triazol-3-on, oder
jeweils durch s Reste aus der Gruppe Methyl, Ethyl, Methoxy, Halogen und Cyanomethyl substituiertes Heteroaryl oder Heterocyclyl,
R¹ bedeutet Wasserstoff, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl oder Phenyl-(C₁-C₆)-alkyl, wobei die 12 letztgenannten Reste durch s Reste aus der Gruppe bestehend aus Cyano, Halogen, Nitro, Rhodano, OR³, S(O)NR⁴, N(R³)₂, NR³OR³, COR³, OCOR³, SCOR³, NR³COR³, CO₂R³, COSR³, CON(R³)₂ und (C₁-C₄)-Alkoxy-(C₂-C₆)-alkoxycarbonyl substituiert sind,
R² bedeutet (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, Phenyl oder Phenyl-(C₁-C₆)-alkyl, wobei die sieben letztgenannten Reste durch s Reste aus der Gruppe bestehend aus Cyano, Halogen, Nitro, Rhodano, OR³, S(O)ₙR⁴, N(R³)₂, NR³OR³, COR³, OCOR³, SCOR³, NR³COR³, CO₂R³, COSR³, CON(R³)₂ und (C₁-C₄)-Alkoxy-(C₂-C₆)-alkoxycarbonyl substituiert sind,
R³ bedeutet Wasserstoff, (C₁-C₆)-Alkyl, (C₂-C₆)-Alkenyl oder (C₂-C₆)-Alkinyl,
R⁴ bedeutet (C₁-C₆)-Alkyl, (C₂-C₆)-Alkenyl oder (C₂-C₆)-Alkinyl,
R⁵ bedeutet Methyl oder Ethyl,
n bedeutet 0, 1 oder 2,
s bedeutet 0, 1, 2 oder 3.

2. Verbindungen nach Anspruch 1, worin
R bedeutet Wasserstoff, (C₁-C₆)-Alkyl, (C₃-C₇)-cyclo-Alkyl, Halogen-(C₁-C₆)-alkyl, (C₁-C₆)-Alkoxy, Halogen-(C₁-C₆)-alkoxy, Cyano, Nitro, Methylsulfenyl, Methylsulfinyl, Methylsulfonyl, Acetylamino, Benzoylamino, Methoxycarbonyl, Ethoxycarbonyl, Benzoyl, Methylcarbonyl, Piperidinylcarbonyl, Trifluormethylcarbonyl, Halogen, Amino, Aminocarbonyl, Methylaminocarbonyl, Dimethylaminocarbonyl, Methoxymethyl,
einen durch s Reste aus der Gruppe Methyl, Methoxy und Halogen substituierten Heterocyclus aus der Gruppe umfassend Pyridin-2-yl, Pyridin-3-yl, Pyridin-4-yl, Piperidin-2-yl, Piperidin-3-yl, Piperidin-4-yl, Benzisoxazol-2-yl, 1,2,4-Oxadiazol-3-yl, 1,2,4-Triazol-3-yl, 1-Ethylbenzimidazol-2-yl, 4-Methylthiazol-2-yl, Thiophen-2-yl, Furan-2-yl, Furan-3-yl, Tetrahydrofuran-2-yl, Tetrahydrofuran-3-yl, Isoxazol-2-yl, Isoxazol-3-yl, Oxazol-2-yl, Oxazol-3-yl, Pyrrol-2-yl, Pyrrol-3-yl, Imidazol-2-yl, Imidazol-5-yl, Imidazol-4-yl, Pyrazol-3-yl, Pyrazol-5-yl, Pyrazol-4-yl, Isoxazol-3-yl, Isoxazol-4-yl, Isoxazol-5-yl, Oxazol-2-yl, Oxazol-4-yl, Oxazol-5-yl, Isothiazol-3-yl, Isothiazol-4-yl, Isothiazol-5-yl, Thiazol-2-yl, Thiazol-4-yl, Thiazol-5-yl 1,2,3-Triazol-4-yl, 1,2,3-Triazol-5-yl, 1,2,5-Triazol-3-yl, 1,3,4-Triazol-2-yl, 1,2,4-Triazol-3-yl, 1,2,4-Triazol-5-yl, 1,2,4-Oxadiazol-3-yl, 1,2,4-Oxadiazol-5-yl, 1,3,4-Oxadiazol-2-yl, 1,2,3-Oxadiazol-4-yl, 1,2,3-Oxadiazol-5-yl, 1,2,5-Oxadiazol-3-yl, 1,2,4-Thiadiazol-3-yl, 1,2,4-Thiadiazol-5-yl, 1,3,4-Thiadiazol-2-yl, 1,2,3-Thiadiazol-4-yl, 1,2,3-Thiadiazol-5-yl, 1,2,5-Thiadiazol-3-yl, 2H-1,2,3,4-Tetrazol-5-yl, 1 H-1,2,3,4-Tetrazol-1-yl, 1,2,3,4-Oxatriazol-5-yl, 1,2,3,5-Oxatriazol-4-yl, 1,2,3,4-Thiatriazol-5-yl, 1,2,3,5-Thiatriazol-4-yl, Pyrazin-2-yl, Pyrazin-3-yl, Pyrimidin-2-yl, Pyrimidin-4-yl, Pyrimidin-5-yl, Pyridazin-3-yl und Pyridazin-4-yl, oder
durch s Reste aus der Gruppe Methyl, Methoxy, Trifluormethyl und Halogen substituiertes Phenyl,
X und Z bedeuten unabhängig voneinander jeweils Nitro, Halogen, Cyano, Rhodano, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₃-C₆)-Halogenalkinyl, (C₃-C₆)-Cycloalkyl, (C₃-C₆)-Halogencycloalkyl, (C₃-C₆)-Cycloalkyl-(C₁-C₆)-alkyl, (C₃-C₆)-Halogencycloalkyl-(C₁-C₆)-alkyl, COR¹, OR¹, OCOR¹,OSO₂R , S(O)ₙR², SO₂OR¹, SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-COOR¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², Benzoxazol-2-yl, 1-Ethyl-benzimidazol-2-yl, Piperidin-1-yl oder 1,2,4-Triazol-1-yl,
Y bedeutet Nitro, Halogen, Cyano, Rhodano, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₃-C₆)-Halogenalkinyl, (C₃-C₆)-Cycloalkyl, (C₃-C₆)-Halogencycloalkyl, (C₃-C₆)-Cycloalkyl-(C₁-C₆)-alkyl, (C₃-C₆)-Halogencycloalkyl-(C₁-C₆)-alkyl, COR¹, OR¹, OCOR¹,OSO₂R ² S(O)ₙR², SO₂OR¹ SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-COOR¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², Tetrahydrofuranyl-oxymethyl, Tetrahydrofuranylmethoxymethyl, O(CH₂)-3,5-dimethyl-1,2-oxazol-4-yl, O(CH₂)₂-O(3,5-dimethoxypyrimidin-2-yl, O(CH₂)-5-pyrrolidin-2-on oder O(CH₂)-5-2,4-dimethyl-2,4-dihydro-3H-1,2,4-triazol-3-on,
R¹ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, Phenyl oder Phenyl-(C₁-C₆)-alkyl, wobei die sieben letztgenannten Reste durch s Reste aus der Gruppe bestehend aus Cyano, Halogen, Nitro, Rhodano, OR³, S(O)ₙR⁴, N(R³)₂, NR³R³, COR³, OCOR³, SCOR³, NR³COR³, CO₂R³, COSR³, CON(R³)₂ und (C₁-C₄)-Alkoxy-(C₂-C₆)-alkoxycarbonyl substituiert sind,
R² bedeutet (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, Phenyl oder Phenyl-(C₁-C₆)-alkyl, wobei die sieben letztgenannten Reste durch s Reste aus der Gruppe bestehend aus Cyano, Halogen, Nitro, Rhodano, OR³, S(O)ₙR⁴. N(R³)₂, NR³R³, COR³, OCOR³, SCOR³, NR³COR³, CO₂R³, COSR³, CON(R³)₂ und (C₁-C₄)-Alkoxy-(C₂-C₆)-alkoxycarbonyl substituiert sind,
R³ bedeutet Wasserstoff, (C₁-C₆)-Alkyl, (C₂-C₆)-Alkenyl oder (C₂-C₆)-Alkinyl,
R⁴ bedeutet (C₁-C₆)-Alkyl, (C₂-C₆)-Alkenyl oder (C₂-C₆)-Alkinyl,
n bedeutet 0, 1 oder 2,
s bedeutet 0, 1, 2 oder 3.

3. Verbindungen nach Anspruch 1 oder 2, worin
R bedeutet Wasserstoff, (C₁-C₆)-Alkyl, (C₃-C₇)-cyclo-Alkyl, Halogen-(C₁-C₆)-alkyl, (C₁-C₆)-Alkoxy, Halogen-(C₁-C₆)-alkoxy, Cyano, Nitro, Methylsulfenyl, Methylsulfinyl, Methylsulfonyl, Acetylamino, Benzoylamino, Methoxycarbonyl, Ethoxycarbonyl, Benzoyl, Methylcarbonyl, Piperidinylcarbonyl, Trifluormethylcarbonyl, Halogen, Amino, Aminocarbonyl, Methylaminocarbonyl, Dimethylaminocarbonyl, Methoxymethyl,
X und Z bedeuten unabhängig voneinander jeweils Nitro, Halogen, Cyano, (C₁-C₆)-Alkyl, (C₁-C₆)-Halogenalkyl, (C₃-C₆)-Cycloalkyl, OR¹, S(O)ₙR², (C₁-C₆)-Alkyl-S(O)ₙR², (C₁-C₆)-Alkyl-OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R² oder 1,2,4-Triazol-1-yl,
Y bedeutet Nitro, Halogen, Cyano, (C₁-C₆)-Alkyl, (C₁-C₆)-Halogenalkyl, OR¹, S(O)ₙR², SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², Tetrahydrofuranyl-oxymethyl, Tetrahydrofuranylmethoxymethyl, O(CH₂)-3,5-dimethyl-1,2-oxazol-4-yl, O(CH₂)₂-O(3,5-dimethoxypyrimidin-2-yl, O(CH₂)-5-pyrrolidin-2-on oder O(CH₂)-5-2,4-dimethyl-2,4-dihydro-3H-1,2,4-triazol-3-on,
R¹ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, Phenyl oder Phenyl-(C₁-C₆)-alkyl, wobei die sieben letztgenannten Reste durch s Reste aus der Gruppe bestehend aus Halogen und OR³ substituiert sind,
R² bedeutet (C₁-C₆)-Alkyl, (C₃-C₆)-Cycloalkyl oder (C₃-C₆)-Cycloalkyl-(C₁-C₆)-alkyl, wobei die drei letztgenannten Reste durch s Reste aus der Gruppe bestehend aus Halogen und OR³ substituiert sind,
R³ bedeutet Wasserstoff oder (C₁-C₆)-Alkyl,
n bedeutet 0, 1 oder 2,
s bedeutet 0, 1, 2 oder 3.

4. Verbindungen nach Anspruch 1 oder 2, worin
R bedeutet Wasserstoff, (C₁-C₆)-Alkyl, (C₃-C₇)-cyclo-Alkyl, Halogen-(C₁-C₆)-alkyl, (C₁-C₆)-Alkoxy, Halogen-(C₁-C₆)-alkoxy, Cyano, Nitro, Methylsulfenyl, Methylsulfinyl, Methylsulfonyl, Acetylamino, Benzoylamino, Methoxycarbonyl, Ethoxycarbonyl, Benzoyl, Methylcarbonyl, Piperidinylcarbonyl, Trifluormethylcarbonyl, Halogen, Amino, Aminocarbonyl, Methylaminocarbonyl, Dimethylaminocarbonyl, Methoxymethyl,
X und Z bedeuten unabhängig voneinander jeweils Nitro, Halogen, Cyano, (C₁-C₆)-Alkyl, (C₁-C₆)-Halogenalkyl, (C₃-C₆)-Cycloalkyl, OR¹, S(O)ₙR², (C₁-C₆)-Alkyl-S(O)ₙR², (C₁-C₆)-Alkyl-OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R² oder 1,2,4-Triazol-1-yl,
Y bedeutet S(O)ₙR², 4,5-Dihydro-1,2-oxazol-3-yl, 5-Cyanomethyl-4,5-dihydro-1,2-oxazol-3-yl oder 5-Methoxymethyl-4,5-dihydro-1,2-oxazol-3-yl,
R¹ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, Phenyl oder Phenyl-(C₁-C₆)-alkyl, wobei die sieben letztgenannten Reste durch s Reste aus der Gruppe bestehend aus Halogen und OR³ substituiert sind,
R² bedeutet (C₁-C₆)-Alkyl, (C₃-C₆)-Cycloalkyl oder (C₃-C₆)-Cycloalkyl-(C₁-C₆)-alkyl, wobei die drei letztgenannten Reste durch s Reste aus der Gruppe bestehend aus Halogen und OR³ substituiert sind,
R³ bedeutet Wasserstoff oder (C₁-C₆)-Alkyl,
n bedeutet 0, 1 oder 2,
s bedeutet 0, 1, 2 oder 3.

5. Herbizide Mittel, **gekennzeichnet durch** einen herbizid wirksamen Gehalt an mindestens einer Verbindung der Formel (I) gemäß einem der Ansprüche 1 bis 4.

6. Herbizide Mittel nach Anspruch 5 in Mischung mit Formulierungshilfsmitteln.

7. Herbizide Mittel nach Anspruch 5 oder 6 enthaltend mindestens einen weiteren pestizid wirksame Stoffen aus der Gruppe Insektizide, Akarizide, Herbizide, Fungizide, Safenern und Wachstumsregulatoren.

8. Herbizide Mittel nach Anspruch 7 enthaltend einen Safener.

9. Herbizide Mittel nach Anspruch 7 oder 8 enthaltend ein weiteres Herbizid.

10. Verfahren zur Bekämpfung unerwünschter Pflanzen, **dadurch gekennzeichnet, daß** man eine wirksame Menge mindestens einer Verbindung der Formel (I) gemäß einem der Ansprüche 1 bis 4 oder eines herbiziden Mittels nach einem der Ansprüche 5 bis 9 auf die Pflanzen oder auf den Ort des unerwünschten Pflanzenwachstums appliziert.

11. Verwendung von Verbindungen der Formel (I) gemäß einem der Ansprüche 1 bis 4 oder von herbiziden Mitteln nach einem der Ansprüche 5 bis 9 zur Bekämpfung unerwünschter Pflanzen.

12. Verwendung nach Anspruch 19, **dadurch gekennzeichnet, daß** die Verbindungen der Formel (I) zur Bekämpfung unerwünschter Pflanzen in Kulturen von Nutzpflanzen eingesetzt werden.

13. Verwendung nach Anspruch 12, **dadurch gekennzeichnet, daß** die Nutzpflanzen transgene Nutzpflanzen sind.

## Claims

1. A compound of the formula (I) or a salt thereof 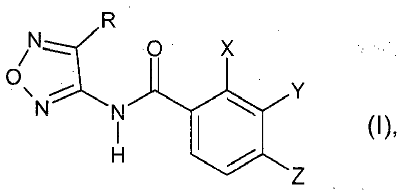 in which
R is hydrogen, (C₁-C₆)-alkyl, (C₃-C₇)-cycloalkyl, halo-(C₁-C₆)-alkyl, (C₁-C₆)-alkoxy, halo-(C₁-C₆)-alkoxy, (C₂-C₆)-alkenyl, (C₂-C₆)-alkenyloxy, (C₂-C₆)-haloalkenyl, (C₂-C₆) -alkynyl, (C₂-C₆)-alkynyloxy, (C₂-C₆)-haloalkynyl, cyano, nitro, methylsulfenyl, methylsulfinyl, methylsulfonyl, acetylamino, benzoylamino, methoxycarbonyl, ethoxycarbonyl, methoxycarbonylmethyl, ethoxycarbonylmethyl, benzoyl, methylcarbonyl, piperidinylcarbonyl, trifluoromethylcarbonyl, halogen, amino, aminocarbonyl, methylaminocarbonyl, dimethylaminocarbonyl, methoxymethyl or
heteroaryl, heterocyclyl or phenyl, each of which is substituted by s radicals selected from the group consisting of methyl, ethyl, methoxy, trifluoromethyl and halogen,
X and Z independently of one another are in each case nitro, halogen, cyano, formyl, rhodano, (C₁-C₆)-alkyl, (C₁-C₆)-haloalkyl, (C₂-C₆) -alkenyl, (C₂-C₆)-haloalkenyl, (C₂-C₆)-alkynyl, (C₃-C₆)-haloalkynyl, (C₃-C₆)-cycloalkyl, (C₃-C₆)-halocycloalkyl, (C₃-C₆)-cycloalkyl-(C₁-C₆)-alkyl, (C₃-C₆)-halocycloalkyl-(C₁-C₆)-alkyl, COR¹, OR¹, OCOR¹, OSO₂R², S(O)ₙR², SO₂OR¹, SO₂N(R¹)₂, NR¹SO₂R², NR¹COR¹, (C₁-C₆)-alkyl-S(O)ₙR², (C₁-C₆)-alkyl-OR¹, (C₁-C₆)-alkyl-OCOR¹, (C₁-C₆)-alkyl-OSO₂R², (C₁-C₆)-alkyl-COOR¹, (C₁-C₆)-alkyl-SO₂OR¹, (C₁-C₆)-alkyl-CON(R¹)₂, (C₁-C₆)-alkyl-SO₂N(R¹)₂, (C₁-C₆)-alkyl-NR¹COR¹, (C₁-C₆)-alkyl-NR¹SO₂R², NR₁R₂, P(O) (OR⁵)₂, or
heteroaryl, heterocyclyl or phenyl, each of which is substituted by s radicals selected from the group consisting of methyl, ethyl, methoxy, nitro, trifluoromethyl and halogen,
Y is nitro, halogen, cyano, rhodano, (C₁-C₆)-alkyl, (C₁-C₆)-haloalkyl, (C₂-C₆)-alkenyl, (C₂-C₆) -haloalkenyl, (C₂-C₆) -alkynyl, (C₃-C₆)-haloalkynyl, (C₃-C₆)-cycloalkyl, (C₃-C₆) -halocycloalkyl, (C₃-C₆) -cycloalkyl- (C₁-C₆)-alkyl, (C₃-C₆) -halocycloalkyl- (C₁-C₆) -alkyl, COR¹, CO₂R¹, OR¹, OCOR¹, OSO₂R², S(O)ₙR², SO₂OR¹, SO₂N(R¹)₂, NR¹SO₂R², NR¹COR¹,
(C₁-C₆)-alkyl-heteroaryl, O-(C₁-C₆)-alkyl-heterocyclyl, O-(C₁-C₆)-alkyl-heteroaryl, (C₁-C₆)-alkyl-heterocyclyl, (C₁-C₆)-alkyl-S (O)ₙR², (C₁-C₆)-alkyl-OR¹, (C₁-C₆)-alkyl-OCOR¹, (C₁-C₆)-alkyl-OSO₂R², (C₁-C₆)-alkyl-COOR¹, (C₁-C₆)-alkyl-CN, (C₁-C₆)-alkyl-SO₂OR¹, (C₁-C₆) -alkyl-CON (R¹)₂, (C₁-C₆)-alkyl-SO₂N(R¹)₂, (C₁-C₆)-alkyl-NR¹COR¹, (C₁-C₆)-alkyl-NR¹SO₂R², NR¹R², P(O) (OR⁵)₂, tetrahydrofuranyloxymethyl, tetrahydrofuranylmethoxymethyl, O(CH₂)-3,5-dimethyl-1,2-oxazol-4-yl, O(CH₂)₂-O(3,5-dimethoxypyrimidin-2-yl, O(CH₂)-5-pyrrolidin-2-one, O(CH₂)-5-2,4-dimethyl-2,4-dihydro-3H-1,2,4-triazol-3-one, or
heteroaryl or heterocyclyl, each of which is substituted by s radicals selected from the group consisting of methyl, ethyl, methoxy, halogen and cyanomethyl,
R¹ is hydrogen, (C₁-C₆)-alkyl, (C₁-C₆)-haloalkyl, (C₂-C₆)-alkenyl, (C₂-C₆)-haloalkenyl, (C₂-C₆)-alkynyl, (C₂-C₆)-haloalkynyl, (C₃-C₆) -cycloalkyl, (C₃-C₆) -halocycloalkyl, (C₁-C₆)-alkyl-O- (C₁-C₆)-alkyl, (C₃-C₆)-cycloalkyl-(C₁-C₆)-alkyl, phenyl or phenyl-(C₁-C₆)-alkyl, where the 12 last-mentioned radicals are substituted by s radicals selected from the group consisting of cyano, halogen, nitro, rhodano, OR³, S(O)ₙR⁴, N(R³)₂, NR³OR³, COR³, OCOR³, SCOR³, NR³COR³, CO₂R³, COSR³, CON(R³)₂ and (C₁-C₄)-alkoxy-(C₂-C₆) -alkoxycarbonyl,
R² is (C₁-C₆) -alkyl, (C₂-C₆)-alkenyl, (C₂-C₆)-alkynyl, (C₃-C₆) -cycloalkyl, (C₃-C₆)-cycloalkyl-(C₁-C₆) -alkyl, phenyl or phenyl-(C₁-C₆)-alkyl, where the seven last-mentioned radicals are substituted by s radicals from the group consisting of cyano, halogen, nitro, thiocyanato, OR³, S(O)ₙR⁴, N(R³)₂, NR³OR³, COR³, OCOR³, SCOR³, NR³COR³, CO₂R³, COSR³, CON(R³)₂ and (C₁-C₄)-alkoxy-(C₂-C₆)-alkoxycarbonyl,
R³ is hydrogen, (C₁-C₆)-alkyl, (C₂-C₆)-alkenyl or (C₂-C₆)-alkynyl,
R⁴ is (C₁-C₆)-alkyl, (C₂-C₆)-alkenyl or (C₂-C₆)-alkynyl,
R⁵ is methyl or ethyl,
n is 0, 1 or 2,
s is 0, 1, 2 or 3.

2. The compound as claimed in claim 1, wherein
R is hydrogen, (C₁-C₆)-alkyl, (C₃-C₇)-cycloalkyl, halo- (C₁-C₆)-alkyl, (C₁-C₆) -alkoxy, halo-(C₁-C₆)-alkoxy, cyano, nitro, methylsulfenyl, methylsulfinyl, methylsulfonyl, acetylamino, benzoylamino, methoxycarbonyl, ethoxycarbonyl, benzoyl, methylcarbonyl, piperidinylcarbonyl, trifluoromethylcarbonyl, halogen, amino, aminocarbonyl, methylaminocarbonyl, dimethylaminocarbonyl, methoxymethyl,
a heterocycle selected from the group consisting of pyridin-2-yl, pyridin-3-yl, pyridin-4-yl, piperidin-2-yl, piperidin-3-yl, piperidin-4-yl, benzisoxazol-2-yl, 1,2,4-oxadiazol-3-yl, 1,2,4-triazol-3-yl, 1-ethylbenzimidazol-2-yl, 4-methylthiazol-2-yl, thiophen-2-yl, furan-2-yl, furan-3-yl, tetrahydrofuran-2-yl, tetrahydrofuran-3-yl, isoxazol-2-yl, isoxazol-3-yl, oxazol-2-yl, oxazol-3-yl, pyrrol-2-yl, pyrrol-3-yl, imidazol-2-yl, imidazol-5-yl, imidazol-4-yl, pyrazol-3-yl, pyrazol-5-yl, pyrazol-4-yl, isoxazol-3-yl, isoxazol-4-yl, isoxazol-5-yl, oxazol-2-yl, oxazol-4-yl, oxazol-5-yl, isothiazol-3-yl, isothiazol-4-yl, isothiazol-5-yl, thiazol-2-yl, thiazol-4-yl, thiazol-5-yl,
1,2,3-triazol-4-yl, 1,2,3-triazol-5-yl, 1,2,5-triazol-3-yl, 1,3,4-triazol-2-yl, 1,2,4-triazol-3-yl, 1,2,4-triazol-5-yl, 1,2,4-oxadiazol-3-yl, 1,2,4-oxadiazol-5-yl, 1,3,4-oxadiazol-2-yl, 1,2,3-oxadiazol-4-yl, 1,2,3-oxadiazol-5-yl, 1,2,5-oxadiazol-3-yl, 1,2,4-thiadiazol-3-yl, 1,2,4-thiadiazol-5-yl, 1,3,4-thiadiazol-2-yl, 1,2,3-thiadiazol-4-yl, 1,2,3-thiadiazol-5-yl, 1,2,5-thiadiazol-3-yl, 2H-1,2,3,4-tetrazol-5-yl, 1H-1,2,3,4-tetrazol-1-yl, 1,2,3,4-oxatriazol-5-yl, 1,2,3,5-oxatriazol-4-yl, 1,2,3,4-thiatriazol-5-yl, 1,2,3,5-thiatriazol-4-yl, pyrazin-2-yl, pyrazin-3-yl, pyrimidin-2-yl, pyrimidin-4-yl, pyrimidin-5-yl, pyridazin-3-yl and pyridazin-4-yl, which heterocycle is substituted by s radicals selected from the group consisting of methyl, methoxy and halogen, or
phenyl which is substituted by s radicals selected from the group consisting of methyl, methoxy, trifluoromethyl and halogen,
X and Z independently of each other are in each case nitro, halogen, cyano, rhodano, (C₁-C₆)-alkyl, (C₁-C₆)-haloalkyl, (C₂-C₆)-alkenyl, (C₂-C₆)-haloalkenyl, (C₂-C₆)-alkynyl, (C₃-C₆)-haloalkynyl, (C₃-C₆)-cycloalkyl, (C₃-C₆)-halocycloalkyl, (C₃-C₆) -cycloalkyl- (C₁-C₆)-alkyl, (C₃-C₆)-halocycloalkyl-(C₁-C₆)-alkyl, COR¹, OR¹, OCOR¹, OSO₂R², S(O)ₙR², SO₂OR¹, SO₂N(R¹)₂, NR¹SO₂R², NR¹COR¹, (C₁-C₆) -alkyl-S (O)ₙR², (C₁-C₆)-alkyl-OR¹-, (C₁-C₆)-alkyl-OCOR¹, (C₁-C₆) -alkyl-OSO₂R², (C₁-C₆) -alkyl-COOR¹, (C₁-C₆)-alkyl-SO₂OR¹, (C₁-C₆)-alkyl-CON (R¹)₂, (C₁-C₆) -alkyl-SO₂N (R¹)₂, (C₁-C₆) -alkyl-NR¹COR¹, (C₁-C₆) -alkyl-NR¹SO₂R², benzoxazol-2-yl, 1-ethylbenzimidazol-2-yl, piperidin-1-yl or 1,2,4-triazol-1-yl,
Y is nitro, halogen, cyano, rhodano, (C₁-C₆)-alkyl, (C₁-C₆)-haloalkyl, (C₂-C₆)-alkenyl, (C₂-C₆)-haloalkenyl, (C₂-C₆) -alkynyl, (C₃-C₆) -haloalkynyl, (C₃-C₆) -cycloalkyl, (C₃-C₆) -halocycloalkyl, (C₃-C₆) -cycloalkyl- (C₁-C₆) -alkyl, (C₃-C₆) -halocycloalkyl- (C₁-C₆) -alkyl, COR¹, OR¹, OCOR¹, OSO₂R², S(O)ₙR², SO₂OR¹, SO₂N(R¹)₂, NR¹SO₂R², NR¹COR¹, (C₁-C₆)-alkyl-S(O)ₙR², (C₁-C₆)-alkyl-OR¹-, (C₁-C₆)-alkyl-OCOR¹, (C₁-C₆) -alkyl-OSO₂R², (C₁-C₆) -alkyl-COOR¹, (C₁-C₆) -alkyl-SO₂OR¹, (C₁-C₆) -alkyl-CON (R¹)₂, (C₁-C₆) -alkyl-SO₂N (R¹)₂, (C₁-C₆) -alkyl-NR¹COR¹, (C₁-C₆) -alkyl-NR¹SO₂R², tetrahydrofuranyloxymethyl, tetrahydrofuranylmethoxymethyl, O(CH₂)-3,5-dimethyl-1,2-oxazol-4-yl, O(CH₂)₂-O(3,5-dimethoxypyrimidin-2-yl, O(CH₂)-5-pyrrolidin-2-one or O(CH₂)-5-2,4-dimethyl-2,4-dihydro-3H-1,2,4-triazol-3-one,
R¹ is hydrogen, (C₁-C₆)-alkyl, (C₂-C₆)-alkenyl, (C₂-C₆)-alkynyl, (C₃-C₆) -cycloalkyl, (C₃-C₆) -cycloalkyl- (C₁-C₆)-alkyl, phenyl or phenyl-(C₁-C₆)-alkyl, where the seven last-mentioned radicals are substituted by s radicals selected from the group consisting of cyano, halogen, nitro, thiocyanato, OR³, S(O)ₙR⁴, N(R³)₂, NR³R³, COR³, OCOR³, SCOR³, NR³COR³, CO₂R³, COSR³, CON(R³)₂ and (C₁-C₄) -alkoxy- (C₂-C₆) -alkoxycarbonyl ,
R² is (C₁-C₆) -alkyl, (C₂-C₆)-alkenyl, (C₂-C₆)-alkynyl, (C₃-C₆) -cycloalkyl, (C₃-C₆) -cycloalkyl-(C₁-C₆)-alkyl, phenyl or phenyl-(C₁-C₆)-alkyl, where the seven last-mentioned radicals are substituted by s radicals selected from the group consisting of cyano, halogen, nitro, thiocyanato, OR³, S(O)ₙR⁴, N(R³)₂, NR³R³, COR³, OCOR³, SCOR³, NR³COR³, CO₂R³, COSR³, CON(R³)₂ and (C₁-C₄)-alkoxy-(C₂-C₆)-alkoxycarbonyl,
R³ is hydrogen, (C₁-C₆)-alkyl, (C₂-C₆)-alkenyl or (C₂-C₆)-alkynyl,
R⁴ is (C₁-C₆)-alkyl, (C₂-C₆)-alkenyl or (C₂-C₆)-alkynyl,
n is 0, 1 or 2,
s is 0, 1, 2 or 3.

3. The compound as claimed in claim 1 or 2, wherein
R is hydrogen, (C₁-C₆)-alkyl, (C₃-C₇)-cycloalkyl, halo- (C₁-C₆) -alkyl, (C₁-C₆) -alkoxy, halogen- (C₁-C₆)-alkoxy, cyano, nitro, methylsulfenyl, methylsulfinyl, methylsulfonyl, acetylamino, benzoylamino, methoxycarbonyl, ethoxycarbonyl, benzoyl, methylcarbonyl, piperidinylcarbonyl, trifluoromethylcarbonyl, halogen, amino, aminocarbonyl, methylaminocarbonyl, dimethylaminocarbonyl, methoxymethyl,
X and Z independently of one another are in each case nitro, halogen, cyano, (C₁-C₆) -alkyl, (C₁-C₆)-haloalkyl, (C₃-C₆)-cycloalkyl, OR¹, S(O)ₙR², (C₁-C₆) -alkyl-S (O)ₙR², (C₁-C₆)-alkyl-OR¹, (C₁-C₆)-alkyl-CON (R¹)₂, (C₁-C₆) -alkyl-SO₂N(R¹)₂, (C₁-C₆)-alkyl-NR¹COR¹, (C₁-C₆)-alkyl-NR¹SO₂R² or 1,2,4-triazol-1-yl,
Y is nitro, halogen, cyano, (C₁-C₆) -alkyl, (C₁-C₆)-haloalkyl , OR¹, S(O)ₙR², SO₂N(R¹)₂, NR¹SO₂R², NR¹COR¹, (C₁-C₆) -alkyl-S (O)ₙR², (C₁-C₆) -alkyl-OR¹, (C₁-C₆) -alkyl-CON(R¹)₂, (C₁-C₆)-alkyl-SO₂N(R¹)₂, (C₁-C₆)-alkyl-NR¹COR¹, (C₁-C₆) -alkyl-NR¹SO₂R², tetrahydrofuranyloxymethyl, tetrahydrofuranylmethoxymethyl, O(CH₂)-3,5-dimethyl-1,2-oxazol-4-yl, O(CH₂)₂-O(3,5-dimethoxypyrimidin-2-yl, O(CH₂)-5-pyrrolidin-2-one or O(CH²)-5-2,4-dimethyl-2,4-dihydro-3H-1,2,4-triazol-3-one,
R¹ is hydrogen, (C₁-C₆) -alkyl, (C₂-C₆) -alkenyl, (C₂-C₆) -alkynyl, (C₃-C₆)-cycloalkyl, (C₃-C₆)-cycloalkyl- (C₁-C₆)-alkyl, phenyl or phenyl-(C₁-C₆)-alkyl, where the seven last-mentioned radicals are substituted by s radicals selected from the group consisting of halogen and OR³,
R² is (C₁-C₆) -alkyl, (C₃-C₆) -cycloalkyl or (C₃-C₆) - cycloalkyl-(C₁-C₆)-alkyl, where the three last-mentioned radicals are substituted by s radicals selected from the group consisting of halogen and OR³,
R³ is hydrogen or (C₁-C₆)-alkyl,
n is 0, 1 or 2,
s is 0, 1, 2 or 3.

4. The compound as claimed in claim 1 or 2, wherein
R is hydrogen, (C₁-C₆) -alkyl, (C₃-C₇)-cycloalkyl, halo-(C₁-C₆)-alkyl, (C₁-C₆)-alkoxy, halo-(C₁-C₆)-alkoxy, cyano, nitro, methylsulfenyl, methylsulfinyl, methylsulfonyl, acetylamino, benzoylamino, methoxycarbonyl, ethoxycarbonyl, benzoyl, methylcarbonyl, piperidinylcarbonyl, trifluoromethylcarbonyl, halogen, amino, aminocarbonyl, methylaminocarbonyl, dimethylaminocarbonyl, methoxymethyl,
X and Z independently of one another are in each case nitro, halogen, cyano, (C₁-C₆) -alkyl, (C₁-C₆)-haloalkyl, (C₃-C₆)-cycloalkyl, OR¹, S(O)ₙR², (C₁-C₆)-alkyl-S(O)ₙR², (C₁-C₆)-alkyl-OR¹, (C₁-C₆)-alkyl-CON (R¹)₂, (C₁-C₆) -alkyl-SO₂N(R¹)₂, (C₁-C₆)-alkyl-NR¹COR¹, (C₁-C₆)-alkyl-NR¹SO₂R² or 1,2,4-triazol-1-yl,
Y is S(O)ₙR², 4,5-dihydro-1,2-oxazol-3-yl, 5-cyanomethyl-4,5-dihydro-1,2-oxazol-3-yl or 5-methoxymethyl-4,5-dihydro-1,2-oxazol-3-yl,
R¹ is hydrogen, (C₁-C₆) -alkyl, (C₂-C₆)-alkenyl, (C₂-C₆)-alkynyl, (C₃-C₆) -cycloalkyl, (C₃-C₆) -cycloalkyl- (C₁-C₆)-alkyl, phenyl or phenyl-(C₁-C₆)-alkyl, where the seven last-mentioned radical are substituted by s radicals selected from the group consisting of halogen and OR³,
R² is (C₁-C₆) -alkyl, (C₃-C₆)-cycloalkyl or (C₃-C₆)-cycloalkyl-(C₁-C₆)-alkyl, where the three last-mentioned radicals are substituted by s radicals selected from the group consisting of halogen and OR₃
R³ is hydrogen or (C₁-C₆)-alkyl,
n ist 0, 1 or 2,
s is 0, 1, 2 or 3.

5. A herbicidal composition which comprises a herbicidally effective amount of at least one compound of the formula (I) as claimed in any of claims 1 to 4.

6. The herbicidal composition as claimed in claim 5 in a mixture with formulation auxiliaries.

7. The herbicidal composition as claimed in claim 5 or 6, comprising at least one further pesticidally active compound from the group of the insecticides, acaricides, herbicides, fungicides, safeners and growth regulators.

8. The herbicidal composition as claimed in claim 7, comprising a safener.

9. The herbicidal composition as claimed in claim 7 or 8, comprising a further herbicide.

10. A method of controlling undesired plants, wherein an effective amount of at least one compound of the formula (I) as claimed in any of claims 1 to 4 or of a herbicidal composition as claimed in any of claims 5 to 9 is applied to the plants or to the location of the undesired plant growth.

11. The use of compounds of the formula (I) as claimed in any of claims 1 to 4 or of herbicidal compositions as claimed in any of claims 5 to 9 for controlling undesirable plants.

12. The use as claimed in claim 11, wherein the compounds of the formula (I) are used for controlling unwanted plants in crops of useful plants.

13. The use as claimed in claim 12, wherein the useful plants are transgenic useful plants.

## Revendications

1. Composés de formule (I) ou leurs sels 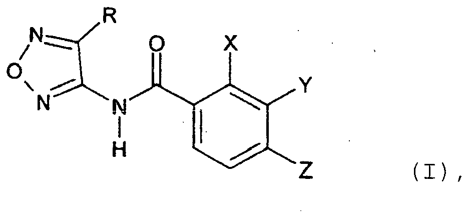 dans laquelle
R représente un atome d'hydrogène, un groupe alkyle en C₁-C₆, cycloalkyle en C₃-C₇, halogéno-alkyle(C₁-C₆), alcoxy en C₁-C₆, halogénoalcoxy(C₁-C₆), alcényle en C₂-C₆, alcényloxy en C₂-C₆, halogénoalcényle (C₂-C₆), alcynyle en C₂-C₆, alcynyloxy(C₂-C₆), halogénoalcynyle(C₂-C₆), cyano, nitro, méthylsulfényle, méthylsulfinyle, méthylsulfonyle, acétylamino, benzoylamino, méthoxycarbonyle, éthoxycarbonyle, méthoxycarbonylméthyle, éthoxycarbonylméthyle, benzoyle, méthylcarbonyle, pipéridinylcarbonyle, trifluorométhylcarbonyle, halogéno, amino, aminocarbonyle, méthylaminocarbonyle, diméthylaminocarbonyle, méthoxyméthyle, ou
un groupe hétéroaryle, hétérocyclyle ou phényle substitués chacun par s radicaux choisis dans l'ensemble constitué par méthyle, éthyle, méthoxy, trifluorométhyle et halogéno,
X et Z représentent chacun, indépendamment l'un de l'autre, un atome d'halogène ou un groupe nitro, cyano, formyle, sulfocyano, alkyle en C₁-C₆, halogénoalkyle(C₁-C₆), alcényle en C₂-C₆, halogénoalcényle(C₂-C₆), alcynyle en C₂-C₆, halogénoalcynyle(C₃-C₆), cycloalkyle en C₃-C₆, halogénocycloalkyle(C3-C6), cycloalkyl(C₃-C₆)-alkyle (C₁-C₆), halogénocycloalkyl (C₃-C₆) -alkyle(C₁-C₆), COR¹, OR¹, OCOR¹, OSO₂R², S(O)ₙR², SO₂OR¹, SO₂N(R¹)₂, NR¹SO₂R², NR¹COR¹, alkyl (C₁-C₆) -S(O)ₙR², alkyl(C₁-C₆)-OR¹, alkyl (C₁-C₆)-OCOR¹, alkyl(C₁-C₆)-OSO₂R², alkyl(C₁-C₆)-COOR¹, alkyl (C₁-C₆)-SO₂OR¹, alkyl (C₁-C₆) -CON (R¹)₂, alkyl (C₁-C₆) -SO₂N(R¹)₂, alkyl(C₁-C₆)-NR¹COR¹, alkyl(C₁-C₆)-NR¹SO₂R², NR₁R₂, P(O)(OR⁵)₂, ou
un groupe hétéroaryle, hétérocyclyle ou phényle substitués chacun par s radicaux choisis dans l'ensemble constitué par méthyle, éthyle, méthoxy, nitro, trifluorométhyle et halogéno,
Y représente un atome d'halogène ou un groupe nitro, cyano, sulfocyano, alkyle en C₁-C₆, halogénoalkyle (C₁-C₆), alcényle en C₂-C₆, halogénoalcényle(C₂-C₆), alcynyle en C₂-C₆, halogénoalcynyle(C₃-C₆), cycloalkyle en C₃-C₆, halogénocycloalkyle(C₃-C₆), cycloalkyl(C₃-C₆)-alkyle(C₁-C₆) halogénocycloalkyl(C₃-C₆)-alkyle(C₁-C₆), COR¹, CO₂R¹, OR¹, OCOR¹, OSO₂R², S(O)ₙR², SO₂OR¹, SO₂N(R¹)₂, NR¹SO₂R², NR¹COR¹, alkyl (C₁-C₆)-hétéroaryle, O-alkyl (C₁-C₆)-hétérocyclyle, O-alkyl(C₁-C₆)-hétéroaryle, alkyl(C₁-C₆)-hétérocyclyle, alkyl(C₁-C₆)-S(O)ₙR², alkyl(C₁-C₆)-OR¹, alkyl (C₁-C₆) -OCOR¹, alkyl (C₁-C₆) -OSO₂R², alkyl (C₁-C₆)-COOR¹, alkyl (C₁-C₆)-CN, alkyl (C₁-C₆) -SO₂OR¹, alkyl (C₁-C₆) CON(R¹)₂, alkyl(C₁-C₆)-SO₂N(R¹)₂, alkyl(C₁-C₆)-NR¹COR¹, alkyl (C₁-C₆) -NR¹SO₂R², NR¹R², P(O) (OR⁵)₂, tétrahydrofuranyloxyméthyle, tétrahydrofuranylméthoxyméthyle, O(CH₂)-3,5-diméthyl-1,2-oxazol-4-yle, O(CH₂)₂-O(3,5-diméthoxypyrimidin-2-yle, O(CH₂)-5-pyrrolidin-2-one, O(CH₂)-5-2,4-diméthyl-2,4-dihydro-3H-1,2,4-triazol-3-one, ou
un groupe hétéroaryle ou hétérocyclyle substitués chacun par s radicaux choisis dans l'ensemble constitué par méthyle, éthyle, méthoxy, halogéno et cyanométhyle,
R¹ représente un atome d'hydrogène, un groupe alkyle en C₁-C₆, halogénoalkyle(C₁-C₆), alcényle en C₂-C₆, halogénoalcényle(C₂-C₆), alcynyle en C₂-C₆, halogénoalcynyle(C₂-C₆), cycloalkyle en C₃-C₆, halogénocycloalkyle(C₃-C₆), alkyl (C₁-C₆)-O-alkyle (C₁-C₆), cycloalkyl(C₃-C₆)-alkyle(C₁-C₆), phényle ou phénylalkyle(C₁-C₆), les 12 radicaux nommés en dernier étant substitués par s radicaux choisis dans l'ensemble constitué par cyano, halogéno, nitro, sulfocyano, OR³, S(O)ₙR⁴, N(R³)₂, NR³OR³, COR³, OCOR³, SCOR³, NR³COR³, CO₂R³, COSR³, CON(R³)et alcoxy(C₁-C₄)-alcoxycarbonyle(C₂-C₆),
R² représente un groupe alkyle en C₁-C₆, alcényle en C₂-C₆, alcynyle en C₂-C₆, cycloalkyle en C₃-C₆, cycloalkyl(C₃-C₆)-alkyle (C₁-C₆), phényle ou phénylalkyle(C₁-C₆), les sept radicaux nommés en dernier étant substitués par s radicaux choisis dans l'ensemble constitué par cyano, halogéno, nitro, sulfocyano, OR³, S(O)ₙR⁴, N(R³)₂, NR³OR³, COR³, OCOR³, SCOR³, NR³COR³, CO₂R³, COSR³, CON(R³)₂ et alcoxy(C₁-C₄)-alcoxycarbonyle(C₂-C₆),
R³ représente un atome d'hydrogène, un groupe alkyle en C₁-C₆, alcényle en C₂-C₆ ou alcynyle en C₂-C₆,
R⁴ représente un groupe alkyle en C₁-C₆, alcényle en C₂-C₆ ou alcynyle en C₂-C₆,
R⁵ représente le groupe méthyle ou éthyle,
n représente 0, 1 ou 2,
s représente 0, 1, 2 ou 3.

2. Composés selon la revendication 1, dans lesquels
R représente un atome d'hydrogène, un groupe alkyle en C₁-C₆, cycloalkyle en C₃-C₇, halogénoalkyle(C₁-C₆), alcoxy(C₁-C₆), halogénoalcoxy(C₁-C₆), cyano, nitro, méthylsulfényle, méthylsulfinyle, méthylsulfonyle, acétylamino, benzoylamino, méthoxycarbonyle, éthoxycarbonyle, benzoyle, méthylcarbonyle, pipéridinylcarbonyle, trifluorométhylcarbonyle, halogéno, amino, aminocarbonyle, méthylaminocarbonyle, diméthylaminocarbonyle, méthoxyméthyle,
un hétérocycle choisi dans l'ensemble comprenant les groupes pyridin-2-yle, pyridin-3-yle, pyridin-4-yle, pipéridin-2-yle, pipéridin-3-yle, pipéridin-4-yle, benzisoxazol-2-yle, 1,2,4-oxadiazol-3-yle, 1,2,4-triazol-3-yle, 1-éthylbenzimidazol-2-yle, 4-méthylthiazol-2-yle, thiophén-2-yle, furan-2-yle, furan-3-yle, tétrahydrofuran-2-yle, tétrahydrofuran-3-yle, isoxazol-2-yle, isoxazol-3-yle, oxazol-2-yle, oxazol-3-yle, pyrrol-2-yle, pyrrol-3-yle, imidazol-2-yle, imidazol-5-yle, imidazol-4-yle, pyrazol-3-yle, pyrazol-5-yle, pyrazol-4-yle, isoxazol-3-yle, isoxazol-4-yle, isoxazol-5-yle, oxazol-2-yle, oxazol-4-yle, oxazol-5-yle, isothiazol-3-yle, isothiazol-4-yle, isothiazol-5-yle, thiazol-2-yle, thiazol-4-yle, thiazol-5-yl 1,2,3-triazol-4-yle, 1,2,3-triazol-5-yle, 1,2,5-triazol-3-yle, 1,3,4-triazol-2-yle, 1,2,4-triazol-3-yle, 1,2,4-triazol-5-yle, 1,2,4-oxadiazol-3-yle, 1,2,4-oxadiazol-5-yle, 1,3,4-oxadiazol-2-yle, 1,2,3-oxadiazol-4-yle, 1,2,3-oxadiazol-5-yle, 1,2,5-oxadiazol-3-yle, 1,2,4-thiadiazol-3-yle, 1,2,4-thiadiazol-5-yle, 1,3,4-thiadiazol-2-yle, 1,2,3-thiadiazol-4-yle, 1,2,3-thiadiazol-5-yle, 1,2,5-thiadiazol-3-yle, 2H-1,2,3,4-tétrazol-5-yle, IH-1,2,3,4-tétrazol-1-yle, 1,2,3,4-oxatriazol-5-yle, 1,2,3,5-oxatriazol-4-yle, 1,2,3,4-thiatriazol-5-yle, 1,2,3,5-thiatriazol-4-yle, pyrazin-2-yle, pyrazin-3-yle, pyrimidin-2-yle, Pyrimidin-4-yle, pyrimidin-5-yle, pyridazin-3-yle et pyridazin-4-yle, substitué par s radicaux choisis dans l'ensemble constitué par méthyle, méthoxy et halogéno ou
un groupe phényle substitué par s radicaux choisis dans l'ensemble constitué par méthyle, méthoxy, trifluorométhyle et halogéno,
X et Z représentent chacun, indépendamment l'un de l'autre, un atome d'halogène ou un groupe nitro, cyano, sulfocyano, alkyle en C₁-C₆, halogénoalkyle(C₁-C₆) , alcényle en C₂-C₆, halogénoalcényle(C₂-C₆), alcynyle en C₂-C₆, halogénoalcynyle(C₃-C₆), cycloalkyle en C₃-C₆, halogénocycloalkyle(C₃-C₆), cycloalkyl(C₃-C₆)-alkyle(C₁-C₆), halogénoalkyl(C₃-C₆)-alkyle (C₁-C₆), COR¹, OR¹, OCOR¹, OSO₂R², S(O)ₙR², SO₂OR¹, SO₂N(R¹)₂, NR¹SO₂R², NR¹COR¹, alkyl (C₁-C₆)-S(O)ₙR², alkyl(C₁-C₆)-OR¹, alkyl (C₁-C₆)-OCOR¹, alkyl (C₁-C₆)-OSO₂R², alkyl(C₁-C₆)-COOR¹, alkyl(C₁-C₆)-SO₂OR¹, alkyl(C₁-C₆)-CON(R¹)₂, alkyl(C₁-C₆)-SO₂N(R¹)₂, alkyl(C₁-C₆)-NR¹COR¹, alkyl(C₁-C₆)-NR¹SO₂R², benzoxazol-2-yle, 1-éthyl-benzimidazol-2-yle, pipéridin-1-yle ou 1,2,4-triazol-1-yle,
Y représente un atome d'halogène ou un groupe nitro, cyano, sulfocyano, alkyle en C₁-C₆, halogénoalkyle(C₁-C₆), alcényle en C₂-C₆, halogénoalcényle(C₂-C₆), alcynyle en C₂-C₆, halogénoalcynyle(C₃-C₆), cycloalkyle en C₃-C₆, halogénocycloalkyle(C₃-C₆), cycloalkyl (C₃-C₆) -alkyle (C₁-C₆), halogénocycloalkyl(C₃-C₆)-alkyle(C₁-C₆), COR¹, OR¹, OCOR¹, OSO₂R², S(O)ₙR², SO₂OR¹, SO₂N(R¹)₂, NR¹SO₂R², NR¹COR¹, alkyl (C₁-C₆)*-*S(O)ₙR², alkyl (C₁-C₆)-OR¹, alkyl (C₁-C₆)-OCOR¹, alkyl (C₁-C₆) -OSO₂R², alkyl (C₁-C₆)-COOR¹, alkyl(C₁-C₆)-SO₂OR¹, alkyl (C₁-C₆)-CON(R¹)₂, alkyl (C₁-C₆)-SO₂N(R¹)₂, alkyl (C₁-C₆)-NR¹COR¹, alkyl (C₁-C₆)-NR¹SO₂R², tétrahydrofuranyloxyméthyle, tétrahydrofuranylméthoxyméthyle, O(CH₂)-3,5-diméthyl-1,2-xazol-4-yle, O(CH₂)₂-O(3,5-diméthoxypyrimidin-2-yle, O(CH₂)-5-pyrrolidin-2-one ou O(CH₂)-5-2,4-diméthyl-2,4-dihydro-3H-1,2,4-triazol-3-one,
R¹ représente un atome d'hydrogène, un groupe alkyle en C₁-C₆, alcényle en C₂-C₆, alcynyle en C₂-C₆, cycloalkyle en C₃-C₆, cycloalkyl (C₃-C₆)-alkyle(C₁-C₆), phényle ou phényl-alkyle(C₁-C₆), les sept radicaux nommés en dernier étant substitués par s radicaux choisis dans l'ensemble constitué par cyano, halogéno, nitro, sulfocyano, OR³, S(O),R⁴, N(R³)₂, NR³OR³, COR³, OCOR³, SCOR³, NR³COR³, CO₂R³ COSR³, CON(R³)₂ et alcoxy(C₁-C₄)-alcoxycarbonyle(C₂-C₆),
R² représente un groupe alkyle en C₁-C₆, alcényle en C₂-C₆, alcynyle en C₂-C₆, cycloalkyle en C₃-C₆, cycloalkyl(C₃-C₆)-alkyle (C₁-C₆), phényle ou phénylalkyle(C₁-C₆), les sept radicaux nommés en dernier étant substitués par s radicaux choisis dans l'ensemble constitué par cyano, halogéno, nitro, sulfocyano, OR³, S(O)ₙR⁴, N(R³)₂, NR³OR³, COR³, OCOR³, SCOR³, NR³COR³, CO₂R³, COSR³, CON(R³)₂ et alcoxy(C₁-C₄)-alcoxycarbonyle(C₂-C₆),
R³ représente un atome d'hydrogène, un groupe alkyle en C₁-C₆, alcényle en C₂-C₆ ou alcynyle en C₂-C₆,
R⁴ représente un groupe alkyle en C₁-C₆, alcényle en C₂-C₆ ou alcynyle en C₂-C₆,
n représente 0, 1 ou 2,
s représente 0, 1, 2 ou 3.

3. Composés selon la revendication 1 ou 2, dans lesquels
R représente un atome d'hydrogène, un groupe alkyle en C₁-C₆, cycloalkyle en C₃-C₇, halogénoalkyle(C₁-C₆), alcoxy en C₁-C₆, halogénoalcoxy(C₁-C₆), cyano, nitro, méthylsulfényle, méthylsulfinyle, méthylsulfonyle, acétylamino, benzoylamino, méthoxycarbonyle, éthoxycarbonyle, benzoyle, méthylcarbonyle, pipéridinylcarbonyle, trifluorométhylcarbonyle, halogéno, amino, aminocarbonyle, méthylaminocarbonyle, diméthylaminocarbonyle, méthoxyméthyle,
X et Z représentent chacun, indépendamment l'un de l'autre, un atome d'halogène ou un groupe nitro, cyano, alkyle en C₁-C₆, halogénoalkyle(C₁-C₆), cycloalkyle en C₃-C₆, OR¹, S(O)ₙR², alkyl(C₁-C₆)-S(O)ₙR², alkyl(C₁-C₆)-OR¹, alkyl(C₁-C₆)-CON(R¹)₂, alkyl(C₁-C₆)-SO₂N(R¹)₂, alkyl(C₁-C₆)-NR¹COR¹, alkyl(C₁-C₆)-NR¹SO₂R² ou 1,2,4-triazol-1-yle,
Y représente un atome d'halogène ou un groupe nitro, cyano, alkyle en C₁-C₆, halogénoalkyle(C₁-C₆), OR¹, S(O)ₙR²_{,} SO₂N(R¹)₂, NR¹SO₂R², NR¹COR¹, alkyl(C₁-C₆)-S(O)ₙR², alkyl (C₁-C₆)-OR¹, alkyl(C₁-C₆)-CON(R¹)₂, alkyl(C₁-C₆)-SO₂N(R¹)₂, alkyl(C₁-C₆)-NR¹COR¹, alkyl(C₁-C₆)-NR¹SO₂R², tétrahydrofuranyl-oxyméthyle, tétrahydrofuranylméthoxyméthyle, O(CH₂)-3,5-diméthyl-1,2-oxazol-4-yle, O(CH₂)₂-O(3,5-diméthoxypyrimidin-2-yle, O(CH₂)-5-pyrrolidin-2-one ou O(CH₂)-5-2,4-diméthyl-2,4-dihydro-3H-1,2,4-triazol-3-one,
R¹ représente un atome d'hydrogène, un groupe alkyle en C₁-C₆, alcényle en C₂-C₆, alcynyle en C₂-C₆, cycloalkyle en C₃-C₆, cycloalkyl (C₃-C₆)-alkyle (C₁-C₆), phényle ou phényl-alkyle(C₁-C₆), les sept radicaux nommés en dernier étant substitués par s radicaux choisis dans l'ensemble constitué par halogéno et OR³,
R² représente un groupe alkyle en C₁-C₆, cycloalkyle en C₃-C₆ ou cycloalkyl(C₃-C₆)-alkyle (C₁-C₆), les trois radicaux nommés en dernier étant substitués par s radicaux choisis dans l'ensemble constitué par halogéno et OR³,
R³ représente un atome d'hydrogène ou un groupe alkyle en C₁-C₆,
n représente 0, 1 ou 2,
s représente 0, 1, 2 ou 3.

4. Composés selon la revendication 1 ou 2, dans lesquels
R représente un atome d'hydrogène, un groupe alkyle en C₁-C₆, cycloalkyle en C₃-C₇, halogénoalkyle(C₁-C₆), alcoxy en C₁-C₆, halogénoalcoxy(C₁-C₆), cyano, nitro, méthylsulfényle, méthylsulfinyle, méthylsulfonyle, acétylamino, benzoylamino, méthoxycarbonyle, éthoxycarbonyle, benzoyle, méthylcarbonyle, pipéridinylcarbonyle, trifluorométhylcarbonyle, halogéno, amino, aminocarbonyle, méthylaminocarbonyle, diméthylaminocarbonyle, méthoxyméthyle,
X et Z représentent chacun, indépendamment l'un de l'autre, un atome d'halogène ou un groupe nitro, cyano, alkyle en C₁-C₆, halogénoalkyle(C₁-C₆), cycloalkyle en C₃-C₆, OR¹, S(O)ₙR², alkyl(C₁-C₆)-S(O)ₙR², alkyl (C₁-C₆) - OR¹, alkyl(C₁-C₆)-CON(R¹)₂, alkyl(C₁-C₆)-SO₂N(R¹)₂, alkyl(C₁-C₆)-NR¹COR¹, alkyl(C₁-C₆)-NR¹SO₂R² ou 1,2,4-triazol-1-yle,
Y représente un groupe S(O)ₙR², 4,5-dihydro-1,2-oxazol-3-yle, 5-cyanométhyl-4,5-dihydro-1,2-oxazol-3-yle ou 5-méthoxyméthyl-4,5-dihydro-1,2-oxazol-3-yle,
R¹ représente un atome d'hydrogène, un groupe alkyle en C₁-C₆, alcényle en C₂-C₆, alcynyle en C₂-C₆, cycloalkyle en C₃-C₆, cycloalkyl (C₃-C₆) -alkyle (C₁-C₆), phényle ou phényl-alkyle(C₁-C₆), les sept radicaux nommés en dernier étant substitués par s radicaux choisis dans l'ensemble constitué par halogéno et OR³,
R² représente un groupe alkyle en C₁-C₆, cycloalkyle en C₃-C₆ ou cycloalkyl (C₃-C₆) -alkyle (C₁-C₆), les trois radicaux nommés en dernier étant substitués par s radicaux choisis dans l'ensemble constitué par les groupes halogéno et OR³,
R³ représente un atome d'hydrogène ou un groupe alkyle en C₁-C₆,
n représente 0, 1 ou 2,
s représente 0, 1, 2 ou 3.

5. Compositions herbicides, **caractérisées par** une teneur à activité herbicide en au moins un composé de formule (I) selon l'une quelconque des revendications 1 à 4.

6. Compositions herbicides selon la revendication 5, en mélange avec des adjuvants de formulation.

7. Compositions herbicides selon la revendication 5 ou 6, contenant au moins une substance à activité pesticide, choisie dans le groupe constitué par les insecticides, acaricides, herbicides, fongicides, phytoprotecteurs et régulateurs de croissance.

8. Compositions herbicides selon la revendication 7, contenant un phytoprotecteur.

9. Compositions herbicides selon la revendication 7 ou 8, contenant un autre herbicide.

10. Procédé pour la lutte contre des plantes indésirables, **caractérisé en ce qu'**on applique sur les plantes ou le lieu de la croissance indésirable de plantes une quantité efficace d'au moins un composé de formule (I) selon l'une quelconque des revendications 1 à 4 ou d'une composition herbicide selon l'une quelconque des revendications 5 à 9.

11. Utilisation de composés de formule (I) selon l'une quelconque des revendications 1 à 4 ou de compositions herbicides selon l'une quelconque des revendications 5 à 9, pour la lutte contre des plantes indésirables.

12. Utilisation selon la revendication 11, **caractérisée en ce que** les composés de formule (I) sont utilisés pour la lutte contre des plantes indésirables dans des cultures de plantes utiles.

13. Utilisation selon la revendication 12, **caractérisée en ce que** les plantes utiles sont des plantes utiles transgéniques.
